# EUROPEAN PATENT APPLICATION

(11) **EP 4 772 536 A1**
(43) Date of publication of application: **08.07.2026**
(21) Application number: 24859879.9
(22) Date of filing: 29.08.2024
(51) Int. Cl.: C07K 16/28, A61K 31/713, A61K 35/12, A61K 35/76, A61K 38/02, A61K 39/395, A61K 45/00, A61K 48/00, A61P 25/00, A61P 25/28, A61P 37/04, C07K 14/705, C12N 1/15, C12N 1/19, C12N 1/21, C12N 5/10, C12N 15/13, C12N 15/63, C12P 21/08

(54) **THERAPEUTIC AGENT FOR NEURODEGENERATIVE DISEASES**

(30) Priority: 30.08.2023 JP 2023140405
(71) Applicant: Teijin Pharma Limited, Tokyo 100-0013 (JP); UNIVERSITY PUBLIC CORPORATION OSAKA, Osaka-shi Osaka 540051 (JP)
(72) Inventor: EGUCHI, Hiroshi, Tokyo 1000013 (JP); TOMIYAMA, Takami, Osaka 5998531 (JP); UMEDA, Tomohiro, Osaka 5998531 (JP)
(74) Representative: Lavoix
(86) International application number: PCT/JP2024/030896
(87) International publication number: WO 2025/047850

(57) **Abstract**

The invention provides an anti-gpNMB antibody that binds to and acts on gpNMB and removes malfunctional microglia, etc., as well as uses thereof. Specifically, the antibody specifically binds to at least one site in a region from the PMEL-CAF-like domain to the PKD domain of sgpNMB.

## Description

### TECHNICAL FIELD

The present invention relates to an anti-gpNMB antibody (especially a humanized antibody) that has the functions of decreasing the number of mal-functional microglia in a subject and/or removing amyloid β oligomers and/or phosphorylated tau and/or synuclein (a-synuclein, β-synuclein, γ-synuclein) in a subject and/or increasing the number of synapses in a subject and/or inhibiting or restoring declines in cognitive functions and/or motor functions, and a pharmaceutical drug comprising an ingredient that has such functions.

### BACKGROUND ART

### 1. Neurodegenerative diseases

Neurodegenerative diseases are progressive neurological disorders in which the accumulation of toxic proteins (proteins that cannot be degraded intracellularly due to multimerization or aggregate formation caused by structural changes) in nerve cells results in impaired neurotransmission and intracellular clearance functions of nerve cells, as well as nerve cell death, leading to cognitive decline, memory impairment, mental impairment, or motor function impairment. Neurodegenerative diseases have no fundamental therapies, and drugs that alleviate the symptoms associated with the diseases are used. Most of these "neurodegenerative diseases" are "central nervous system degenerative diseases."

Examples classified as neurodegenerative diseases include (1) tauopathies (diseases which occur mainly because Tau accumulates and causes neurodegeneration): specifically, Alzheimer's disease, frontotemporal lobar degeneration (FTLD-Tau), frontotemporal dementia (FTD), primary age-related tauopathy (PART), chronic traumatic encephalopathy (CTE), Pick's disease, cerebral cortex basal ganglia degeneration (CBD or CBS), progressive supranuclear paralysis (PSP), globular glial tauopathy (GGT), argyrophilic grain dementia (AGD) (argyrophilic grain disease ), age-related Tau astroglial tauopathy (ARTAG), familial British dementia (FBD), familial Danish dementia (FDD), FTDP17, multisystem tauopathy with dementia (MSTD), neurofibrillary tangle dementia, neurofibrillary tangle with calcinosis (DNTC), and white matter tauopathy with globular glial inclusion (WMT-GGI); and (2) neurodegenerative diseases other than tauopathies: specifically, Parkinson's disease (accumulation of α-synuclein, etc.), Huntington's disease (accumulation of mutant huntingtin, etc.), spinal cord cerebellum degeneration (accumulation of polyglutamic acid protein, etc.), hereditary spinal cord cerebellar ataxia (accumulation of polyglutamic acid protein, etc.), spinobulbar muscular atrophy (accumulation of polyglutamic acid protein, etc.), Lewy body dementia (accumulation of α-synuclein, etc.), Creutzfeldt-Jakob disease (accumulation of abnormal prion protein and β-synuclein, etc.), amyotrophic lateral sclerosis (ALS) (accumulation of SOD, TDP43, FUS (fused in sarcoma), α-synuclein, γ-synuclein, etc.), and frontotemporal lobar degeneration (accumulation of FTLD-TDP, FTLD-FUS:TDP43, FUS, etc.).

### 2. Alzheimer's disease (Alzheimer-type dementia)

Dementia is recognized as a major problem in the global aging population. According to a study conducted by Alzheimer's Disease International, the number of patients worldwide with dementia is projected to increase to approximately 76 million by 2030 and then to 139 million by 2050.

According to the World Health Organization (WHO), about 60 to 70% of all dementias are reported to be Alzheimer's disease (https://www.who.int/news-room/fact-sheets/detail/dementia). Two major pathologies have been reported in Alzheimer's disease: senile plaques (amyloid deposition) and neurofibrillary changes (tau accumulation).

Because patients with familial Alzheimer's disease have genetic mutations that are associated with increased production of amyloid, research is being conducted on antibodies and vaccines against amyloid beta (Aβ), a component of amyloid. Various anti-Aβ antibodies that eliminate amyloid deposition (Non-Patent Literature 1) and drugs targeting Aβ oligomers (Non-Patent Literature 2), which are considered highly toxic, are already in clinical development, and Lecanemab (Non-Patent Literature 3, Non-Patent Literature 72) received approval from the FDA in 2023. However, these drugs against amyloid are reported to exhibit only limited efficacies and also involve high risks of side effects.

Tau, another component of neurofibrillary tangles, is also attracting attention as a drug discovery target, as recent brain imaging analysis has confirmed a correlation between Tau accumulation and clinical symptoms and disease progression. Clinical trials of anti-Tau antibodies against various epitopes are currently underway (Non-Patent Literature 4, Non-Patent Literature 84).

Other techniques to efficiently transfer antibodies into the brain for the treatment of Alzheimer's disease includes antibodies against membrane proteins expressed in the blood-brain barrier (BBB), such as transferrin receptors, or antibodies that can efficiently pass through the BBB by adding peptides with affinity to the constant region or by introducing amino acid mutations (Non-Patent Literature 5).

However, there are currently no effective drugs for inhibiting or improving declined cognitive function, and the creation of effective Alzheimer's disease drugs is a pressing issue worldwide.

### 3. Microglia

Microglia belong to the glial cell family, which also includes astrocytes and oligodendrocytes. However, unlike astrocytes and oligodendrocytes, microglia originate from progenitor cells that develop in the embryonic yolk sac. Microglia are macrophage-like cells in the brain and are thought to act as immunocompetent cells in the central nervous system, for example, to remove toxic proteins such as amyloid-β and aggregated Tau in the brain. It has also been reported that microglia can act in a neuroprotective or reparative manner in the event of neurological disorders. Furthermore, microglia have been shown to play an important role in the maintenance of neural networks, such as synaptic pruning. On the other hand, activated microglia release inflammatory cytokines and induce inflammation in the brain. Recent advances in genetic analysis technology have led to reports of the existence of various microglial subtypes, and the relationship of each microglial subtype to diseases is an issue for future research.

With regard to the relationship between microglia and Alzheimer's pathology, it was reported that degenerated microglia are associated with tau accumulation in immunohistochemistry using Alzheimer's disease patient brains (Non-Patent Literature 6). There is also a report which identified a subtype of dysfunctional microglia in Alzheimer's disease by immunohistology (single cell histology) (Non-Patent Literature 7). However, it is unclear how dysfunctional microglia modify diseases. In addition, although dysfunctional microglia are generally identified by their morphology, some researchers refer to dysfunctional microglia-like microglia as "degenerating microglia" or "aging microglia" (Non-Patent Literature 8), and the meaning of these terms have not been necessarily settled.

Brain imaging analysis of Alzheimer's disease patients has also been conducted using ligands for TSPO (translocator protein), whose expression increases when microglia are activated, and it was reported that the signal detected by TSPO-PET (Positron Emission (TSPO-PET) is higher in patients with Alzheimer's disease (Non-Patent Literature 9) and correlates with tau and amyloid pathology (Non-Patent Literature 10). However, research on the specificity of these TSPO-PET ligands for activated microglia is still in its infancy (Non-Patent Literature 11), and no common view has yet been reached.

Recently, GWAS (Genome Wide Association Study) analysis of Alzheimer's disease (Non-Patent Literature 12) revealed that many disease-related genes are expressed in microglia. Therefore, research and development of therapeutic agents for Alzheimer's disease targeting microglia is underway. For example, humans with a loss of function (LOF) gene mutation (R47H), which causes loss of function of TREM2, are at increased risk of developing Alzheimer's disease (Non-Patent Literature 13). Currently, clinical trials are underway against anti-TREM2 agonist antibodies (Non-Patent Literature 14) and anti-CD33 inhibitory antibodies (Non-Patent Literature 15) that have the ability to activate microglia (NCT04592874, NCT03822208), with the hope that activating microglia will promote clearance of toxic proteins such as Aβ and tau as candidate antibody drugs for microglia targeting. On the other hand, anti-Sema4D antibodies (Non-Patent Literature 16), which inhibit activated microglia in terms of suppressing gliosis, are also in clinical trials (NCT04381468).

Recently, scRNAseq (single-cell RNA sequencing) analysis of microglia in mouse models of Alzheimer's disease has revealed the existence of microglial subtypes that appear in association with Alzheimer's pathology. Such microglial subtypes include DAM (Disease Associated Microglia, Non-Patent Literature 17), ARM (Activated Response Microglia, Non-Patent Literature 18), and MGnD (microglial neurodegenerative phenotype, Non-Patent Literature 19). However, it is still unclear how these disease-associated microglia modify Alzheimer's disease, i.e., how they exert their effects, since there is no way to specifically regulate these DAMs and other disease-associated microglia. Furthermore, it is unknown which of the disease-specific microglia subtypes that have been identified suppress the pathogenesis and which subtypes enhance the pathogenesis. It is also unclear which dysfunctional microglia are classified or included within which subtypes, since mRNA expression levels do not directly provide information on whether cells accumulate denatured proteins or not.

### 4. gpNMB

gpNMB (Glycoprotein nonmetastatic melanoma protein B) (also known as Osteoactivin, DC-HIL, Non-Patent Literature 20) is a single transmembrane glycoprotein and has as many as 12 glycosylation sites in humans. gpNMB is normally expressed in intracellular organelles (endoplasmic reticulum, lysosomes, Golgi apparatus, melanosomes), etc. (Non-Patent Literature 21). However, gpNMB has also been reported to be expressed on the plasma membrane when overexpressed (Non-Patent Literature 22). It has also been reported that gpNMB is expressed in cancer cells (Non-Patent Literature 23), and that KLD (kringle-like domain) is important for cell growth and that intracellular ITIM motifs induce signaling (Non-Patent Literature 24).

gpNMB has a region homologous to the CAF (Core Amyloid Fragment) region of the family protein PMEL (Premelanosome Protein) (Non-Patent Literature 25) (hereinafter referred to as the amyloid core domain) and PKD (Polycystic Kidney Disease homologous) domain. The PKD domain has a β-sandwich structure formed by a β-sheet consisting of three strands and a β-sheet consisting of four strands that fold together. It was named the PKD domain because it is a characteristic structure possessed by polycystin-1, the gene responsible for polycystic kidney disease (Non-Patent Literature 26).

gpNMB is important for the function of phagosomes and lysosomes (Non-Patent Literature 27), as it has been reported that lysosome function is impaired in gpNMB-deficient mice (Non-Patent Literature 28). Abnormalities of the iris and the development of glaucoma have been reported in mice lacking gpNMB (Non-Patent Literature 29), and humans lacking gpNMB have been reported to have cutaneous amyloidosis (Non-Patent Literature 30).

It is known that gpNMB is cleaved by membrane proteases such as ADAM10 and released as soluble gpNMB (Non-Patent Literature 31), and soluble gpNMB has been reported to be involved in neuroprotection (Non-Patent Literature 32), anti-inflammation (Non-Patent Literature 21) and cell proliferation (Non-Patent Literature 33). It has also been reported that neural function was improved in transgenic mice overexpressing soluble gpNMB (extracellular region gpNMB) (Non-Patent Literature 34), and cognitive function was increased in an Alzheimer's disease model mice (APP/PS1) when autophagy was enhanced by using lentiviral vectors to express gpNMB (Non-Patent Literature 35).

On the other hand, it has been reported that administration of a mouse gpNMB-specific vaccine peptide with some amino acid sequences of mouse gpNMB reduces aged vascular endothelial cells and fibroblasts, improves insulin resistance and atherosclerosis in a high-fat diet load model mice, and extends survival in mice with accelerated aging. It has also been reported that antibody-dependent cellular cytotoxicity (ADCC) activity is important in this process, and gpNMB partial peptide sequences that are effective in removing senescent cells have been disclosed (Patent Literature 1, Non-Patent Literature 36). Likewise, it has been reported that administration of a polyclonal antibody against mouse gpNMB in a high-fat diet-loaded mouse model improved obesity and insulin resistance by inhibiting soluble gpNMB produced by the liver (Non-Patent Literature 37).

Glembatumumab (Patent Literature 2), an anti-human gpNMB-specific human antibody, is known as an anti-gpNMB antibody. Glembatumumab Vedotin, a conjugate of Glembatumumab and an anticancer drug, advanced to Phase II trials as a potential cancer drug, but its development was discontinued. Another anti-gpNMB antibody that binds to gpNMB expressed on the surface of cancer cells and treats cancer with immunotoxins has also been disclosed (Patent Literature 3). There is also a report on the acquisition of another anti-human gpNMB-specific antibody, which describes diagnostic and therapeutic applications against human-derived cancer cells (Non-Patent Literature 38). Anti-rat gpNMB-specific antibodies specific for activated rat microglia have also been reported (Non-Patent Literature 39). It has also been reported that anti-mouse DC-HIL specific antibodies put signals into the cells (Non-Patent Literature 40).

With regard to gpNMB expression in Alzheimer's disease (AD), immunohistochemistry and RNA expression analysis and cerebrospinal fluid concentrations have been reported. Regarding immunohistochemistry, accumulation of gpNMB-expressing microglia in ApoE-positive Aβ plaques was reported in the brains of AD patients (Non-Patent Literature 41).

Regarding RNA expression, scRNAseq analysis of AppNL-G-F mice showed that microglia subtypes were classified into Activated Response Microglia (ARM), which are tissue repair genes such as gpNMB, etc., and increased TREM2 production and decreased CD33 production have been reported (Non-Patent Literature 18). There has also been a report that gpNMB expression is upregulated in MGnD in 5XFAD, a mouse model of Alzheimer's disease, suggesting that it may have a neuroprotective role (Non-Patent Literature 19). On the other hand, it has been reported that scRNAseq analysis of Alzheimer's disease model mice showed the expression of gpNMB in mal-functional microglia (Non-Patent Literature 42). There has also been a report that snRNAseq (single-nucleus RNA sequencing) using Alzheimer's disease patient brain samples showed the expression of TREM2, ITGAX, GPNMB, FLT1, SPP1, etc., in activated microglia with phagocytic ability, which are thought to work to remove toxic proteins (Non-Patent Literature 43). The association of type 1 microglia with Alzheimer's disease and the possible two-fold function of gpNMBs have also been reported (Non-Patent Literature 44). However, many microglial subtypes are known to exist, as shown in Non-Patent Literature 18 and Non-Patent Literature 43.

Furthermore, it has been reported that gpNMB is involved in autophagy function, reduces Aβ deposition in AD model mice (APP-PS1), and improves cognitive function as demonstrated by Morris water maze test (Non-Patent Literature 35).

Regarding the relationship between soluble gpNMB concentrations in cerebrospinal fluid and Alzheimer's disease, some reports (Non-Patent Literature 19) indicate that the concentrations are increased in AD, while others (Non-Patent Literature 45) indicate that there is no relationship. Recent reports have indicated that gpNMB in cerebrospinal fluid correlates with pathophysiology and clinical symptoms of diseases such as ALS (amyotrophic lateral sclerosis), PD, and AD (Non-Patent Literature 80, Non-Patent Literature 81, Non-Patent Literature 82).

In addition, recent genome-wide association studies (GWAS) analysis has reported gpNMB as a risk gene for Parkinson's disease (Non-Patent Literature 46), and its involvement in the propagation of α-synuclein, a causative agent of Parkinson's disease, has also been reported (Non-Patent Literature 47). On the other hand, another report has indicated that loss of gpNMB does not alter synuclein-associated pathology (Non-Patent Literature 48). With regard to immunohistochemistry, the detection of gpNMB-expressing microglia has been reported in the brains of patients with Alzheimer's disease and Nasu-Hakola disease (Non-Patent Literature 41). Increased expression of gpNMB in the substantia nigra has also been reported in Parkinson's disease at the immunohistochemical staining and protein level (Non-Patent Literature 49).

### 5. Alzheimer's disease model mice

Various mouse models of Alzheimer's disease have been developed and are currently used in research (Non-Patent Literature 50). One representative model mice is the 5XFAD mouse (Non-Patent Literature 51), which produces Aβ oligomers, which are considered the most toxic form of amyloid-β, and also accumulates Aβ oligomers in neurons. The APPosk mouse (Non-Patent Literature 52) is another model mice that accumulates Aβ oligomers in neurons, like the 5XFAD model mice, and has been reported to improve pathology, restore synapse number, and improve cognitive function after intranasal administration of rifampicin (Non-Patent Literature 53). However, there have been no reports of clearance of intracellularly accumulated Aβ oligomers by antibodies such as anti-Aβ antibodies. There have also been no reports of antibodies that showed recovery of the number of synapses in neurons, an indicator of neuronal function and neurotransmission.

Tg2576 is a Tg mouse that overexpresses the Swedish mutant (KM670/671NL) somatic gene of the APP (Amyloid precursor protein, isoform 695) gene. Tg2576 is a model mice for AD that has been reported to exhibit amyloid plaques (senile plaques in the brain of Alzheimer's disease patients) at about 9 months of age (Non-Patent Literature 54), and is used for general purpose studies of amyloid pathology.

Tau264 is a Tg mouse expressing human Tau. Although it does not exhibit Tau pathology by itself (Non-Patent Literature 55), it has been reported that its crossbreeding with APPosk mice induces phosphorylated tau accumulation (Non-Patent Literature 56), and is considered a model mice with pathology similar to human Alzheimer's disease in that it exhibits phosphorylation and accumulation of wild-type Tau without mutations.

### 6. Evaluation of nerve function

Recovery of neural functions such as spatial reference function and memory functions is generally evaluated in vivo using behavioral tests, such as the Morris water maze test, the Y-maze, and novel substance search.

In vitro evaluation of the number of neurons is commonly carried out by immunohistochemical staining such as NeuN staining, which is an indicator of the number of neurons, and synaptophysin staining, which is an indicator of the number of synapses.

In many mouse models of Alzheimer's disease, evaluation is performed in a state that does not lead to neuronal cell death, and therefore, evaluation of neural functions is carried out by analysis of the number of synapses using synaptophysin staining.

Many papers have reported a correlation between synaptophysin levels in vitro and neurological functions in vivo (Non-Patent Literature 57, Non-Patent Literature 58, and Non-Patent Literature 59), and quantification of synaptophysin protein by synaptophysin staining or other means can be used to assess recovery of neurological functions.

### 7. α-Synucleinopathy

It is known that in Parkinson's disease (PD), dementia with Lewy bodies (DLB), and multiple system atrophy (MSA; a Parkinson's syndrome), aggregates of α-synuclein protein pathologically appear in the brain and throughout the body, causing neuronal cell death. These diseases are collectively referred to as α-synucleinopathies, in which aggregation-prone abnormal structural forms of α-synuclein accumulate in the brain and cause neuronal cell loss.

Among these, Parkinson's disease is characterized by various behavioral disorders and autonomic nervous system symptoms and is caused by a lack of dopamine due to progressive loss of neurons in the substantia nigra of the midbrain. In Japan, Parkinson's disease occurs in approximately 100 people per 100,000 population, with no difference between men and women, and most cases occur in elderly people aged 50 to 60 years or older. Most cases are solitary and their causes are unknown, although multiple factors, including aging, environmental factors, and genetic factors, are presumed to be involved. Rarely, hereditary PD is also observed. No substantial therapy exists for PD, and there are only drugs that ameliorate symptoms. Accordingly, the creation of a fundamental treatment based on the disease status is an urgent global challenge.

According to a 2018 report in JAMA Neurology, the increase in the number of Parkinson's disease patients has been very rapid, surpassing the increase in Alzheimer's disease; between 1990 and 2015, the prevalence of Parkinson's disease worldwide more than doubled, as did the mortality rate. Since Parkinson's disease increases with aging, it is estimated that the number of people with the disease will increase exponentially in the future; a 2014 meta-analysis estimated that the number of people with Parkinson's disease worldwide will more than double from 6.9 million in 2015 to 14.2 million in 2040 (Non-Patent Literature 73).

### 8. Parkinson's disease mouse models

Currently, various mouse models of Parkinson's disease have been developed and used in research (Non-Patent Literature 74). One representative model mice is the A53T mutant human α-synuclein-Tg mouse (Non-Patent Literature 75). This is a transgenic model mice with A53T mutant human α-synuclein that produces α-synuclein oligomers, which are considered the most toxic of all synuclein aggregates, and accumulates α-synuclein oligomers in the hippocampus and substantia nigra (Non-Patent Literature 76). According to this mouse model, since α-synuclein oligomers first accumulates in the hippocampus before in the substantia nigra, cognitive dysfunction appears first, followed by behavioral disorders due to accumulation of α-synuclein oligomers in the substantia nigra.

Some anti-α-synuclein antibodies and other drugs have been reported to have therapeutic effects in model mice (Non-Patent Literature 77), but these drugs require long-term administration, and their therapeutic effects are not apparent. Several anti-α-synuclein antibody drugs have already advanced to clinical trials for Parkinson's disease, but two antibodies have already been discontinued (Non-Patent Literature 78, Non-Patent Literature 79).

### 9. Evaluation of motor function

The Rotarod test and the Inverted Screen test are known as methods for evaluating the motor function of model mice.

### <Rotarod test>

This test is used to measure locomotor coordination and motor learning in rodents, and is especially used to examine neurological diseases and drug effects in laboratory animals. A mouse is placed on a rotating rod, and the mouse walks on the rod without falling off. The speed of the rod's rotation is gradually increased, and the time until the mouse falls is measured.

### <Inverted Screen test>

The inverted screen test is used to measure locomotor strength/coordination. Untrained mice are individually placed on a square (7.5 cm x 7.5 cm) wire mesh screen (mesh consisting of 12 mm squares) mounted horizontally on a metal rod. The screen is then flipped 180 degrees so that the mouse is at the bottom of the screen and the time until it falls is measured.

### LIST OF CITATIONS

### Patent Literature

[Patent Literature 1] WO2021/020047 A
[Patent Literature 2] JP6334496 B
[Patent Literature 3] WO2007/053718 A

### Non-Patent Literature

[Non-Patent Literature 1] Avgerinos et al., Ageing Res. Rev., (2021), 68:101339
[Non-Patent Literature 2] Tolar et al., Int. J. Mol. Sci. (2021), 22[12]:6355
[Non-Patent Literature 3] Jeremic et al., Ageing Res. Rev., (2021), 72:101496
[Non-Patent Literature 4] Ji et al., Drugs, (2021), 81[10]:1135-1152
[Non-Patent Literature 5] Pardridge et al., Pharmaceuticals (2022), Vol.15Article 3, Internet <https://doi.org/10.3390/ph15010003>
[Non-Patent Literature 6] Streit et al., Acta Neuropathol., (2009), 118[4]:475-85
[Non-Patent Literature 7] Swanson et al., Acta Neuropathol. Commun., (2020), 8:170
[Non-Patent Literature 8] Shahidehpour et al., Neurobiol. Aging., (2021), 99:19-27
[Non-Patent Literature 9] Malpetti et al., Brain, (2020), 143:1588-1602
[Non-Patent Literature 10] Dani et al., Brain, (2018), 141[9]:2740-2754
[Non-Patent Literature 11] Gouilly et al., Eur. J. Neurosci., (2022), doi: 10.1111/ejn.15613
[Non-Patent Literature 12] Jansen et al., Nat. Genet., (2019), 51[3]:404-413
[Non-Patent Literature 13] Wang et al., Cell, (2015), 160[6]:1061-1071
[Non-Patent Literature 14] Wang et al., J. Exp. Med., (2020), .217[9]:e20200785
[Non-Patent Literature 15] Griciuc et al., Curr. Opin. Neurol., (2021), 34[2]:228-236
[Non-Patent Literature 16] Mao et al., Int. J. Mol. Sci., (2021), 22:9465
[Non-Patent Literature 17] Keren-Shaul et al., Cell, (2017), 169:1276-1290
[Non-Patent Literature 18] Frigerio et al., Cell Rep., (2019), .27[4]:1293-1306
[Non-Patent Literature 19] Huettenrauch et al., Acta Neuropathol. Commun., (2018), 6:108
[Non-Patent Literature 20] Shikano et al., J. Biol. Chem., (2001), 276[11]:8125-8134
[Non-Patent Literature 21] Ripoll et al., J. Immunol., (2007), 178:6557-6566
[Non-Patent Literature 22] Tse et al., Clin. Cancer Res., (2006), 12[4]:1373-1382
[Non-Patent Literature 23] Maric et al., OncoTargets Ther., (2013), 6:839-852
[Non-Patent Literature 24] Xie et al., Cancer Sci., (2019), 110[7]:2237-2246
[Non-Patent Literature 25] Hee et al., Scientific Reports, (2017), 7:44064
[Non-Patent Literature 26] Bycroft et al., EMBO J., (1999), 18[2]:297-305
[Non-Patent Literature 27] Li et al., FASEB J., (2010), 24[12]:4767-4781
[Non-Patent Literature 28] Robinet et al., Scientific Reports, (2021), 11:10249
[Non-Patent Literature 29] Anderson et al., Nat. Genet., (2002), 30:81-85
[Non-Patent Literature 30] Yang et al., Am. J. Hum. Genet., (2018), 102[2]:219-232
[Non-Patent Literature 31] Rose et al., PLoS One, (2010), 5[8]:e12093
[Non-Patent Literature 32] Nakano et al., Neuroscience, (2014), 277:123-131
[Non-Patent Literature 33] Wang et al., Cancer Sci.. (2021), 112:4187-4197
[Non-Patent Literature 34] Murata et al., J. Neurochem., (2015), 132:583-594
[Non-Patent Literature 35] Zhu et al., Neurosci. Lett., (2022), 767:136300
[Non-Patent Literature 36] Suda et al., Nat. Aging, (2021), 1:1117-1126
[Non-Patent Literature 37] Gong et al., Nat. Metab., (2019), 1[5]:570-583
[Non-Patent Literature 38] Zhang et al., Monoclon. Antibodies Immunodiagn. Immunother., (2013), .32[4]:265-269
[Non-Patent Literature 39] Kawahara et al., Glia, (2016), 64[11]:1938-1961
[Non-Patent Literature 40] Chung et al., J. Immunol., (2019), 183[5]:5190-5198
[Non-Patent Literature 41] Satoh et al., Intractable Rare Dis. Res., (2019), 8[2]:120-128
[Non-Patent Literature 42] Krasemann et al., Immunity, (2017), 47:566-581
[Non-Patent Literature 43] Gerrits et al., Acta Neuropathol., (2021), 141:681-696
[Non-Patent Literature 44] 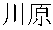 , 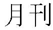 "cell ", (2022), 54[2]:110-114
[Non-Patent Literature 45] Aichholzer et al., Alzheimers Res. Ther., (2021), 13:94
[Non-Patent Literature 46] Murthy et al., Neurogenetics, 2017, 18:121-133
[Non-Patent Literature 47] Diaz-Ortiz et al., Science, 2022, 377:833
[Non-Patent Literature 48] Grendza et al., Neurobiol. Dis., 2021, 159:105494
[Non-Patent Literature 49] Moloney et al., Neurobiol. Dis., 2018, 120:1-11
[Non-Patent Literature 50] FBRI LLC, "ALZFORUM: NETWORKING FOR A CURE", "RESEARCH MODELS", [online], (1996), [Seached in 2022], Internet <https://www.alzforum.org/research-models/alzheimers-disease>
[Non-Patent Literature 51] Oakley et al., J. Neurosci., (2006), 26[40]:10129-10140
[Non-Patent Literature 52] Umeda et al., J. Neurosci. Res., (2011), 89:1031-1042
[Non-Patent Literature 53] Umeda et al., Front. Neurosci., (2021), 15:763476
[Non-Patent Literature 54] Hsiao et al., Science, (1996), 274[5284]:99-102
[Non-Patent Literature 55] Umeda et al., Am. J. Clin. Pathol., 2013, 183[1]:211-225
[Non-Patent Literature 56] Umeda et al., Acta Neuropathol., (2014), 127:685-698
[Non-Patent Literature 57] Shi et al., Front. Pharmacol., (2021), 12:794458
[Non-Patent Literature 58] Umeda et al., Biomedicines 2022, 10, 297
[Non-Patent Literature 59] Hafez et al., Neuroscience, (2012), 223:465-472
[Non-Patent Literature 60] EMBL-EBI, "AlphaFold Protein Structure Database", "Transmembrane glycoprotein NMB", [online], (2021), [Seached in 2022], Internet <https://alphafold.ebi.ac.uk/entry/Q99P91>
[Non-Patent Literature 61] Woollacott et al., J. Neuroinflammation, (2020), 17:234
[Non-Patent Literature 62] Kushwaha et al., Ann. Neurosci., (2018), 25:223-233
[Non-Patent Literature 63] Quek et al., J. Neuroinflammation, (2022), 19:58
[Non-Patent Literature 64] Boi et al., Int. J. Mol. Sci., (2020), 21[22]:8535
[Non-Patent Literature 65] Ishijima et al., Science Progress, (2021), 104[4]:1-21
[Non-Patent Literature 66] Cai et al., Bioengineered, (2021), 12[2]:11390-11398
[Non-Patent Literature 67] Paasila et al., Brain Pathology, (2019), 29:726-740
[Non-Patent Literature 68] Hajj et al., J. Neuroinflammation, (2019), 16:87
[Non-Patent Literature 69] Stratoulias et al., EMBO J., (2019), 38:e101997
[Non-Patent Literature 70] Alexander et al., Pigment Cell Melanoma Res. (2013), 26[4]:470-486
[Non-Patent Literature 71] Chrystal et al., Molecules (2021), 26, Article_3529
[Non-Patent Literature 72] Yadollahikhales et al., Neurotherapeutics (2023) 20:914-931
[Non-Patent Literature 73] Dorsey et al., JAMA Neurol., 2018, 75:9-10
[Non-Patent Literature 74] Dovonov et al., Transl. Neurodegener., 2023, 12:Art_36
[Non-Patent Literature 75] Lee et al., PNAS USA, 2022, 99[13]:8968-8973
[Non-Patent Literature 76] Umeda et al., Int. J. Mol. Sci., 2021, 22[16]:8453
[Non-Patent Literature 77] Nordstrom et al. Neurobiology of Disease, 2021, Vol.161, Art_105543.
[Non-Patent Literature 78] Xiao et al. J Transl Med 2023, Vol.21, Art_178.
[Non-Patent Literature 79] Chatterjee et al. Neurobiol Dis 2019, Vol.132, Art_104587.
[Non-Patent Literature 80] Zhu et al., J Mol Neurosci 2019, Vol.69, pp643-657.
[Non-Patent Literature 81] Hansson et al., EMBO Mol Med 2023, Vol.15, e16359.
[Non-Patent Literature 82] Doroszkiewicz et al., J Clin Med 2023, 12:4689.
[Non-Patent Literature 83] Kawahara et al., J. Neurochem., (2024), Vol.00, 1-23
[Non-Patent Literature 84] Panza et al., Expert Opin. Investig. Drugs, 2023, Vol. 32, pp.625-634.
[Non-Patent Literature 85] Barreto et al., Glia, 2024, Vol.72, No.7, pp.1319-1339

### SUMMARY OF INVENTION

### PROBLEM TO BE SOLVED BY THE INVENTION

There are some drugs that improve symptoms of neurodegenerative diseases, but there have been no therapeutic drugs. On the other hand, scRNAseq and other methods have revealed the existence of disease-associated microglia that appear specifically in neurodegenerative diseases, but it is unclear as to which microglia are responsible for disease progression. It is also unclear as to whether the identification and specific elimination of the disease-causing mal-functional microglia would lead to therapeutic effects such as elimination of toxic proteins in neurons and restoration of neuronal function.

In this context, as described in the BACKGROUND ART section above, although gpNMB is one of the genes expressed in mal-functional microglia that are involved in autophagosome and lysosome function, it is unclear as to whether gpNMB-high microglia are involved in the pathogenesis of the disease or not, or even whether they are pathogenic or protective.

As mentioned above, the presence of disease-associated microglia has been reported in several mouse models of neurodegenerative diseases such as Alzheimer's disease and Parkinson's disease. However, it was unclear as to how disease-associated microglia modulate the disease, since there was no way to specifically regulate disease-associated microglia.

Although it has been reported that gpNMBs expressed in disease-associated microglia act in a neuroprotective manner and that they are expressed in mal-functional microglia, as described above, there have been no reports of anti-gpNMB monoclonal antibodies that bind with high affinity under physiological conditions in vivo, which can be administered to animal models of diseases to evaluate their efficacy. Furthermore, there have been no report that a high-affinity anti-gpNMB monoclonal antibodies, which can act in the brain, was administered to a mouse model of Alzheimer's disease to analyze its effects.

gpNMB is a glycoprotein rich in glycans, with 12 sites of glycosylation in humans. Therefore, it is not easy to create monoclonal antibodies that can recognize gpNMBs expressed on cells, and as mentioned above, the clinical trials conducted so far were those for glembatumumab vedotin, a conjugate of glembatumumab and an anticancer drug, have only been conducted for cancer types that highly express gpNMBs (melanoma, breast cancer, etc.). Because glembatumumab is a human gpNMB-specific antibody, it was not possible to evaluate its effect on endogenous gpNMB in mouse or rat disease models.

On the other hand, there have been reports of improvement of obesity and insulin resistance in high-fat diet-loaded mouse models by inhibition of liver-expressed soluble gpNMB with an anti-mouse gpNMB polyclonal antibody (Non-Patent Literature 38) and improvement of atherosclerosis and insulin resistance and prolongation of survival in accelerated aging model mice by removal of senescent cells with mouse gpNMB specific vaccine peptide (Patent Literature 1 and Non-Patent Literature 36).

With regard to the production of anti-gpNMB antibodies with mouse-human cross-reactivity for the purpose of developing a therapeutic agent for Alzheimer's disease, there have been no reports to date as mentioned above, since gpNMB has a very large number of glycosylation sites, as low a homology of as about 70% between mouse and human, and a complex conformation (Non-Patent Literature 57), rendering the attempt to obtain antibodies that recognize the conformation in vivo very challenging. Furthermore, because the modification of glycans differs depending on the cells and tissues in which they are expressed, it was assumed to be extremely difficult to obtain antibodies that bind specifically to gpNMBs expressed in microglia, and even more difficult to create anti-gpNMB antibodies (among others, humanized antibodies) that bind to gpNMBs expressed on the cell membrane and have mouse/human cross-reactivity.

The present invention has been made in view of the above issues, and an objective of the present invention is to provide a means to specifically regulate mal-functional microglia, such as anti-gpNMB antibodies (among others, humanized antibodies), and also to provide a pharmaceutical drug based on a new mechanism of action using such means.

### MEANS TO SOLVE THE PROBLEM

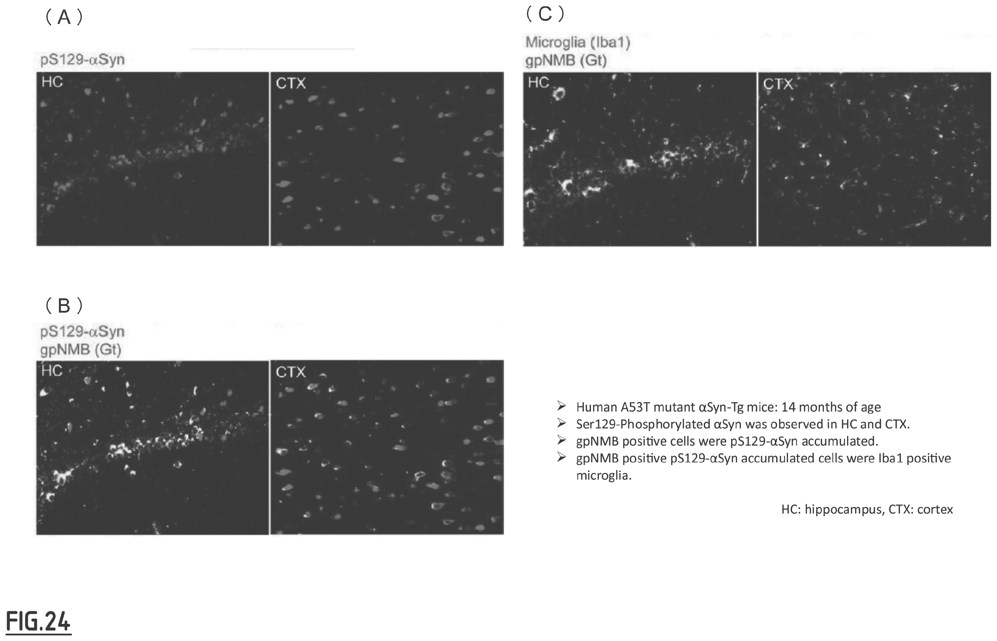

The present inventors have confirmed the presence of microglia with intracellular accumulation of Aβ, Tau, or α-synuclein in Alzheimer's disease model mice, tauopathy model mice, and Parkinson's disease model mice. Furthermore, the present inventors have found that most of these microglia are gpNMB-positive cells, i.e., cells that are double-stained by immunohistochemical staining with anti-gpNMB and anti-Iba1 antibodies (Figure 24).

There has also been a report of a method for scoring the level of degeneration (dysfunction) based on the ramified structure Non-Patent Literature 61), where these microglia had a reduced ramified structure in morphology and were evaluated as degenerated. In addition, accumulation of lipofuscin, which is observed in senescent cells, was also observed. These findings suggest that these microglia are mal-functional microglia that are unable to degrade aggregation proteins. It has been proposed that such mal-functional microglia accumulating aggregate proteins lose their neuroprotective effects and phagocytosis, leading to neurodegenerative pathologies (Non-Patent Literature 62 and Non-Patent Literature 7).

The present inventors have also found that most of these mal-functional microglia are gpNMB-positive, i.e., cells that are double-stained by immunohistochemistry with anti-gpNMB and anti-Iba1 antibodies. For example, based on the immunohistological analysis of the brain of APPosk mice, the inventors found that gpNMB-positive cells with intracellular Aβ accumulation were anti-gpNMB and anti-Iba1 antibody-positive cells, i.e., gpNMB-high expressing microglia. The term "mal-functional microglia" herein refers to a cell that is double-stained by immunohistochemistry with anti-gpNMB and anti-Iba1 antibodies.

Although researchers do not always agree on what is meant by "mal-functional microglia" as mentioned above, the term "mal-functional microglia" as used herein refers to microglia that have at least one of the following characteristics.
(1) Their phagocytic and/or toxic-protein degradation functions are reduced or abnormally enhanced.
(2) They release proinflammatory cytokines that may progress diseases (e.g., inflammation in the brain).
(3) They have reduced ramified structures compared to normal microglia.

Among these, the first characteristic (1) can be confirmed by the fact that a variety of aggregate proteins are known to be subject to phagocytosis, which vary by disease, e.g., Aβ, tau, alpha-synuclein, Lipofuscin, L-Ferritin, TDP43, SOD, polyglutamate proteins, and their accumulation is observed in the microglia (Non-Patent Literature 63 and Non-Patent Literature 64). Specifically, this characteristic can be identified by staining with Iba1, a marker for microglia, as well as by detection with antibodies against aggregating proteins (e.g., anti-Aβ or anti-tau antibodies) or by autofluorescence (lipofuscin). The second characteristic (2) can be confirmed by the detection of the cytokines concerned (Non-Patent Literature 65 and Non-Patent Literature 66). The third characteristic (3) can be confirmed by immunohistochemical staining (Non-Patent Literature 67) and morphological observation using electron microscopy (Non-Patent Literature 68) (Non-Patent Literature 69).

On the other hand, the term "normal microglia" used herein refers to microglia that do not have any of the aforementioned characteristics of mal-functional microglia and are working to maintain homeostasis in the body.

Through intensive investigations in view of the above background, the present inventors have succeeded in obtaining anti-gpNMB antibodies (among others, humanized antibodies) that specifically binds to at least one site in a region from a PMEL-CAF-like domain to a PKD domain of human gpNMB, and have found that these antibodies have unexpected functions, e.g., the effects of decreasing the number of mal-functional microglia in a subject and/or removing amyloid β oligomers and/or phosphorylated tau and/or synuclein oligomers in a subject and/or increasing the number of synapses in a subject and/or inhibiting or restoring declines in cognitive functions or motor functions, and can be used for a variety of applications based on these functions, e.g., for treating or preventing neurodegenerative diseases such as Alzheimer's disease and Parkinson's disease in a subject. The present inventors have also found that using any means to reduce the number of dysfunctional microglia, such as antibodies makes it possible to achieve unexpected effects, e.g., the effects of decreasing the number of mal-functional microglia in a subject and/or removing amyloid β oligomers and/or phosphorylated tau and/or synuclein oligomers in a subject and/or increasing the number of synapses in a subject and/or inhibiting or restoring declines in cognitive functions or motor functions, and even the unexpected effect of treating or preventing various neurodegenerative diseases. Based on these findings, the inventors have completed the following inventions.

Specifically, aspects of the present invention include the following.
[Aspect 1] An anti-gpNMB antibody (e.g., humanized antibody) or its fragment or a derivative thereof which binds specifically to at least one site in a region from a PMEL-CAF-like (PMEL core amyloid fragment-like) domain to a PKD domain of human gpNMB (glycoprotein nonmetastatic melanoma protein B).
[Aspect 2] The anti-gpNMB antibody (e.g., humanized antibody) or its fragment or a derivative thereof according to Aspect 1, which binds specifically to a region containing amino acid residue(s) D287 and/or H301 of human gpNMB having the amino acid sequence defined in SEQ ID NO 209.
[Aspect 3] The anti-gpNMB antibody (e.g., humanized antibody) or its fragment or a derivative thereof according to Aspect 1 or 2, which binds specifically to a region containing amino acid residue(s) R214 and/or R215 of human gpNMB having the amino acid sequence defined in SEQ ID NO 209.
[Aspect 4] The anti-gpNMB antibody (e.g., humanized antibody) or its fragment or a derivative thereof according to Aspect 2 or 3, which also binds specifically to one or more regions selected from of a region containing amino acid residue(s) K257 and/or D258, a region containing amino acid residue(s) H268 and/or D269, a region containing an amino acid residue K282, a region containing an amino acid residue K316, a region containing amino acid residue K186, and a region containing amino acid residue(s) H216 and/or R218, of human gpNMB having the amino acid sequence defined in SEQ ID NO 209.
[Aspect 5] The anti-gpNMB antibody (e.g., humanized antibody) or its fragment or a derivative thereof according to any one of Aspects 1 to 4, which also binds specifically to at least one site in a region from a PMEL-CAF-like domain to a PKD domain of mouse gpNMB.
[Aspect 6] The anti-gpNMB antibody (e.g., humanized antibody) or its fragment or a derivative thereof according to any one of Aspects 1 to 5, having one or more activities selected from: an activity to reduce the number of mal-functional microglia; an activity to remove amyloid beta oligomers and/or phosphorylated tau and/or synuclein oligomers; an activity to increase the number of synapses; and an activity to inhibit or restore declines in cognitive functions or motor functions.
[Aspect 7] The anti-gpNMB antibody (e.g., humanized antibody) or its fragment or a derivative thereof according to any one of Aspects 1 to 6, which is a monoclonal antibody or its fragment or a derivative thereof.
[Aspect 8] The anti-gpNMB antibody (e.g., humanized antibody) or its fragment or a derivative thereof according to any one of Aspects 1 to 7, which comprises at least a heavy chain variable region comprising:
   (1) as a CDR-H1 sequence, the amino acid sequence defined in SEQ ID NO 1, or an amino acid sequence derived from SEQ ID NO 1 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 1,
      as a CDR-H2 sequence, the amino acid sequence defined in SEQ ID NO 3, or an amino acid sequence derived from SEQ ID NO 3 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 3, and
      as a CDR-H3 sequence, the amino acid sequence defined in SEQ ID NO 5, or an amino acid sequence derived from SEQ ID NO 5 via substitution, deletion, or insertion of any one, two, three, or four amino acid residues, or an amino acid sequence having a homology (preferably an identity) of 66.6 % or more, 75.0 % or more, 83.3 % or more, or 91.6 % or more to the amino acid sequence defined in SEQ ID NO 5, or
   (2) as a CDR-H1 sequence, the amino acid sequence defined in SEQ ID NO 17, or an amino acid sequence derived from SEQ ID NO 17 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 17,
      as a CDR-H2 sequence, the amino acid sequence defined in SEQ ID NO 19, or an amino acid sequence derived from SEQ ID NO 19 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 19, and
      as a CDR-H3 sequence, the amino acid sequence defined in SEQ ID NO 21, or an amino acid sequence derived from SEQ ID NO 21 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 21, or
   (3) as a CDR-H1 sequence, the amino acid sequence defined in SEQ ID NO 33, or an amino acid sequence derived from SEQ ID NO 33 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 33,
      as a CDR-H2 sequence, the amino acid sequence defined in SEQ ID NO 35, or an amino acid sequence derived from SEQ ID NO 35 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 35, and
      as a CDR-H3 sequence, the amino acid sequence defined in SEQ ID NO 37, or an amino acid sequence derived from SEQ ID NO 37 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 37, or
   (4) as a CDR-H1 sequence, the amino acid sequence defined in SEQ ID NO 49, or an amino acid sequence derived from SEQ ID NO 49 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 49,
      as a CDR-H2 sequence, the amino acid sequence defined in SEQ ID NO 51, or an amino acid sequence derived from SEQ ID NO 51 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 51, and
      as a CDR-H3 sequence, the amino acid sequence defined in SEQ ID NO 53, or an amino acid sequence derived from SEQ ID NO 53 via substitution, deletion, or insertion of any one, two, three, four, five, or six amino acid residues, or an amino acid sequence having a homology (preferably an identity) of 62.5 % or more, 68.7 % or more, 75.0 % or more, 81.2 % or more, 87.5 % or more, or 93.7 % or more to the amino acid sequence defined in SEQ ID NO 53, or
   (5) as a CDR-H1 sequence, the amino acid sequence defined in SEQ ID NO 65, or an amino acid sequence derived from SEQ ID NO 65 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 65,
      as a CDR-H2 sequence, the amino acid sequence defined in SEQ ID NO 67, or an amino acid sequence derived from SEQ ID NO 67 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 67, and
      as a CDR-H3 sequence, the amino acid sequence defined in SEQ ID NO 69, or an amino acid sequence derived from SEQ ID NO 69 via substitution, deletion, or insertion of any one, two, three, four, or five amino acid residues, or an amino acid sequence having a homology (preferably an identity) of 64.2 % or more, 71.4 % or more, 78.5 % or more, 85.7 % or more, or 92.8 % or more to the amino acid sequence defined in SEQ ID NO 69, or
   (6) as a CDR-H1 sequence, the amino acid sequence defined in SEQ ID NO 81, or an amino acid sequence derived from SEQ ID NO 81 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 81,
      as a CDR-H2 sequence, the amino acid sequence defined in SEQ ID NO 83, or an amino acid sequence derived from SEQ ID NO 83 via substitution, deletion, or insertion of any one or two amino acid residues, or an amino acid sequence having a homology (preferably an identity) of 71.4 % or more or 85.7 % or more to the amino acid sequence defined in SEQ ID NO 83, and
      as a CDR-H3 sequence, the amino acid sequence defined in SEQ ID NO 85, or an amino acid sequence derived from SEQ ID NO 85 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 85, or
   (7) as a CDR-H1 sequence, the amino acid sequence defined in SEQ ID NO 97, or an amino acid sequence derived from SEQ ID NO 97 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 97,
      as a CDR-H2 sequence, the amino acid sequence defined in SEQ ID NO 99, or an amino acid sequence derived from SEQ ID NO 99 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 99, and
      as a CDR-H3 sequence, the amino acid sequence defined in SEQ ID NO 101, or an amino acid sequence derived from SEQ ID NO 101 via substitution, deletion, or insertion of any one or two amino acid residues, or an amino acid sequence having a homology (preferably an identity) of 60.0 % or more or 80.0 % or more to the amino acid sequence defined in SEQ ID NO 101, or
   (8) as a CDR-H1 sequence, the amino acid sequence defined in SEQ ID NO 113, or an amino acid sequence derived from SEQ ID NO 113 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 113,
      as a CDR-H2 sequence, the amino acid sequence defined in SEQ ID NO 115, or an amino acid sequence derived from SEQ ID NO 115 via substitution, deletion, or insertion of any one or two amino acid residues, or an amino acid sequence having a homology (preferably an identity) of 71.4 % or more or 85.7 % or more to the amino acid sequence defined in SEQ ID NO 115, and
      as a CDR-H3 sequence, the amino acid sequence defined in SEQ ID NO 117, or an amino acid sequence derived from SEQ ID NO 117 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 117, or
   (9) as a CDR-H1 sequence, the amino acid sequence defined in SEQ ID NO 129, or an amino acid sequence derived from SEQ ID NO 129 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 129,
      as a CDR-H2 sequence, the amino acid sequence defined in SEQ ID NO 131, or an amino acid sequence derived from SEQ ID NO 131 via substitution, deletion, or insertion of any one or two amino acid residues, or an amino acid sequence having a homology (preferably an identity) of 71.4 % or more or 85.7 % or more to the amino acid sequence defined in SEQ ID NO 131, and
      as a CDR-H3 sequence, the amino acid sequence defined in SEQ ID NO 133, or an amino acid sequence derived from SEQ ID NO 133 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 133, or
   (10) as a CDR-H1 sequence, the amino acid sequence defined in SEQ ID NO 145, or an amino acid sequence derived from SEQ ID NO 145 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 145,
      as a CDR-H2 sequence, the amino acid sequence defined in SEQ ID NO 147, or an amino acid sequence derived from SEQ ID NO 147 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 147, and
      as a CDR-H3 sequence, the amino acid sequence defined in SEQ ID NO 149, or an amino acid sequence derived from SEQ ID NO 149 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 149, or
   (11) as a CDR-H1 sequence, the amino acid sequence defined in SEQ ID NO 161, or an amino acid sequence derived from SEQ ID NO 161 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 161,
      as a CDR-H2 sequence, the amino acid sequence defined in SEQ ID NO 163, or an amino acid sequence derived from SEQ ID NO 163 via substitution, deletion, or insertion of any one or two amino acid residues, or an amino acid sequence having a homology (preferably an identity) of 71.4 % or more or 85.7 % or more to the amino acid sequence defined in SEQ ID NO 163, and
      as a CDR-H3 sequence, the amino acid sequence defined in SEQ ID NO 165, or an amino acid sequence derived from SEQ ID NO 165 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 165, or
   (12) as a CDR-H1 sequence, the amino acid sequence defined in SEQ ID NO 255, or an amino acid sequence derived from SEQ ID NO 255 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 255,
      as a CDR-H2 sequence, the amino acid sequence defined in SEQ ID NO 257, or an amino acid sequence derived from SEQ ID NO 257 via substitution, deletion, or insertion of any one or two amino acid residues, or an amino acid sequence having a homology (preferably an identity) of 71.4 % or more or 85.7 % or more to the amino acid sequence defined in SEQ ID NO 257, and
      as a CDR-H3 sequence, the amino acid sequence defined in SEQ ID NO 259, or an amino acid sequence derived from SEQ ID NO 259 via substitution, deletion, or insertion of any one or two amino acid residues, or an amino acid sequence having a homology (preferably an identity) of 66.6 % or more or 83.3 % or more to the amino acid sequence defined in SEQ ID NO 259, or
   (13) as a CDR-H1 sequence, the amino acid sequence defined in SEQ ID NO 283, or an amino acid sequence derived from SEQ ID NO 283 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 283,
      as a CDR-H2 sequence, the amino acid sequence defined in SEQ ID NO 285, or an amino acid sequence derived from SEQ ID NO 285 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 285,
      as a CDR-H3 sequence, the amino acid sequence defined in SEQ ID NO 287, or an amino acid sequence derived from SEQ ID NO 287 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 287, or
   (14) as a CDR-H1 sequence, the amino acid sequence defined in SEQ ID NO 311, or an amino acid sequence derived from SEQ ID NO 311 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 311,
      as a CDR-H2 sequence, the amino acid sequence defined in SEQ ID NO 313, or an amino acid sequence derived from SEQ ID NO 313 via substitution, deletion, or insertion of any one or two amino acid residues, or an amino acid sequence having a homology (preferably an identity) of 71.4 % or more or 85.7 % or more to the amino acid sequence defined in SEQ ID NO 313,
      as a CDR-H3 sequence, the amino acid sequence defined in SEQ ID NO 315, or an amino acid sequence derived from SEQ ID NO 315 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 315, or
   (15) as a CDR grafting region H1 sequence, the amino acid sequence defined in SEQ ID NO 271, or an amino acid sequence derived from SEQ ID NO 271 via substitution, deletion, or insertion of any one, two, three, or four amino acid residues other than tyrosine at position 27, asparagine at position 32, and tryptophan at position 33,
      as a CDR grafting region H2 sequence, the amino acid sequence defined in SEQ ID NO 273, or an amino acid sequence derived from SEQ ID NO 273 via substitution, deletion, or insertion of any one, two, three, four, five, or six amino acid residues other than aspartic acid at position 55, phenylalanine at position 57, threonine at position 58, asparagine at position 59, tyrosine at position 60, and asparagine at position 61,
      as a CDR grafting region H3 sequence, the amino acid sequence defined in SEQ ID NO 275, or an amino acid sequence derived from SEQ ID NO 275 via substitution, deletion, or insertion of any one, two, three, four, five, or six amino acid residues other than arginine at position 98, glycine at position 100, and glycine at position 109, or
   (16) as a CDR grafting region H1 sequence, the amino acid sequence defined in SEQ ID NO 299, or an amino acid sequence derived from SEQ ID NO 299 via substitution, deletion, or insertion of any one, two, three, or four amino acid residues,
      as a CDR grafting region H2 sequence, the amino acid sequence defined in SEQ ID NO 301, or an amino acid sequence derived from SEQ ID NO 301 via substitution, deletion, or insertion of any one, two, three, four, five, or six amino acid residues other than aspartic acid at position 52, tryptophan at position 54, aspartic acid at position 58, and proline at position 63,
      as a CDR grafting region H3 sequence, the amino acid sequence defined in SEQ ID NO 303, or an amino acid sequence derived from SEQ ID NO 303 via substitution, deletion, or insertion of any one, two, three, four, five, or six amino acid residues other than arginine at position 99, threonine at position 100, tyrosine at position 102, tyrosine at position 105, tyrosine at position 107, and methionine at position 110.
[Aspect 9] The anti-gpNMB antibody (e.g., humanized antibody) or its fragment or a derivative thereof according to any one of Aspects 1 to 7, which comprises at least a heavy chain variable region comprising:
   (1) the amino acid sequence defined in SEQ ID NO 13, or an amino acid sequence having a homology of 60 % or more to the amino acid sequence defined in SEQ ID NO 13, or
   (2) the amino acid sequence defined in SEQ ID NO 29, or an amino acid sequence having a homology of 60 % or more to the amino acid sequence defined in SEQ ID NO 29, or
   (3) the amino acid sequence defined in SEQ ID NO 45, or an amino acid sequence having a homology of 60 % or more to the amino acid sequence defined in SEQ ID NO 45, or
   (4) the amino acid sequence defined in SEQ ID NO 61, or an amino acid sequence having a homology of 60 % or more to the amino acid sequence defined in SEQ ID NO 61, or
   (5) the amino acid sequence defined in SEQ ID NO 77, or an amino acid sequence having a homology of 60 % or more to the amino acid sequence defined in SEQ ID NO 77, or
   (6) the amino acid sequence defined in SEQ ID NO 93, or an amino acid sequence having a homology of 60 % or more to the amino acid sequence defined in SEQ ID NO 93, or
   (7) the amino acid sequence defined in SEQ ID NO 109, or an amino acid sequence having a homology of 60 % or more to the amino acid sequence defined in SEQ ID NO 109, or
   (8) the amino acid sequence defined in SEQ ID NO 125, or an amino acid sequence having a homology of 60 % or more to the amino acid sequence defined in SEQ ID NO 125, or
   (9) the amino acid sequence defined in SEQ ID NO 141, or an amino acid sequence having a homology of 60 % or more to the amino acid sequence defined in SEQ ID NO 141, or
   (10) the amino acid sequence defined in SEQ ID NO 157, or an amino acid sequence having a homology of 60 % or more to the amino acid sequence defined in SEQ ID NO 157, or
   (11) the amino acid sequence defined in SEQ ID NO 173, or an amino acid sequence having a homology of 60 % or more to the amino acid sequence defined in SEQ ID NO 173, or
   (12) the amino acid sequence defined in SEQ ID NO 267, or an amino acid sequence having a homology of 60 % or more to the amino acid sequence defined in SEQ ID NO 267, or
   (13) the amino acid sequence defined in SEQ ID NO 295, or an amino acid sequence having a homology of 60 % or more to the amino acid sequence defined in SEQ ID NO 295, or
   (14) the amino acid sequence defined in SEQ ID NO 323, or an amino acid sequence having a homology of 60 % or more to the amino acid sequence defined in SEQ ID NO 323.
[Aspect 10] An anti-gpNMB antibody (e.g., humanized antibody) or its fragment or a derivative thereof according to Aspect 8 or 9, wherein the heavy chain variable region comprises, as a framework sequence, a framework sequence of a class of human immunoglobulin.
[Aspect 11] The anti-gpNMB antibody (e.g., humanized antibody) or its fragment or a derivative thereof according to any one of Aspects 8 to 10, which further comprises a heavy chain constant region having an amino acid sequence of a heavy chain constant region of a class of human immunoglobulin.
[Aspect 12] The anti-gpNMB antibody (e.g., humanized antibody) or its fragment or a derivative thereof according to any one of Aspects 1 to 11, which comprises at least a light chain variable region comprising:
   (1) as a CDR-L1 sequence, the amino acid sequence defined in SEQ ID NO 7, or an amino acid sequence derived from SEQ ID NO 7 via substitution, deletion, or insertion of any one or two amino acid residues, or an amino acid sequence having a homology (preferably an identity) of 66.6 % or more or 83.3 % or more to the amino acid sequence defined in SEQ ID NO 7,
      as a CDR-L2 sequence, the amino acid sequence defined in SEQ ID NO 9, or an amino acid sequence derived from SEQ ID NO 9 via substitution, deletion, or insertion of any one amino acid residue, or an amino acid sequence having a homology (preferably an identity) of 66.6 % or more to the amino acid sequence defined in SEQ ID NO 9, and
      as a CDR-L3 sequence, the amino acid sequence defined in SEQ ID NO 11, or an amino acid sequence derived from SEQ ID NO 11 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 11, or
   (2) as a CDR-L1 sequence, the amino acid sequence defined in SEQ ID NO 23, or an amino acid sequence derived from SEQ ID NO 23 via substitution, deletion, or insertion of any one or two amino acid residues, or an amino acid sequence having a homology (preferably an identity) of 60.0 % or more or 80.0 % or more to the amino acid sequence defined in SEQ ID NO 23,
      as a CDR-L2 sequence, the amino acid sequence defined in SEQ ID NO 25, or an amino acid sequence derived from SEQ ID NO 25 via substitution, deletion, or insertion of any one amino acid residue, or an amino acid sequence having a homology (preferably an identity) of 66.6 % or more to the amino acid sequence defined in SEQ ID NO 25, and
      as a CDR-L3 sequence, the amino acid sequence defined in SEQ ID NO 27, or an amino acid sequence derived from SEQ ID NO 27 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 27, or
   (3) as a CDR-L1 sequence, the amino acid sequence defined in SEQ ID NO 39, or an amino acid sequence derived from SEQ ID NO 39 via substitution, deletion, or insertion of any one or two amino acid residues, or an amino acid sequence having a homology (preferably an identity) of 60.0 % or more or 80.0 % or more to the amino acid sequence defined in SEQ ID NO 39,
      as a CDR-L2 sequence, the amino acid sequence defined in SEQ ID NO 41, or an amino acid sequence derived from SEQ ID NO 41 via substitution, deletion, or insertion of any one amino acid residue, or an amino acid sequence having a homology (preferably an identity) of 66.6 % or more to the amino acid sequence defined in SEQ ID NO 41, and
      as a CDR-L3 sequence, the amino acid sequence defined in SEQ ID NO 43, or an amino acid sequence derived from SEQ ID NO 43 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 43, or
   (4) as a CDR-L1 sequence, the amino acid sequence defined in SEQ ID NO 55, or an amino acid sequence derived from SEQ ID NO 55 via substitution, deletion, or insertion of any one or two amino acid residues, or an amino acid sequence having a homology (preferably an identity) of 66.6 % or more or 83.3 % or more to the amino acid sequence defined in SEQ ID NO 55,
      as a CDR-L2 sequence, the amino acid sequence defined in SEQ ID NO 57, or an amino acid sequence derived from SEQ ID NO 57 via substitution, deletion, or insertion of any one amino acid residue, or an amino acid sequence having a homology (preferably an identity) of 66.6 % or more to the amino acid sequence defined in SEQ ID NO 57, and
      as a CDR-L3 sequence, the amino acid sequence defined in SEQ ID NO 59, or an amino acid sequence derived from SEQ ID NO 59 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 59, or
   (5) as a CDR-L1 sequence, the amino acid sequence defined in SEQ ID NO 71, or an amino acid sequence derived from SEQ ID NO 71 via substitution, deletion, or insertion of any one or two amino acid residues, or an amino acid sequence having a homology (preferably an identity) of 60.0 % or more or 80.0 % or more to the amino acid sequence defined in SEQ ID NO 71,
      as a CDR-L2 sequence, the amino acid sequence defined in SEQ ID NO 73, or an amino acid sequence derived from SEQ ID NO 73 via substitution, deletion, or insertion of any one amino acid residue, or an amino acid sequence having a homology (preferably an identity) of 66.6 % or more to the amino acid sequence defined in SEQ ID NO 73, and
      as a CDR-L3 sequence, the amino acid sequence defined in SEQ ID NO 75, or an amino acid sequence derived from SEQ ID NO 75 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 75, or
   (6) as a CDR-L1 sequence, the amino acid sequence defined in SEQ ID NO 87, or an amino acid sequence derived from SEQ ID NO 87 via substitution, deletion, or insertion of any one or two amino acid residues, or an amino acid sequence having a homology (preferably an identity) of 66.6 % or more or 83.3 % or more to the amino acid sequence defined in SEQ ID NO 87,
      as a CDR-L2 sequence, the amino acid sequence defined in SEQ ID NO 89, or an amino acid sequence derived from SEQ ID NO 89 via substitution, deletion, or insertion of any one amino acid residue, or an amino acid sequence having a homology (preferably an identity) of 66.6 % or more to the amino acid sequence defined in SEQ ID NO 89, and
      as a CDR-L3 sequence, the amino acid sequence defined in SEQ ID NO 91, or an amino acid sequence derived from SEQ ID NO 91 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 91, or
   (7) as a CDR-L1 sequence, the amino acid sequence defined in SEQ ID NO 103, or an amino acid sequence derived from SEQ ID NO 103 via substitution, deletion, or insertion of any one or two amino acid residues, or an amino acid sequence having a homology (preferably an identity) of 66.6 % or more or 83.3 % or more to the amino acid sequence defined in SEQ ID NO 103,
      as a CDR-L2 sequence, the amino acid sequence defined in SEQ ID NO 105, or an amino acid sequence derived from SEQ ID NO 105 via substitution, deletion, or insertion of any one amino acid residue, or an amino acid sequence having a homology (preferably an identity) of 66.6 % or more to the amino acid sequence defined in SEQ ID NO 105, and
      as a CDR-L3 sequence, the amino acid sequence defined in SEQ ID NO 107, or an amino acid sequence derived from SEQ ID NO 107 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 107, or
   (8) as a CDR-L1 sequence, the amino acid sequence defined in SEQ ID NO 119, or an amino acid sequence derived from SEQ ID NO 119 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 119,
      as a CDR-L2 sequence, the amino acid sequence defined in SEQ ID NO 121, or an amino acid sequence derived from SEQ ID NO 121 via substitution, deletion, or insertion of any one amino acid residue, or an amino acid sequence having a homology (preferably an identity) of 66.6 % or more to the amino acid sequence defined in SEQ ID NO 121, and
      as a CDR-L3 sequence, the amino acid sequence defined in SEQ ID NO 123, or an amino acid sequence derived from SEQ ID NO 123 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 123, or
   (9) as a CDR-L1 sequence, the amino acid sequence defined in SEQ ID NO 135, or an amino acid sequence derived from SEQ ID NO 135 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 135,
      as a CDR-L2 sequence, the amino acid sequence defined in SEQ ID NO 137, or an amino acid sequence derived from SEQ ID NO 137 via substitution, deletion, or insertion of any one amino acid residue, or an amino acid sequence having a homology (preferably an identity) of 66.6 % or more to the amino acid sequence defined in SEQ ID NO 137, and
      as a CDR-L3 sequence, the amino acid sequence defined in SEQ ID NO 139, or an amino acid sequence derived from SEQ ID NO 139 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 139, or
   (10) as a CDR-L1 sequence, the amino acid sequence defined in SEQ ID NO 151, or an amino acid sequence derived from SEQ ID NO 151 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 151,
      as a CDR-L2 sequence, the amino acid sequence defined in SEQ ID NO 153, or an amino acid sequence derived from SEQ ID NO 153 via substitution, deletion, or insertion of any one amino acid residue, or an amino acid sequence having a homology (preferably an identity) of 66.6 % or more to the amino acid sequence defined in SEQ ID NO 153, and
      as a CDR-L3 sequence, the amino acid sequence defined in SEQ ID NO 155, or an amino acid sequence derived from SEQ ID NO 155 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 155, or
   (11) as a CDR-L1 sequence, the amino acid sequence defined in SEQ ID NO 167, or an amino acid sequence derived from SEQ ID NO 167 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 167,
      as a CDR-L2 sequence, the amino acid sequence defined in SEQ ID NO 169, or an amino acid sequence derived from SEQ ID NO 169 via substitution, deletion, or insertion of any one amino acid residue, or an amino acid sequence having a homology (preferably an identity) of 66.6 % or more to the amino acid sequence defined in SEQ ID NO 169, and
      as a CDR-L3 sequence, the amino acid sequence defined in SEQ ID NO 171, or an amino acid sequence derived from SEQ ID NO 171 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 171, or
   (12) as a CDR-L1 sequence, the amino acid sequence defined in SEQ ID NO 261, or an amino acid sequence derived from SEQ ID NO 261 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 261,
      as a CDR-L2 sequence, the amino acid sequence defined in SEQ ID NO 263, or an amino acid sequence derived from SEQ ID NO 263 via substitution, deletion, or insertion of any one amino acid residue, or an amino acid sequence having a homology (preferably an identity) of 66.6 % or more to the amino acid sequence defined in SEQ ID NO 263, and
      as a CDR-L3 sequence, the amino acid sequence defined in SEQ ID NO 265, or an amino acid sequence derived from SEQ ID NO 265 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 265, or
   (13) as a CDR-L1 sequence, the amino acid sequence defined in SEQ ID NO 289, or an amino acid sequence derived from SEQ ID NO 289 via substitution, deletion, or insertion of any one or two amino acid residues, or an amino acid sequence having a homology (preferably an identity) of 60.0 % or more or 80.0 % or more to the amino acid sequence defined in SEQ ID NO 289,
      as a CDR-L2 sequence, the amino acid sequence defined in SEQ ID NO 291, or an amino acid sequence derived from SEQ ID NO 291 via substitution, deletion, or insertion of any one amino acid residue, or an amino acid sequence having a homology (preferably an identity) of 66.6 % or more to the amino acid sequence defined in SEQ ID NO 291, and
      as a CDR-L3 sequence, the amino acid sequence defined in SEQ ID NO 293, or an amino acid sequence derived from SEQ ID NO 293 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 293, or
   (14) as a CDR-L1 sequence, the amino acid sequence defined in SEQ ID NO 317, or an amino acid sequence derived from SEQ ID NO 317 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 317,
      as a CDR-L2 sequence, the amino acid sequence defined in SEQ ID NO 319, or an amino acid sequence derived from SEQ ID NO 319 via substitution, deletion, or insertion of any one amino acid residue, or an amino acid sequence having a homology (preferably an identity) of 66.6 % or more to the amino acid sequence defined in SEQ ID NO 319, and
      as a CDR-L3 sequence, the amino acid sequence defined in SEQ ID NO 321, or an amino acid sequence derived from SEQ ID NO 321 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 321, or
   (15) as a CDR grafting region L1 sequence, the amino acid sequence defined in SEQ ID NO 277, or an amino acid sequence derived from SEQ ID NO 277 via substitution, deletion, or insertion of any one, two, three, or four amino acid residues other than isoleucine at position 29, tyrosine at position 31, and histidine at position 33,
      as a CDR grafting region L2 sequence, the amino acid sequence defined in SEQ ID NO 279, or an amino acid sequence derived from SEQ ID NO 279 via substitution, deletion, or insertion of any one or two amino acid residue other than threonine at position 50,
      as a CDR grafting region L3 sequence, the amino acid sequence defined in SEQ ID NO 281, or an amino acid sequence derived from SEQ ID NO 281 via substitution, deletion, or insertion of any one, two, or three amino acid residues other than histidine at position 88, glutamine at position 89, tryptophan at position 90, serine at position 92, tyrosine at position 93, proline at position 94, and cysteine at position 95, or
   (16) as a CDR grafting region L1 sequence, the amino acid sequence defined in SEQ ID NO 305, or an amino acid sequence derived from SEQ ID NO 305 via substitution, deletion, or insertion of any one, two, three, four, five, or six amino acid residues other than leucine at position 31, tyrosine at position 37 and glutamic acid at position 39,
      as a CDR grafting region L2 sequence, the amino acid sequence defined in SEQ ID NO 307, or an amino acid sequence derived from SEQ ID NO 307 via substitution, deletion, or insertion of any one or two amino acid residue other than lysine at position 55,
      as a CDR grafting region L3 sequence, the amino acid sequence defined in SEQ ID NO 309, or an amino acid sequence derived from SEQ ID NO 309 via substitution, deletion, or insertion of any one, two, or three amino acid residues other than phenylalanine at position 94.
[Aspect 13] The anti-gpNMB antibody (e.g., humanized antibody) or its fragment or a derivative thereof according to any one of Aspects 1 to 11, which comprises at least a light chain variable region comprising:
   (1) the amino acid sequence defined in SEQ ID NO 15, or an amino acid sequence having a homology of 60 % or more to the amino acid sequence defined in SEQ ID NO 15, or
   (2) the amino acid sequence defined in SEQ ID NO 31, or an amino acid sequence having a homology of 60 % or more to the amino acid sequence defined in SEQ ID NO 31, or
   (3) the amino acid sequence defined in SEQ ID NO 47, or an amino acid sequence having a homology of 60 % or more to the amino acid sequence defined in SEQ ID NO 47, or
   (4) the amino acid sequence defined in SEQ ID NO 63, or an amino acid sequence having a homology of 60 % or more to the amino acid sequence defined in SEQ ID NO 63, or
   (5) the amino acid sequence defined in SEQ ID NO 79, or an amino acid sequence having a homology of 60 % or more to the amino acid sequence defined in SEQ ID NO 79, or
   (6) the amino acid sequence defined in SEQ ID NO 95, or an amino acid sequence having a homology of 60 % or more to the amino acid sequence defined in SEQ ID NO 95, or
   (7) the amino acid sequence defined in SEQ ID NO 111, or an amino acid sequence having a homology of 60 % or more to the amino acid sequence defined in SEQ ID NO 111, or
   (8) the amino acid sequence defined in SEQ ID NO 127, or an amino acid sequence having a homology of 60 % or more to the amino acid sequence defined in SEQ ID NO 127, or
   (9) the amino acid sequence defined in SEQ ID NO 143, or an amino acid sequence having a homology of 60 % or more to the amino acid sequence defined in SEQ ID NO 143, or
   (10) the amino acid sequence defined in SEQ ID NO 159, or an amino acid sequence having a homology of 60 % or more to the amino acid sequence defined in SEQ ID NO 159, or
   (11) the amino acid sequence defined in SEQ ID NO 175, or an amino acid sequence having a homology of 60 % or more to the amino acid sequence defined in SEQ ID NO 175, or
   (12) the amino acid sequence defined in SEQ ID NO 269, or an amino acid sequence having a homology of 60 % or more to the amino acid sequence defined in SEQ ID NO 269, or
   (13) the amino acid sequence defined in SEQ ID NO 297, or an amino acid sequence having a homology of 60 % or more to the amino acid sequence defined in SEQ ID NO 297, or
   (14) the amino acid sequence defined in SEQ ID NO 325, or an amino acid sequence having a homology of 60 % or more to the amino acid sequence defined in SEQ ID NO 325.
[Aspect 14] The anti-gpNMB antibody (e.g., humanized antibody) or its fragment or a derivative thereof according to Aspect 12 or 13, wherein the light chain variable region comprises, as a framework sequence, a framework sequence of a class of human immunoglobulin.
[Aspect 15] The anti-gpNMB antibody (e.g., humanized antibody) or its fragment or a derivative thereof according to any one of Aspects 12 to 14, which further comprises a light chain constant region having an amino acid sequence of a light chain constant region of a class of human immunoglobulin.
[Aspect 16] The anti-gpNMB antibody (e.g., humanized antibody) or its fragment or a derivative thereof according to any one of Aspects 1 to 15, which is a Fab, scFv, Diabody, Nanobody, VHH, bispecific antibody, or multispecific antibody, or a derivative thereof.
[Aspect 17] An anti-gpNMB antibody (e.g., humanized antibody) or its fragment or a derivative thereof which binds to at least one site in a region from a PMEL-CAF-like domain to a PKD domain competitively with an anti-gpNMB antibody or its fragment or a derivative thereof according to any one of Aspects 1 to 16.
[Aspect 18] A nucleic acid molecule comprising a polynucleotide sequence encoding an anti-gpNMB antibody (e.g., humanized antibody) or its fragment or a derivative thereof according to any one of Aspects 1 to 17.
[Aspect 19] A cloning vector or expression vector carrying at least one nucleic acid molecule according to Aspect 18.
[Aspect 20] A recombinant cell transformed with a vector according to Aspect 19.
[Aspect 21] A method for producing an anti-gpNMB antibody (e.g., humanized antibody) or its fragment or a derivative thereof according to any one of Aspects 1 to 17, comprising culturing a recombinant cell according to Aspect 20.
[Aspect 22] A method for producing an anti-gpNMB antibody (e.g., humanized antibody) or its fragment or a derivative thereof according to any one of Aspects 1 to 17, comprising:
   administering to an animal a polypeptide having the same amino acid sequence as at least one region selected from:
      a region containing amino acid residue(s) D287 and/or H301,
      a region containing amino acid residue(s) R214 and/or R215,
      a region containing amino acid residue(s) K257 and/or D258,
      a region containing amino acid residue(s) H268 and/or D269,
      a region containing amino acid residue K282,
      a region containing amino acid residue K316,
      a region containing amino acid residue K186, and
      a region containing amino acid residue(s) H216 and/or R218
   of human gpNMB having the amino acid sequence defined in SEQ ID NO 209; and
   collecting an antibody or its fragment or a derivative thereof produced in the body of the animal.
[Aspect 23] A vaccine having an activity to stimulate the production of an anti-gpNMB antibody (e.g., humanized antibody) or its fragment or a derivative thereof according to any one of Aspects 1 to 17, comprising a polypeptide having the same amino acid sequence as a region containing amino acid residue(s) D287 and/or H301 and /or a region containing amino acid residue(s) R214 and /or R215 of human gpNMB having the amino acid sequence defined in SEQ ID NO 209.
[Aspect 24] A pharmaceutical composition comprising, as an active ingredient, one or more selected from the group consisting of an anti-gpNMB antibody (e.g., humanized antibody) or its fragment or a derivative thereof according to any one of Aspects 1 to 17, a nucleic acid molecule according to Aspect 18, a vector according to Aspect 19, and a recombinant cell according to Aspect 20.
[Aspect 25] The pharmaceutical composition according to Aspect 24, for at least one use selected from;
   decreasing the number of mal-functional microglia in a subject;
   removing amyloid β oligomers in a subject;
   increasing the number of synapses in a subject;
   removing phosphorylated tau and/or inhibiting accumulation of tau in a subject;
   removing synuclein and/or synuclein aggregates in a subject; and
   inhibiting or restoring declines in cognitive functions in a subject,
   inhibiting or restoring declines in motor functions in a subject,
   treating or preventing neurodegenerative disease in a subject.
[Aspect 26] A pharmaceutical composition for at least one use selected from;
   removing amyloid β oligomers in a subject;
   increasing the number of synapses in a subject;
   removing phosphorylated tau and/or inhibiting accumulation of tau in a subject;
   removing synuclein and/or synuclein aggregates in a subject; and
   inhibiting or restoring declines in cognitive functions in a subject,
   inhibiting or restoring declines in motor functions in a subject,
   treating or preventing neurodegenerative disease in a subject,
   the pharmaceutical composition comprising, as an active ingredient, an agent for decreasing the number of mal-functional microglia.
[Aspect 27] The pharmaceutical composition according to Aspect 24, for use in treating or preventing neurodegenerative disease in a subject.
[Aspect 28] The pharmaceutical composition according to any one of Aspects 25 to 27, wherein the neurodegenerative disease is a disease associated with accumulation of at least one protein selected from amyloid β, tau, synuclein, polyglutamine, RAN translation-generated protein, and prion.
[Aspect 29] The pharmaceutical composition according to any one of Aspects 25 to 27, wherein the neurodegenerative disease is at least one disease selected from Alzheimer's disease, frontotemporal lobar degeneration (FTLD-Tau), frontotemporal dementia (FTD), primary age-related tauopathy (PART), chronic traumatic encephalopathy (CTE), Pick's disease, cerebral cortex basal ganglia degeneration (CBD or CBS), progressive supranuclear paralysis (PSP), globular glial tauopathy (GGT), argyrophilic grain dementia (AGD) (argyrophilic grain disease), aging-related tau astroglial tauopathy (ARTAG), familial British dementia (FBD), familial Danish dementia (FDD), FTDP17, multisystem tauopathy with dementia (MSTD), dementia of the neurofibrillary tangle type, diffuse neurofibrillary tangles with calcification (DNTC), white matter tauopathy with globular glial inclusions (WMT-GGI), Parkinson's disease, Huntington's disease, spinal cord cerebellum degeneration, hereditary spinocerebellar ataxia, spinal and bulbar muscular atrophy, Lewy body dementia, Creutzfeldt-Jakob disease, amyotrophic lateral sclerosis (ALS), frontotemporal lobar degeneration (FTLD-TDP, FTLD-FUS), and repeat diseases.
[Aspect 30] The pharmaceutical composition according to Aspect 29, wherein the neurodegenerative disease is Alzheimer's disease.
[Aspect 31] The pharmaceutical composition according to Aspect 29, wherein the neurodegenerative disease is Parkinson's disease, Lewy body dementia, multisystem atrophy (MSA), or amyotrophic lateral sclerosis (ALS).
[Aspect 32] The pharmaceutical composition according to Aspect 30 or 31, further comprising a second active ingredient.
[Aspect 33] The pharmaceutical composition according to Aspect 32, wherein the second active ingredient one or more selected from an anti-Tau antibody, anti-amyloid β antibody, anti-CD33 antibody, anti-semaphorin 4D antibody, anti-TNFα antibody, anti-sortilin antibody, anti-galactose-specific lectin (galectin) 3 antibody, anti-TREM2 (Triggering receptor expressed on myeloid cells 2) antibody, anti-IL-1β antibody, anti-CD38 antibody, anti-MS4A antibody, and anti-synuclein antibody.
[Aspect 34] The pharmaceutical composition according to Aspect 32, wherein the second active ingredient is a vaccine comprising a full-length or partial-length polypeptide of one or more proteins selected from Tau, amyloid β, CD33, semaphorin 4D, TNFα, sortilin, galactose-specific lectin (galectin) 3, and TREM2 (triggering receptor expressed on myeloid cells 2), or a nucleic acid encoding the polypeptide.

The term "pharmaceutical drug" used herein is to be interpreted as encompassing both the concepts of "therapeutic agent" and "prophylactic agent," unless it is inconsistent with the context.

### EFFECT OF THE INVENTION

The anti-gpNMB antibodies (among others, humanized antibodies) of the present invention can exhibit extremely unexpected effects, e.g., the effects of decreasing the number of mal-functional microglia in a subject and/or removing amyloid β oligomers and/or phosphorylated tau and/or synuclein oligomers in a subject and/or increasing the number of synapses in a subject and/or inhibiting or restoring declines in cognitive functions or motor functions, by specifically binding to at least one site in a region from a PMEL-CAF-like domain to a PKD domain of human gpNMB. The use of such anti-gpNMB antibodies will also provide pharmaceutical drugs for treating or preventing various diseases including neurodegenerative diseases such as Alzheimer's disease and Parkinson's disease based on new mechanisms of action.

### BRIEF EXPLANATION OF FIGURES

[Figure 1] Figure 1 shows an amino acid sequence alignment of human gpNMB (isotype a) and mouse gpNMB, in which the region at amino acid residues 256 to 319 is shown with dotted lines as an example of the PKD domain, and the region at amino acid residues 172 to 246 is shown with solid lines as an example of the PMEL-CAF-like domain.
[Figure 2] Figure 2 shows immunostained photographs indicating the effect of administration of anti-gpNMB antibodies (GPN05-1 and GPN06-1) on reducing gpNMB-positive microglia in Alzheimer's disease model mice. (A) Control, (B) GPN05-1-treated group, and (C) GPN06-1-treated group. L: left brain, R: right brain, DG: dentate gyrus, CA: cornu Ammonis sectors, CA3: hippocampal CA3 region, CA 23: hippocampal CA2-CA3 areas, Ent: Entorhinal Cortex, PPtA: cortical somatosensory cortex.
[Figure 3] Figure 3 is a graph showing the statistical analysis of the immunostained photographs in Figure 2.
[Figure 4] Figure 4 shows immunostained photographs indicating the effect of administration of anti-gpNMB antibodies (GPN05-1 and GPN06-1) on removing of Aβ oligomers in Alzheimer's disease model mice. (A) Control, (B) GPN05-1-treated group, and (C) GPN06-1-treated group.
[Figure 5] Figure 5 shows stained photographs indicating the effect of administration of anti-gpNMB antibodies (GPN05-1 and GPN06-1) on restoring synapses in Alzheimer's disease model mice. (A) Control 1 (Non-Tg mice), (B) Control 2 (APPosk mice), (C) GPN05-1-treated group, and (D) GPN06-1-treated group.
[Figure 6] Figure 6 shows immunostained photographs of brain sections of Alzheimer's disease model mice after antibody administration. (A) Control antibody 11E10-treated group, (B) GPN09-1-treated group, (C) GPN11-10-treated group, (D) GPN15-2-treated group, (E) GPN15-3-treated group, and (F) GPN18 -2 treated group.
[Figure 7] Figure 7 is a graph showing the statistical analysis of the immunostained photographs in Figure 6.
[Figure 8] Figure 8 shows stained photographs indicating the effect of administration of an anti-gpNMB antibody (GPN18-2) on restoring synapses in Alzheimer's disease model mice. (A) Control (Non-Tg mice), and (B) GPN18-2-treated group.
[Figure 9] Figure 9 shows immunostained photographs of brain sections of Alzheimer's disease model mice after antibody administration. (A) Control antibody-treated group, (B) GPN18-2-treated group, (C) GPN06-1-treated group, (D) GPN18-5-treated group, and (E) 1-5E-treated group.
[Figure 10] Figure 10 is a graph showing the statistical analysis of the immunostained photographs in Figure 9.
[Figure 11] Figure 11 shows immunostained photographs of Alzheimer's disease model mice (APPosk mice) after antibody administration. (A) Control antibody-treated group, (B) GPN18-2-treated group, (C) GPN06-1-treated group, (D) GPN18-5-treated group, and (E) 1-5E-treated group.
[Figure 12] Figure 12 is a graph showing the statistical analysis of the immunostained photographs in Figure 11.
[Figure 13] Figure 13 is a graph showing the results of Morris water maze test (Acquisition test) on Alzheimer's disease model mice (APP/Tau-Tg mice) after administration with anti-gpNMB antibodies (GPN06-1, GPN18-2).
[Figure 14] Figure 14 is a graph showing the results of Morris water maze test (Probe test) on Alzheimer's disease model mice (APP/Tau-Tg mice) after administration with anti-gpNMB antibodies (GPN06-1, GPN18-2).
[Figure 15] Figure 15 is a graph showing the results of Morris water maze test (Probe test) on Alzheimer's disease model mice (APP/Tau-Tg mice) after administration with an anti-gpNMB antibody (GPN18-2).
[Figure 16] Figure 16 is a graph showing the results of Morris water maze test (Probe test) on Alzheimer's disease model mice (APP/Tau-Tg mice) after administration with an anti-gpNMB antibody (GPN18-2).
[Figure 17] Figure 17 shows double immunostained photographs of Iba1 and gpNMB in brain sections of Alzheimer's disease model mice (APP/Tau-Tg mice) after antibody administration. (A1) Control antibody-treated group, hippocampus; (A2) Control antibody-treated group, hippocampus; (B1) Anti-gpNMB antibody (GPN18-2)-treated group, olfactory endocortex; (B2) Anti-gpNMB antibody (GPN18-2)-treated group, olfactory endocortex.
[Figure 18] Figure 18 shows double immunostained photographs of Aβ and phosphorylated tau in brain sections of Alzheimer's disease model mice (APP/Tau-Tg mice) after antibody administration. (A1) Control antibody-treated group, hippocampus; (A2) Control antibody-treated group, hippocampus; (B1) Anti-gpNMB antibody (GPN18-2)-treated group, olfactory endocortex; (B2) Anti-gpNMB antibody (GPN18-2)-treated group, olfactory endocortex.
[Figure 19] Figure 19 shows immunostained photographs of brain sections Alzheimer's disease model mice (APP/Tau-Tg mice) after antibody administration. (A) Control antibody-treated group, olfactory endocortex, (B) Anti-gpNMB antibody (GPN18-2)-treated group, olfactory endocortex.
[Figure 20] Figure 20 shows an alignment of humanized mutation sequences hGPN06-1_VH1 to VH5 to mouse GPN06-1_VH.

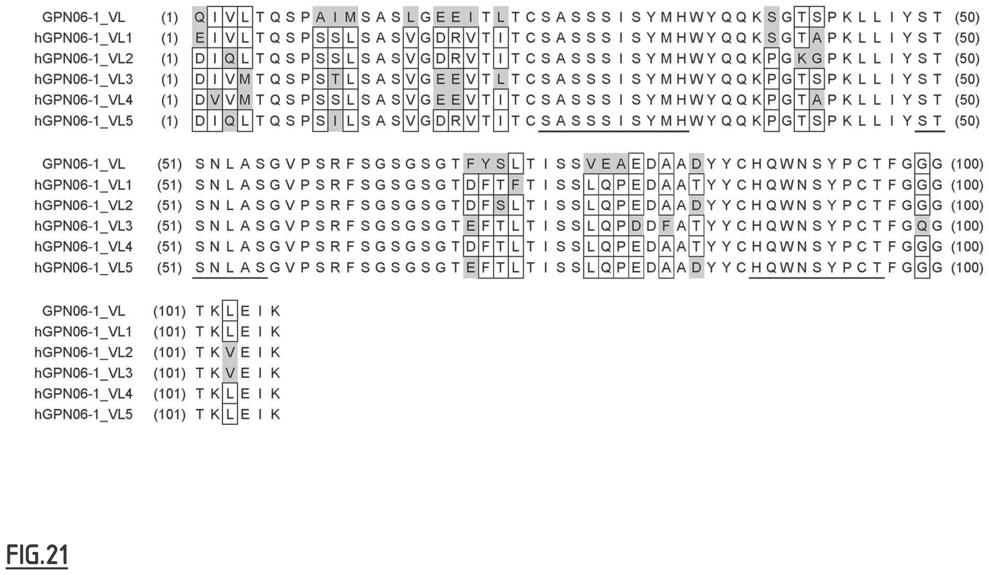

[Figure 21] Figure 21 shows an alignment of humanized mutation sequences hGPN06-1_VL1 to VL5 to mouse GPN06-1_VL.
[Figure 22] Figure 22 shows an alignment of humanized mutation sequences hGPN18-2_VH1 to VH5 to mouse GPN18-2_VH.
[Figure 23] Figure 23 shows an alignment of humanized mutation sequences hGPN18-2_VL1 to VL5 to mouse GPN18-2_VL.
[Figure 24] Figure 24 shows fluorescent immunohistochemistry tissue-stained photographs of the hippocampus and cerebral cortex of Parkinson's disease model mice (A53T mutation human α-synuclein-Tg mice). (A) is a photograph with staining with an anti-pS129 α-synuclein antibody, (B) is a photograph with double staining with an anti-gpNMB antibody polyclonal antibody and an anti-pS129α synuclein antibody, and (C) is a photograph with double staining with an Iba1 antibody and an anti-gpNMB polyclonal antibody.
[Figure 25] Figure 25 is a graph showing the results of Morris water maze test (Acquisition) on Parkinson's disease model mice (A53T mutation human α-synuclein-Tg mice) after administration with anti-gpNMB antibodies (GPN06-1, GPN18-2).
[Figure 26] Figure 26 is a graph showing the results of Rotarod test on Parkinson's disease model mice (A53T mutation human α-synuclein-Tg mice) after administration with anti-gpNMB antibodies (GPN06-1, GPN18-2).
[Figure 27] Figure 27 is a graph showing the results of Inverted Screen test on Parkinson's disease model mice (A53T mutation human α-synuclein-Tg mice) after administration with anti-gpNMB antibodies (GPN06-1, GPN18-2).
[Figure 28] Figure 28 shows double immunostained photographs of Iba1 and gpNMB in brain sections of Parkinson's disease model mice (A53T mutation human α-synuclein-Tg mice) after antibody administration with anti-gpNMB antibodies (GPN06-1, GPN18-2).
[Figure 29] Figure 29 is a graph showing the statistical analysis of the immunostained photographs in Figure 28.
[Figure 30] Figure 30 shows double immunostained photographs of phosphorylated α-synuclein in brain sections of Parkinson's disease model mice (A53T mutation human α-synuclein-Tg mice) after antibody administration with anti-gpNMB antibodies (GPN06-1, GPN18-2).
[Figure 31] Figure 31 is a graph showing the statistical analysis of the immunostained photographs in Figure 30.
[Figure 32] Figure 32 shows double immunostained photographs of synuclein oligomers in brain sections of Parkinson's disease model mice (A53T mutation human α-synuclein-Tg mice) after antibody administration with anti-gpNMB antibodies (GPN06-1, GPN18-2).
[Figure 33] Figure 33 is a graph showing the statistical analysis of the immunostained photographs in Figure 32.
[Figure 34] Figure 34 shows double immunostained photographs of synaptophysin in brain sections of Parkinson's disease model mice (A53T mutation human α-synuclein-Tg mice) after antibody administration with anti-gpNMB antibodies (GPN06-1, GPN18-2).
[Figure 35] Figure 35 is a graph showing the statistical analysis of the immunostained photographs in Figure 34.

### EMBODIMENTS

The present invention will now be described based on specific embodiments. These embodiments should not be construed to limit the scope of the present invention. All references, including patent publications, unexamined patent publications, and non-patent publications cited in this specification, can be incorporated by reference in their entirety for all purposes.

### [Anti-gpNMB antibody]

An embodiment of the present invention relates to an anti-gpNMB antibody or its fragment or a derivative thereof which binds specifically to at least one site in a region from a PMEL-CAF-like (PMEL core amyloid fragment-like) domain to a PKD domain of human gpNMB (glycoprotein nonmetastatic melanoma protein B) (hereinafter also referred to as "the anti-gpNMB antibody of the present invention" or "the antibody of the present invention"). The statement "at least one site in a region from a PMEL-CAF-like domain to a PKD domain of gpNMB" herein refers to at least one site in a region with, e.g., the amino acid residues at positions 172 to 319 in human gpNMB having the amino acid sequence defined in SEQ ID NO 209.

### * Antibody:

The term "antibody" herein refers to a glycoprotein containing at least two heavy (H) chains and two light (L) chains interconnected through disulfide bonds. Each heavy chain contains a heavy chain variable region (abbreviated as VH) and a heavy chain constant region, and the heavy chain constant region contains three domains, CH1, CH2 and CH3. Each light chain contains a light chain variable region (abbreviated as VL) and a light chain constant region. The light chain constant region contains one domain, CL. The light chain constant region has two chains called a λ chain and a κ chain. The heavy chain constant region has a γ chain, a µ chain, an α chain, a δ chain, and an ε chain, and has isotypes of antibodies called IgG, IgM, IgA, IgD and IgE, respectively, depending on the difference in the heavy chain. The VH and VL regions are further divided into four regions (FR-1, FR-2, FR-3, FR-4) having higher preservability called framework regions (FR) and three regions (CDR-1, CDR-2, CDR-3) called complementarity determining regions (CDR). The VH region comprises three CDRs and four FRs, in detail, a sequence of FR-1, CDR-1 (CDR-H1), FR-2, CDR-2 (CDR-H2), FR-3, CDR-3 (CDR-H3), and FR4 from the amino terminal to the carboxy terminal. The VL region comprises three CDRs and four FRs, in detail, a sequence of FR-1, CDR-1 (CDR-L1), FR-2, CDR-2 (CDR-L2), FR-3, CDR-3 (CDR-L3), and FR4 from the amino terminal to the carboxy terminal. The variable regions of the heavy and light chains contain binding domains that interact with an antigen.

The antibody of the present invention may be a fragment (e.g., an antigen-binding fragment) and/or a derivative of an antibody, as long as it has the activity to bind specifically to at least one site in a region from a PMEL-CAF-like domain to a PKD domain of gpNMB. Examples of fragments of antibodies include F (ab') 2, Fab, and Fv. Examples of derivatives of antibodies include an antibody in which an amino acid mutation is artificially incorporated in the constant region, an antibody in which the domain configuration of the constant region is modified, an antibody in the form having two or more Fc fragments per molecule, an antibody composed of only a heavy chain or only a light chain, a sugar chain modified antibody, a bispecific antibody, an antibody conjugate combined with an antibody or a fragment compound of an antibody or a protein other than antibodies, an antibody enzyme, a nanobody, a tandem scFv fragment, a bispecific tandem scFv fragment, a diabody, and a VHH body. In the present invention, the term "antibody" simply includes a fragment and/or a derivative of an antibody unless otherwise specified.

The antibody of the present invention may be either a monoclonal antibody or a polyclonal antibody, but may preferably be a monoclonal antibody. The term monoclonal antibody classically refers to an antibody molecule obtained from a clone derived from a single antibody producing cell, and is a single type of antibody molecule containing a combination of VH and VL each consisting of a specific amino acid sequence. It is possible to obtain from a monoclonal antibody a nucleic acid molecule having a gene sequence encoding the amino acids of each of the proteins constituting the antibody, and it is also possible to produce the antibody using such nucleic acid molecules using genetic engineering methods. It is well known to a person skilled in the art that genetic information on the sequences of, e.g., the H and L chains, their variable regions, and their CDRs can be used to modify the antibody to improve its binding and specificity, etc., and that antibodies from animals such as mice can be modified into human-type antibodies to produce antibodies with a structure suitable for use as therapeutic agents. A human monoclonal antibody can be also produced with a transgenic animal into which a human antibody gene has been introduced to be sensitized with an antigen. Other methods that do not require sensitization of animals include the use of phage libraries expressing antigen-binding regions of human antibodies or portions thereof (human antibody phage display) to obtain antibodies that bind specifically to the corresponding antigens or phage clones consisting of specific amino acid sequences, and those skilled in the art can use such information to produce human antibodies as appropriate (see, e.g., the review by Taketo Tanaka et al., in Keio J. Med., (2011), 60:37-46). Those skilled in the art can also design antibodies to be administered to non-human animals using the amino acid sequence information of CDRs and variable regions as appropriate, using a technique similar to the humanization technique.

The specificity of an antibody herein means that the antibody exhibits a high antigen-antibody reaction to a specific antigen. Those skilled in the art can determine the antigen-antibody reaction by appropriately selecting a binding measurement method in a solid phase or liquid phase system. Examples of such methods include, although not limited to, enzyme-linked immunosorbent assay (ELISA), enzyme immunoassay (EIA), surface plasmon resonance (SPR), fluorescence resonance energy transfer (FRET), and luminescence resonance energy transfer (LRET). Before the binding between an antigen and an antibody is measured, the antibody and/or the antigen may be labeled with, e.g., an enzyme, a fluorescent substance, a luminescent substance, or a radioisotope, and then the antigen-antibody reaction may be detected by a method suitable for the physical and/or chemical properties of the labeled substance.

### *Specific binding to at least one site in a region from a PMEL-CAF-like domain to a PKD domain of gpNMB:

The antibody of the present invention specifically binds to at least one site in a region from a PMEL-CAF-like domain to a PKD domain of gpNMB. The term "gpNMB" (glycoprotein nonmetastatic melanoma protein B; also referred to as Osteoactivin or DC-HIL) herein refers to the single transmembrane glycoprotein mentioned above. For more details, see each of the documents mentioned above (especially Non-Patent Literature 20).

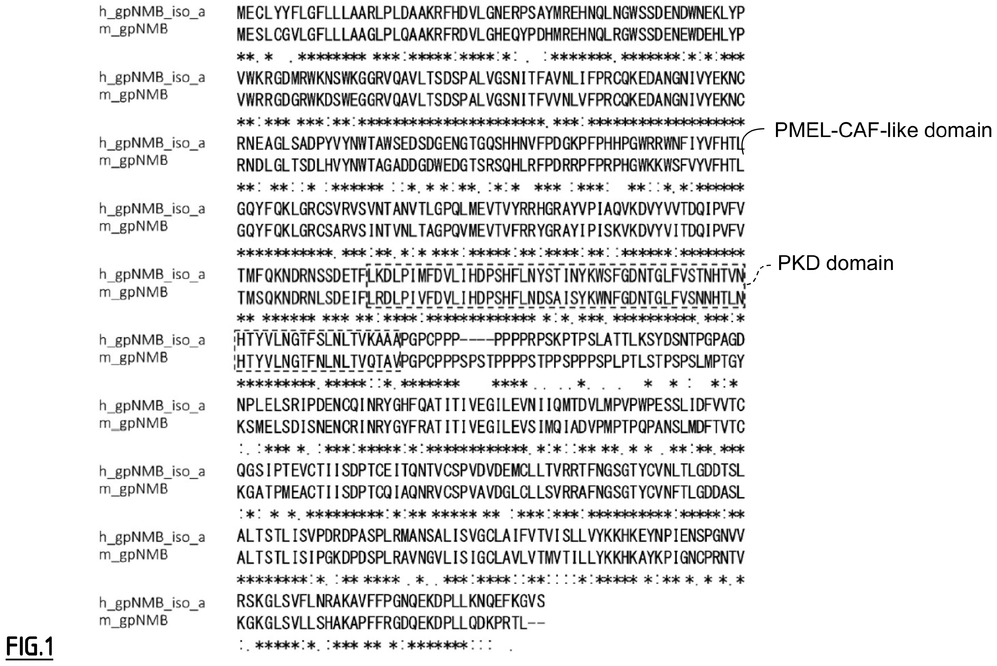

The amino acid sequence of the total length protein of human gpNMB (isotype a) is shown in SEQ ID NO 209, and the nucleic acid sequence of a gene encoding the protein is shown in SEQ ID NO 210. The amino acid sequence of the total length protein of mouse gpNMB is shown in SEQ ID NO 211, and the nucleic acid sequence of a gene encoding the protein is shown in SEQ ID NO 212. The amino acid sequence of the total length protein of rat gpNMB is shown in SEQ ID NO 213, and the nucleic acid sequence of a gene encoding the protein is shown in SEQ ID NO 214. The alignment of the amino acid sequences of human gpNMB (isotype a) and mouse gpNMB is shown in Figure 1.

The PKD (Polycystic Kidney Disease homology) domain of gpNMB is a domain containing a beta sheet consisting of three strands and a beta sheet consisting of four strands folded together to form a beta sandwich structure. It was named the PKD domain because it is a characteristic structure possessed by polycystin-1 (polycystin-1), the gene responsible for polycystic kidney disease (Non-Patent Literature 26). According to an embodiment, in the case of the human gpNMB defined in SEQ ID NO 209 and the mouse gpNMB defined in SEQ ID NO 211, the region containing the amino acid residues at positions 256 to 319 corresponds to the PKD domain. According to an embodiment, the PKD domain may preferably be a region containing the amino acid residues at positions 256 to 316, more preferably a region containing positions 257 to 316, still more preferably a region containing 268 to 316, even more preferably a region containing 270 to 316, particularly preferably a region containing 282 to 316, especially preferably a region containing 287 to 316. According to an embodiment, the PKD domain may preferably be a region containing the amino acid residues at positions 257 to 319, more preferably a region containing positions 257 to 316, still more preferably a region containing positions 257 to 301. According to an embodiment, the PKD domain may preferably be a region containing the amino acid residues at positions 287 to 301. The region at amino acid residues 256 to 319 surrounded by a solid line in Figure 1 is an example of the PDK domain of each of human gpNMB and mouse gpNMB.

The PMEL-CAF-like (PMEL Core Amyloid Fragment-like) domain of gpNMB is a region that is homologous to a CAF region in PMEL of a protein of the same family as described above, which is a region that induces the formation of amyloid-like aggregates in a PMEL protein (Non-Patent Literature 71). The PMEL-CAF-like domain of gpNMB is considered to prevent aggregation formation by having many N-type glycan binding sites in the neighboring PKD region (Non-Patent Literature 70). According to an embodiment, in the case of the human gpNMB defined in SEQ ID NO 209 and the mouse gpNMB defined in SEQ ID NO 211, the region containing the amino acid residues at positions 172 to 246 corresponds to the PMEL-CAF-like domain. The PMEL-CAF-like domain may preferably be a region containing the amino acid residues at positions178 to 228, more preferably a region containing the amino acid residues at positions186 to 218. The region at amino acid residues 172 to 246 surrounded by a solid line in Figure 1 is an example of the PMEL-CAF-like domain of each of human gpNMB and mouse gpNMB.

The term "region from a PMEL-CAF-like domain to a PKD domain of gpNMB" herein refers to a region containing not only the PMEL-CAF-like domain and the PKD domain defined above, but also a region intervening between the PMEL-CAF-like domain and the PKD domain. According to an embodiment, in the case of the human gpNMB defined in SEQ ID NO 209 and the mouse gpNMB defined in SEQ ID NO 211, the region containing the amino acid residues at positions 172 to 319 corresponds to a region from a PMEL-CAF-like domain to a PKD domain.

The antibody of the present invention may have the ability to specifically bind to at least one site in a region from a PMEL-CAF-like domain to a PKD domain of gpNMB of any vertebrate. However, in view of human pharmaceutical uses, the antibody of the present invention may preferably have the ability to specifically bind to at least one site in a region from a PMEL-CAF-like domain to a PKD domain of at least the human gpNMB defined in SEQ ID NO 209. From the viewpoint of evaluating the effect on endogenous gpNMB in disease model mice and disease model rats, the antibody of the present invention may more preferably have the ability to specifically bind to at least one site in a region from the PMEL-CAF-like domain to the PKD domain of the mouse gpNMB defined in SEQ ID NO 211, in addition to at least one site in a region from a PMEL-CAF-like domain to a PKD domain of human gpNMB. The statement that an antibody "specifically" binds to gpNMB herein refers to the binding ability of the antibody to gpNMB with a binding strength of 10-fold or more, preferably 100-fold or more, more preferably 1000-fold or more, compared to the binding ability of the antibody to proteins other than gpNMB (e.g. albumin). In addition, the "region from the PMEL-CAF-like domain to the PKD domain" herein refers to a region including the PMEL-CAF-like domain, the PKD domain, and the region intervening between the PMEL-CAF-like domain and the PKD domain.

When the antibody of the present invention specifically binds to at least one site in a region from a PMEL-CAF-like domain to a PKD domain of human gpNMB, the epitope in the region to which the antibody binds is not particularly limited. However, the antibody of the present invention may preferably have the ability to specifically bind to at least one site or more in a region from the PMEL-CAF-like domain to the PKD domain, and may more preferably have the specifically ability to bind to one or more sites selected from a site containing amino acid residue(s) K257 and/or D258 of the PKD domain, a site containing amino acid residue(s) H268 and/or D269 of the PKD domain, a site containing an amino acid residue K282 of the PKD domain, a site containing amino acid residue(s) D287 and/or H301 of the PKD domain, a site containing an amino acid residue K316 of the PKD domain, a site containing an amino acid residue K186 of the PMEL-CAF-like domain, a site containing amino acid residue(s) R214 and/or R215 of the PMEL-CAF-like domain, and a site containing amino acid residue(s) H216 and/or R218 of the PMEL-CAF-like domain of the human gpNMB defined in SEQ ID NO 209. Among them, the antibody of the present invention may preferably have the ability to specifically bind to at least either or both of a site containing amino acid residue(s) D287 and/or H301 of the PKD domain and a site containing amino acid residue(s) R214 and/or R215 of the PMEL-CAF-like domain of the human gpNMB defined in SEQ ID NO 209. Regardless of whether or not to have the ability to bind to these sites, it may preferably have the ability to also specifically bind to one or more sites selected from a site containing amino acid residue(s) K257 and/or D258, a site containing amino acid residue(s) H268 and/or D269, a site containing an amino acid residue K282, a site containing an amino acid residue K316, K186 a region containing an amino acid residue, and a site containing amino acid residue(s) H216 and/or R218 of human gpNMB.

The epitope of human gpNMB to which the antibody of the present invention binds may be a linear epitope, which consists of a contiguous region on the primary structure of human gpNMB (amino acid sequence shown in SEQ ID NO 209), or a steric epitope, which is formed in a steric structure by two or more discrete subregions of the primary structure, as long as the epitope includes at least one site within the region from the PMEL-CAF-like domain to the PKD domain. The region constituting the linear epitope and each of the subregions constituting the steric epitope may be located in the PMEL-CAF-like domain, the PKD domain, or the region between the PMEL-CAF-like domain and the PKD domain, or may span more than one of these regions. They may also be present only within the region from the PMEL-CAF-like domain to the PKD domain, or they may span other regions as well. Also, of the two or more partial regions constituting the steric epitope, if at least one partial region is partially or entirely within the region from the PMEL-CAF-like domain to the PKD domain, the other partial regions may be outside the region from the PMEL-CAF-like domain to the PKD domain.

The binding strength of the antibody of the present invention to the region from the PMEL-CAF-like domain to the PKD domain of gpNMB is not particularly limited. However, as measured by antigen ELISA using recombinant gpNMB and/or cell ELISA using gpNMB-expressing cells, the antibody of the present invention may preferably have a 50% effective concentration (EC₅₀) to the region from the PMEL-CAF-like domain to the PKD domain of gpNMB of typically 1×10⁻⁷M or lower, or 1×10⁻⁸M or lower, or 1×10⁻⁹M or lower. The "50% effective concentration" (EC₅₀) of an antibody herein refers to the concentration of the antibody that exhibits 50% of the maximum binding affinity with which that antibody binds to the antigen. Specific conditions for measuring EC₅₀ by antigen ELISA using recombinant gpNMBs and/or cell ELISA using gpNMB-expressing cells include those employed in the Examples described below.

The antibody of the present invention may also bind to one or more sites in any region other than the region from the PMEL-CAF-like domain to the PKD domain of gpNMB. Examples of amino acid sites to which the antibody of the present invention may bind other than the region from the PMEL-CAF-like domain to the PKD domain of gpNMB may preferably include, although not limited to, E385 of the human gpNMB defined in SEQ ID NO 209.

### *Activities of the antibody:

According to an embodiment, the antibody of the present invention may preferably have various activities explained below.

The antibody of the present invention may preferably have the activity to bind to microglia. It has been reported that gpNMB is expressed in mail-functional microglia that have become mal-functional in their ability to degrade toxic or unwanted proteins under stress (Non-Patent Literature 42). The antibody of the present invention may preferably have the activity to bind to at least one site in a region from the PMEL-CAF-like domain to the PKD domain of gpNMB expressed on the plasma membrane of such mal-functional microglia.

The antibody of the present invention may also preferably have various activities against microglia. Specifically, the antibody of the present invention may preferably have at least one or more of:
(a) the activity to reduce or remove mal-functional microglia;
(b) the activity to promote normal microglia's functions to phagocytize mal-functional microglia or the activity to normalize the mal-function of mal-functional microglia; and
(c) the activity to enhance the effect of normal microglia in the vicinity of mal-functional microglia.

The relationship between the aforementioned activities of the antibody of the present invention, i.e., (a) the activity to reduce or remove mal-functional microglia, (b) the activity to promote normal microglia's functions to phagocytize mal-functional microglia or the activity to normalize the mal-function of mal-functional microglia, and (c) the activity to enhance the effects of normal microglia in the vicinity of mal-functional microglia, is unknown. However, although not necessarily bound by the theory, the relationship is inferred as follows. Specifically, it is assumed that binding of the anti-gpNMB antibody to mal-functional microglia may have them recognized by normal microglia with phagocytic activity, which phagocytize and remove the mal-functional microglia to create a space, in which normal microglia proliferate to promote normalization of the brain environment, thereby inducing removal of toxic proteins from neurons and restoration of the number of synapses. Alternatively, it is assumed that the anti-gpNMB antibody may promote phagocytic activation or proliferation of microglia and enhance phagocytosis of mal-functional microglia to promote normalization of the brain environment, thereby inducing removal of toxic proteins from neurons and restoration of the number of synapses.

The antibody of the present invention may also preferably have the activity to improve or restore the brain environment or neuronal functions. Specifically, the antibody of the present invention may preferably have at least one or more of:
(x) the activity to remove amyloid beta oligomers;
(y) the activity to increase the number of synapses;
(p) the activity to remove phosphorylated tau and/or to inhibit accumulation of tau;
(q) the activity to inhibit NFT (neurofibrillary tangle) formation;
(r) the activity to remove amyloid β; and
(s) the activity to remove synuclein and/or synuclein aggregates.

The antibody of the present invention may also preferably have (z) the activity to treat or prevent neurodegenerative diseases. Specifically, the antibody of the present invention may preferably have the activity to treat or prevent central neurodegenerative diseases. Specifically, the antibody of the present invention the antibody of the present invention may preferably have the activity to treat or prevent central neurodegenerative diseases explained below.

According to an embodiment of the present invention, diseases classified as neurodegenerative diseases are those caused by the accumulation of one or more proteins selected from amyloid β, tau, synuclein, polyglutamine, RAN translation-generated protein, and prion.

According to an embodiment of the present invention, diseases classified as neurodegenerative diseases can be divided into tauopathy and other neurodegenerative diseases. Specific examples of each categories are as follows.

Specific examples of tauopathy (diseases in which tau accumulates and causes neurodegeneration) include Alzheimer's disease, frontotemporal lobar degeneration (FTLD-Tau), frontotemporal dementia (FTD), primary age-related tauopathy (PART), chronic traumatic encephalopathy (CTE), Pick's disease, cerebral cortex basal ganglia degeneration (CBD or CBS), progressive supranuclea palsy (PSP), globular glial tauopathy (GGT), argyrophilic grain dementia (AGD) (argyrophilic grain disease), aging-related tau astrogliopathy (ARTAG), familial British dementia (FBD), familial Danish dementia (FDD), FTDP17, multisystem tauopathy with dementia (MSTD), dementia of the neurofibrillary tangle type, diffuse neurofibrillary tangles with calcification (DNTC), and white matter tauopathy with globular glial inclusions (WMT-GGI).

Specific examples of neurodegenerative diseases other than tauopathy include Parkinson's disease (accumulation of α-synuclein, etc.), Huntington's disease (accumulation of mutant huntingtin, etc.), spinal cord cerebellum degeneration (accumulation of polyglutamic acid protein, etc.), hereditary spinocerebellar ataxia (accumulation of polyglutamic acid protein, etc.), spinobulbar muscular atrophy (accumulation of polyglutamic acid protein, etc.), Lewy body dementia (accumulation of α-synuclein, etc.), multiple system atrophy (MSA) (accumulation of α-synuclein, etc.), Creutzfeldt-Jakob disease (CJD) (accumulation of abnormal prion protein, etc.), amyotrophic lateral sclerosis (ALS) (accumulation of SOD, TDP43, C9orf72, α-synuclein, γ-synuclein, etc.), frontotemporal lobar degeneration (accumulation of FTLD-TDP, FTLD-FUS:TDP43, FUS, etc.), and repeat diseases. In particular, with regard to amyotrophic lateral sclerosis (ALS), it has been reported that the expression of gpNMB in microglia in the spinal cord increases as the disease progresses in mouse models (Non-Patent Literature 85: Barreto et al., Glia, 2024, Vol.72, No.7, pp.1319-1339). Accordingly, the present invention is expected to have a therapeutic effect on Alzheimer's disease as well as Parkinson's disease.

In the Examples described below, the present inventors analyzed the effect of the antibodies of the present invention by administering antibodies according to an embodiment of the present invention to senile Alzheimer's disease (AD) model mice, and evaluating their brain sections by immunohistochemistry. The results showed that administration of their antibodies reduced gpNMB-positive microglia in the brains of AD model mice. More surprisingly, it was found that Aβ oligomers, the most toxic form of amyloid-β in the brains of AD model mice, were removed from neurons. Even more surprisingly, it was found that administration of the antibodies restored the number of neuronal synapses in the brains of AD model mice to normal levels. Still more surprisingly, it was found that administration of the antibodies restored cognitive function in the Morris water maze test using the present inventors' newly constructed AD model mice (APP/Tau-Tg mice). Furthermore, the effect of the antibodies of the present invention was analyzed by administering antibodies according to an embodiment of the present invention to AD model mice (APP/Tau-Tg mice) and subjecting the mice to pathological analysis. As a result, it was found that administration of these antibodies eliminated phosphorylated tau and amyloid-β in neurons. More surprisingly, NFT (neurofibrillary tangles) formation was suppressed.

In the Examples described below, the effect of the antibodies of the present invention was analyzed by administering antibodies according to an embodiment of the present invention to Parkinson's disease (PD) model mice (A53T mutation human α-synuclein-Tg mice) and evaluating the mice for their cognitive and motor functions. The Morris water maze test was carried out for evaluating cognitive function. The results showed that, surprisingly, the antibodies were shown to restore cognitive function in the Morris water maze test when administered once a week, for a total of just five times. In addition, the Rotarod test and the Inverted Screen test were carried out for evaluating motor function. The results showed that, even more surprisingly, the antibodies were effective in suppressing motor dysfunction when administered once a week, for a total of only five times.

The effect of the antibodies of the present invention was also analyzed by evaluating the brains of the Parkinson's disease (PD) model mice (A53T mutant human α-synuclein-Tg mice), which were administered with antibodies according to an embodiment of the present invention as mentioned above, by pathological analysis. The results showed that administration of these antibodies reduced gpNMB-positive microglia in the brains of PD model mice. More surprisingly, αsyn oligomers, which are considered to be the most toxic of α-synuclein (αSyn) in the PD model mouse brain, were found to be removed from the neurons. Furthermore, it was confirmed that the administration of these antibodies restored the number of synapses of neurons in the PD model mouse brain to normal levels.

As described above, in several mouse models of neurodegenerative diseases, microglia accumulating toxic proteins appeared as the disease progressed, and the expression of gpNMB was upregulated in the microglia, indicating that administration of the antibodies of the present invention inhibited the progression of, or even recovered, neurodegenerative disease pathology and symptoms. Based on the above, it is highly expected that the antibodies of the present invention, which can eliminate pathologically involved microglia, will exert therapeutic effects in various neurodegenerative diseases such as Alzheimer's disease, Parkinson's disease, and ALS by removing microglia that have accumulated toxic substances and become mal-functional.

The antibody of the present invention may exert the aforementioned various activities either in vivo or ex vivo, or both in vivo and ex vivo. Either in vivo or ex vivo, the subjects in which the aforementioned various activities are exhibited is not limited, but the antibody of the present invention may preferably exert the aforementioned various activities at least in vertebrates, more preferably at least in mammals, especially in humans or non-human animals.

### *Amino acid sequence and nucleic acid sequence of each region of each chain of the antibody:

The amino acid sequence of the antibody of the present invention is not particularly limited, as long as it has the ability to specifically bind to at least one site in a region from a PMEL-CAF-like domain to a PKD domain of gpNMB. However, the antibody of the present invention may preferably have a specific amino acid sequence as each CDR sequence. The details will be explained below. Incidentally, the "identity" of amino acid sequences herein refers to the ratio of matching amino acid residues, and the "homology" of amino acid sequences herein refers to the ratio of matching or similar amino acid residues. The homology and identity of amino acid sequences can be determined by, e.g., BLAST method (default conditions of NCBI's PBLAST). It is also clear that when the phrase "a homology of 80% or more" is used, for example, it includes "an identity of 80% or more."

The term "similar amino acid residues" herein refers to amino acid residues having side chains with similar chemical characteristics (e.g., charge or hydrophobicity). Similar amino acid residues include, for example, the following combinations.
(1) Amino acid residues with aliphatic side chains: glycine (Gly or G), alanine (Ala or A), valine (Val or V), leucine (Leu or L), and isoleucine (Ile or I) residue.
(2) Amino acid residues with aliphatic hydroxyl side chains: serine (Ser or S) and threonine (Thr or T) residue.
(3) Amino acid residues with amide-containing side chains: asparagine(Asn or N)and glutamine(Gln or Q) residue.
(4) Amino acid residues with aromatic side chains: phenylalanine (Phe or F), tyrosine (Tyr or Y), and tryptophan (Trp or W) residue.
(5) Amino acid residues with basic side chains: lysine (Lys or K), arginine(Arg or R)and histidine (His or H) residue.
(6) Amino acid residues with acidic side chains: aspartic acid (Asp or D)and glutamic acid (Glu or E) residue.
(7) Amino acid residues with sulfur-containing side chains: cysteine (Cys or C), and methionine (Met or M) residue.

In addition, the combination of (1) and methionine (Met or M) and the combination of (4) and histidine (His or H) are also treated as similar amino acid residues.

In some cases, substitutions can be made regardless of similarity if the corresponding variants exist with reference to the germline sequence. Germline-referenced variants include substitutions, deletions, or additions. Substitutions include cases where amino acid residues after substitution are presumed to affect the direction of the side chain such as proline (Pro or P) and glycine (Gly or G).

An embodiment of the antibody of the present invention relates to antibodies an antibody containing a heavy chain variable region having any of the combination of the CDR-H1 to -H3 sequences specified in (1) to (14) below. In this context, the phrase "CDR grafting region" herein refers to a region of the amino acid sequence of an antibody that has an amino acid sequence identical to, or overlapping with, the amino acid sequence of a CDR as defined herein and that, like the CDR, is in contact with the antigen and determines antigen specificity, to thereby play a role in binding to the antigen.
(1) As a CDR-H1 sequence, the amino acid sequence defined in SEQ ID NO 1, or an amino acid sequence derived from SEQ ID NO 1 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 1,
   as a CDR-H2 sequence, the amino acid sequence defined in SEQ ID NO 3, or an amino acid sequence derived from SEQ ID NO 3 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 3, and
   as a CDR-H3 sequence, the amino acid sequence defined in SEQ ID NO 5, or an amino acid sequence derived from SEQ ID NO 5 via substitution, deletion, or insertion of any one, two, three, or four amino acid residues, or an amino acid sequence having a homology (preferably an identity) of 66.6 % or more, 75.0 % or more, 83.3 % or more, or 91.6 % or more to the amino acid sequence defined in SEQ ID NO 5.
(2) As a CDR-H1 sequence, the amino acid sequence defined in SEQ ID NO 17, or an amino acid sequence derived from SEQ ID NO 17 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 17,
   as a CDR-H2 sequence, the amino acid sequence defined in SEQ ID NO 19, or an amino acid sequence derived from SEQ ID NO 19 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 19, and
   as a CDR-H3 sequence, the amino acid sequence defined in SEQ ID NO 21, or an amino acid sequence derived from SEQ ID NO 21 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 21.
(3) As a CDR-H1 sequence, the amino acid sequence defined in SEQ ID NO 33, or an amino acid sequence derived from SEQ ID NO 33 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 33,
   as a CDR-H2 sequence, the amino acid sequence defined in SEQ ID NO 35, or an amino acid sequence derived from SEQ ID NO 35 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 35, and
   as a CDR-H3 sequence, the amino acid sequence defined in SEQ ID NO 37, or an amino acid sequence derived from SEQ ID NO 37 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 37.
(4) As a CDR-H1 sequence, the amino acid sequence defined in SEQ ID NO 49, or an amino acid sequence derived from SEQ ID NO 49 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 49,
   as a CDR-H2 sequence, the amino acid sequence defined in SEQ ID NO 51, or an amino acid sequence derived from SEQ ID NO 51 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 51, and
   as a CDR-H3 sequence, the amino acid sequence defined in SEQ ID NO 53, or an amino acid sequence derived from SEQ ID NO 53 via substitution, deletion, or insertion of any one, two, three, four, five, or six amino acid residues, or an amino acid sequence having a homology (preferably an identity) of 62.5 % or more, 68.7 % or more, 75.0 % or more, 81.2 % or more, 87.5 % or more, or 93.7 % or more to the amino acid sequence defined in SEQ ID NO 53.
(5) As a CDR-H1 sequence, the amino acid sequence defined in SEQ ID NO 65, or an amino acid sequence derived from SEQ ID NO 65 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 65,
   as a CDR-H2 sequence, the amino acid sequence defined in SEQ ID NO 67, or an amino acid sequence derived from SEQ ID NO 67 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 67, and
   as a CDR-H3 sequence, the amino acid sequence defined in SEQ ID NO 69, or an amino acid sequence derived from SEQ ID NO 69 via substitution, deletion, or insertion of any one, two, three, four, or five amino acid residues, or an amino acid sequence having a homology (preferably an identity) of 64.2 % or more, 71.4 % or more, 78.5 % or more, 85.7 % or more, or 92.8 % or more to the amino acid sequence defined in SEQ ID NO 69.
(6) As a CDR-H1 sequence, the amino acid sequence defined in SEQ ID NO 81, or an amino acid sequence derived from SEQ ID NO 81 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 81,
   as a CDR-H2 sequence, the amino acid sequence defined in SEQ ID NO 83, or an amino acid sequence derived from SEQ ID NO 83 via substitution, deletion, or insertion of any one or two amino acid residues, or an amino acid sequence having a homology (preferably an identity) of 71.4 % or more or 85.7 % or more to the amino acid sequence defined in SEQ ID NO 83, and
   as a CDR-H3 sequence, the amino acid sequence defined in SEQ ID NO 85, or an amino acid sequence derived from SEQ ID NO 85 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 85.
(7) As a CDR-H1 sequence, the amino acid sequence defined in SEQ ID NO 97, or an amino acid sequence derived from SEQ ID NO 97 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 97,
   as a CDR-H2 sequence, the amino acid sequence defined in SEQ ID NO 99, or an amino acid sequence derived from SEQ ID NO 99 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 99, and
   as a CDR-H3 sequence, the amino acid sequence defined in SEQ ID NO 101, or an amino acid sequence derived from SEQ ID NO 101 via substitution, deletion, or insertion of any one or two amino acid residues, or an amino acid sequence having a homology (preferably an identity) of 60.0 % or more or 80.0 % or more to the amino acid sequence defined in SEQ ID NO 101.
(8) As a CDR-H1 sequence, the amino acid sequence defined in SEQ ID NO 113, or an amino acid sequence derived from SEQ ID NO 113 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 113,
   as a CDR-H2 sequence, the amino acid sequence defined in SEQ ID NO 115, or an amino acid sequence derived from SEQ ID NO 115 via substitution, deletion, or insertion of any one or two amino acid residues, or an amino acid sequence having a homology (preferably an identity) of 71.4 % or more or 85.7 % or more to the amino acid sequence defined in SEQ ID NO 115, and
   as a CDR-H3 sequence, the amino acid sequence defined in SEQ ID NO 117, or an amino acid sequence derived from SEQ ID NO 117 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 117.
(9) As a CDR-H1 sequence, the amino acid sequence defined in SEQ ID NO 129, or an amino acid sequence derived from SEQ ID NO 129 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 129,
   as a CDR-H2 sequence, the amino acid sequence defined in SEQ ID NO 131, or an amino acid sequence derived from SEQ ID NO 131 via substitution, deletion, or insertion of any one or two amino acid residues, or an amino acid sequence having a homology (preferably an identity) of 71.4 % or more or 85.7 % or more to the amino acid sequence defined in SEQ ID NO 131, and
   as a CDR-H3 sequence, the amino acid sequence defined in SEQ ID NO 133, or an amino acid sequence derived from SEQ ID NO 133 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 133.
(10) As a CDR-H1 sequence, the amino acid sequence defined in SEQ ID NO 145, or an amino acid sequence derived from SEQ ID NO 145 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 145,
   as a CDR-H2 sequence, the amino acid sequence defined in SEQ ID NO 147, or an amino acid sequence derived from SEQ ID NO 147 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 147, and
   as a CDR-H3 sequence, the amino acid sequence defined in SEQ ID NO 149, or an amino acid sequence derived from SEQ ID NO 149 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 149.
(11) As a CDR-H1 sequence, the amino acid sequence defined in SEQ ID NO 161, or an amino acid sequence derived from SEQ ID NO 161 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 161,
   as a CDR-H2 sequence, the amino acid sequence defined in SEQ ID NO 163, or an amino acid sequence derived from SEQ ID NO 163 via substitution, deletion, or insertion of any one or two amino acid residues, or an amino acid sequence having a homology (preferably an identity) of 71.4 % or more or 85.7 % or more to the amino acid sequence defined in SEQ ID NO 163, and
   as a CDR-H3 sequence, the amino acid sequence defined in SEQ ID NO 165, or an amino acid sequence derived from SEQ ID NO 165 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 165.
(12) As a CDR-H1 sequence, the amino acid sequence defined in SEQ ID NO 255, or an amino acid sequence derived from SEQ ID NO 255 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 255,
   as a CDR-H2 sequence, the amino acid sequence defined in SEQ ID NO 257, or an amino acid sequence derived from SEQ ID NO 257 via substitution, deletion, or insertion of any one or two amino acid residues, or an amino acid sequence having a homology (preferably an identity) of 71.4 % or more or 85.7 % or more to the amino acid sequence defined in SEQ ID NO 257, and
   as a CDR-H3 sequence, the amino acid sequence defined in SEQ ID NO 259, or an amino acid sequence derived from SEQ ID NO 259 via substitution, deletion, or insertion of any one or two amino acid residues, or an amino acid sequence having a homology (preferably an identity) of 66.6 % or more or 83.3 % or more to the amino acid sequence defined in SEQ ID NO 259.
(13) As a CDR-H1 sequence, the amino acid sequence defined in SEQ ID NO 283, or an amino acid sequence derived from SEQ ID NO 283 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 283,
   as a CDR-H2 sequence, the amino acid sequence defined in SEQ ID NO 285, or an amino acid sequence derived from SEQ ID NO 285 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 285,
   as a CDR-H3 sequence, the amino acid sequence defined in SEQ ID NO 287, or an amino acid sequence derived from SEQ ID NO 287 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 287.
(14) As a CDR-H1 sequence, the amino acid sequence defined in SEQ ID NO 311, or an amino acid sequence derived from SEQ ID NO 311 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 311,
   as a CDR-H2 sequence, the amino acid sequence defined in SEQ ID NO 313, or an amino acid sequence derived from SEQ ID NO 313 via substitution, deletion, or insertion of any one or two amino acid residues, or an amino acid sequence having a homology (preferably an identity) of 71.4 % or more or 85.7 % or more to the amino acid sequence defined in SEQ ID NO 313,
   as a CDR-H3 sequence, the amino acid sequence defined in SEQ ID NO 315, or an amino acid sequence derived from SEQ ID NO 315 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 315.
   As shown in Example 17 below, the present inventors have investigated the importance of each amino acid residue of each CDR grafting region sequence in maintaining the binding ability of humanized anti-gpNMB antibody H1L1 (clone hGPN06-1_H1L1) by performing alanine scanning. As a result, the present inventors have identified the following amino acid residues in the heavy chain variable region (SEQ ID NO 295) as being important in maintaining the binding ability of humanized anti-gpNMB antibody hGPN06-1_H1L1: tyrosine at position 27, asparagine at position 32, and tryptophan at position 33 in CDR grafting region H1 (SEQ ID NO 271); aspartic acid at position 55, phenylalanine at position 57, threonine at position 58, asparagine at position 59, tyrosine at position 60, and asparagine at position 61 in CDR grafting region H2 (SEQ ID NO 273); and arginine at position 98, glycine at position 100, and glycine at position 109 in CDR grafting region H3 (SEQ ID NO 275). On the other hand, amino acid residues of each CDR grafting region sequence in the heavy chain variable region other than the above were identified as having little effect on the maintenance of its binding ability. Based on these findings, an embodiment of the antibody of the present invention also relates to an antibody having a heavy chain variable region containing the CDR grafting region-H1 to - H3 sequences in (15) below. (The amino acid positions below are based on the amino acid sequence of the heavy chain variable region of humanized anti-gpNMB antibody hGPN06-1_H1L1 (SEQ ID NO 295).)
(15) As a CDR grafting region H1 sequence, the amino acid sequence defined in SEQ ID NO 271, or an amino acid sequence derived from SEQ ID NO 271 via substitution, deletion, or insertion of any one, two, three, or four amino acid residues other than tyrosine at position 27, asparagine at position 32, and tryptophan at position 33,
   as a CDR grafting region H2 sequence, the amino acid sequence defined in SEQ ID NO 273, or an amino acid sequence derived from SEQ ID NO 273 via substitution, deletion, or insertion of any one, two, three, four, five, or six amino acid residues other than aspartic acid at position 55, phenylalanine at position 57, threonine at position 58, asparagine at position 59, tyrosine at position 60, and asparagine at position 61,
   as a CDR grafting region H3 sequence, the amino acid sequence defined in SEQ ID NO 275, or an amino acid sequence derived from SEQ ID NO 275 via substitution, deletion, or insertion of any one, two, three, four, five, or six amino acid residues other than arginine at position 98, glycine at position 100, and glycine at position 109.
   As shown in Example 17 below, the present inventors have investigated the importance of each amino acid residue of each CDR grafting region sequence in maintaining the binding ability of humanized anti-gpNMB antibody H2L3 (clone hGPN18-2_H2L3) by performing alanine scanning. As a result, the present inventors have identified the following amino acid residues in the heavy chain variable region (SEQ ID NO 323) as being important in maintaining the binding ability of humanized anti-gpNMB antibody H2L3: aspartic acid at position 52, tryptophan at position 54, aspartic acid at position 58, and proline at position 63 in CDR grafting region H2 (SEQ ID NO 301); and arginine at position 99, threonine at position 100, tyrosine at position 102, tyrosine at position 105, tyrosine at position 107, and methionine at position 110 in CDR grafting region H3 (SEQ ID NO 303). On the other hand, amino acid residues of each CDR grafting region sequence in the heavy chain variable region other than the above were identified as having little effect on the maintenance of its binding ability. Based on these findings, an embodiment of the antibody of the present invention also relates to an antibody having a heavy chain variable region containing the CDR grafting region-H1 to -H3 sequences in (16) below. (The amino acid positions below are based on the amino acid sequence of the heavy chain variable region of humanized anti-gpNMB antibody H2L3 (SEQ ID NO 323).)
(16) As a CDR grafting region H1 sequence, the amino acid sequence defined in SEQ ID NO 299, or an amino acid sequence derived from SEQ ID NO 299 via substitution, deletion, or insertion of any one, two, three, or four amino acid residues,
   as a CDR grafting region H2 sequence, the amino acid sequence defined in SEQ ID NO 301, or an amino acid sequence derived from SEQ ID NO 301 via substitution, deletion, or insertion of any one, two, three, four, five, or six amino acid residues other than aspartic acid at position 52, tryptophan at position 54, aspartic acid at position 58, and proline at position 63,
   as a CDR grafting region H3 sequence, the amino acid sequence defined in SEQ ID NO 303, or an amino acid sequence derived from SEQ ID NO 303 via substitution, deletion, or insertion of any one, two, three, four, five, or six amino acid residues other than arginine at position 99, threonine at position 100, tyrosine at position 102, tyrosine at position 105, tyrosine at position 107, and methionine at position 110.

An embodiment of the antibody of the present invention relates to antibodies an antibody containing a heavy chain variable region having any of the combination of the CDR-L1 to - L 3 sequences specified in (1) to (14) below.
(1) As a CDR-L1 sequence, the amino acid sequence defined in SEQ ID NO 7, or an amino acid sequence derived from SEQ ID NO 7 via substitution, deletion, or insertion of any one or two amino acid residues, or an amino acid sequence having a homology (preferably an identity) of 66.6 % or more or 83.3 % or more to the amino acid sequence defined in SEQ ID NO 7,
   as a CDR-L2 sequence, the amino acid sequence defined in SEQ ID NO 9, or an amino acid sequence derived from SEQ ID NO 9 via substitution, deletion, or insertion of any one amino acid residue, or an amino acid sequence having a homology (preferably an identity) of 66.6 % or more to the amino acid sequence defined in SEQ ID NO 9, and
   as a CDR-L3 sequence, the amino acid sequence defined in SEQ ID NO 11, or an amino acid sequence derived from SEQ ID NO 11 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 11.
(2) As a CDR-L1 sequence, the amino acid sequence defined in SEQ ID NO 23, or an amino acid sequence derived from SEQ ID NO 23 via substitution, deletion, or insertion of any one or two amino acid residues, or an amino acid sequence having a homology (preferably an identity) of 60.0 % or more or 80.0 % or more to the amino acid sequence defined in SEQ ID NO 23,
   as a CDR-L2 sequence, the amino acid sequence defined in SEQ ID NO 25, or an amino acid sequence derived from SEQ ID NO 25 via substitution, deletion, or insertion of any one amino acid residue, or an amino acid sequence having a homology (preferably an identity) of 66.6 % or more to the amino acid sequence defined in SEQ ID NO 25, and
   as a CDR-L3 sequence, the amino acid sequence defined in SEQ ID NO 27, or an amino acid sequence derived from SEQ ID NO 27 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 27 .
(3) As a CDR-L1 sequence, the amino acid sequence defined in SEQ ID NO 39, or an amino acid sequence derived from SEQ ID NO 39 via substitution, deletion, or insertion of any one or two amino acid residues, or an amino acid sequence having a homology (preferably an identity) of 60.0 % or more or 80.0 % or more to the amino acid sequence defined in SEQ ID NO 39,
   as a CDR-L2 sequence, the amino acid sequence defined in SEQ ID NO 41, or an amino acid sequence derived from SEQ ID NO 41 via substitution, deletion, or insertion of any one amino acid residue, or an amino acid sequence having a homology (preferably an identity) of 66.6 % or more to the amino acid sequence defined in SEQ ID NO 41, and
   as a CDR-L3 sequence, the amino acid sequence defined in SEQ ID NO 43, or an amino acid sequence derived from SEQ ID NO 43 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 43.
(4) As a CDR-L1 sequence, the amino acid sequence defined in SEQ ID NO 55, or an amino acid sequence derived from SEQ ID NO 55 via substitution, deletion, or insertion of any one or two amino acid residues, or an amino acid sequence having a homology (preferably an identity) of 66.6 % or more or 83.3 % or more to the amino acid sequence defined in SEQ ID NO 55,
   as a CDR-L2 sequence, the amino acid sequence defined in SEQ ID NO 57, or an amino acid sequence derived from SEQ ID NO 57 via substitution, deletion, or insertion of any one amino acid residue, or an amino acid sequence having a homology (preferably an identity) of 66.6 % or more to the amino acid sequence defined in SEQ ID NO 57, and
   as a CDR-L3 sequence, the amino acid sequence defined in SEQ ID NO 59, or an amino acid sequence derived from SEQ ID NO 59 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 59.
(5) As a CDR-L1 sequence, the amino acid sequence defined in SEQ ID NO 71, or an amino acid sequence derived from SEQ ID NO 71 via substitution, deletion, or insertion of any one or two amino acid residues, or an amino acid sequence having a homology (preferably an identity) of 60.0 % or more or 80.0 % or more to the amino acid sequence defined in SEQ ID NO 71,
   as a CDR-L2 sequence, the amino acid sequence defined in SEQ ID NO 73, or an amino acid sequence derived from SEQ ID NO 73 via substitution, deletion, or insertion of any one amino acid residue, or an amino acid sequence having a homology (preferably an identity) of 66.6 % or more to the amino acid sequence defined in SEQ ID NO 73, and
   as a CDR-L3 sequence, the amino acid sequence defined in SEQ ID NO 75, or an amino acid sequence derived from SEQ ID NO 75 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 75 .
(6) As a CDR-L1 sequence, the amino acid sequence defined in SEQ ID NO 87, or an amino acid sequence derived from SEQ ID NO 87 via substitution, deletion, or insertion of any one or two amino acid residues, or an amino acid sequence having a homology (preferably an identity) of 66.6 % or more or 83.3 % or more to the amino acid sequence defined in SEQ ID NO 87,
   as a CDR-L2 sequence, the amino acid sequence defined in SEQ ID NO 89, or an amino acid sequence derived from SEQ ID NO 89 via substitution, deletion, or insertion of any one amino acid residue, or an amino acid sequence having a homology (preferably an identity) of 66.6 % or more to the amino acid sequence defined in SEQ ID NO 89, and
   as a CDR-L3 sequence, the amino acid sequence defined in SEQ ID NO 91, or an amino acid sequence derived from SEQ ID NO 91 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 91.
(7) As a CDR-L1 sequence, the amino acid sequence defined in SEQ ID NO 103, or an amino acid sequence derived from SEQ ID NO 103 via substitution, deletion, or insertion of any one or two amino acid residues, or an amino acid sequence having a homology (preferably an identity) of 66.6 % or more or 83.3 % or more to the amino acid sequence defined in SEQ ID NO 103,
   as a CDR-L2 sequence, the amino acid sequence defined in SEQ ID NO 105, or an amino acid sequence derived from SEQ ID NO 105 via substitution, deletion, or insertion of any one amino acid residue, or an amino acid sequence having a homology (preferably an identity) of 66.6 % or more to the amino acid sequence defined in SEQ ID NO 105, and
   as a CDR-L3 sequence, the amino acid sequence defined in SEQ ID NO 107, or an amino acid sequence derived from SEQ ID NO 107 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 107.
(8) As a CDR-L1 sequence, the amino acid sequence defined in SEQ ID NO 119, or an amino acid sequence derived from SEQ ID NO 119 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 119,
   as a CDR-L2 sequence, the amino acid sequence defined in SEQ ID NO 121, or an amino acid sequence derived from SEQ ID NO 121 via substitution, deletion, or insertion of any one amino acid residue, or an amino acid sequence having a homology (preferably an identity) of 66.6 % or more to the amino acid sequence defined in SEQ ID NO 121, and
   as a CDR-L3 sequence, the amino acid sequence defined in SEQ ID NO 123, or an amino acid sequence derived from SEQ ID NO 123 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 123.
(9) As a CDR-L1 sequence, the amino acid sequence defined in SEQ ID NO 135, or an amino acid sequence derived from SEQ ID NO 135 via substitution, deletion, or insertion of any one, two, three, or four amino acid residues, or to the amino acid sequence defined in SEQ ID NO 135 an amino acid sequence having a homology (preferably an identity) of 63.6 % or more, 72.7 % or more, 81.8 % or more, or 90.9 % or more,
   as a CDR-L2 sequence, the amino acid sequence defined in SEQ ID NO 137, or an amino acid sequence derived from SEQ ID NO 137 via substitution, deletion, or insertion of any one amino acid residue, or to the amino acid sequence defined in SEQ ID NO 137 an amino acid sequence having a homology (preferably an identity) of 66.6 % or more, and
   as a CDR-L3 sequence, the amino acid sequence defined in SEQ ID NO 139, or an amino acid sequence derived from SEQ ID NO 139 via substitution, deletion, or insertion of any one, two, or three amino acid residues, or to the amino acid sequence defined in SEQ ID NO 139 an amino acid sequence having a homology (preferably an identity) of 66.6 % or more, 77.7 % or more, or 88.8 % or more .
(10) As a CDR-L1 sequence, the amino acid sequence defined in SEQ ID NO 151, or an amino acid sequence derived from SEQ ID NO 151 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 151,
   as a CDR-L2 sequence, the amino acid sequence defined in SEQ ID NO 153, or an amino acid sequence derived from SEQ ID NO 153 via substitution, deletion, or insertion of any one amino acid residue, or an amino acid sequence having a homology (preferably an identity) of 66.6 % or more to the amino acid sequence defined in SEQ ID NO 153, and
   as a CDR-L3 sequence, the amino acid sequence defined in SEQ ID NO 155, or an amino acid sequence derived from SEQ ID NO 155 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 155.
(11) As a CDR-L1 sequence, the amino acid sequence defined in SEQ ID NO 167, or an amino acid sequence derived from SEQ ID NO 167 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 167,
   as a CDR-L2 sequence, the amino acid sequence defined in SEQ ID NO 169, or an amino acid sequence derived from SEQ ID NO 169 via substitution, deletion, or insertion of any one amino acid residue, or an amino acid sequence having a homology (preferably an identity) of 66.6 % or more to the amino acid sequence defined in SEQ ID NO 169, and
   as a CDR-L3 sequence, the amino acid sequence defined in SEQ ID NO 171, or an amino acid sequence derived from SEQ ID NO 171 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 171.
(12) As a CDR-L1 sequence, the amino acid sequence defined in SEQ ID NO 261, or an amino acid sequence derived from SEQ ID NO 261 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 261,
   as a CDR-L2 sequence, the amino acid sequence defined in SEQ ID NO 263, or an amino acid sequence derived from SEQ ID NO 263 via substitution, deletion, or insertion of any one amino acid residue, or an amino acid sequence having a homology (preferably an identity) of 66.6 % or more to the amino acid sequence defined in SEQ ID NO 263, and
   as a CDR-L3 sequence, the amino acid sequence defined in SEQ ID NO 265, or an amino acid sequence derived from SEQ ID NO 265 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 265.
(13) As a CDR-L1 sequence, the amino acid sequence defined in SEQ ID NO 289, or an amino acid sequence derived from SEQ ID NO 289 via substitution, deletion, or insertion of any one or two amino acid residues, or an amino acid sequence having a homology (preferably an identity) of 60.0 % or more or 80.0 % or more to the amino acid sequence defined in SEQ ID NO 289,
   as a CDR-L2 sequence, the amino acid sequence defined in SEQ ID NO 291, or an amino acid sequence derived from SEQ ID NO 291 via substitution, deletion, or insertion of any one amino acid residue, or an amino acid sequence having a homology (preferably an identity) of 66.6 % or more to the amino acid sequence defined in SEQ ID NO 291, and
   as a CDR-L3 sequence, the amino acid sequence defined in SEQ ID NO 293, or an amino acid sequence derived from SEQ ID NO 293 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 293.
(14) As a CDR-L1 sequence, the amino acid sequence defined in SEQ ID NO 317, or an amino acid sequence derived from SEQ ID NO 317 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 317,
   as a CDR-L2 sequence, the amino acid sequence defined in SEQ ID NO 319, or an amino acid sequence derived from SEQ ID NO 319 via substitution, deletion, or insertion of any one amino acid residue, or an amino acid sequence having a homology (preferably an identity) of 66.6 % or more to the amino acid sequence defined in SEQ ID NO 319, and
   as a CDR-L3 sequence, the amino acid sequence defined in SEQ ID NO 321, or an amino acid sequence derived from SEQ ID NO 321 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 321 .
   As shown in Example 17 below, the present inventors have investigated the importance of each amino acid residue of each CDR grafting region sequence in maintaining the binding ability of humanized anti-gpNMB antibody H1L1 (clone hGPN06-1_H1L1) by performing alanine scanning. As a result, the present inventors have identified the following amino acid residues in the light chain variable region (SEQ ID NO 297) as being important in maintaining the binding ability of humanized anti-gpNMB antibody H1L1: isoleucine at position 29, tyrosine at position 31 and histidine at position 33 in CDR grafting region L1 (SEQ ID NO 277); threonine at position 50 in CDR grafting region L2 (SEQ ID NO 279); and histidine at position 88, glutamine at position 89, tryptophan at position 90, serine at position 92, tyrosine at position 93, proline at position 94, and cysteine at position 95 in CDR grafting region L3 (SEQ ID NO 281). On the other hand, amino acid residues of each CDR grafting region sequence in the light chain variable region other than the above were identified as having little effect on the maintenance of its binding ability. Based on these findings, an embodiment of the antibody of the present invention also relates to an antibody having a light chain variable region containing the CDR grafting region-L1 to -L3 sequences in (15) below. (The amino acid positions below are based on the amino acid sequence of the light chain variable region of humanized anti-gpNMB antibody H1L1 (SEQ ID NO 297).)
(15) As a CDR grafting region L1 sequence, the amino acid sequence defined in SEQ ID NO 277, or an amino acid sequence derived from SEQ ID NO 277 via substitution, deletion, or insertion of any one, two, three, or four amino acid residues other than isoleucine at position 29, tyrosine at position 31, and histidine at position 33,
   as a CDR grafting region L2 sequence, the amino acid sequence defined in SEQ ID NO 279, or an amino acid sequence derived from SEQ ID NO 279 via substitution, deletion, or insertion of any one or two amino acid residue other than threonine at position 50,
   as a CDR grafting region L3 sequence, the amino acid sequence defined in SEQ ID NO 281, or an amino acid sequence defined in SEQ ID NO 281 via substitution, deletion, or insertion of any one, two, or three amino acid residues other than histidine at position 88, glutamine at position 89, tryptophan at position 90, serine at position 92, tyrosine at position 93, proline at position 94, and cysteine at position 95.
   As shown in Example 21 below, the present inventors have investigated the importance of each amino acid residue of each CDR grafting region sequence in maintaining the binding ability of humanized anti-gpNMB antibody H2L3 (clone hGPN18-2_H2L3) by performing alanine scanning. As a result, the present inventors have identified the following amino acid residues in the light chain variable region (SEQ ID NO 325) as being important in maintaining the binding ability of humanized anti-gpNMB antibody H2L3: leucine at position 31 and tyrosine at position 37 in CDR grafting region L1 (SEQ ID NO 305); lysine at position 55 in CDR grafting region L2 (SEQ ID NO 307); and phenylalanine at position 94 in CDR grafting region L3 (SEQ ID NO 309). On the other hand, amino acid residues of each CDR grafting region sequence in the light chain variable region other than the above were identified as having little effect on the maintenance of its binding ability. Based on these findings, an embodiment of the antibody of the present invention also relates to an antibody having a light chain variable region containing the CDR grafting region-L1 to -L3 sequences in (16) below. (The amino acid positions below are based on the amino acid sequence of the light chain variable region of humanized anti-gpNMB antibody H2L3 (SEQ ID NO 325).)
(16) As a CDR grafting region L1 sequence, the amino acid sequence defined in SEQ ID NO 305, or an amino acid sequence derived from SEQ ID NO 305 via substitution, deletion, or insertion of any one, two, three, four, five, or six amino acid residues other than leucine at position 31, tyrosine at position 37 and glutamic acid at position 39,
   as a CDR grafting region L2 sequence, the amino acid sequence defined in SEQ ID NO 307, or an amino acid sequence derived from SEQ ID NO 307 via substitution, deletion, or insertion of any one or two amino acid residue other than lysine at position 55,
   as a CDR grafting region L3 sequence, the amino acid sequence defined in SEQ ID NO 309, or an amino acid sequence derived from SEQ ID NO 309 via substitution, deletion, or insertion of any one, two, or three amino acid residues other than phenylalanine at position 94.

An embodiment of the antibody of the present invention relates to an antibody having a heavy chain variable region having any of the combinations of the CDR-H1 to H3 sequences described above and a light chain variable region having any of the combinations of the CDR-L1 to L3 sequences described above.

An embodiment of the antibody of the present invention relates to an antibody having any of the following amino acid sequences (a) to (c) as the sequence of the heavy chain variable region.
(a) An amino acid sequence selected from SEQ ID NO 13, SEQ ID NO 29, SEQ ID NO 45, SEQ ID NO 61, SEQ ID NO 77, SEQ ID NO 93, SEQ ID NO 109, SEQ ID NO 125, SEQ ID NO 141, SEQ ID NO 157, SEQ ID NO 173, and SEQ ID NO 267.
(b) An amino acid sequence having a homology (preferably identity) of at least 60 % or higher, or 65 % or higher, or 70 % or higher, or 75 % or higher, or 80 % or higher, or 85 % or higher, or 90 % or higher, or 95 % or higher, or 96 % or higher, or 97 % or higher, or 98 % or higher, or 99 % or higher to any of the amino acid sequences specified in (a) above.
(c) An amino acid sequence derived from any of the amino acid sequences specified in (a) above via substitution, deletion, or addition of 1 to 12 (preferably 1 to 11, more preferably 1 to 10, 1 to 9, 1 to 8, 1 to 7, 1 to 6, 1 to 5, 1 to 4, 1 to 3, 1 to 2, or 1) amino acid residues.

An embodiment of the antibody of the present invention relates to an antibody having any of the following amino acid sequences (a) to (c) as the sequence of the heavy chain variable region.
(a) An amino acid sequence selected from SEQ ID NO 295 and SEQ ID NO 323.
(b) An amino acid sequence having a homology (preferably identity) of at least 60 % or higher, or 65 % or higher, or 70 % or higher, or 75 % or higher, or 80 % or higher, or 85 % or higher, or 90 % or higher, or 95 % or higher, or 96 % or higher, or 97 % or higher, or 98 % or higher, or 99 % or higher to any of the amino acid sequences specified in (a) above.
(c) An amino acid sequence derived from any of the amino acid sequences specified in (a) above via substitution, deletion, or addition of 1 to 12 (preferably 1 to 11, more preferably 1 to 10, 1 to 9, 1 to 8, 1 to 7, 1 to 6, 1 to 5, 1 to 4, 1 to 3, 1 to 2, or 1) amino acid residues.

An embodiment of the antibody of the present invention relates to an antibody having any of the following amino acid sequences (a) to (c) as the sequence of the light chain variable region.
(a) An amino acid sequence selected from SEQ ID NO 15, SEQ ID NO 31, SEQ ID NO 47, SEQ ID NO 63, SEQ ID NO 79, SEQ ID NO 95, SEQ ID NO 111, SEQ ID NO 127, SEQ ID NO 143, SEQ ID NO 159, SEQ ID NO 175, and SEQ ID NO 269.
(b) An amino acid sequence having a homology (preferably identity) of at least 60 % or higher, or 65 % or higher, or 70 % or higher, or 75 % or higher, or 80 % or higher, or 85 % or higher, or 90 % or higher, or 95 % or higher, or 96 % or higher, or 97 % or higher, or 98 % or higher, or 99 % or higher to any of the amino acid sequences specified in (a) above.
(c) An amino acid sequence derived from any of the amino acid sequences specified in (a) above via substitution, deletion, or addition of 1 to 10 (preferably 1 to 9, more preferably 1 to 8, 1 to 7, 1 to 6, 1 to 5, 1 to 4, 1 to 3, 1 to 2, or 1) amino acid residues.

An embodiment of the antibody of the present invention relates to an antibody having any of the following amino acid sequences (a) to (c) as the sequence of the light chain variable region.
(a) An amino acid sequence selected from SEQ ID NO 297 and SEQ ID NO 325.
(b) An amino acid sequence having a homology (preferably identity) of at least 60 % or higher, or 65 % or higher, or 70 % or higher, or 75 % or higher, or 80 % or higher, or 85 % or higher, or 90 % or higher, or 95 % or higher, or 96 % or higher, or 97 % or higher, or 98 % or higher, or 99 % or higher to any of the amino acid sequences specified in (a) above.
(c) An amino acid sequence derived from any of the amino acid sequences specified in (a) above via substitution, deletion, or addition of 1 to 10 (preferably 1 to 9, more preferably 1 to 8, 1 to 7, 1 to 6, 1 to 5, 1 to 4, 1 to 3, 1 to 2, or 1) amino acid residues.

An embodiment of the antibody of the present invention relates to an antibody having any of the heavy chain variable region sequences described above and any of the light chain variable region sequences described above.

The CDR-H1, CDR-H2, CDR-H3, CDR-L1, CDR-L2, and CDR-L3 sequences in the antibody can be identified by the Kabat method (NIH Publication, (1991) No. 91-3242) or the Chothia method (J. Mol. Biol., (1997), 273). These methods are within the technical common knowledge for those skilled in the art, and are also outlined in, e.g., the Dr. Andrew C.R. Martin's Group Internet home page (http://www.bioinf.org.uk/abs/). The CDRs described herein are indicated as Kabat's CDRs.

Framework sequences of each of the heavy chain variable region and the light chain variable region of the immunoglobulin as the antibody of the present invention may preferably be framework sequences in each class of vertebrate immunoglobulin and, more preferably, framework sequences in each class of immunoglobulin of human or a non-human animal such as mouse or rat.

A person skilled in the art would be able to design the anti-gpNMB-specific antibody of the present invention by combining the amino acid sequence of each of the CDRs and/or each of the heavy and light chain variable regions described above with the amino acid sequence of each of the framework regions and/or each of the heavy and light chain constant regions of an antibody derived from human or non-human animals including mouse or rat, as appropriate. In this regard, a humanized anti-gpNMB-specific antibody can be designed by using the amino acid sequence of each of the framework regions and/or each of the heavy and light chain constant regions of a human antibody. The amino acid sequence of each of the framework regions and/or each of the heavy and light chain constant regions of such a humanized antibody can be selected from, e.g., each class of human IgG, IgA, IgM, IgE, and IgD, and their variants.

The antibody of the present invention may preferably be an antibody of the IgG class or its variant, more preferably of the human IgG class or its variant, of the human IgG4 subclass or its variant, or the human IgG1 subclass or its variant. In one example, the stabilized IgG4 constant region contains a proline at position 241 in the hinge region, according to Kabat's system. This position corresponds to position 228 of the hinge region according to the EU numbering scheme (Proc. Natl. Acad. Sci. USA, (1969), 63[1]:78-85), Sequences of proteins of immunological interest (Washington DC United States Department of Health and Human Services, 2001and NIH Publication, (1991), No. 91-3242). This residue is generally serine in human IgG4, but its stabilization can be induced by substituting the serine with proline. In one example, the N297A mutation can be incorporated into the constant region of IgG1 to suppress its ability to bind to the Fc receptor and/or anchor complement as much as possible.

### *Competitive binding:

An embodiment of the present invention relates to an antibody that binds to at least one site in a region from a PMEL-CAF-like domain to a PKD domain of gpNMB competitively with the binding of the antibody of the present invention to the same site. Such a competitively binding antibody is also included in the scope of the present invention. The term "competitive binding" herein refers to phenomenon where when at least two monoclonal antibodies coexist with an antigen, the binding of one antibody to the antigen is inhibited by the binding of the other antibody to the antigen. Competitive binding ability between two monoclonal antibodies can generally be measured by using a fixed amount (concentration) of a first monoclonal antibody, adding varying amounts (concentrations) of a second monoclonal antibody thereto, and measuring the amount (concentration) of the second monoclonal antibody added that decreases the binding of the fixed amount of the first monoclonal antibody to the antigen. The degree of inhibition can be expressed in terms of IC50 or Ki. A monoclonal antibody that competitively binds to the antibody of the present invention may refer to, e.g., an antibody whose IC50 measured in terms of competitive antigen-antibody binding to at least one site in a region from a PMEL-CAF-like domain to a PKD domain of gpNMB using 10 nM of the antibody of the invention is usually 1000nM or lower, particularly 100nM or lower, more particularly 10nM or lower. Competitive binding ability can be measured by, e.g., labelling the antibody with an enzyme, fluorescent substance, luminescent substance, radioisotope, etc., and carrying out detection using a measurement method suitable for the physical and/or chemical properties of the labeled substance, or by using a biosensor based on, e.g., surface plasmon resonance (SPR) and bio-layer interferometry (BLI).

### [Production method of the anti-gpNMB antibody]

The antibody according to the present invention can be obtained using techniques known to those skilled in the art. The antibody according to the present invention may be either a polyclonal antibody or a monoclonal antibody (Nature, (1983), 305(5934): 537-40). A polyclonal antibody according to the present invention can be produced, e.g., by preparing a human gpNMB protein having the amino acid sequence defined in SEQ ID NO 209 or a gpNMB partial peptide having a part of the amino acid sequence (e.g., a peptide containing a PKD domain with the amino acid residues 256 to 319) as an antigen, or preparing an antigen-expressing polynucleotide which encodes the amino acid sequence of such a human gpNMB protein or gpNMB partial peptide, administering the antigen or antigen-expressing polynucleotide to a mammal intramuscularly or subcutaneously to express the antigen in the animal body and thereby sensitize the animal, and recovering the antibody produced in the animal body from, e.g., serum of the animal. When a peptide is used as an antigen, it may be bound to a carrier protein such as BSA or KLH or a polylysine.

A monoclonal humanized antibody according to the present invention can be produced, e.g., by administering the human gpNMB protein or the gpNMB partial peptide or the antigen-expressing polynucleotide encoding the human gpNMB protein or gpNMB partial peptide mentioned above to a mammal to thereby sensitize the animal, collecting immune cells from the mammal and fusing them with myeloma cells to produce hybridomas, cloning each hybridoma, and recovering the antibody produced by a culture of the cloned hybridoma. Such methods for obtaining monoclonal antibodies (Nature, (1992), 356[6365]:152-4) and cell fusion techniques (Nature, (1975), 256[5517]:495-7) have already been reported and generalized, and the administration of immunostimulants can further enhance the acquisition efficiency (Cancer Gene Ther., (2007), 14[11]:904-17). Monoclonal antibodies obtained by this method include, although not limited to, the following.

*An antibody containing a heavy chain variable region the amino acid sequence defined in SEQ ID NO 13 and a light chain variable region the amino acid sequence defined in SEQ ID NO 15 (GPN05-1).
* An antibody containing a heavy chain variable region the amino acid sequence defined in SEQ ID NO 29 and a light chain variable region the amino acid sequence defined in SEQ ID NO 31 (GPN06-1).
* An antibody containing a heavy chain variable region the amino acid sequence defined in SEQ ID NO 45 and a light chain variable region the amino acid sequence defined in SEQ ID NO 47 (GPN07-1).
* An antibody containing a heavy chain variable region the amino acid sequence defined in SEQ ID NO 61 and a light chain variable region the amino acid sequence defined in SEQ ID NO 63 (GPN11-10).
* An antibody containing a heavy chain variable region the amino acid sequence defined in SEQ ID NO 77 and a light chain variable region the amino acid sequence defined in SEQ ID NO 79 (GPN15-2).
* An antibody containing a heavy chain variable region the amino acid sequence defined in SEQ ID NO 93 and a light chain variable region the amino acid sequence defined in SEQ ID NO 95 (GPN15-3).
* An antibody containing a heavy chain variable region the amino acid sequence defined in SEQ ID NO 109 and a light chain variable region the amino acid sequence defined in SEQ ID NO 111 (GPN18-4).
* An antibody containing a heavy chain variable region the amino acid sequence defined in SEQ ID NO 125 and a light chain variable region the amino acid sequence defined in SEQ ID NO 127 (GPN18-1).
* An antibody containing a heavy chain variable region the amino acid sequence defined in SEQ ID NO 141 and a light chain variable region the amino acid sequence defined in SEQ ID NO 143 (GPN18-2).
* An antibody containing a heavy chain variable region the amino acid sequence defined in SEQ ID NO 157 and a light chain variable region the amino acid sequence defined in SEQ ID NO 159 (GPN18-6).
* An antibody containing a heavy chain variable region the amino acid sequence defined in SEQ ID NO 173 and a light chain variable region the amino acid sequence defined in SEQ ID NO 175 (GPN18-7).
* An antibody containing a heavy chain variable region the amino acid sequence defined in SEQ ID NO 267 and a light chain variable region the amino acid sequence defined in SEQ ID NO 269 (GPN09-1).

Another production method includes producing a gene encoding the heavy chain and/or the light chain of the antibody, or more specifically, a nucleic acid molecule encoding the heavy chain and/or the light chain of immunoglobulin of the antibody, from a hybridoma which produces the antibody to be obtained, or from a phage clone obtained by human antibody phage display. Here, the nucleic acid molecule may be introduced into various vectors or plasmids to create a vector or plasmid containing the nucleic acid molecule. Host cells are then transformed with the nucleic acid molecule, vector, or plasmid described above. The host cells may be selected from eukaryotic cells, such as mammalian, insect, yeast, or plant cells, or from bacterial cells. The transformed host cells are then cultured under appropriate conditions for producing the anti-gpNMB-specific antibody of the present invention. If necessary, the anti-gpNMB-specific antibody of the present invention produced may be isolated from the host cells. The various methods used in these procedures are all well known to those skilled in the art.

A person skilled in the art can also produce antibody chimeric proteins, small molecule antibodies, scaffolded antibodies, etc., using known techniques, by genetic modification of genes encoding heavy and/or light chains of immunoglobulins to introduce desired traits or by using structural information of variable regions or CDR regions of heavy and/or light chains of immunoglobulins. A person skilled in the art can also make any modifications to the structure of the constant regions or to the glycosylation sites of the antibody using techniques well known to those skilled in the art in order to improve the performance of the antibody or to avoid any side effects.

### [Humanized antibody]

According to an embodiment, the anti-gpNMB antibody of the present invention is a humanized antibody. The humanized the anti-gpNMB antibody of the present invention may also be referred to as "the anti-gpNMB humanized antibody of the present invention" or simply as "the humanized antibody of the present invention." It should also be noted that "the anti-gpNMB antibody of the present invention" encompasses "the anti-gpNMB humanized antibody of the present invention."

Humanization of the non-humanized anti-gpNMB antibody of the present invention, i.e., production of the anti-gpNMB humanized antibody of the present invention, can be carried out by, e.g., the following procedure.

Specifically, for the amino acid sequences of the non-humanized (e.g., mouse) anti-gpNMB antibody, CDR regions are identified using IMGT (Lefranc, M.-P., Pommie, C., Ruiz, M., Giudicelli, V., Foulquier, E., Truong, L., Thouvenin-Contet, V. and Lefranc, G. "IMGT unique numbering for immunoglobulin and T cell receptor variable domains and Ig superfamily V-like domains". Dev. Comp. Immunol., 27, 55-77 (2003) PMID: 12477501 LIGM:268) and Kabat numbering. The combined IMGT/Kabat method is used to ensure that the CDR loop structure is optimized. Next, the human antibody frames are selected to maintain the CDR loop structure by the CDR grafting method to obtain antibody variants. A humanized antibody can be obtained by selecting an antibody variant that has the desired binding affinity (e.g., binding affinity for at least one region from the PMEL-CAF-like region to the PKD region of gpNMB) from among the antibody variants obtained.

Specifically, a human germline gene is selected that most closely approximates the amino acid sequences of the heavy and light chain variable regions of non-humanized (e.g., mouse) anti-gpNMB antibodies obtained by the various methods described above. On the other hand, BLAST search is used to select about 200 human IgG sequences that are homologous to the heavy and light chain variable regions as candidates. Then, four framework sequences are selected, for example, in terms of framework homology, retention of key amino acids in the framework, and the loop structure. CDR grafting is performed on these sequences to obtain four humanized variants. In addition, CDR grafting is performed on the human germline sequence as a framework to obtain one humanized variant. The resulting five humanized variants of heavy and light chains are combined to express 25 humanized antibody variants, which are purified and evaluated for their binding activity. Variants with a given (e.g., three-fold or one-third) binding affinity are selected via activity comparison using SPR with human chimeric antibodies with mouse heavy and light chains of the parent antibody, whereby humanized antibodies can be obtained.

As another example, anti-gpNMB antibodies that recognize at least one site within a region from the PMEL-CAF-like region to the PKD region, which is also recognized by the anti-gpNMB antibodies disclosed herein, can be obtained directly from human antibody-producing mice or human antibody phagemid libraries.

Examples of humanized anti-gpNMB monoclonal antibodies obtained by such methods include, although are not limited to:
* An antibody containing a heavy chain variable region the amino acid sequence defined in SEQ ID NO 295 and a light chain variable region the amino acid sequence defined in SEQ ID NO 297 (hGPN06-1_H1L1).
* An antibody containing a heavy chain variable region the amino acid sequence defined in SEQ ID NO 323 and a light chain variable region the amino acid sequence defined in SEQ ID NO 325 (hGPN18-2_H2L3).

### [Pharmaceutical composition]

The anti-gpNMB-specific antibody of the present invention may preferably be used as an active ingredient of a pharmaceutical composition. Accordingly, an embodiment of the present invention relates to a pharmaceutical composition containing the anti-gpNMB-specific antibody of the present invention as an active ingredient (hereinafter also collectively referred to as "the first pharmaceutical composition of the present invention").

As mentioned above, the anti-gpNMB-specific antibody of the present invention may be expected to express one or more of the activities specified in (a) to (c), (w) to (z), and (p) to (s) below when administered to a subject such as human. Therefore, the first pharmaceutical composition of the present invention may preferably be a pharmaceutical composition intended for one or more of the applications specified in (a) to (c) , (w) to (z), and (p) to (s) below.
(a) To reduce or remove mal-functional microglia.
(b) To promote normal microglia's functions to phagocytize mal-functional microglia or the activity to normalize the mal-function of mal-functional microglia.
(c) To enhance the effect of normal microglia in the vicinity of mal-functional microglia.
(w) To inhibit or restore declines in cognitive functions or motor functions.
(x) To remove amyloid β oligomers.
(y) To increase the number of synapses.
(z) To treat or prevent neurodegenerative diseases.
(p) To remove phosphorylated tau and/or to inhibit accumulation of tau;
(q) To inhibit NFT (neurofibrillary tangle) formation;
(r) To remove amyloid β; and
(s) To remove synuclein and/or synuclein aggregates.

As mentioned above, one embodiment of the anti-gpNMB-specific antibody of the present invention is estimated to exhibit one or more of the activities specified in (w) to (z) and (p) to (s) above as a result of exhibiting one or more of the activities specified in (a) to (c) above. Therefore, when the anti-gpNMB-specific antibody of the present invention is an antibody that expresses one or more of the activities specified in (a) to (c) above, the first pharmaceutical composition of the present invention may preferably be a pharmaceutical composition intended for one or more of the applications specified in (w) to (z) and (p) to (s) above. Preferable among the activities specified in (a) to (c) above is the activity specified in (a) above.

Based on the results of the Examples described herein, it is very likely that one or more of the activities specified in (w) to (z) and (p) to (s) above are exhibited as a result of one or more of the activities specified in (a) to (c) above. This is a novel finding discovered by the present inventors. Based on this finding, it is also possible to prepare a pharmaceutical compositions that exhibit one or more of the activities specified in (w) to (z) and (p) to (s) above by not using the anti-gpNMB specific antibody of the present invention, but using any other agent that has one or more of the applications specified in (a) to (c) above (e.g., a small molecule compound or a macromolecular drug such as an antibody). Accordingly, an embodiment of the present invention relates to a pharmaceutical composition intended for one or more of the activities specified in (w) to (z) and (p) to (s) above, containing an agent that has one or more of the applications specified in (a) to (c) above (which may be, e.g., a small molecule compound or a macromolecular drug such as an antibody) as an active ingredient (hereinafter also collectively referred to as "the second pharmaceutical composition of the present invention"). Preferable among the activities specified in (a) to (c) above is the activity specified in (a) above.

The details of the first pharmaceutical composition of the present invention and the second pharmaceutical composition of the present invention (hereinafter also collectively referred to as "the pharmaceutical composition of the present invention") are, without limitation, as follows.

The pharmaceutical composition of the present invention may be formulated in the form of a pharmaceutical composition containing, in addition to the active ingredient (the anti-gpNMB-specific antibody of the present invention in the case of the first pharmaceutical composition of the present invention, or an agent that has one or more of the activities specified in (a) to (c) above in the case of the second pharmaceutical composition of the present invention), a pharmaceutically acceptable carrier and/or other additive. Formulation using a pharmaceutically acceptable carrier and/or other additive may be carried out using, e.g., the methods described in University of the Sciences in Philadelphia, "Remington: The Science and Practice of Pharmacy, 20th EDITION", Lippincott Williams & Wilkins, (2000).

One form of such a therapeutic or prophylactic agent is a liquid or lyophilized form prepared by dissolving, suspending, or emulsifying the ingredients in a sterile aqueous or oily solution. Examples of such solvents or dissolving solutions as aqueous media include distilled water for injection and saline. In addition, osmotics (e.g., D-glucose, D-sorbitol, D-mannitol, sodium chloride, etc.) may be added, as well as suitable dissolution aids, such as alcohols (e.g., ethanol), poly alcohols (e.g., propylene glycol, polyethylene glycol), nonionic surfactants (e.g., polysorbate 80, polyoxyethylene hardened castor oil 50), etc., may be used in combination. Oil-based liquids may also be used as solvents or dissolving solutions. Examples of such oily liquids include sesame oil and soybean oil. Benzyl benzoate, benzyl alcohol, etc., may also be used as a dissolution aid. In such formulations, additives may be used as appropriate. Examples of additives include: buffers (e.g., phosphate buffer and acetate buffer), soothing agents (e.g., benzalkonium chloride and procaine hydrochloride), stabilizers (e.g., human serum albumin and polyethylene glycol), preservatives (e.g., ascorbic acid, erythorbic acid, and their salts), colorants (e.g., copper chlorophyll, β-carotene, red #2, and blue #1), antiseptics (e.g., paraoxybenzoate, phenol, benzethonium chloride, and benzalkonium chloride), thickeners (e.g., hydroxy propyl cellulose, carboxyl methyl cellulose, and their salts), stabilizing agents (e.g., human serum albumin, mannitol, and sorbitol), and deodorants (e.g., menthol and citrus flavoring).

Other forms of therapeutic or prophylactic agents include solid dosage forms such as dispersions, tablets, granules, capsules, pills, suppositories, and lozenges. Solid dosage forms administered in the form of oral preparations may contain additives such as excipients (e.g., crystalline cellulose, lactose, and starch), lubricants (e.g., magnesium stearate and talc), binding agents (e.g., hydroxypropyl cellulose, hydroxypropyl methylcellulose, and macrogol), and disintegrants (e.g., starch and calcium carboxymethylcellulose). In addition, additives such as preservatives (e.g., benzyl alcohol, chlorobutanol, methyl paraoxibenzoate, propyl paraoxibenzoate, etc.), antioxidants, colorants, and sweetening agents can be used as needed. Another form of the therapeutic or prophylactic agent is intended for mucosal application. This type of formulation may contain adhesives, adhesion enhancers, viscosity agents, and viscosity enhancers (e.g., mucin, agar, gelatin, pectin, carrageenan, sodium alginate, locust bean gum, xanthan gum, tragacanth gum, gum arabic, chitosan, pullulan, waxy starch, sucralfate, cellulose, and their derivatives) are contained as additives mainly to provide absorption and retention on the mucosa. gum arabic, chitosan, pullulan, waxy starches, sucralfate, cellulose, and their derivatives). However, the form of the therapeutic or prophylactic agent to be provided to the living body and the solvent and additives used therein are not limited to these, and can be selected by a person skilled in the art as appropriate.

The pharmaceutical composition of the present invention may contain, in addition to the active ingredient (the anti-gpNMB-specific antibody of the present invention in the case of the first pharmaceutical composition of the present invention, or an agent that has one or more of the activities specified in (a) to (c) above in the case of the second pharmaceutical composition of the present invention), another known drug (active ingredient). Examples of such other known drugs (active ingredients) include, although not limited to, various therapeutic drugs for central neurological diseases, therapeutic drugs for neurodegenerative diseases, nerve function recovery drugs, and therapeutic drugs for Alzheimer's disease. Specific examples include anti-Tau antibodies, anti-amyloid β antibodies, anti-CD33 antibodies, anti-semaphorin 4D antibodies, anti-TNFα antibodies, anti-sortilin antibodies, anti-galactose-specific-lectin (galectin) 3 antibodies, and anti-TREM2 (Triggering receptor expressed on myeloid cells 2) antibodies anti-IL-1β antibodies, anti-CD38 antibodies, anti-MS4A antibodies, and anti-synuclein antibodies. They may be used singly or in combination of any two or more.

The pharmaceutical composition of the present invention may also be combined with another known drug in the form of a kit. Examples of other drugs (active ingredients) that can be combined with the anti-gpNMB-specific antibody of the present invention include the known drugs (active ingredients) described above for combination drugs. The dose of the drugs other than the anti-gpNMB-specific antibody used in the form of the combination drug or kit can be determined based on doses normally used for treatment, but can be increased or decreased depending on the circumstances.

The pharmaceutical compositions of the present invention may be administered parenterally for the purpose of improving symptoms. For parenteral administration, it can be administered as, e.g., an intranasal formulation, and liquid, turbid, or solid formulations can be selected. Another parenteral dosage form may be an injectable form, which may be administered by subcutaneous, intravenous, intravenous, intramuscular, intracerebroventricular, or intraperitoneal injection. Other parenteral formulations include those for transmucosal administration other than suppositories, sublingual, transdermal, and other than nasal administration. In addition, the antibody can be locally administered intravascularly as contained in or applied to a stent or intravascular occlusive agent. In some cases, it is also possible to administer the antibody in the form of DNA or RNA encoding the antibody, or in the form of cells or commensal bacteria producing the antibody.

The dosage of the pharmaceutical composition of the present invention varies depending on the age, sex, weight, symptoms, therapeutic effect, method of administration, treatment time, or type of active ingredient contained in the pharmaceutical composition, etc. of the patient, but may usually be from 0.1 mg to 1 g, preferably from 0.5 mg to 300 mg, of the active ingredient per adult per dose. It may be administered once every week to every four weeks or once every month to every six months. However, the dosage and frequency of administration may vary depending on various conditions, and a lower dosage and/or frequency of administration than the aforementioned dosage and/or frequency may be sufficient, or a higher dosage and/or frequency of administration than the aforementioned dosage and/or frequency may be necessary. In addition, the therapeutic or prophylactic agent according to the present invention can be effective in a short period of administration or can be administered for a long period of time by reducing side effects.

### [Others]

As explained above, the anti-gpNMB-specific antibody of the present invention may be expected to express one or more of the activities specified in (a) to (c) and (w) to (z) and (p) to (s) below when administered to a subject such as human.
(a) The activity to reduce or remove mal-functional microglia.
(b) The activity to promote normal microglia's functions to phagocytize mal-functional microglia or the activity to normalize the mal-function of mal-functional microglia.
(c) The activity to enhance the effect of normal microglia in the vicinity of mal-functional microglia.
(w) The activity to inhibit or restore declines in cognitive functions or motor functions.
(x) The activity to remove amyloid β oligomers.
(y) The activity to increase the number of synapses.
(z) The activity to treat or prevent neurodegenerative diseases.
(p) The activity to remove phosphorylated tau and/or to inhibit accumulation of tau;
(q) The activity to inhibit NFT (neurofibrillary tangle) formation;
(r) The activity to remove amyloid β; and
(s) The activity to remove synuclein and/or synuclein aggregates.

Accordingly, in addition to the pharmaceutical composition of the present invention, the subject of the present invention may include the following embodiments.

Specifically, an embodiment of the present invention relates to a method for achieving one or more of the activities specified in (a) to (c), (w) to (z) and (p) to (s) above in a subject, comprising administering an effective amount of the anti-gpNMB-specific antibody of the present invention to a subject in need thereof. When the anti-gpNMB-specific antibody of the present invention is an antibody expressing one or more of the activities specified in (a) to (c) above, the method may preferably be intended to achieve one or more of the activities in (w) to (z) and (p) to (s) above in the subject. Other details are as explained above. Preferable among the activities specified in (a) to (c) above is the activity specified in (a) above.

An embodiment of the present invention relates to a method for achieving one or more of the activities in (w) to (z) and (p) to (s) above in a subject, comprising administering an effective amount of an agent that has one or more of the activities specified in (a) to (c) above (which may be, e.g., a small molecule compound or a macromolecular drug such as an antibody) to a subject in need thereof. Other details are as explained above. Preferable among the activities specified in (a) to (c) above is the activity specified in (a) above.

An embodiment of the present invention relates to the anti-gpNMB-specific antibody of the present invention for use in achieving one or more of the activities specified in (a) to (c), (w) to (z) and (p) to (s) above in a subject. When the anti-gpNMB-specific antibody of the present invention is an antibody expressing one or more of the activities specified in (a) to (c) above, the antibody may preferably be for use in achieving one or more of the activities in (w) to (z) and (p) to (s) above in the subject. Other details are as explained above. Preferable among the activities specified in (a) to (c) above is the activity specified in (a) above.

An embodiment of the present invention relates to an agent that has one or more of the activities specified in (a) to (c) above (which may be, e.g., a small molecule compound or a macromolecular drug such as an antibody) for use in achieving one or more of the activities in (w) to (z) and (p) to (s) above in a subject. Other details are as explained above. Preferable among the activities specified in (a) to (c) above is the activity specified in (a) above.

An embodiment of the present invention relates to the use of the anti-gpNMB-specific antibody of the present invention in the manufacture of a medicament for achieving one or more of the activities specified in (a) to (c), (w) to (z) and (p) to (s) above in a subject. When the anti-gpNMB-specific antibody of the present invention is an antibody expressing one or more of the activities specified in (a) to (c) above, the medicament may preferably be for use in achieving one or more of the activities in (w) to (z) and (p) to (s) above in the subject. Other details are as explained above. Preferable among the activities specified in (a) to (c) above is the activity specified in (a) above.

An embodiment of the present invention relates to the use of an agent that has one or more of the activities specified in (a) to (c) above (which may be, e.g., a small molecule compound or a macromolecular drug such as an antibody) in the manufacture of a medicament for achieving one or more of the activities in (w) to (z) and (p) to (s)above in a subject. Other details are as explained above. Preferable among the activities specified in (a) to (c) above is the activity specified in (a) above.

### EXAMPLES

The present invention will now be described in further detail by way of Examples. These examples are shown merely for convenience of the description, and should not be construed as limitations to the present invention in any sense.

### [Common procedures]

### *Antigen ELISA (Enzyme-Linked Immunosorbent Assay):

Recombinant soluble human or mouse gpNMB was diluted by PBS to 2 µg/mL, added to a 96-well plate (Nunc, MaxiSorp) at 50 µL/well, and allowed to stand overnight at 4°C. The 96-well plate was blocked with 3% BSA/PBS and used for ELISA as recombinant soluble gpNMB-fixed 96-well plate.

Hybridoma culture supernatant containing an anti-gpNMB antibody or 3% BSA/PBS solution was added at 30 µL/well to the above recombinant soluble gpNMB-fixed 96-well plate and allowed to react for 1 hour at room temperature. After washing with washing buffer (TBS-T: Tris Buffered Saline, 0.025% Tween 20), anti-mouse IgG antibody-ALP conjugate (SBA, 1050 -04), diluted 1000-fold in 3% BSA/PBS, was added at 30 µL/well and allowed to react for 1 hour at room temperature. Substrate (PNPP (p-nitrophenyl phosphate), 1 mg/mL ) solution was added at 100 µL/well and allowed to react for 1 hour at room temperature, and absorbance at 405-550 nm was calculated. The absorbance values obtained were evaluated as binding activity.

### *Cell-based ELISA:

The gpNMB-expressing cells were seeded into a polylysine-coated 96-well plate and allowed to adhere, and the culture supernatant was removed. The cells were then fixed with a 10% formalin-containing solution for 10 minutes at room temperature, and the supernatant was removed. The cells were then blocked with 3% BSA/PBS solution, and stored under refrigeration.

After the blocking solution was removed from the cell-fixed plate obtained above, and antibody-containing culture medium or solution was added at 30 µL/well, and incubated at room temperature for about 1 hour to 1 hour 30 minutes. The cells were washed 2 to 4 times with washing buffer (TBS-T), and a 1000-fold dilution of anti-mouse IgG-HRP labeled secondary antibody solution was added to the plate, and incubated at room temperature for 1 hour. The cells were washed 2 to 4 times with washing buffer (TBS-T), and substrate TMB solution was then added at 50 µL/well, and incubated at room temperature for 30 minutes. The reaction was then stopped by adding 1N sulfuric acid at 50 µL/well, and the absorbance at 450-650 nm was calculated. The absorbance value obtained was evaluated as binding activity.

### [Example 1] Production and screening of mouse or rat monoclonal antibodies (antigen ELISA and cell-based ELISA):

Mouse or rat monoclonal antibodies can be produced by the hybridoma method described in, e.g., Koehler et al., Nature, (1975), 256:495-497. Anti-gpNMB antibodies were produced by inducing immunity in mice or rats using nucleic acids, proteins, or peptides containing the full length or part of the amino acid sequence (SEQ ID NO 209) or the nucleic acid sequence (SEQ ID NO 210) of human gpNMB (isotype a), or the amino acid sequence (SEQ ID NO 211) or the nucleic acid sequence (SEQ ID NO 212) of mouse gpNMB, and collecting lymphocyte cells from the mice or rats with elevated antibody titer. All animal experiments were performed according to institutional regulations. Hybridoma generation by fusion of lymphocyte cells from mice or rats with mouse myeloma cell lines (P3U1 (P3X63Ag8U.1) or SP2/0 (SP2/0-Ag14)) was performed using standard hybridoma techniques. Hybridomas were selected using so-called HAT medium, which contains hypoxanthine, aminopterin, and thymidine. An amino acid sequence alignment of human gpNMB (isotype a) (SEQ ID NO 209) and mouse gpNMB (SEQ ID NO 211) is shown in Figure 1, in which the region at amino acid residues 256 to 319 is shown with dotted lines as an example of the PKD domain, and the region at amino acid residues 172 to 246 is shown with solid lines as an example of the PMEL-CAF-like domain.

The resulting hybridoma cultures were subjected to antigen ELISA with fixed recombinant human soluble gpNMB (SEQ ID NO 221) or mouse soluble gpNMB (SEQ ID NO 223) or cell-based ELISA with cells expressing proteins containing human gpNMB (SEQ ID NO 209) to evaluate binding ability, and the wells containing positive hybridomas were selected. Hybridomas in these wells were single-cloned by limiting dilution. The single-cloned positive hybridomas were cultured, and monoclonal antibodies were purified from the culture media using Protein A column (Ab-Capture, ProteNova) to obtain multiple anti-gpNMB antibodies.

### [Example 2] Sequencing of the antibodies:

Each anti-gpNMB antibody clone obtained in Example 1 was subjected to the SMARTer^{™} RACE method to determine the gene sequences of its light and heavy chains. RNA derived from the hybridoma producing the antibody was subjected to the SMARTer^{™} RACE method to thereby obtain gene fragments of the heavy and light chains of the antibody, including the start and end codons, and their nucleotide sequences were determined. Specifically, the 1st strand cDNA was synthesized with the SMARTer ^{™} RACE 5'/3' Kit (634859, Clontech) using total RNA from the hybridoma as a template, and the cDNA was amplified by PCR reaction. Using the cDNA as a template, PCR reactions were performed using primers for the universal sequence provided in the kit and primers specific for each of the heavy and light chains of the antibody. The resulting PCR products were used as 5'RACE PCR products for TA cloning.

When sequencing a rat monoclonal antibody gene, PCR reactions were performed on the variable regions of the heavy and light chains of the rat antibody, and the resulting PCR fragments were bound to the mouse constant regions to produce a mouse chimeric antibody, which was then subjected to sequencing.

For TA cloning, the 5' RACE PCR products were electrophoresed, and cDNA fragments having desired molecular weights were purified using the QIAEX II Gel Extraction Kit (20021, Qiagen). The purified cDNA fragments were subjected to reaction using TaKaRa-Taq (R001A, Takara) at 72°C for 5 minutes to add adenine at the 3' end. The cDNA fragments were then cloned into the pMD20-T vector (hereafter referred to as MD20 vector) using the Mighty TA-cloning Kit (6028, Takara) according to the attached protocol. The MD20 vector in which the target cDNA fragments were cloned was transformed into E. coli TOP10, and cultured on agar medium containing 100 µg/mL ampicillin. Insertion of the target cDNA fragments into the MD20 vector was confirmed by colony PCR. The nucleotide sequences of the cloned cDNA fragments were determined. Similarly, the nucleotide sequences of the 3' RACE PCR products were determined, whereby the full-length sequence of the gene for each antibody was determined.

Of the anti-gpNMB antibody clones obtained in Example 1, GPN05-1, GPN06-1, GPN07-1, GPN09-1, GPN11-10, GPN15-2, GPN15-3, GPN18-1, GPN18-2, GPN18-4, GPN18-5, GPN18-6, GPN18-7, and 1-5E are shown in Tables 1-1 to 1-5, along with the Sequence Identification Nos. of the amino acid sequences and nucleotide sequences of their heavy chain and light chain variable regions (hereinafter also referred to as VH and VL, respectively) and the complementarity determining regions included therein (hereinafter also referred to as CDR-H1 to H3 and CDR-L1 to L3, respectively), as determined by the methods explained above.

**[Table 1-1]**

| Antibody clone | Description | Amino acid sequence | | Nucleic acid sequence | |
|---|---|---|---|---|---|
| GPN05-1 | CDR-H1 | SEQ ID NO | 1 | SEQ ID NO | 2 |
| | CDR-H2 | SEQ ID NO | 3 | SEQ ID NO | 4 |
| | CDR-H3 | SEQ ID NO | 5 | SEQ ID NO | 6 |
| | CDR-L1 | SEQ ID NO | 7 | SEQ ID NO | 8 |
| | CDR-L2 | SEQ ID NO | 9 | SEQ ID NO | 10 |
| | CDR-L3 | SEQ ID NO | 11 | SEQ ID NO | 12 |
| | Heavy chain variable region (VH) | SEQ ID NO | 13 | SEQ ID NO | 14 |
| | Light chain variable region (VL) | SEQ ID NO | 15 | SEQ ID NO | 16 |
| GPN06-1 | CDR-H1 | SEQ ID NO | 17 | SEQ ID NO | 18 |
| | CDR-H2 | SEQ ID NO | 19 | SEQ ID NO | 20 |
| | CDR-H3 | SEQ ID NO | 21 | SEQ ID NO | 22 |
| | CDR-L1 | SEQ ID NO | 23 | SEQ ID NO | 24 |
| | CDR-L2 | SEQ ID NO | 25 | SEQ ID NO | 26 |
| | CDR-L3 | SEQ ID NO | 27 | SEQ ID NO | 28 |
| | Heavy chain variable region (VH) | SEQ ID NO | 29 | SEQ ID NO | 30 |
| | Light chain variable region (VL) | SEQ ID NO | 31 | SEQ ID NO | 32 |
| GPN07-1 | CDR-H1 | SEQ ID NO | 33 | SEQ ID NO | 34 |
| | CDR-H2 | SEQ ID NO | 35 | SEQ ID NO | 36 |
| | CDR-H3 | SEQ ID NO | 37 | SEQ ID NO | 38 |
| | CDR-L1 | SEQ ID NO | 39 | SEQ ID NO | 40 |
| | CDR-L2 | SEQ ID NO | 41 | SEQ ID NO | 42 |
| | CDR-L3 | SEQ ID NO | 43 | SEQ ID NO | 44 |
| | Heavy chain variable region (VH) | SEQ ID NO | 45 | SEQ ID NO | 46 |
| | Light chain variable region (VL) | SEQ ID NO | 47 | SEQ ID NO | 48 |

**[Table 1-2]**

| Antibody clone | Description | Amino acid sequence | | Nucleic acid sequence | |
|---|---|---|---|---|---|
| GPN11-10 | CDR-H1 | SEQ ID NO | 49 | SEQ ID NO | 50 |
| | CDR-H2 | SEQ ID NO | 51 | SEQ ID NO | 52 |
| | CDR-H3 | SEQ ID NO | 53 | SEQ ID NO | 54 |
| | CDR-L1 | SEQ ID NO | 55 | SEQ ID NO | 56 |
| | CDR-L2 | SEQ ID NO | 57 | SEQ ID NO | 58 |
| | CDR-L3 | SEQ ID NO | 59 | SEQ ID NO | 60 |
| | Heavy chain variable region (VH) | SEQ ID NO | 61 | SEQ ID NO | 62 |
| | Light chain variable region (VL) | SEQ ID NO | 63 | SEQ ID NO | 64 |
| GPN15-2 | CDR-H1 | SEQ ID NO | 65 | SEQ ID NO | 66 |
| | CDR-H2 | SEQ ID NO | 67 | SEQ ID NO | 68 |
| | CDR-H3 | SEQ ID NO | 69 | SEQ ID NO | 70 |
| | CDR-L1 | SEQ ID NO | 71 | SEQ ID NO | 72 |
| | CDR-L2 | SEQ ID NO | 73 | SEQ ID NO | 74 |
| | CDR-L3 | SEQ ID NO | 75 | SEQ ID NO | 76 |
| | Heavy chain variable region (VH) | SEQ ID NO | 77 | SEQ ID NO | 78 |
| | Light chain variable region (VL) | SEQ ID NO | 79 | SEQ ID NO | 80 |
| GPN15-3 | CDR-H1 | SEQ ID NO | 81 | SEQ ID NO | 82 |
| | CDR-H2 | SEQ ID NO | 83 | SEQ ID NO | 84 |
| | CDR-H3 | SEQ ID NO | 85 | SEQ ID NO | 86 |
| | CDR-L1 | SEQ ID NO | 87 | SEQ ID NO | 88 |
| | CDR-L2 | SEQ ID NO | 89 | SEQ ID NO | 90 |
| | CDR-L3 | SEQ ID NO | 91 | SEQ ID NO | 92 |
| | Heavy chain variable region (VH) | SEQ ID NO | 93 | SEQ ID NO | 94 |
| | Light chain variable region (VL) | SEQ ID NO | 95 | SEQ ID NO | 96 |

**[Table 1-3]**

| Antibody clone | Description | Amino acid sequence | | Nucleic acid sequence | |
|---|---|---|---|---|---|
| GPN18-4 | CDR-H1 | SEQ ID NO | 97 | SEQ ID NO | 98 |
| | CDR-H2 | SEQ ID NO | 99 | SEQ ID NO | 100 |
| | CDR-H3 | SEQ ID NO | 101 | SEQ ID NO | 102 |
| | CDR-L1 | SEQ ID NO | 103 | SEQ ID NO | 104 |
| | CDR-L2 | SEQ ID NO | 105 | SEQ ID NO | 106 |
| | CDR-L3 | SEQ ID NO | 107 | SEQ ID NO | 108 |
| | Heavy chain variable region (VH) | SEQ ID NO | 109 | SEQ ID NO | 110 |
| | Light chain variable region (VL) | SEQ ID NO | 111 | SEQ ID NO | 112 |
| GPN18-1 | CDR-H1 | SEQ ID NO | 113 | SEQ ID NO | 114 |
| | CDR-H2 | SEQ ID NO | 115 | SEQ ID NO | 116 |
| | CDR-H3 | SEQ ID NO | 117 | SEQ ID NO | 118 |
| | CDR-L1 | SEQ ID NO | 119 | SEQ ID NO | 120 |
| | CDR-L2 | SEQ ID NO | 121 | SEQ ID NO | 122 |
| | CDR-L3 | SEQ ID NO | 123 | SEQ ID NO | 124 |
| | Heavy chain variable region (VH) | SEQ ID NO | 125 | SEQ ID NO | 126 |
| | Light chain variable region (VL) | SEQ ID NO | 127 | SEQ ID NO | 128 |
| GPN18-2 | CDR-H1 | SEQ ID NO | 129 | SEQ ID NO | 130 |
| | CDR-H2 | SEQ ID NO | 131 | SEQ ID NO | 132 |
| | CDR-H3 | SEQ ID NO | 133 | SEQ ID NO | 134 |
| | CDR-L1 | SEQ ID NO | 135 | SEQ ID NO | 136 |
| | CDR-L2 | SEQ ID NO | 137 | SEQ ID NO | 138 |
| | CDR-L3 | SEQ ID NO | 139 | SEQ ID NO | 140 |
| | Heavy chain variable region (VH) | SEQ ID NO | 141 | SEQ ID NO | 142 |
| | Light chain variable region (VL) | SEQ ID NO | 143 | SEQ ID NO | 144 |

**[Table 1-4]**

| Antibody clone | Description | Amino acid sequence | | Nucleic acid sequence | |
|---|---|---|---|---|---|
| GPN18-6 | CDR-H1 | SEQ ID NO | 145 | SEQ ID NO | 146 |
| | CDR-H2 | SEQ ID NO | 147 | SEQ ID NO | 148 |
| | CDR-H3 | SEQ ID NO | 149 | SEQ ID NO | 150 |
| | CDR-L1 | SEQ ID NO | 151 | SEQ ID NO | 152 |
| | CDR-L2 | SEQ ID NO | 153 | SEQ ID NO | 154 |
| | CDR-L3 | SEQ ID NO | 155 | SEQ ID NO | 156 |
| | Heavy chain variable region (VH) | SEQ ID NO | 157 | SEQ ID NO | 158 |
| | Light chain variable region (VL) | SEQ ID NO | 159 | SEQ ID NO | 160 |
| GPN18-7 | CDR-H1 | SEQ ID NO | 161 | SEQ ID NO | 162 |
| | CDR-H2 | SEQ ID NO | 163 | SEQ ID NO | 164 |
| | CDR-H3 | SEQ ID NO | 165 | SEQ ID NO | 166 |
| | CDR-L1 | SEQ ID NO | 167 | SEQ ID NO | 168 |
| | CDR-L2 | SEQ ID NO | 169 | SEQ ID NO | 170 |
| | CDR-L3 | SEQ ID NO | 171 | SEQ ID NO | 172 |
| | Heavy chain variable region (VH) | SEQ ID NO | 173 | SEQ ID NO | 174 |
| | Light chain variable region (VL) | SEQ ID NO | 175 | SEQ ID NO | 176 |
| GPN18-5 | CDR-H1 | SEQ ID NO | 177 | SEQ ID NO | 178 |
| | CDR-H2 | SEQ ID NO | 179 | SEQ ID NO | 180 |
| | CDR-H3 | SEQ ID NO | 181 | SEQ ID NO | 182 |
| | CDR-L1 | SEQ ID NO | 183 | SEQ ID NO | 184 |
| | CDR-L2 | SEQ ID NO | 185 | SEQ ID NO | 186 |
| | CDR-L3 | SEQ ID NO | 187 | SEQ ID NO | 188 |
| | Heavy chain variable region (VH) | SEQ ID NO | 189 | SEQ ID NO | 190 |
| | Light chain variable region (VL) | SEQ ID NO | 191 | SEQ ID NO | 192 |

**[Table 1-5]**

| Antibody clone | Description | Amino acid sequence | | Nucleic acid sequence | |
|---|---|---|---|---|---|
| 1-5E | CDR-H1 | SEQ ID NO | 193 | SEQ ID NO | 194 |
| | CDR-H2 | SEQ ID NO | 195 | SEQ ID NO | 196 |
| | CDR-H3 | SEQ ID NO | 197 | SEQ ID NO | 198 |
| | CDR-L1 | SEQ ID NO | 199 | SEQ ID NO | 200 |
| | CDR-L2 | SEQ ID NO | 201 | SEQ ID NO | 202 |
| | CDR-L3 | SEQ ID NO | 203 | SEQ ID NO | 204 |
| | Heavy chain variable region (VH) | SEQ ID NO | 205 | SEQ ID NO | 206 |
| | Light chain variable region (VL) | SEQ ID NO | 207 | SEQ ID NO | 208 |
| GPN09-1 | CDR-H1 | SEQ ID NO | 255 | SEQ ID NO | 256 |
| | CDR-H2 | SEQ ID NO | 257 | SEQ ID NO | 258 |
| | CDR-H3 | SEQ ID NO | 259 | SEQ ID NO | 260 |
| | CDR-L1 | SEQ ID NO | 261 | SEQ ID NO | 262 |
| | CDR-L2 | SEQ ID NO | 263 | SEQ ID NO | 264 |
| | CDR-L3 | SEQ ID NO | 265 | SEQ ID NO | 266 |
| | Heavy chain variable region (VH) | SEQ ID NO | 267 | SEQ ID NO | 268 |
| | Light chain variable region (VL) | SEQ ID NO | 269 | SEQ ID NO | 270 |

### [Example 3] Analysis of the binding activity of the anti-gpNMB antibodies to microglia (cell-based ELISA):

To evaluate the binding ability of each anti-gpNMB antibody obtained in Example 1 to microglia, brain cells were harvested from 1- to 2-day-old mouse or rat brains, cultured for about 1 week, and mouse or rat microglia were collected by shaking the adherent cells for 1 hour to collect detached cells. The collected mouse or rat microglia were suspended in 20% FBS/DMEM-GlutaMax, seeded into 96-well plates at 2×10⁴cell /well, cultured in a CO₂ incubator at 37°C for 1 to 2 days for attachment, fixed with Mild form, and then blocked with 3% BSA/PBS to prepare a cell ELISA plate.

After the blocking solution was removed from the mouse or rat microglia-fixed ELISA plate, a diluent of the anti-gpNMB antibody (3% BSA/PBS solution) was added at 30 µL/well, and allowed to react for 1 hour at room temperature. Then, after washing with washing buffer, an antibody that specifically reacts with mouse IgG, i.e., either anti-mouse IgG antibody-HRP conjugate (MBL, Code No. 330) at 50 µL/well or an anti-mouse IgG antibody-ALP conjugate (SBA (SBA, Code No. 1050-04) at 30 µL/well, was added and allowed to react for 1 hour at room temperature. Substrate for HRP (ELISA-Star^{™} peroxidase chemiluminescent substrate, FUJIFILM Wako Pure Chemicals Corporation) was added at 100 µL/well, and the luminescence intensity was immediately measured for 1 to 10 seconds, and the value of luminescence intensity obtained was evaluated as binding activity. Alternatively, substrate for ALP (PNPP) was added at 100 µL/well and reacted at room temperature for 1 hour, and the absorbance at 405 to 550 nm was measured and evaluated as binding activity. The results confirmed the binding activity of the anti-gpNMB antibodies to mouse and rat microglia.

### [Example 4] Analysis of the binding sites of the anti-gpNMB antibodies (antigen ELISA):

To analyze the binding sites of each anti-gpNMB antibody obtained in Example 1 to the human gpNMB protein, the following experiment was performed.

First, based on the amino acid sequence of the human gpNMB extracellular domain (SEQ ID NO 215, in which the amino acid residues at positions 1 to 21 constitute a signal peptide, while the amino acid residues at positions 22 and onward constitute an isolated peptide of the human gpNMB extracellular domain), an N-terminal deletion variant (hgpNMB _d07_76-498) and C-terminal deletion variants (hgpNMB _d03_1-251, hgpNMB_d04_1-321, hgpNMB_d05_1-375, hgpNMB_d06_1-418, hgpNMB_d13_1-412, hgpNMB_d14_1-406, hgpNMB_d15_1-400, hgpNMB_d16_1-394, hgpNMB_d17_1-388, hgpNMB_d18_1-382, and hgpNMB _d19_1-234) of the human gpNMB extracellular domain were produced as shown in Table 2 below. Each purified gpNMB protein was obtained by constructing a transient animal cell expression vector into which each gpNMB gene had been inserted, and having it expressed in a medium using the Expi293 Expression System (A14635, Thermo Fisher Scientific). The expressed protein was then purified based on affinity with Ni-NTA agarose (143-09763, FUJIFILM Wako) using a histidine tag added to the C-terminus to thereby obtain the purified protein.

**[Table 2]**

| Table 2: N-terminal or C-terminal deletion variants of human gpNMB extracellular domain | |
|---|---|
| Variant No. | Description |
| hgpNMB_d03_1-251 | C-terminal deletion variant consisting of 1 to 251 of hgpNMB |
| hgpNMB_d04_1-321 | C-terminal deletion variant consisting of 1 to 321 of hgpNMB |
| hgpNMB_d05_1-375 | C-terminal deletion variant consisting of 1 to 375 of hgpNMB |
| hgpNMB d06_ 1-418 | C-terminal deletion variant consisting of 1 to 418 of hgpNMB |
| hgpNMB_d07_76-498 | N-terminal deletion variant consisting of 76 to 498 of hgpNMB |
| hgpNMB_d13_1-412 | C-terminal deletion variant consisting of 1 to 412 of hgpNMB |
| hgpNMB_d14_1-406 | C-terminal deletion variant consisting of 1 to 406 of hgpNMB |
| hgpNMB_d15_1-400 | C-terminal deletion variant consisting of 1 to 400 of hgpNMB |
| hgpNMB_d16_1-394 | C-terminal deletion variant consisting of 1 to 394 of hgpNMB |
| hgpNMB_d17_1-388 | C-terminal deletion variant consisting of 1 to 388 of hgpNMB |
| hgpNMB_d18_1-382 | C-terminal deletion variant consisting of 1 to 382 of hgpNMB |
| hgpNMB_d19_1-234 | C-terminal deletion variant consisting of 1 to 234 of hgpNMB |

Next, 17 nmol/L of each of purified recombinant human soluble gpNMB (SEQ ID NO 221), the N-terminal deletion variant (hgpNMB_d07_76-498) and the C-terminal deletion variants (hgpNMB_d03_1-251, hgpNMB_d04_1-321, hgpNMB_d05_1-375, hgpNMB_d06_1-418, hgpNMB_d13_1-412, hgpNMB_d14_1-406, hgpNMB_d15_1-400, hgpNMB_d16_1-394, hgpNMB_d17_1-388, hgpNMB_d18_1-382, and hgpNMB_dl9_1-234) of the human gpNMB extracellular domain shown in Table 2 above, and purified recombinant mouse soluble gpNMB (SEQ ID NO 223) was added to a 96-well plate for ELISA at 100 µL/well, and allowed to stand at room temperature for 90 minutes. The plate was then blocked with 3% BSA/PBS to thereby obtain an antigen ELISA plate.

After the blocking solution was removed from the antigen ELISA plate, a diluent of the anti-gpNMB antibody (3% BSA/PBS solution) was added at 50 µL/well, and allowed to react for 1 hour at room temperature. Then, after washing with washing buffer, an antibody that specifically reacts with mouse IgG, anti-mouse IgG antibody-ALP conjugate (SBA, 1050-04), was added at 50 µL/well and allowed to react for 1 hour at room temperature. Substrate (PNPP) was added at 100 µL/well and allowed to react for 1 hour at room temperature, and the absorbance at 405 to 550 nm was measured and evaluated as binding activity.

### [Example 5] Determination of the epitopes of the anti-gpNMB antibodies (antigen ELISA):

To determine the epitope of each anti-gpNMB antibody obtained in Example 1 in the human gpNMB protein, the following experiment was performed.

First, a C-terminal deletion variant (hgpNMB _d19_1-234) and point mutants (hgpNMB_d22_K245A, hgpNMB_d23_D252A, hgpNMB_d24_E253A, hgpNMB_d25_D264A, hgpNMB_d26_H272A, hgpNMB_d28_H297A, hgpNMB_d30_H376A, hgpNMBd31_D247A_R248A, hgpNMB_d34_D287A, hgpNMB_d35_H301A, hgpNMB _d36_R331A_K334A, hgpNMB _d37_K344A_D347A, hgpNMB_d38_D356A, hgpNMB_d39_E360A, hgpNMB_d40_E367A, and hgpNMB_d41_R373A) of human gpNMB extracellular domain as shown in Table 3 below were produced. Each purified gpNMB protein was obtained by constructing a transient animal cell expression vector into which each gpNMB gene had been inserted, and having it expressed in a medium using the Expi293 Expression System (A14635, Thermo Fisher Scientific). The expressed protein was then purified based on affinity with Ni-NTA agarose (143-09763, FUJIFILM Wako) using a histidine tag added to the C-terminus to thereby obtain the purified protein. The C-terminal deletion variant and the point mutants of human gpNMB extracellular domain are summarized in Table 3.

**[Table 3]**

| Table 3: C-terminal deletion variants and point mutants of human gpNMB extracellular domain | |
|---|---|
| Variant No. | Description |
| hgpNMB_d19_1-234 | C-terminal deletion variant consisting of 1 to 234 of hgpNMB |
| hgpNMB_d22_K245A | K245A point mutant of hgpNMB |
| hgpNMB_d23_D252A | D252A point mutant of hgpNMB |
| hgpNMB_d24_E253A | E253A point mutant of hgpNMB |
| hgpNMB_d25_D264A | D264A point mutant of hgpNMB |
| hgpNMB_d26_H272A | H272A point mutant of hgpNMB |
| hgpNMB_d28_H297A | H297A point mutant of hgpNMB |
| hgpNMB_d30_H376A | H376A point mutant of hgpNMB |
| hgpNMBd31_D247A_R248A | D247A and R248A point mutant of hgpNMB |
| hgpNMB_d34_D287A | D287A point mutant of hgpNMB |
| hgpNMB_d35_H301A | H301A point mutant of hgpNMB |
| hgpNMB_d36_R331A_K334A | R331A and K334A point mutant of hgpNMB |
| hgpNMB_d37_K344A_D347A | K344A and D347A point mutant of hgpNMB |
| hgpNMB_d38_D356A | D356A point mutant of hgpNMB |
| hgpNMB_d39_E360A | E360A point mutant of hgpNMB |
| hgpNMB_d40_E367A | E367A point mutant of hgpNMB |
| hgpNMB_d41_R373A | R373A point mutant of hgpNMB |

These alanine point mutants of human gpNMB extracellular domain were subjected to antigen ELISA analysis using the same procedure explained in Example 4 to evaluate the binding ability of each anti-gpNMB antibody. Specifically, each of the C-terminal deletion variant and the alanine point mutants of human gpNMB extracellular domain shown in Table 2 above was added to a 96-well plate for ELISA at a concentration of 17 nmol/L at 100 µL/well and allowed to stand at room temperature for 90 minutes. The antigen ELISA plate was then blocked with 3% BSA/PBS to obtain an antigen ELISA plate.

After the blocking solution was removed from the antigen ELISA plate, a diluent of the anti-gpNMB antibody (3% BSA/PBS solution) was added at 50 µL/well and allowed to react for 1 hour at room temperature. Then, after washing with washing buffer, an antibody that specifically reacts with mouse IgG, i.e., an anti-mouse IgG antibody-ALP conjugate (SBA, 1050-04), was added at 50 µL/well and allowed to react for 1 hour at room temperature. Substrate (PNPP) was added at 100 µL/well and reacted at room temperature for 1 hour, and the absorbance at 405 to 550 nm was calculated and evaluated as binding activity. Compared to the mutant that showed the maximum absorbance at 2 nM concentration, mutants that showed a decrease in absorbance of 50% or more are marked as +, and those that showed an absorbance of 50% or more are marked as -. The results are shown in Table 4.

**[Table 4]**

| Table 4: Results of ELISA binding analysis of each anti-gpNMB antibody clone to human gpNMB extracellular domain variants | | | | | | | | |
|---|---|---|---|---|---|---|---|---|
| | GPN 05-1 | GPN 06-1 | GPN 07-1 | GPN 09-1 | GPN 11-10 | GPN 15-2 | GPN 15-3 | GPN 18-4 |
| 22-234_N terminal | - | - | - | - | - | - | - | - |
| K245A | - | - | - | - | - | - | - | - |
| D252A | - | - | - | - | - | - | - | - |
| E253A | - | - | - | - | - | - | - | - |
| K257A,D258A | - | - | - | + | + | - | - | - |
| D264A | - | - | - | - | - | - | - | - |
| H268A,D269A | - | - | + | - | - | - | - | - |
| H272A | - | - | - | - | - | - | - | - |
| K282A | - | + | + | - | - | - | - | - |
| H297A | - | - | - | - | - | - | - | - |
| H376A | - | - | - | - | - | - | - | - |
| D247A,R248A | - | - | - | - | - | - | - | - |
| **D287A** | + | + | + | + | + | + | + | + |
| **H301A** | + | + | + | + | + | + | + | + |
| K316A | - | + | + | - | - | - | - | - |
| R331A,K334A | - | - | - | - | - | - | - | - |
| K344A,D347A | - | - | - | - | - | - | - | - |
| D356A | - | - | - | - | - | - | - | - |
| E360A | - | - | - | - | - | - | - | - |
| E367A | - | - | - | - | - | - | - | - |
| R373A | - | - | - | - | - | - | - | - |
| E385A | - | - | - | + | + | - | - | - |

In addition, the anti-gpNMB antibodies that were bound to a C-terminal deletion mutant of the human gpNMB extracellular domain (hgpNMB_d19_1-234) were subjected to analysis for binding ability to alanine point mutants of N-terminal human gpNMB extracellular domain using antigen ELISA in a similar manner.

First, a C-terminal deletion variant (hgpNMB_d19_1-234) and point mutants (hgpNMB_d19_1-234, hgpNMB_d43_1-234_R77A, hgpNMB_d44_1-234_D85A, hgpNMB_d45_1-234_R104A, hgpNMB_d47_1-234_E117A,K118A, hgpNMB_d48_1-234_R121A,E123A, hgpNMB_d49_1-234_D129A, hgpNMB_d50_1-234_E140A,D141A, hgpNMB _d51_1-234_D143A,E145A, hgpNMB_d52_1-234_H152A,H153A, hgpNMB _d53_1-234_D158A,K160A, hgpNMB _d54_1-234_H164A,H165A, hgpNMB _d55_1-234_R169A, hgpNMB _d58_1-234_R189A, hgpNMB _d59_1-234_R193A, hgpNMB_d61_1-234_R214A,R215A, and hgpNMB_d64_1-234_D109A) of human gpNMB extracellular domain shown in Table 5 below were generated. Each purified gpNMB protein was obtained by constructing a transient animal cell expression vector into which each gpNMB gene had been inserted, and having it expressed in a medium using the Expi293 Expression System (A14635, Thermo Fisher Scientific). The expressed protein was then purified based on affinity with Ni-NTA agarose (143-09763, FUJIFILM Wako) using a histidine tag added to the C-terminus to thereby obtain the purified protein. The C-terminal deletion variant and the point mutants of human gpNMB extracellular domain are summarized in Table 5.

**[Table 5]**

| Table 5: C-terminal deletion variant and point mutants of human gpNMB extracellular domain | |
|---|---|
| Variant No. | Description |
| hgpNMB_d19_1-234 | C-terminal deletion variant consisting of 1 to 234 of hgpNMB |
| hgpNMB_d43_1-234_R77A | R77A point mutant of 1 to 234 of hgpNMB |
| hgpNMB_d44_1-234_D85A | D85A point mutant of 1 to 234 of hgpNMB |
| hgpNMB_d45_1-234_R104A | R104A point mutant of 1 to 234 of hgpNMB |
| hgpNMB_d47_1-234_E117A,K118A | E117A and K118A point mutant of 1 to 234 of hgpNMB |
| hgpNMB_d48_1-234_R121A,E123A | R121A and E123A point mutant of 1 to 234 of hgpNMB |
| hgpNMB_d49_1-234_D129A | D129A point mutant of 1 to 234 of hgpNMB |
| hgpNMB_d50_1-234_E140A,D141A | E140A and D141A point mutant of 1 to 234 of hgpNMB |
| hgpNMB_d51_1-234_D143A,E145A | D143A and E145A point mutant of 1 to 234 of hgpNMB |
| hgpNMB_d52_1-234_H152A,H153A | H152A and H153A point mutant of 1 to 234 of hgpNMB |
| hgpNMB_d53_1-234_D158A,K160A | D158A and K160A point mutant of 1 to 234 of hgpNMB |
| hgpNMB_d54_1-234_H164A,H165A | H164A and H165A point mutant of 1 to 234 of hgpNMB |
| hgpNMB_d55_1-234_R169A | R169A point mutant of 1 to 234 of hgpNMB |
| hgpNMB_d58_1-234_R189A | R189A point mutant of 1 to 234 of hgpNMB |
| hgpNMB_d59_1-234_R193A | R193A point mutant of 1 to 234 of hgpNMB |
| hgpNMB_d61_1-234_R214A,R215A | R214A and R215A point mutant of 1 to 234 of hgpNMB |
| hgpNMB_d64_1-234_D109A | D109A point mutant of 1 to 234 of hgpNMB |

These alanine point mutants of human gpNMB extracellular domain were subjected to antigen ELISA analysis using the same procedure explained in Example 4 to evaluate the binding ability of each anti-gpNMB antibody. Specifically, each of the C-terminal deletion variant and the alanine point mutants of human gpNMB extracellular domain shown in Table 5 above was added to a 96-well plate for ELISA at a concentration of 17 nmol/L at 100 µL/well and allowed to stand at room temperature for 90 minutes. The antigen ELISA plate was then blocked with 3% BSA/PBS to obtain an antigen ELISA plate.

After the blocking solution was removed from the antigen ELISA plate, a diluent of the anti-gpNMB antibody (3% BSA/PBS solution) was added at 50 µL/well and allowed to react for 1 hour at room temperature. Then, after washing with washing buffer, an antibody that specifically reacts with mouse IgG, i.e., an anti-mouse IgG antibody-ALP conjugate (SBA, 1050-04), was added at 50 µL/well and allowed to react for 1 hour at room temperature. Substrate (PNPP) was added at 100 µL/well and reacted at room temperature for 1 hour, and the absorbance at 405 to 550 nm was calculated and evaluated as binding activity. The results are shown in Table 6 (As mentioned above, in the amino acid sequence shown in SEQ ID NO 215, the amino acid residues at positions 1 to 21 constitute a signal peptide, while the amino acid residues at positions 22 and onward constitute an isolated peptide of the human gpNMB extracellular domain. Accordingly, each mutant protein shown in Table 6 starts at amino acid residue at position 22.). The binding activity to the C-terminal deletion mutant (hgpNMB_d19_22-234) is set as 100%, mutants that showed an absorbance of 50% or less are marked as +, and those that showed an absorbance of 50% or more are marked as -.

**[Table 6]**

| Table 6: Results of ELISA binding analysis of each anti-gpNMB antibody clone to human gpNMB extracellular domain variants | | | | | |
|---|---|---|---|---|---|
| gpNMB variant protein | GPN 18-1 | GPN 18-2 | GPN 18-5 | GPN 18-6 | GPN 18-7 |
| 22-234_N terminal | - | - | - | - | - |
| 22-234_R77A | - | - | - | - | - |
| 22-234_D85A | - | - | + | - | - |
| 22-234_R104A | - | - | - | - | - |
| 22-234_D109A | - | - | - | - | - |
| 22-234_E117A,K118A | - | - | - | - | - |
| 22-234_R121A,E123A | - | - | - | - | - |
| 22-234_D129A | - | - | - | - | - |
| 22-234_E140A,D141A | - | - | - | - | - |
| 22-234_D143A,E145A | - | - | - | - | - |
| 22-234_H152A,H153A | - | - | - | - | - |
| 22-234_D158A,K160A | - | - | - | - | - |
| 22-234_H164A,H165A | - | - | - | - | - |
| 22-234_R169A | - | - | - | - | - |
| 22-234_K186A | - | - | - | + | - |
| 22-234_R189A | - | - | - | - | - |
| 22-234_R193A | - | - | - | - | - |
| 22-234_R214A,R215A | + | + | - | + | + |
| 22-234_H216A, R218A | + | + | - | - | + |

The epitope of each anti-gpNMB antibody was determined by considering the stability of the purified gpNMB mutant and the mutation sites that affected the binding.

The results showed that antibody clones GPN18-1, GPN18-2, GPN18-6, and GPN18-7 interact with amino acids present in the PMEL-CAF-like region (R214 and/or R215). On the other hand, antibody clone GPN18-5 was found to further recognize the N-terminal portion. Antibody clone 1-5E is an anti-gpNMB antibody obtained by immunization with the vaccine peptide identified in a previous report (Non-Patent Literature 36), and the epitope recognized by this antibody is "RRGDGRWKD" at amino acid residues 63 to 71 at the N-terminus.

### [Example 6] Production of recombinant anti-gpNMB antibodies:

Plasmids were prepared by adding a gene sequence encoding a signal sequence to the 5' end of each of a gene sequence encoding the H chain and a gene sequence encoding the L chain of the anti-gpNMB antibody obtained in Example 2, and inserting each of the gene sequences into the animal cell expression vector pcDNA3.4. The prepared plasmids were then transiently expressed using the ExpiCHO Expression System (A29133, Thermo Fisher Scientific) such that the antibodies were secreted into the medium. The culture supernatant was then collected and purified using a Protein A column (Ab-Capture, Protenova) and a gel filtration column (Superdex 200 Increase, Cytiva).

### [Example 7(1)] Interaction analysis of the anti-gpNMB antibodies by surface plasmon resonance method - 1

To compare the binding properties (binding and dissociation kinetics) of each anti-gpNMB antibody clone obtained in Example 1 to gpNMB, surface plasmon resonance (SPR) measurement was performed. Specifically, the following conditions were used.

The BIACORE T200 system was used as the measurement system. An anti-His tag monoclonal antibody was fixed at around 5000 RU in all flow cells of the sensor chip CM5 (BR-1005-30, Cytiva) with the Amine Coupling Kit (BR-1000-50, GE) and His Capture Kit (28 -9950-56, Cytiva). The running buffer used was HBS-EP+ (BR-1006-69, Cytiva).

Recombinant human gpNMB-FLAG-His (SEQ ID NO 221) or recombinant mouse gpNMB-FLAG-His (SEQ ID NO 223) was captured in the measurement system and used as the ligand. Each anti-gpNMB antibody clone was used at a concentration of 100 nmol/L as an analyte. Shiga toxin 2 (Shiga toxin 2) antibody (11E10) or control mouse IgG1 (leinco: Pro# M1411) was used as an analyte negative control.

The temperature of the measurement system was set at 25°C. As the ligand, recombinant human gpNMB-FLAG-His was reacted with the anti-His monoclonal antibody in the flow cell (2) so as to achieve 100 RU or lower, and recombinant mouse gpNMB-FLAG-His was reacted with the anti-His monoclonal antibody in the flow cell (4) so as to achieve 100 RU or lower. The flow rate was set to 20 µL/min, and 10 nmol/L of purified mouse IgG2a, κ, Isotype Ctrl, Clone: MG2a-53 (401502, BioLegend, hereinafter ctrl IgG2a) was reacted for 1 min, and HBS-EP+ was flowed for at least 10 minutes. The analyte was diluted with HBS-EP+ (100 nmol/L) and reacted with all flow cells for 600 seconds each to obtain a binding curve, and then reacted with HBS-EP+ for 600 seconds to obtain a dissociation curve.

After the reaction, regeneration buffer 1 (0.2% SDS), regeneration buffer 2 (100 mmol/L Tris-HCl (pH 8.5), 1 mol/L NaCl, 15 mmol/L MgCl2), and regeneration buffer 3 (10 mmol/L glycine -HCl (pH 1.5)) were reacted for 1 minute each to remove and wash gpNMB-FLAG-His in the measurement system. Biacore T200 Evaluation software (ver. 2.0) was used to analyze the data with the 1:1 Binding Model, and the binding rate value (Ass: 1/Ms), binding stability value (Diss: 1/s) and equilibrium dissociation value (Diss/Ass: M) were quantified as ordinal numbers. The results are shown in Table 7.

**[Table 7]**

| Table 7: Results of SPR interaction analysis of each anti-gpNMB antibody clone against human gpNMB and mouse gpNMB - 1 | | | | | | | |
|---|---|---|---|---|---|---|---|
| Antibody clone | | Human gpNMB | | | Mouse gpNMB | | |
| Sample | Recognized domain | Ass. (1/Ms) | Diss (1/s) | Diss/Ass (M) | Ass (1/Ms) | Diss (1/s) | Diss/Ass (M) |
| GPN05-1 | PKD | 4.8.E+04 | 1.6.E-04 | 3.3.E-09 | 6.1. E+04 | 1.3.E-03 | 2.0.E-08 |
| GPN06-1 | PKD | 3.4.E+05 | 4.9.E-04 | 1.4.E-09 | 1.5.E+05 | 1.8.E-03 | 1.2.E-08 |
| GPN09-1 | PKD | - | - | - | 1.6.E+05 | 4.2.E-04 | 2.6.E-09 |
| GPN11-10 | PKD | 8.1.E+04 | 1.0.E-03 | 1.3.E-08 | 7.5.E+04 | 3.7.E-04 | 4.9.E-09 |
| GPN15-2 | PKD | 1.7.E+05 | 6.0.E-07 | 3.5.E-12 | 1.4.E+05 | 2.8.E-04 | 2.0.E-09 |
| GPN15-3 | PKD | 1.3.E+05 | 3.7.E-04 | 2.9.E-09 | 1.3.E+05 | 3.6.E-04 | 2.9.E-09 |
| GPN18-4 | PKD | 4.7.E+04 | 1.5.E-05 | 3.2.E-10 | 3.0.E+04 | 1.2.E-04 | 3.8.E-09 |
| GPN18-1 | PMEL -CAF-like | 3.8.E+04 | 5.4.E-04 | 1.4.E-08 | 3.8.E+04 | 9.1.E-04 | 2.4.E-08 |
| GPN18-2 | PMEL -CAF-like | 3.1.E+04 | 6.8.E-05 | 2.2.E-09 | 2.4.E+04 | 7.9.E-05 | 3.3.E-09 |
| GPN18-6 | PMEL -CAF-like | 2.7.E+04 | 6.5.E-04 | 2.4.E-08 | 3.2.E+04 | 9.8.E-04 | 3.1.E-08 |
| GPN18-7 | PMEL -CAF-like | 2.6.E+04 | 5.9.E-04 | 2.3.E-08 | 2.0.E+04 | 9.9.E-04 | 4.9.E-08 |
| GPN18-5 | N-frg. | 1.5.E+05 | 3.0.E-04 | 2.0.E-09 | 9.1.E+04 | 7.0.E-04 | 7.7.E-09 |
| 1-5E | N-terminal vaccine | - | - | - | 6.7.E+05 | 6.1.E-03 | 9.1.E-09 |

### [Example 7(2)] Interaction analysis of the anti-gpNMB antibodies by surface plasmon resonance method - 2

Two of the anti-gpNMB antibody clones obtained in Example 1, GPN05-1 and GPN06-1, were subjected to measurement for concentration-dependent binding properties (binding and dissociation kinetics) to gpNMB by surface plasmon resonance (SPR) for investigation. Specifically, the same conditions as in Example 7(1) were used except for the conditions described below.

The sensor chip CM3 (BR-1005-36, Cytiva) was used, and anti-gpNMB antibody clones GPN05-1 and GPN06-1 were used as analytes at various concentrations each. The measurement conditions were determined in accordance with the single-cycle kinetics method. The analyte at each concentration was reacted for 450 seconds to obtain a binding curve, and HBS-EP+ was reacted for 600 seconds to obtain a dissociation curve. After the reaction, regeneration buffer 1 (0.2% SDS), regeneration buffer 2 (100 mmol/L Tris-HCl (pH 8.5), 1 mol/L NaCl, 15 mmol/L MgCl2), and regeneration buffer 3 (10 mmol/L glycine -HCl (pH 1.5)) were reacted for 1 minute each to remove and wash gpNMB-FLAG-His in the measurement system. Biacore T200 Evaluation software (ver. 2.0) was used to analyze the data with the 1:1 Binding Model, and the binding rate value (Ass: 1/Ms), binding stability value (Diss: 1/s) and equilibrium dissociation value (Diss/Ass: M) were calculated. The results are shown in Table 8.

**[Table 8]**

| Table 8: Results of SPR interaction analysis of each anti-gpNMB antibody clone against human gpNMB and mouse gpNMB - 2 | | | | |
|---|---|---|---|---|
| Ligand | Clone | ka (1/Ms) | kd (1/s) | KD (M) |
| Human gpNMB | GPN05-1 | 9.69E+04 | 3.55E-05 | 3.66E-10 |
| | GPN06-1 | 5.19E+05 | 2.53E-04 | 4.88E-10 |
| Mouse gpNMB | GPN05-1 | 3.61E+04 | 3.07E-04 | 8.50E-09 |
| | GPN06-1 | 1.74E+05 | 8.79E-04 | 5.05E-09 |

### [Example 8] Binding analysis of anti-gpNMB antibody (commercial polyclonal antibody) in Alzheimer's disease model mice (APPosk mice)

APPosk mice aged 21 to 25 months were reflux-fixed, brains were excised, and 5-µm-thick paraffin sections were prepared. After deparaffinization, the brain sections were boiled in citrate buffer (pH 6) for 30 minutes to activate the antigen, and then double-stained with commercially available anti-gpNMB polyclonal antibody AF2330 (2 µg/mL) + FITC-donkey anti-goat IgG antibody (Jackson Lab) and Iba1 antibody (WAKO, 5 µg/mL) + Rhodamine-donkey anti-rabbit IgG antibody (Jackson Lab). After treatment with lipofuscin quencher TrueBlackplus (Biotium) for 10 minutes at room temperature, the sections were encapsulated with DAPI-containing encapsulant (Vector).

The results showed that the brain sections from the APPosk mice aged 21 to 25 months included cells stained with the anti-gpNMB antibody (AF2330, R&D) among Iba1 - positive microglia, and that almost all gpNMB-positive cells were Iba1-positive microglia. The results also showed that the microglia that underwent morphological changes were gpNMB-positive microglia. These findings confirm that gpNMB-positive microglia appear in the Alzheimer's disease model mice (APPosk mice).

### [Example 9] Reduction of gpNMB-positive microglia by administration of anti-gpNMB antibodies (GPN05-1 and GPN06-1) to Alzheimer's disease model mice (APPosk mice)

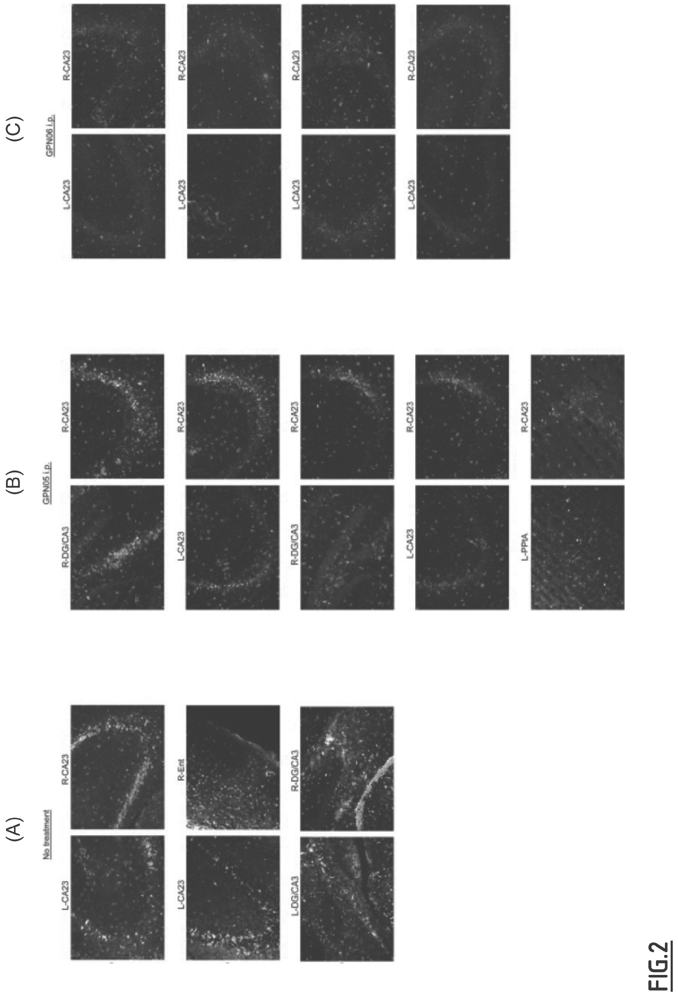

APPosk mice aged 19 to 20 months were divided into two groups (two males and three females in each group), and each group was intraperitoneally injected with a different anti-gpNMB antibody clone obtained in Example 1 (GPN05-1 or GPN06-1) once a week for a total of five times (1 mg/400 µL in PBS each time). Four days after the last dose, mice were reflux-fixed, brains were excised, and 5-µm-thick paraffin sections were prepared. After deparaffinization, the brain sections were boiled in citrate buffer (pH 6) for 30 minutes to activate the antigen, and then double-stained with anti-mouse gpNMB goat polyclonal antibody (R&D systems, #AF2330, 2 µg/mL) + FITC-donkey anti-goat IgG antibody (Jackson Lab) and Iba1 antibody (WAKO, 5 µg/mL) + Rhodamine-donkey anti-rabbit IgG antibody (Jackson Lab). After treatment with lipofuscin quencher TrueBlackplus (Biotium) for 10 minutes at room temperature, the sections were encapsulated with DAPI-containing encapsulant (Vector). One male in the GPN06-1 group died the week after the fourth dose. As controls, brain sections from APPosk mice aged 21 to 25 months (3 mice) were stained in the same manner. The resulting immunostained photographs are shown in Figure 2. Figure 2(A) shows the control, Figure 2(B) shows the GPN05-1-treated group, and Figure 2(C) shows the GPN06-1-treated group.

The results showed that the control APPosk mice had numerous gpNMB-positive microglia (Iba1-positive) detected with the anti-mouse gpNMB goat polyclonal antibody in the hippocampus (CA2-CA3), entorhinal cortex (Ent) and dentate gyrus (DG). On the other hand, gpNMB-positive microglia were reduced in the GPN05-1-treated group, and were almost absent in the GPN06-1-treated group. These results confirm that administration of the anti-gpNMB monoclonal antibodies GPN05-1 and GPN06-1 reduce gpNMB-positive microglia in the brain.

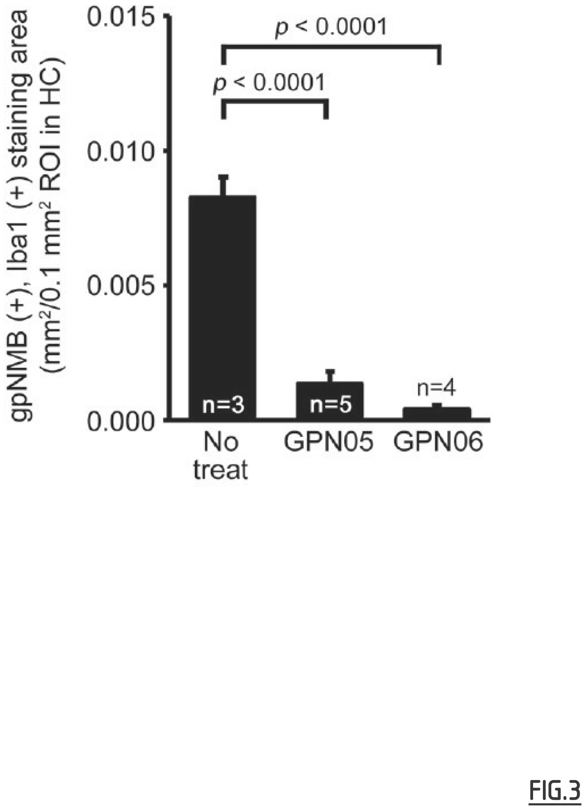

In addition, statistical analysis was performed on the above immunostaining results. Specifically, the staining signal of Iba1 and the co-staining signal of gpNMB and Iba1 were extracted, and the area of each staining region was quantified using ImageJ image processing software. The values obtained were made into a graph as shown in Figure 3. The significant difference test was performed by ANOVA between the three groups using StatView, and only those results that were below the p-value of 0.05 by Fisher's PLSD were considered "significantly different," and their p-values are indicated in the graph.

These analysis results confirm that administration of the anti-gpNMB monoclonal antibodies GPN05-1 and GPN06-1 reduced gpNMB-positive microglia in the brain with statistically significant differences. This means that mal-functional microglia, which account for at least part of gpNMB-positive microglia, were reduced. Such reduction of mal-functional microglia is also supported by the removal of Aβ oligomers as shown in Example 10 and the restoration of the number of synapses as shown in Example 11.

### [Example 101 Removal of Aβ oligomers by administration of anti-gpNMB antibodies (GPN05-1 and GPN06-1) to Alzheimer's disease model mice (APPosk mice)

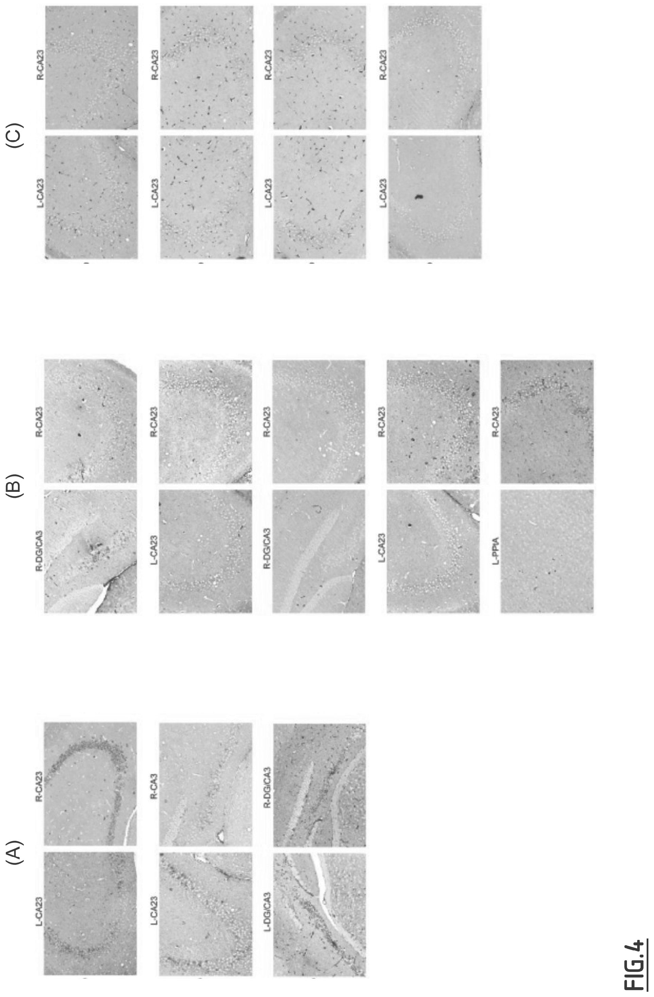

APPosk mice aged 19 to 20 months were divided into two groups (two males and three females in each group), and each group was intraperitoneally administered a different anti-gpNMB antibody obtained in Example 1 (GPN05-1 or GPN06-1) once a week for a total of five times (1 mg/400 µL in PBS each time). Four days after the last dose, the mice were reflux-fixed, brains were excised, and 5-µm-thick paraffin sections were prepared. After deparaffinization, the brain sections were boiled in hydrochloric acid (pH 2) for 10 minutes, and stained with Aβ oligomeric antibody 11A1 (IBL, 1 µg/mL) + Biotin-horse anti-mouse IgG antibody (Vector) + ABC elite (Vector) + DAB. As controls, brain sections from APPosk mice aged 21 to 25 months (3 mice) were stained in the same manner. The resulting immunostained photographs are shown in Figure 4. Figure 4(A) shows the control, Figure 4(B) shows the GPN05-1-treated group, and Figure 4(C) shows the GPN06-1-treated group.

The results showed that Aβ oligomer-positive cells were found in the hippocampus and dentate gyrus in the control APPosk mice. On the other hand, 11A1-positive cells were reduced in the GPN05-1-treated group, and were almost absent in the GPN06-1-treated group. These results confirm that the administration of the anti-gpNMB antibodies removed Aβ oligomers in the brain.

### [Example 111 Restoration of synapses by administration of anti-gpNMB antibodies (GPN05-1 and GPN06-1) to Alzheimer's disease model mice (APPosk mice)

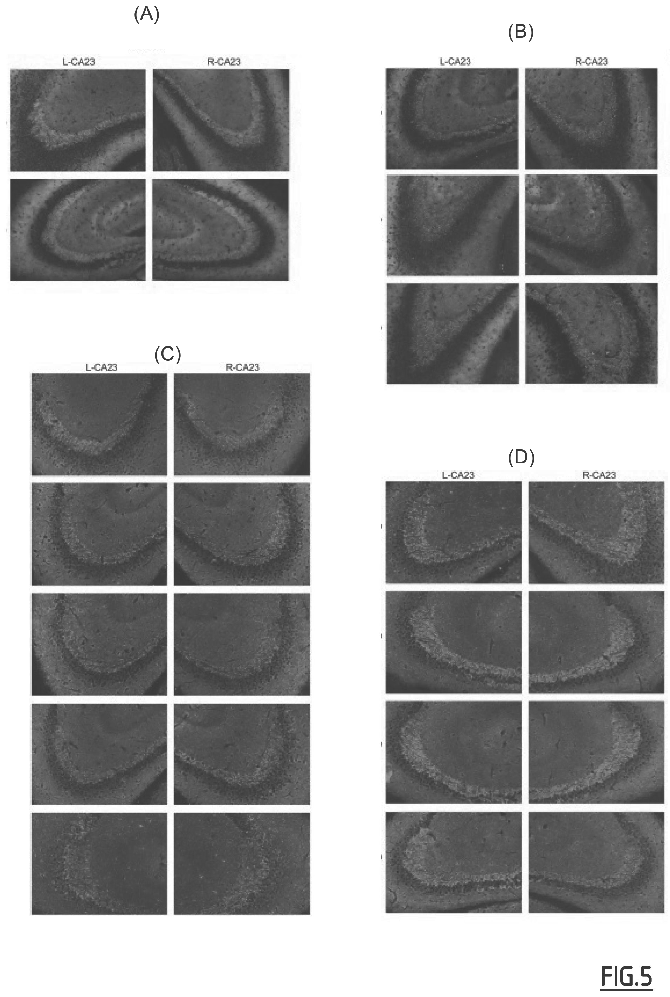

APPosk mice aged 19 to 20 months were divided into two groups (two males and three females in each group), and each group was intraperitoneally administered a different anti-gpNMB antibody obtained in Example 1 (GPN05-1 or GPN06-1) once a week for a total of five times (1 mg/400 µL in PBS each time). Four days after the last dose, the mice were reflux-fixed, brains were excised, and 5-µm-thick paraffin sections were prepared. After deparaffinization, the brain sections were boiled in citrate buffer (pH 6) for 30 minutes, and stained with synaptophysin antibody SVP-38 (Sigma, 200x dilution) + FITC-goat anti-mouse IgG antibody (Jackson Lab). Brain sections from 20-month-old non-transgenic (Non-Tg) mice (2 mice) as Control 1 and brain sections from APPosk mice aged 21 to 25 months (3 mice) as Control 2 were stained in the same manner. The resulting immunostained photographs are shown in Figure 5. Figure 5(A) shows the Control 1 group (Non-Tg mice), Figure 5(B) shows the Control 2 group (APPosk mice), Figure 5(C) shows the GPN05-1-treated group, and Figure 5(D) shows the GPN06-1-treated group.

The results showed that synaptophysin in hippocampal mossy fibers in the Control 2 APPosk mice decreased compared to the Control 1 non-transgenic (Non-Tg) mice. The synaptophysin level recovered slightly in the GPN05-1-treated group and markedly in the GPN06-1-treated group. The recovery of synaptophysin appeared to correlate with the removal of Aβ oligomers. These results suggest that the administration of the anti-gpNMB antibodies increased the number of synapses and restored neural functions in the brains of Alzheimer's disease model mice.

### [Example 12-11 Evaluation of drug efficacy of anti-gpNMB antibodies (GPN09-1, GPN11-10, GPN15-2, GPN15-3, GPN18-2, and 11E10) in Alzheimer's disease model mice (APPosk mice) (removal of Aβ oligomers)

APPosk mice aged 19-20 months were divided into 6 groups (4 mice in each group), and each group was intraperitoneally administered a different anti-gpNMB antibody obtained in Example 1 (GPN09-1, GPN11-10, GPN15-2, GPN15-3, or GPN18-2) or control antibody 11E10 (anti-Shiga toxin mouse monoclonal antibody), once weekly for a total of 5 times (1 mg/400 µL in PBS for each dose). Four days after the last dose, the mice were reflux-fixed, brains were excised, and 5-µm-thick paraffin sections were prepared. After deparaffinization, the brain sections were boiled in hydrochloric acid (pH 2) for 10 minutes, and stained with Aβ oligomeric antibody 11A1 (IBL, 1 µg/mL) + Biotin-horse anti-mouse IgG antibody (Vector) + ABC elite (Vector) + DAB.

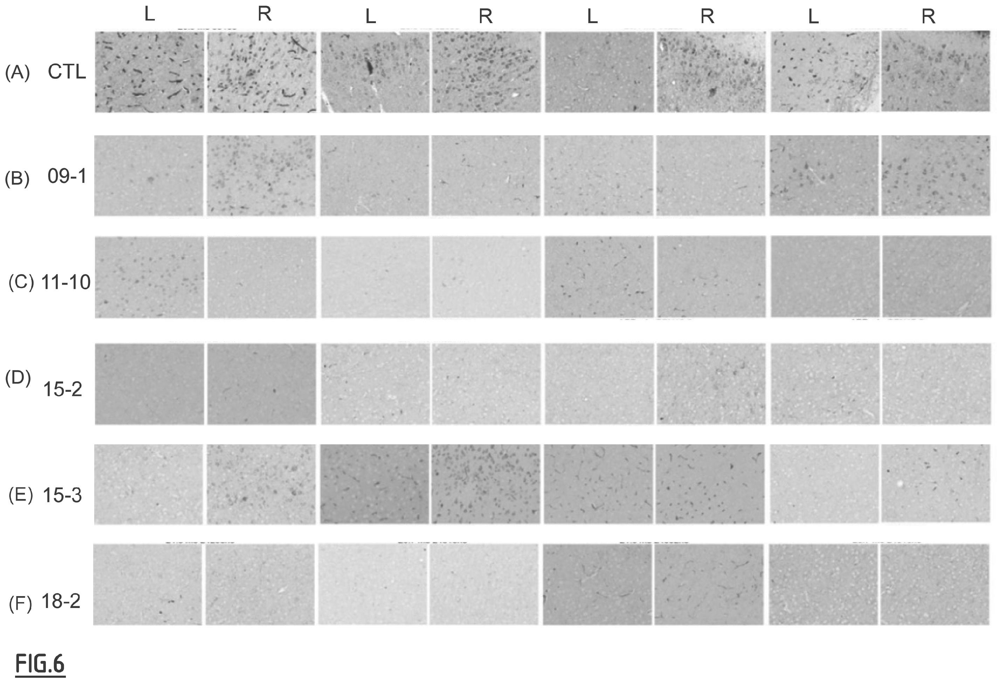

The obtained immunostained photographs are shown in Figure 6. Figure 6(A) shows the Control antibody 11E10-treated group, Figure 6(B) shows the GPN09-1-treated group, Figure 6(C) shows the GPN11-10-treated group, Figure 6(D) shows the GPN15-2-treated group, Figure 6(E) shows the GPN15-3-treated group, and Figure 6(F) shows the GPN18 - 2 treated group.

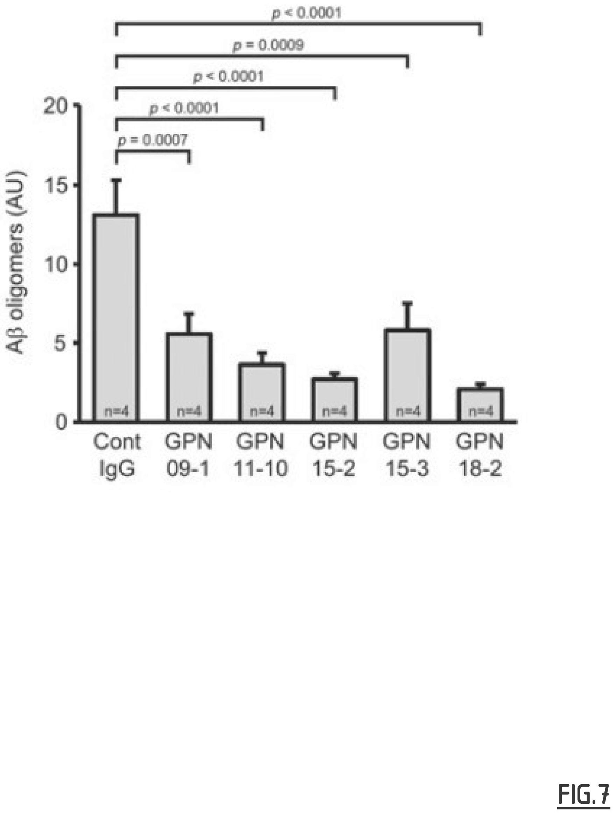

In addition, the intracellular Aβ oligomer staining was quantified for the immunostained photographs in Figure 6, and significance difference test was carried out by ANOVA for the three groups using StatView. The results are shown in the graph in Figure 7. Only the data with p-values below 0.05 by Fisher's PLSD were considered "significantly different," and their p-values are indicated in the graph. The results showed that the APPosk mice in the control antibody-treated group showed 11A1 staining-positive cells in the cerebral cortex. On the other hand, the number of cells positive for 11A1 staining decreased in the GPN09-1-, GPN11-10-, GPN15-2-, and GPN15-3-treated groups, and was almost absent in the GPN18-2-treated group. These results confirm that the administration of the anti-gpNMB antibodies removed Aβ oligomers in the brain.

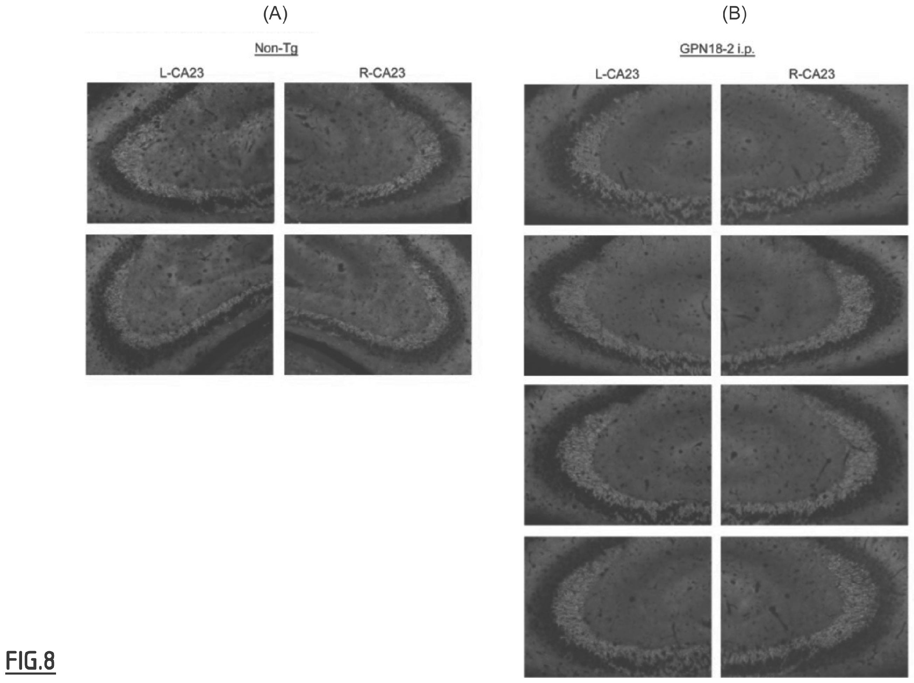

In addition, hippocampal sections from the GPN18-2-treated APPosk mice and those from Non-Tg mice around the same age were subjected to synaptophysin staining according to the method of Example 11. The resulting stained photographs are shown in Figure 8. Figure 8(A) and Figure 8(B) show the control (Non-Tg mice) group and the GPN18-2-treated group, respectively. The results show that the number of synapses in the hippocampus of the GPN18-2-treated APPosk mice was restored to the Non-Tg level.

### [Examples 12-2] Evaluation of drug efficacy of anti-gpNMB antibodies (GPN18-1, GPN18-6, GPN18-7) in Alzheimer's disease model mice (APPosk mice) (removal of Aβ oligomer):

APPosk mice aged 18-20 months were divided into 3 groups (5 mice in each group), and each group was intraperitoneally administered a different anti-gpNMB antibody obtained in Example 1 (GPN18-1, GPN18-6, GPN18-7) once weekly for a total of 5 times (1 mg/400 µL in PBS for each dose). Four days after the last dose, the mice were reflux-fixed, brains were excised, and 5-µm-thick paraffin sections were prepared. After deparaffinization, the brain sections were boiled in hydrochloric acid (pH 2) for 10 minutes, and stained with Aβ oligomeric antibody 11A1 (IBL, 1 µg/mL) + Biotin-horse anti-mouse IgG antibody (Vector) + ABC elite (Vector) + DAB.

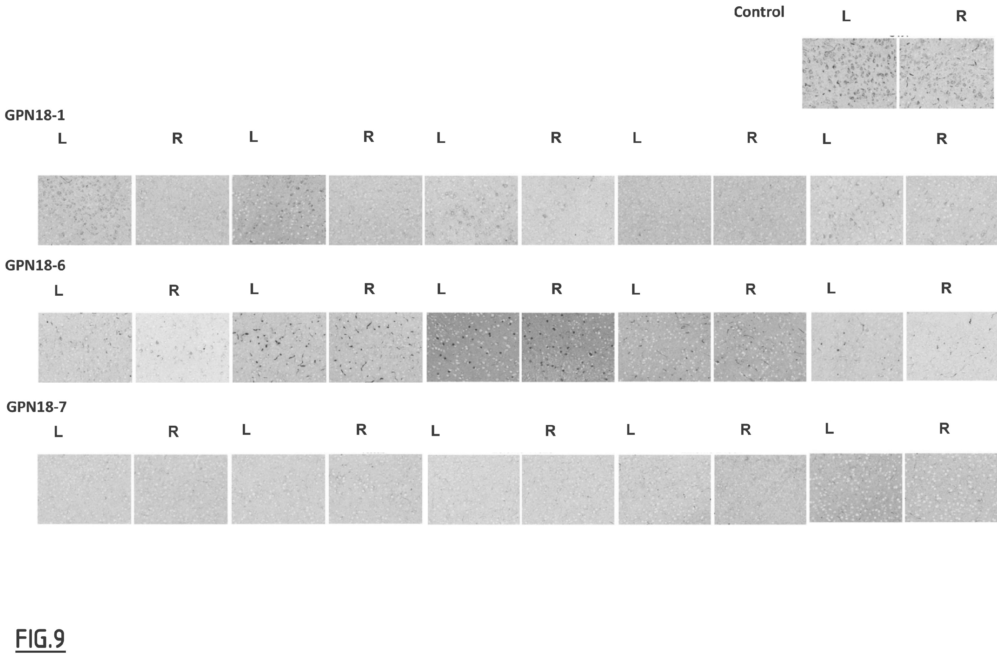

The obtained stained photographs are shown in Figure 9. Figure 9(A) shows the non-treated control of APPosk mice, Figure 9(B) shows the GPN18-1-treated group, Figure 9(C) shows the GPN18-6-treated group, and Figure 9(D) shows the GPN18-7-treated group.

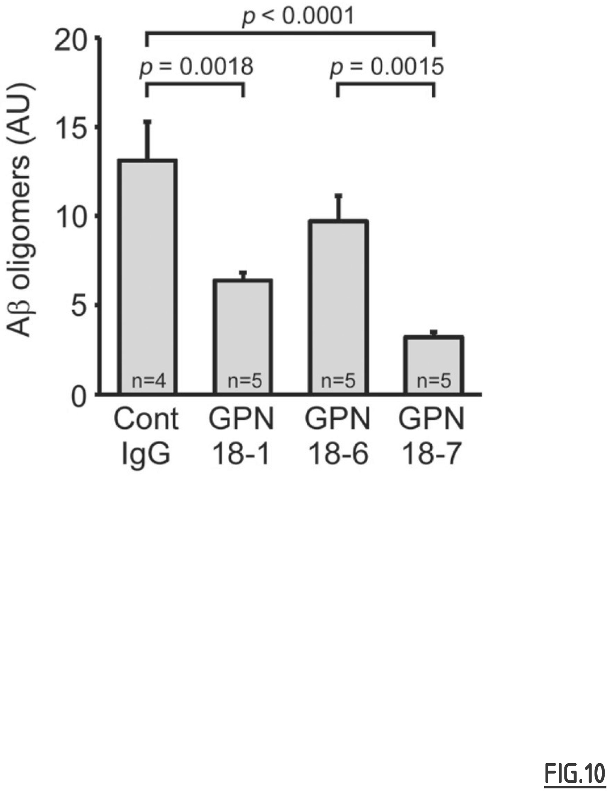

In addition, the intracellular Aβ oligomer staining was quantified for the immunostained photographs in Figure 9, and significance difference test was carried out by ANOVA for the three groups using StatView. The results are shown in the graph in Figure 10. Only the data with p-values below 0.05 by Fisher's PLSD were considered "significantly different," and their p-values are indicated in the graph. For statistical analysis, a "correction factor" was calculated for the non-treated control, assuming that the quantitative value obtained from the individual used in this experiment corresponds to the average of the data from the four animals in the control antibody treatment group in Example 12-1, and all quantitative values obtained in this experiment were multiplied by this factor to create a graph and perform a statistical test. In the graphs, the control is also shown with the data of the four animals in the control antibody group of the previous Example 12-1.

As a result, a large number of 11A1 staining-positive cells were observed in the cerebral cortex of the APPosk mice in the control antibody-treated group. In contrast, a statistically significant decrease in the 11A1-staining positive cells was observed in the GPN18-1 and GPN18-7-treated groups. A decreasing trend was also observed in the GPN18-6-treated group. These results indicate that GPN18-1, GPN18-6, and GPN18-7 antibodies, which recognize the PMEL-CAF-like region, have the same effect as GPN18-2 antibodies in removing A β oligomer in the brain.

### [Example 12-3] Evaluation of drug efficacy of anti-gpNMB antibodies (GPN18-2, GPN06-1, GPN18-5, 1-5E, and anti-BTV mouse monoclonal antibody) in Alzheimer's disease model mice (APPosk mice) (removal of Aβ oligomer)

APPosk mice aged 18 to 20 months were divided into 5 groups (5 mice per group), and each group was treated with a different anti-gpNMB antibody obtained in Example 1 (GPN18-2, GPN06-1, GPN18-5, or 1 -5E) or a control antibody (anti-BTV mouse monoclonal antibody, Leinco Technologies, Inc.) intraperitoneally once a week for a total of five times (1 mg/400 µL in PBS for each dose). Four days after the last dose, mice were reflux-fixed, brains were excised, and 5-µm-thick paraffin sections were prepared. After deparaffinization, the brain sections were boiled in hydrochloric acid (pH 2) for 10 minutes and stained with Aβ oligomeric antibody 11A1 (IBL, 1 µg/mL) + Biotin-horse anti-mouse IgG antibody (Vector) + ABC elite (Vector) + DAB.

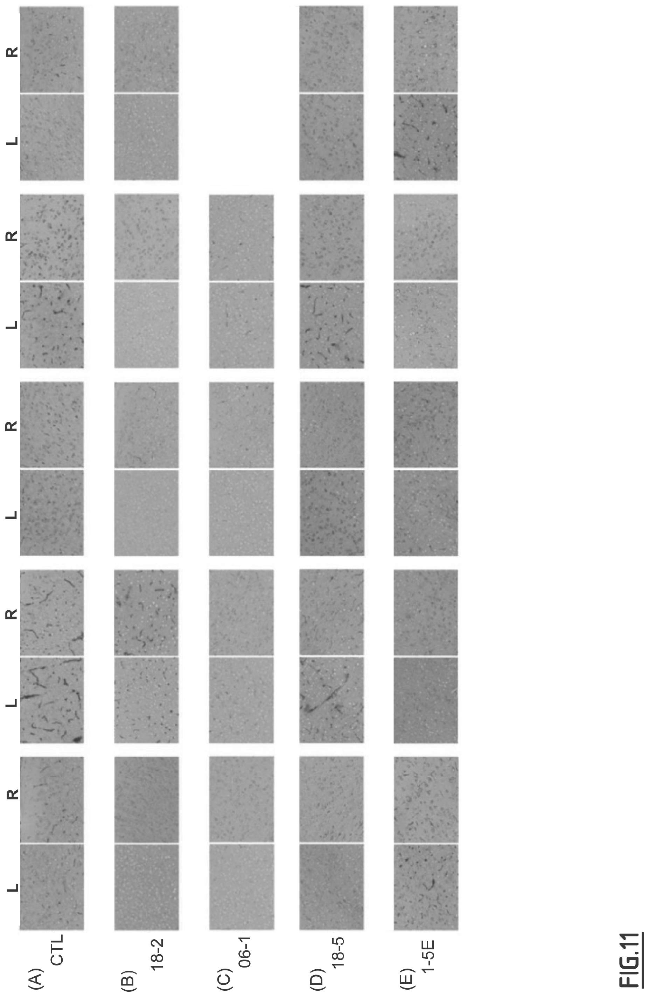

The obtained immunostained photographs are shown in Figure 11. Figure 11(A) shows the Control antibody-treated group, Figure 11(B) shows the GPN18-2-treated group, Figure 11(C) shows the GPN06-1-treated group, Figure 11(D) shows the GPN18-5-treated group, and Figure 11(E) shows the 1-5E-treated group.

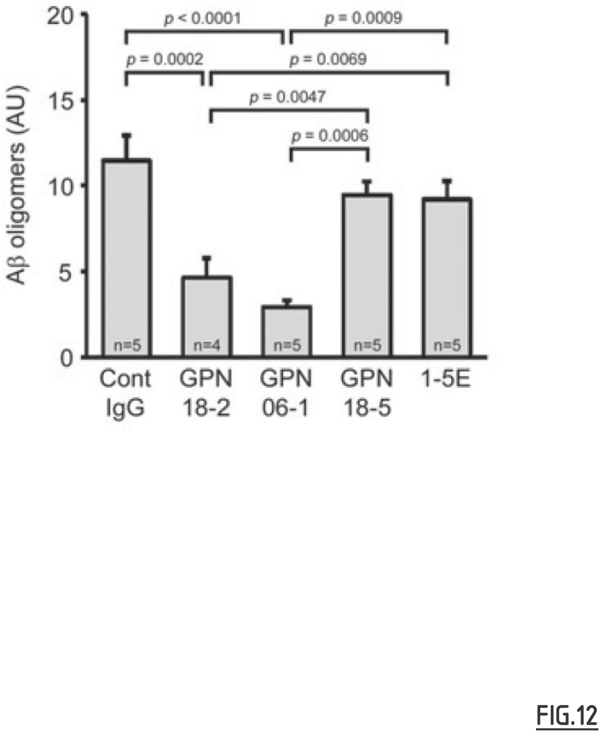

In addition, intracellular Aβ oligomer staining was quantified for the immunostained photographs in Figure 11, and significance difference test was carried out by ANOVA for the three groups using StatView. The results are shown in the graph in Figure 12. Only the data with p-values below 0.05 by Fisher's PLSD were considered "significantly different," and their p-values are indicated in the graph.

The results show that a large number of 11A1 staining-positive cells were observed in the cerebral cortex of the APPosk mice in the control antibody-treated group. On the other hand, the GPN18-2- and GPN06-1-treated groups showed a statistically significant decrease in 11A1 staining-positive cells. The decrease was statistically significant even compared to the GPN18-5- and 1-5E-treated groups. These results indicate that the GPN18-2 antibody, which recognizes the PMEL-CAF-like region, and the GPN06-1 antibody, which recognizes the PKD region, have stronger medicinal effects in removing Aβ oligomers in the brain than the GPN18-5 antibody, which recognizes the N-terminal region, and the 1-5E antibody, which recognizes the vaccine peptide in the N-terminal region.

These comparative experiments showed that the antibodies recognizing the PMEL-CAF-like and PKD regions were more effective in removing Aβ oligomers in the brain than the antibodies recognizing the N-terminal region.

### [Reference Example 1] Production of novel Alzheimer's disease model mice:

A novel mouse model of Alzheimer's disease (Tg2576/Tau264 mice (APP/Tau-Tg)) was produced by crossing Tg2576 mice (Swedish mutant APP transgenic mice), which exhibit amyloid plaques, with Tau264 mice, which expresses human tau (wild type).

Pathological analysis in 9-month-old APP/Tau-Tg showed amyloid plaque formation as detected by an anti-Aβ antibody and accumulation of phosphorylated tau as detected by an AT8 antibody. Appearance of gpNMB-positive microglia was also confirmed by an anti-gpNMB antibody (R & D, goat polyclonal antibody) and an Iba1 antibody, which detects microglia.

### [Example 13] Evaluation of cognitive function improvement by the anti-gpNMB antibody in Alzheimer's disease model mice (APP/Tau-Tg)

The Alzheimer's disease model mice APP/Tau-Tg aged 14 months described in Reference Example were used for analyzing the cognitive function-improving effect of the anti-gpNMB antibody by Morris water maze test. 14-month-old littermate non-Tg mice (wild-type mice) were used as controls for comparison.

### <Group configuration>

*Evaluation antibody group 1: Anti-gpNMB mouse monoclonal antibody GPN06-1, 2.5 mg/mL in 150mM NaCl buffer (pH6.5) containing 0.02M histidine, was administered intraperitoneally at 0.4 mL once weekly for a total of 5 times (n=8).
*Evaluation antibody group 2: Anti-gpNMB mouse monoclonal antibody GPN18-2, 2.5 mg/mL in 150mM NaCl buffer (pH6.5) containing 0.02M histidine, was administered intraperitoneally at 0.4 mL once weekly for a total of 5 times (n=7).
*Control antibody group: An anti-BTV mouse monoclonal antibody (Leinco Technologies, Inc.), 2.5 mg/mL in 150mM NaCl buffer (pH6.5) containing 0.02M histidine, was administered intraperitoneally at 0.4 mL once weekly for a total of 5 times (n=8).
*Non-Tg group: 150mM NaCl buffer (pH6.5) containing 0.02M histidine, was administered intraperitoneally at 0.4 mL once weekly for a total of 5 times (n=12).

### <Water maze test>

*Preparation: A black pool with an inner diameter of 100 cm and a height of 45 cm was filled with water 16 cm deep. The water temperature was adjusted to 21 to 23°C, and the water was kept clear and colorless. After each trial day, feces were removed, and approximately 10 liters of water was replaced.
*Acquisition test: A 15 cm high transparent platform was sunk 20 cm from the wall (30 cm from the center). The pool was divided into four quadrants, including the area where the platform was sunk, and mice were randomly placed only in one of the three quadrants without the platform. Each trial was limited to 60 seconds and was conducted at a rate of 5 trials/day, with an interval of approximately 5 minutes between trials. The escape time to reach the platform (escape time) was recorded as data. Mice that did not escape within 60 seconds were guided to the platform by an operator, and the escape time was recorded as 60 seconds. Mice on the platform were removed from the pool after 10 seconds, and allowed to act freely and dry their bodies until the next trial. The average seconds of escape time for five trials was used for the daily mouse performance. Trials were conducted for four days.

The acquisition test was finished when the performance of the control group (Non-Tg) stabilized, and a probe test was conducted the next day.
*Probe test: The day after the final day of the acquisition test, the platform was removed from the pool, and a 60-second free swim of each mouse was captured by video camera. The time that the free-swimming mouse spent swimming in the target quadrant of the four quadrants was measured and expressed as a percentage of the 60-second period.

Significant differences for each test were determined using repeated measure and Fisher's least significant difference test (Fisher's PLSD), and significant differences for the probe test were determined using Fisher's least significant difference test (Fisher's PLSD).

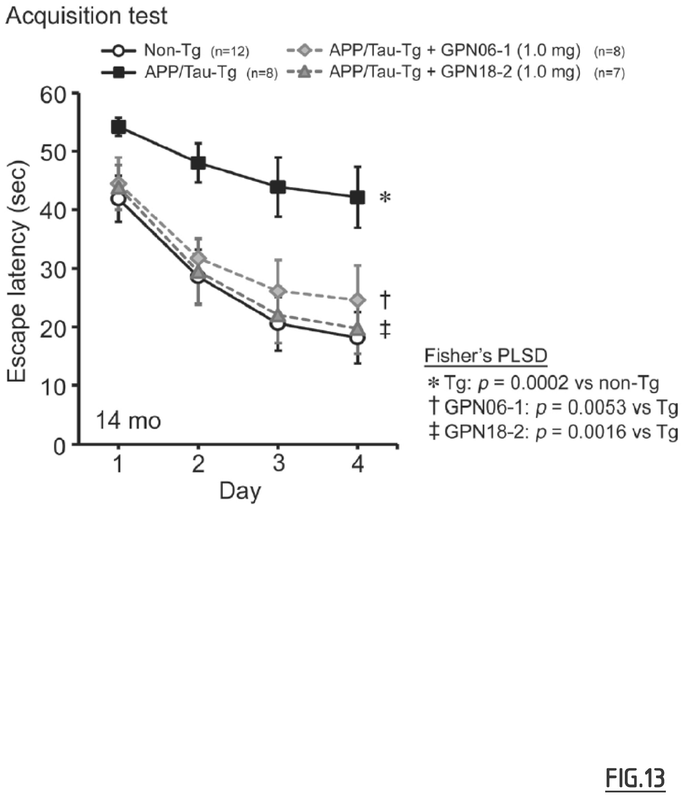

The results of the Acquisition test are shown in the graph in Figure 13. The analysis showed that the control antibody group to APP/Tau-Tg had a significantly longer Escape Latency in the Morris water maze test compared to Non-Tg (wild type). On the other hand, the APP/Tau-Tg group that received anti-gpNMB antibody GPN06-1 or GPN18-2 showed a statistically significant reduction in the time to reach the hidden platform.

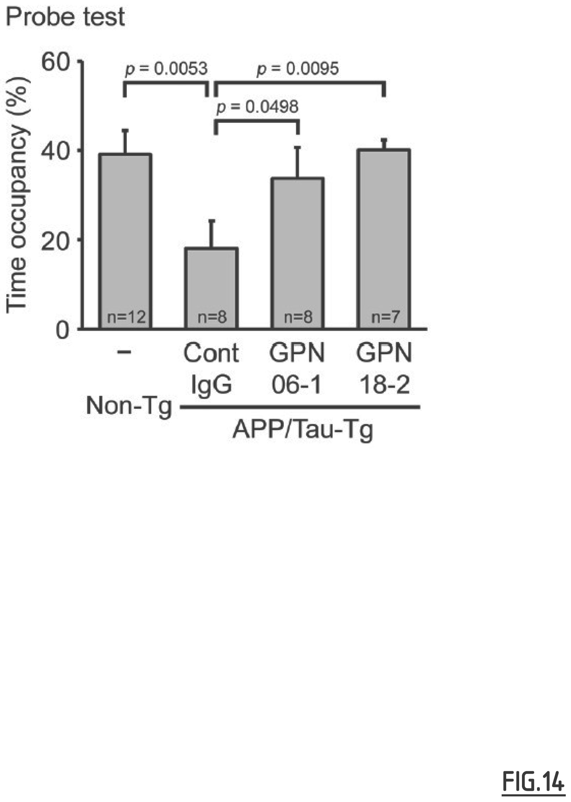

The results of the probe test are shown in the graph in Figure 14. The results of the probe test were similar to those of the acquisition test. Specifically, in the APP/Tau-Tg group treated with the control antibody, the mice spent statistically significantly less time swimming around the quadrant where the hidden platform was present. The mice in the groups treated with GPN06-1 or GPN18-2 spent statistically significantly more time swimming around the quadrant where the hidden platform was located than the control antibody-treated group.

These results indicate that the anti-gpNMB antibodies GPN06-1 and GPN18-2 inhibit or restore declines in cognitive functions and motor functions in APP/Tau-Tg mice with statistically significant differences.

### [Example 14] Dose-dependent and pathological analysis of anti-gpNMB antibodies in improving cognitive function in novel Alzheimer's disease model mice (APP/Tau-Tg)

Fourteen-month-old APP/Tau-Tg mice, the Alzheimer's disease model described in Example 13, were used to analyze the dose-dependency of the anti-gpNMB antibodies of the invention in improving cognitive function by the Morris water maze test. 14-month-old non-Tg (wild-type mice) born in the same abdomen were used as comparison subjects.

### <Group configuration>

*Evaluation antibody group 1: Anti-gpNMB mouse monoclonal antibody GPN18-2, 0.25 mg/mL solution in 150mM NaCl buffer containing 0.02M histidine (pH6.5), was administered intraperitoneally at 0.4 mL once weekly for a total of 5 times (n=7).
*Evaluation antibody group 2: Anti-gpNMB mouse monoclonal antibody GPN18-2, 0.25 mg/mL solution in 150mM NaCl buffer containing 0.02M histidine (pH6.5), was administered intraperitoneally at 0.4 mL once weekly for a total of 5 times (n=7).
*Control antibody group: An anti-BTV mouse monoclonal antibody (Leinco Technologies, Inc.), 2.5 mg/mL solution in 150mM NaCl buffer containing 0.02M histidine (pH6.5), was administered intraperitoneally at 0.4 mL once weekly for a total of 5 times (n=6).
*Non-Tg group: 150mM NaCl buffer containing 0.02M histidine (pH6.5) was administered intraperitoneally at 0.4 mL once weekly for a total of 5 times (n=12).

### <Water maze test>

*Preparation: A black pool with an inner diameter of 100 cm and a height of 45 cm was filled with water 16 cm deep. The water temperature was adjusted to 21 to 23°C, and the water was kept clear and colorless. After each trial day, feces were removed, and approximately 10 liters of water was replaced.
*Acquisition test: A 15 cm high transparent platform was sunk 20 cm from the wall (30 cm from the center). The pool was divided into four quadrants, including the area where the platform was sunk, and mice were randomly placed only in one of the three quadrants without the platform. Each trial was limited to 60 seconds and was conducted at a rate of 5 trials/day, with an interval of approximately 5 minutes between trials. The escape time to reach the platform (escape time) was recorded as data. Mice that did not escape within 60 seconds were guided to the platform by an operator, and the escape time was recorded as 60 seconds. Mice on the platform were removed from the pool after 10 seconds, and allowed to act freely and dry their bodies until the next trial. The average seconds of escape time for five trials was used for the daily mouse performance. Trials were conducted for four days.

The acquisition test was finished when the performance of the control group (Non-Tg) stabilized, and a probe test was conducted the next day.
*Probe test: The day after the final day of the acquisition test, the platform was removed from the pool, and a 60-second free swim of each mouse was captured by video camera. The time that the free-swimming mouse spent swimming in the target quadrant of the four quadrants was measured and expressed as a percentage of the 60-second period.

Significant differences for each test were determined using repeated measure and Fisher's least significant difference test (Fisher's PLSD), and significant differences for the probe test were determined using Fisher's least significant difference test (Fisher's PLSD).

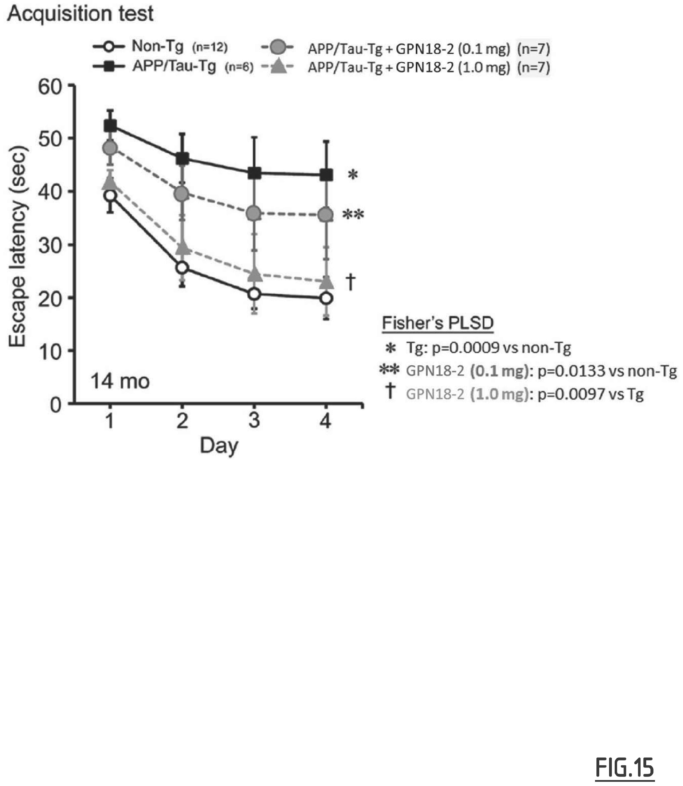

The results of the Acquisition test are shown in the graph in Figure 15. The analysis showed that the control antibody group to APP/Tau-Tg had a significantly longer Escape Latency in the Morris water maze test compared to Non-Tg (wild type). On the other hand, the APP/Tau-Tg group that received anti-gpNMB antibody GPN18-2 showed a statistically significant reduction in the time to reach the hidden platform. The group that received 0.1 mg/head of GPN18-2 also showed a trend toward shorter time to reach the hidden step.

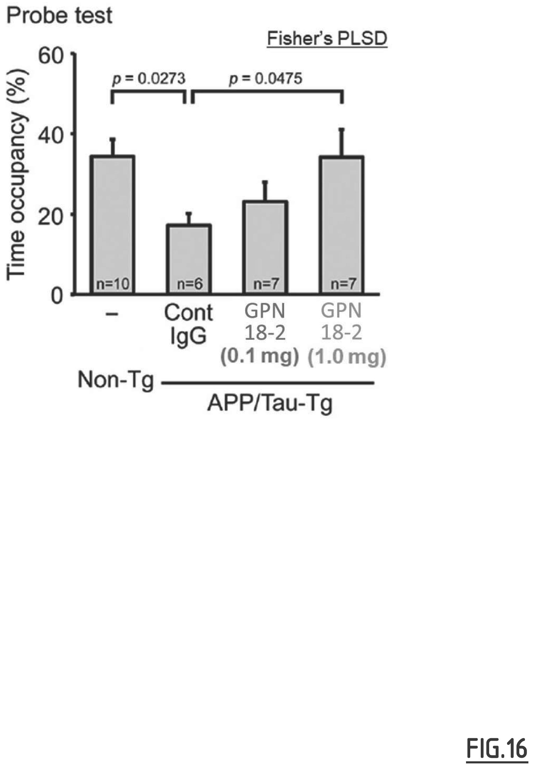

The results of the probe test are shown in the graph in Figure 16. The results of the probe test were similar to those of the acquisition test. Specifically, in the APP/Tau-Tg group treated with the control antibody, the mice spent statistically significantly less time swimming around the quadrant where the hidden platform was present. The mice in the groups treated with 1 mg/head of antibody GPN18-2 spent statistically significantly more time swimming around the quadrant where the hidden platform was located than the control antibody-treated group. The group that received 0.1 mg/head of GPN18-2 also showed a tendency to spend more time swimming around the quadrant where the hidden step was located.

These results indicate that anti-gpNMB antibody: GPN18-2 inhibits or restores declines in cognitive functions and motor functions in APP/Tau-Tg mice in a dose-dependent manner.

### <Pathology analysis>

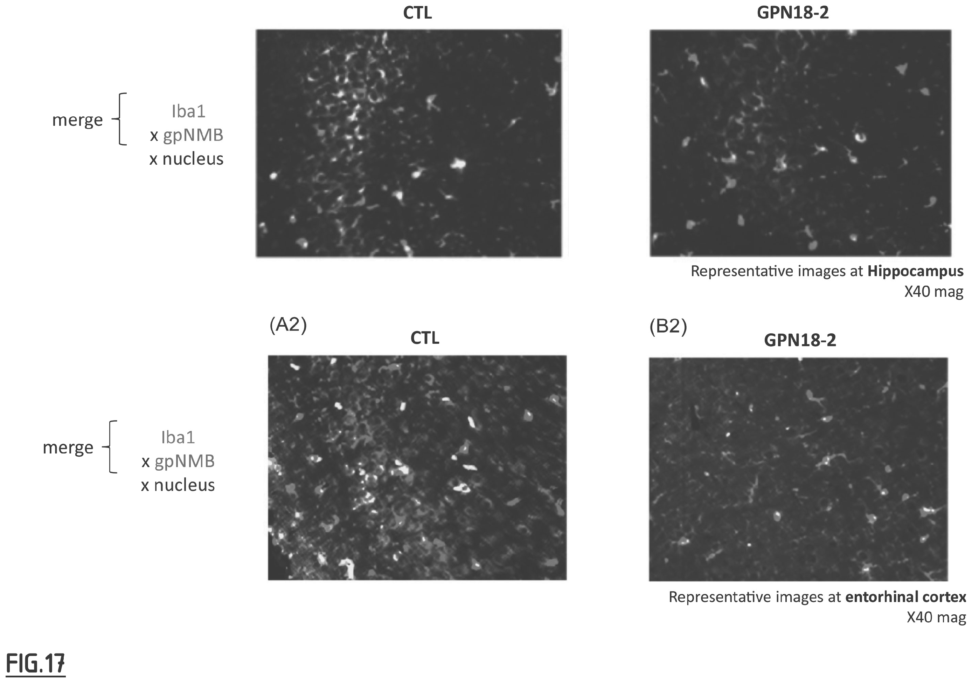

Anti-gpNMB antibody: 14-month-old APP/Tau-Tg mice treated with 1 mg/head of GPN18-2 and 14-month-old APP/Tau-Tg mice treated with 1 mg/head of control antibody (comparative control) were subjected to fixation of the brains with perfusion. The fixed brains were removed, and 5-µm-thick paraffin sections of the brains were prepared. After deparaffinization, the brain sections were boiled in citrate buffer (pH 6) for 30 minutes to activate the antigen, and then double stained with anti-gpNMB antibody AF2330 (R&D, 2 µg/mL) + a FITC-donkey anti-goat IgG antibody (Jackson Lab) and an anti-Iba1 antibody (WAKO, 5µg/mL) + a rhodamine-donkey anti-rabbit IgG antibody (Jackson Lab). After treatment with lipofuscin quencher True Black plus (Biotium) for 10 minutes at room temperature, the tissue was mounted with DAPI-containing mountant (Vector). The results of staining of the hippocampus and intraorbital cortex are shown in Figure 17.

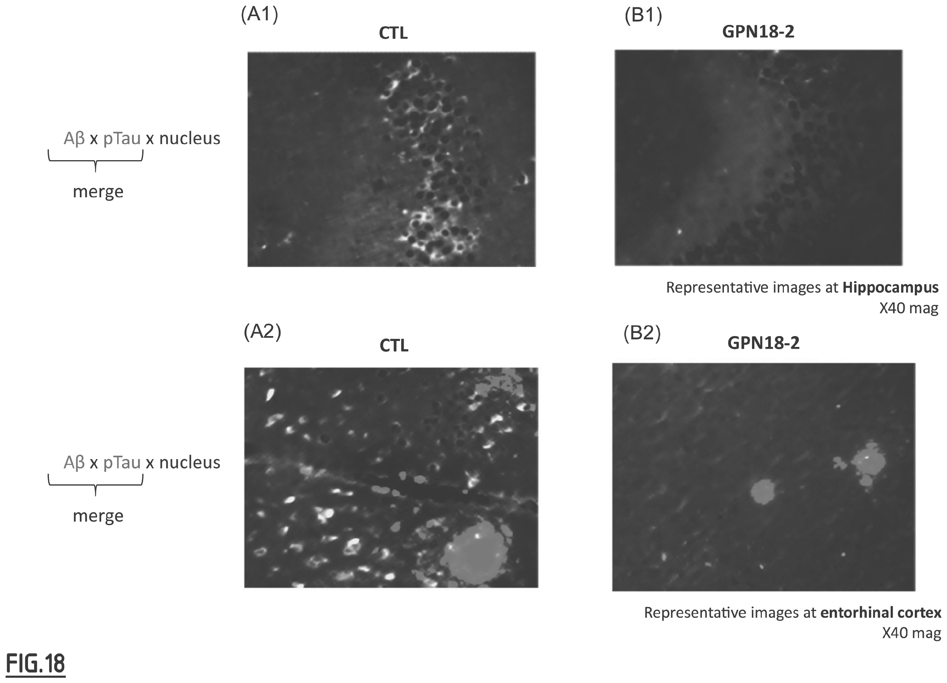

Aβ and phosphorylated tau staining was also performed in parallel. Brain sections were boiled in HCl (pH 2) for 10 minutes and then double stained with antibody AT8 (phosphorylated tau, Invitrogen #MN1020) + a FITC-goat anti-mouse IgG antibody (Jackson Lab) and anti-Aβ antibody β001 (Aβ, Rb pAb: Lab made) + a Rhodamine-goat anti-rabbit IgG antibody (Jackson Lab). After treatment with lipofuscin quencher True Black plus (Biotium) for 10 minutes at room temperature, the tissue was mounted with DAPI-containing mountant (Vector). The results of staining of the hippocampus and intraorbital cortex are shown in Figure 18.

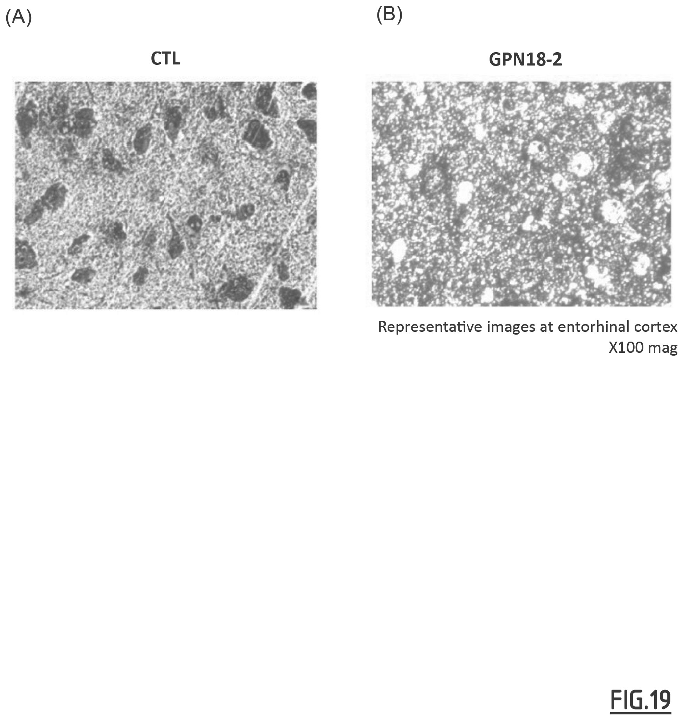

In addition, NFT (neurofibrillary tangles) were detected by Gallyas silver staining. The results showed that NFT formation was suppressed in 14-month-old APP/Tau-Tg mice treated with 1 mg/head of GPN18-2, compared to 14-month-old APP/Tau-Tg mice treated with 1 mg/head of control antibody. The results are shown in Figure 19.

### [Example 15] Humanization design of mouse anti-gpNMB antibody: GPN06-1

The amino acid sequence of the anti-gpNMB antibody GPN06-1 was analyzed for identifying its CDR regions, in view of IMGT (Lefranc, M.-P., Pommié, C., Ruiz, M., Giudicelli, V., Foulquier, E., Truong, L., Thouvenin-Contet, V. and Lefranc, G. "IMGT unique numbering for immunoglobulin and T cell receptor variable domains and Ig superfamily V-like domains". Dev. Comp. Immunol., 27, 55-77 (2003) PMID: 12477501 LIGM:268.) and Kabat numbering. Combined IMGT/Kabat method was used to keep the CDR loop structure optimal.

### <Heavy chain humanization design>

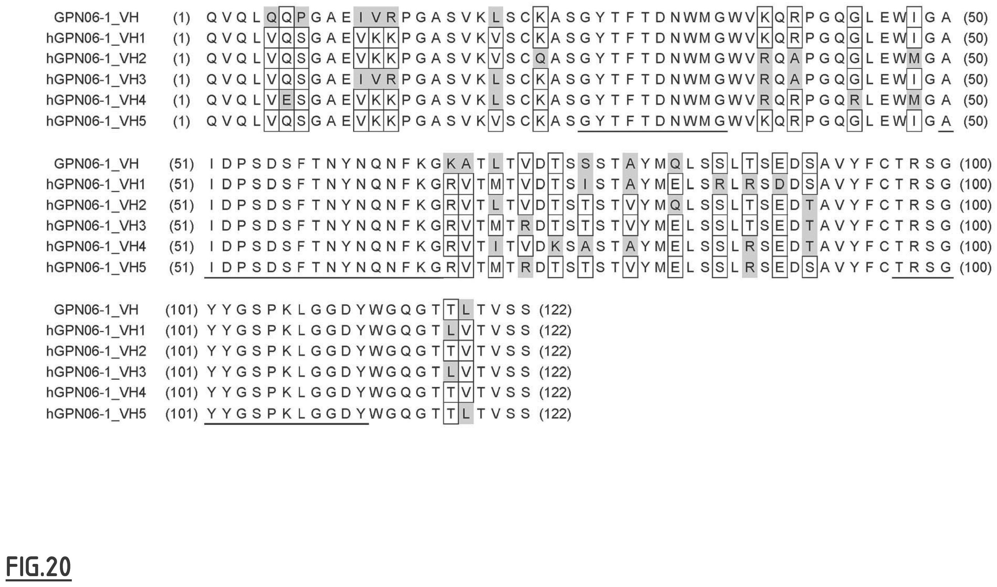

The human germline gene that most closely approximated the amino acid sequence of the mouse heavy chain variable region (GPN06-1_VH) (SEQ ID NO 29) was IGHV1-46*01. BLAST search was used to select 200 candidate human IgG sequences with high homology to GPN06-1_VH. Then, four framework sequences were finally selected in terms of framework homology, retention of key amino acids in the framework, and loop structure. These were subjected to CDR grafting to thereby obtain humanized mutant sequences hGPN06-1_VH1, hGPN06-1_VH2, hGPN06-1_VH3, and hGPN06-1_VH4. In addition, a sequence designed via CDR-grafting of IGHV1-46*01 as a framework was designated as humanized mutant sequence hGPN06-1_VH5. The alignment of these humanized mutant sequences hGPN06-1_VH1 to VH5 to mouse GPN06-1_VH is shown in Figure 20, in which the identified CDR regions are underlined. Table 9 shows the identity and homology of these humanized mutant sequences hGPN06-1_VH1 to VH5 to the mouse sequence GPN06-1_VH. The order of identity and homology of the humanized mutant sequences hGPN06-1_VH1 to VH5 to mouse GPN06-1_VH was VH3 = VH5 > VH2 > VH1 > VH4.

**[Table 9]**

| Table 9: Identity and homology of humanized mutation sequence VH1 to VH5 to mouse sequence VH0 | | |
|---|---|---|
| | Amino acid sequence identity | Amino acid sequence homology |
| hGPN06-1_VH1 | 86.9% | 92.6% |
| hGPN06-1_VH2 | 86.9% | 94.3% |
| hGPN06-1_VH3 | 88.5% | 93.4% |
| hGPN06-1_VH4 | 85.2% | 92.6% |
| hGPN06-1_VH5 | 88.5% | 93.4% |

### <Light chain humanized design>

The human germline gene that most closely approximated the amino acid sequence of the mouse light chain variable region (GPN06-1_VL) (SEQ ID NO 31) was IGKV1-9*01. BLAST search was used to select 200 candidate human IgG sequences with high homology to GPN06-1_VL. Then, four framework sequences were finally selected in terms of framework homology, retention of key amino acids in the framework, and loop structure. These were subjected to CDR grafting to thereby obtain humanized mutant sequences hGPN06-1_VL1, hGPN06-1_VL2, hGPN06-1_VL3, and hGPN06-1_VL4. In addition, a sequence designed via CDR-grafting of IGHV1-9*01 as a framework was designated as humanized mutant sequence hGPN06-1_VL5. The alignment of these humanized mutant sequences hGPN06-1_VL1 to VL5 to mouse GPN06-1_VL is shown in Figure 14, in which the identified CDR regions are underlined. Table 10 shows the identity and homology of these humanized mutant sequences hGPN06-1_VL1 to VL5 to the mouse sequence GPN06-1_VL. The order of identity and homology of the humanized mutant sequences hGPN06-1_VL1 to VL5 to mouse GPN06-1_VL was VL5 > VL4 > VL1 > VL3 > VL2.

**[Table 10]**

| Table 10: Identity and homology of humanized mutation sequence VL1 to VL5 to mouse sequence VL0 | | |
|---|---|---|
| | Amino acid sequence identity | Amino acid sequence homology |
| hGPN06-1_VL1 | 83.0% | 92.5% |
| hGPN06-1_VL2 | 82.1% | 90.6% |
| hGPN06-1_VL3 | 82.1% | 91.5% |
| hGPN06-1_VL4 | 83.0% | 93.4% |
| hGPN06-1_VL5 | 84.9% | 93.4% |

### [Example 16] Selection of GPN06-1 humanized antibodies

The five humanized heavy chain sequences and the five humanized light chain sequences derived from the anti-gpNMB antibody GPN06-1 designed in Example 15 were combined to prepare twenty-five humanized antibodies. These humanized antibodies were evaluated for binding by antigen ELISA to select humanized antibodies whose binding activity was similar to that of human chimeric antibodies.

Specifically, a gene sequence encoding a signal sequence was added to the 5' end of each of the gene sequences encoding the H chains and the L chains of the humanized anti-gpNMB antibodies designed in Example 15, and the resulting sequence was inserted into the animal cell expression vector pcDNA3.4 to form a plasmid, which was transiently expressed using the ExpiCHO Expression System (A29133, Thermo Fisher Scientific) to secrete antibodies into the medium. The culture supernatant was then collected and purified using a Protein A column (Ab-Capture, Protenova) and a gel filtration column (Superdex 200 Increase, Cytiva).

Antigen ELISA was performed by adding each of serially-diluted solutions of the purified humanized antibody to a plate fixed with recombinant human soluble gpNMB (SEQ ID NO 221). The results are shown in Table 11. The results showed that hGPN06-1_H1L1, hGPN06-1_H1L2, hGPN06-1_H2L1, hGPN06-1_H2L2, hGPN06-1_H3L1, hGPN06-1_H3L2, hGPN06-1_H4L1, hGPN06-1_H5L2, hGPN06-1_H5L4, and hGPN06-1_H5L5 had similar levels of binding ability to human chimeric antibody (hGPN06-1_H0L0) (i.e., EC₅₀ values were within 3-fold compared to chimeric antibody hGPN06-1_H0L0).

**[Table 11]**

| Table 11: Evaluation results of the binding ability of each anti-gpNMB antibody clone to human gpNMB by antigen ELISA | | |
|---|---|---|
| Antibody name | EC₅₀ (M) | Relative binding activity |
| hGPN06-1_H0L0 | 3.83E-10 | 1 |
| **hGPN06-1_H1L1** | **5.75E-10** | **0.92** |
| hGPN06-1_H1L2 | **5.18E-10** | 0.88 |
| hGPN06-1_H1L3 | 1.58E-09 | 0.83 |
| hGPN06-1_H1L4 | 1.64E-09 | 0.86 |
| hGPN06-1_H1L5 | 2.32E-09 | 0.81 |
| hGPN06-1_H2L1 | **5.57E-10** | 0.91 |
| hGPN06-1_H2L2 | **4.44E-10** | 0.89 |
| hGPN06-1_H2L3 | 3.50E-09 | 0.68 |
| hGPN06-1_H2L4 | 1.38E-09 | 0.82 |
| hGPN06-1_H2L5 | 1.15E-09 | 0.79 |
| hGPN06-1_H3L1 | **6.74E-10** | 0.85 |
| hGPN06-1_H3L2 | **7.99E-10** | 0.85 |
| hGPN06-1_H3L3 | 1.48E-09 | 0.7 |
| hGPN06-1_H3L4 | 1.45E-09 | 0.69 |
| hGPN06-1_H3L5 | 1.32E-08 | 0.68 |
| hGPN06-1_H4L1 | **4.31E-10** | 0.87 |
| hGPN06-1_H4L2 | 5.04E-09 | 0.71 |
| hGPN06-1_H4L3 | 1.42E-08 | 0.37 |
| hGPN06-1_H4L4 | 1.69E-08 | 0.49 |
| hGPN06-1_H4L5 | 1.90E-09 | 0.57 |
| hGPN06-1_H5L1 | 1.76E-09 | 0.65 |
| hGPN06-1_H5L2 | **5.94E-10** | 0.84 |
| hGPN06-1_H5L3 | 1.87E-09 | 0.7 |
| hGPN06-1_H5L4 | **8.60E-10** | 0.77 |
| hGPN06-1_H5L5 | **5.00E-10** | 0.78 |

### [Example 17] Identification of amino acids in the CDR regions in GPN06-1 humanized antibodies critical for binding by alanine substitution

Among the humanized GPN06 antibody variants obtained in Example 16, hGPN06-1_H1L1, hGPN06-1_H1L2, hGPN06-1_H1L4,hGPN06-1_H2L1, hGPN06-1_H2L2, hGPN06-1_H3L1, hGPN06-1_H3L2, hGPN06-1_H4L1, hGPN06-1_H5L2 were compared in terms of the binding properties (binding and dissociation kinetics) to human soluble gpNMB (SEQ ID NO 221) by means of surface plasmon resonance (SPR). Specifically, the same measurement and analysis conditions as in Example 7 were used, except for the conditions described below.

The humanized GPN06 antibody variants hGPN06-1_H1L1, hGPN06-1_H1L2, hGPN06-1_H1L4, hGPN06-1_H2L1, hGPN06-1_H2L2, hGPN06-1_H3L1, hGPN06-1_H3L2, hGPN06-1_H4L1, hGPN06-1_H5L2 were used as analytes. Each analyte at each concentration was reacted for 600 seconds to obtain a binding curve, and HBS-EP+ was reacted for 1200 seconds to obtain a dissociation curve. After the reaction finished, regeneration buffer 1 (0.2% SDS), regeneration buffer 2 (100 mmol/L Tris-HCl (pH 8.5), 1 mol/L NaCl, 15 mmol/L MgCl2) and regeneration buffer 3 (10 mmol/L glycine -HCl (pH 1.5)) were reacted for 1 minute each to remove and wash gpNMB-FLAG-His in the measurement system. The dissociation rate constant (ka, 1/Ms), binding rate constant (kd, 1/s), and dissociation constant (kd, M) were calculated using BIACORE T200 Evaluation software (ver 2.0) with a Model of 1:1 Binding. The results are shown in Table 12.

**[Table 12]**

| Table 12: Results of SPR kinetics analysis of each anti-gpNMB antibody clone against human gpNMB | | | |
|---|---|---|---|
| Clone | ka (1/Ms) | kd (1/s) | KD (M) |
| hGPN06-1_H0L0 | 7.89E+05 | 2.09E-04 | 2.65E-10 |
| hGPN06-1_H1L1 | 9.16E+05 | 5.67E-04 | 6.20E-10 |
| hGPN06-1_H1L2 | 3.24E+06 | 8.08E-04 | 2.49E-10 |
| hGPN06-1_H1L4 | 4.98E+05 | 2.13E-04 | 4.28E-10 |
| hGPN06-1_H2L1 | 4.79E+05 | 3.30E-04 | 6.89E-10 |
| hGPN06-1_H2L2 | 5.06E+05 | 3.54E-04 | 7.00E-10 |
| hGPN06-1_H3L1 | 3.51E+06 | 1.54E-03 | 4.38E-10 |
| hGPN06-1_H3L2 | 9.83E+05 | 6.36E-04 | 6.46E-10 |
| hGPN06-1_H4L1 | 7.29E+05 | 4.15E-04 | 5.70E-10 |
| hGPN06-1_H5L2 | 6.02E+05 | 3.54E-04 | 5.87E-10 |

### [Example 18] Identification of amino acids in the CDR regions in GPN06-1 humanized antibodies critical for binding by alanine substitution

Each amino acid in the CDR regions of humanized anti-gpNMB antibody hGPN06-1_H1L1 produced in Example 16 was substituted with alanine to produce sixty-five CDR substitution mutants of humanized anti-gpNMB antibody hGPN06-1_H1L1. The binding of these alanine-substituted antibodies to recombinant human soluble gpNMB (SEQ ID NO 221) was evaluated by antigen ELISA to determine which amino acids in the CDR grafting regions are critical for binding. The alanine-substitution mutants of the humanized anti-gpNMB antibody hGPN06-1_H1L1 and their binding activity results are shown in Tables 13, 14-1 and 14-2 below.

The results show that alanine-substitution of the following amino acids in each CDR region caused reduced binding activity and are therefore critical for maintaining the binding ability: in the light chain variable region of the humanized anti-gpNMB antibody hGPN06-1_H1L1 (SEQ ID NO 297), in CDR grafting region-L1 (SEQ ID NO 277), isoleucine at position 29, tyrosine at position 31, and histidine at position 33; in CDR grafting region-L2 (SEQ ID NO 279), threonine at position 50; and in CDR grafting region-L3 (SEQ ID NO 281), histidine at position 88, glutamine at position 89, tryptophan at position 90, serine at position 92, tyrosine at position 93, proline at position 94, and cysteine at position 95; and in the heavy chain variable region of the humanized anti-gpNMB antibody hGPN06-1_H1L1 (SEQ ID NO 295), in CDR grafting region-H1 (SEQ ID NO 271), tyrosine at position 27, asparagine at position 32 and tryptophan at position 33; in CDR grafting region-H2 (SEQ ID NO 273), aspartic acid at position 55, phenylalanine at position 57, threonine at position 58, asparagine at position 59, tyrosine at position 60, and asparagine at position 61; and in CDR grafting region-H3 (SEQ ID NO 275), arginine at position 98, glycine at position 100, and glycine at position 109.

**[Table 13]**

| Table 13: Evaluation results of binding activity of alanine substitutions in the light chain CDR regions | | | | |
|---|---|---|---|---|
| | Alanine substitution position | Amino acid before substitution | EC₅₀ (M) | Relative binding activity |
| hGPN06-1 _H1L1 | - | - | 1.98E-09 | 1.00 |
| CDR grafting region-L1 | 24 | S | 1.99E-09 | 0.98 |
| | 25 | A | No data | No data |
| | 26 | S | 1.09E-09 | 0.75 |
| | 27 | S | 1.56E-09 | 0.99 |
| | 28 | S | 1.27E-09 | 1.18 |
| | 29 | I | 2.30E-08 | 0.27 |
| | 30 | S | 8.63E-09 | 0.58 |
| | **31** | **Y** | **1.49E-08** | **0.36** |
| | 32 | M | 6.40E-09 | 0.65 |
| | **33** | **H** | **4.29E-08** | **0.28** |
| CDR grafting region-L2 | 49 | S | 7.00E-10 | 0.79 |
| | **50** | **T** | **3.19E-09** | **0.30** |
| | 51 | S | 9.27E-10 | 0.82 |
| | 52 | N | 8.50E-10 | 0.74 |
| | 53 | L | 5.64E-10 | 0.98 |
| | 54 | A | No data | No data |
| | 55 | S | 6.03E-10 | 1.00 |
| CDR grafting region-L3 | **88** | **H** | **1.47E-07** | **0.08** |
| | **89** | **Q** | **2.75E-08** | **0.06** |
| | **90** | **W** | **1.22E-07** | **0.13** |
| | 91 | N | 1.84E-09 | 0.58 |
| | **92** | **S** | **1.86E-09** | **0.19** |
| | **93** | **Y** | **2.53E-08** | **0.00** |
| | **94** | **P** | **2.64E-08** | **0.05** |
| | **95** | **C** | **3.15E-09** | **0.45** |
| | 96 | T | 1.03E-09 | 0.83 |

**[Table 14-1]**

| Table 14-1: Evaluation results of binding activity of alanine substitutions in the heavy chain CDR regions | | | | |
|---|---|---|---|---|
| | Alanine substitution position | Amino acid before substitution | EC₅₀ (M) | Relative binding activity |
| hGPN06-1 _H1L1 | - | - | 1.98E-09 | 1.00 |
| CDR grafting region-H1 | 26 | G | 1.21E-09 | 1.08 |
| | **27** | **Y** | **6.99E-08** | **0.02** |
| | 28 | T | 1.98E-09 | 1.49 |
| | 29 | F | 4.80E-09 | 0.54 |
| | 30 | T | 2.02E-09 | 1.23 |
| | 31 | D | 5.15E-10 | 1.26 |
| | **32** | **N** | **7.83E-09** | **0.35** |
| | **33** | **W** | **2.81E-08** | **0.06** |
| | 34 | M | 3.55E-09 | 1.06 |
| | 35 | G | 3.55E-09 | 1.40 |
| CDR grafting region-H2 | 51 | I | 9.93E-10 | 0.89 |
| | 52 | D | 2.79E-09 | 0.51 |
| | 53 | P | 8.01E-10 | 0.85 |
| | 54 | S | 6.52E-10 | 0.92 |
| | **55** | **D** | **3.15E-08** | **0.00** |
| | 56 | S | 4.29E-10 | 1.14 |
| | **57** | **F** | **3.25E-08** | **0.09** |
| | **58** | **T** | **2.80E-08** | **0.04** |
| | **59** | **N** | **2.94E-08** | **0.04** |
| | **60** | **Y** | **1.28E-07** | **0.04** |
| | **61** | **N** | **2.61E-08** | **0.04** |
| | 62 | Q | 4.06E-10 | 1.14 |
| | 63 | N | 4.39E-10 | 1.07 |
| | 64 | F | 4.48E-10 | 1.11 |
| | 65 | K | 4.06E-10 | 1.10 |
| | 66 | G | 3.73E-10 | 1.09 |

**[Table 14-2]**

| Table 14-2: Evaluation results of binding activity of alanine substitutions in the heavy chain CDR regions (continued) | | | | |
|---|---|---|---|---|
| | Alanine substitution position | Amino acid before substitution | EC₅₀ (M) | Relative binding activity |
| hGPN06-1 _H1L1 | - | - | 1.98E-09 | 1.00 |
| CDR grafting region-H3 | 97 | T | 3.60E-10 | 0.98 |
| | **98** | **R** | **5.32E-08** | **0.05** |
| | 99 | S | 5.87E-10 | 0.92 |
| | **100** | **G** | **6.58E-08** | **0.06** |
| | 101 | Y | 5.02E-10 | 1.04 |
| | 102 | Y | 2.21E-09 | 0.68 |
| | 103 | G | 7.62E-10 | 1.14 |
| | 104 | S | 7.12E-10 | 0.88 |
| | 105 | P | 1.18E-09 | 1.09 |
| | 106 | K | 4.41E-10 | 1.00 |
| | 107 | L | 1.05E-09 | 0.71 |
| | 108 | G | 4.76E-10 | 0.96 |
| | **109** | **G** | **4.89E-09** | **0.34** |
| | 110 | D | 9.56E-10 | 0.79 |
| | 111 | Y | 5.84E-10 | 0.99 |

### [Example 19] Humanization design of mouse anti-gpNMB antibody: GPN18-2

The amino acid sequence of the anti-gpNMB antibody GPN18-2 was analyzed for identifying its CDR regions, in view of IMGT (Lefranc, M.-P., Pommié, C., Ruiz, M., Giudicelli, V., Foulquier, E., Truong, L., Thouvenin-Contet, V. and Lefranc, G. "IMGT unique numbering for immunoglobulin and T cell receptor variable domains and Ig superfamily V-like domains". Dev. Comp. Immunol., 27, 55-77 (2003) PMID: 12477501 LIGM:268.) and Kabat numbering. Combined IMGT/Kabat method was used to keep the CDR loop structure optimal.

### <Heavy chain humanization design>

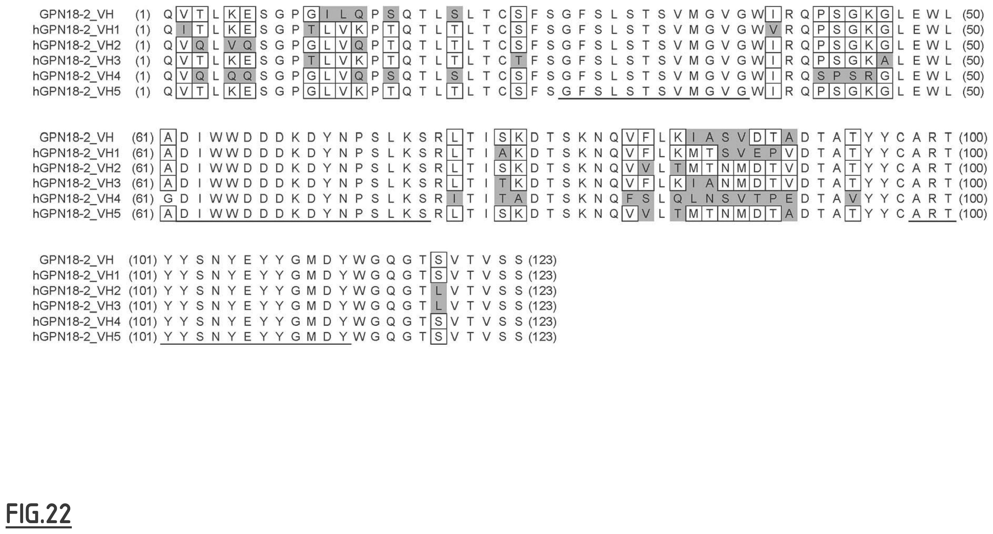

The human germline gene that most closely approximated the amino acid sequence of the mouse heavy chain variable region (GPN18-2 _VH) (SEQ ID NO 141) was IGHV2-05*09. BLAST search was used to select 200 candidate human IgG sequences with high homology to GPN18-2_VH. Then, four framework sequences were finally selected in terms of framework homology, retention of key amino acids in the framework, and loop structure. These were subjected to CDR grafting to thereby obtain humanized mutant sequences hGPN18-2_VH1, hGPN18-2_VH2, hGPN18-2_VH3, and hGPN18-2_VH4. In addition, a sequence designed via CDR-grafting of IGHV2-05*09 as a framework was designated as humanized mutant sequence hGPN18-2_VH5. The alignment of these humanized mutant sequences hGPN18-2_VH1 to VH5 to mouse GPN06-1_VH is shown in Figure 22, in which the identified CDR regions are underlined. Table 15 shows the identity and homology of these humanized mutant sequences hGPN18-2_VH1 to VH5 to the mouse sequence GPN18-2_VH. The order of identity and homology of the humanized mutant sequences hGPN18-2_VH1 to VH5 to mouse GPN18-2_VH was VH5 > VH3 > VH1 > VH2 > VH4.

**[Table 15]**

| Table 15: Identity and homology of humanized mutation sequence hGPN18-2_VH1 to VH5 to mouse sequence hGPN18-2_VH0 | | |
|---|---|---|
| | Amino acid sequence identity | Amino acid sequence homology |
| hGPN18-2_VH1 | 88.6% | 96.7% |
| hGPN18-2_ VH2 | 87.8% | 94.3% |
| hGPN18-2_VH3 | 89.4% | 96.7% |
| hGPN18-2_VH4 | 82.1% | 89.4% |
| hGPN18-2_VH5 | 91.1% | 97.6% |

### <Light chain humanized design>

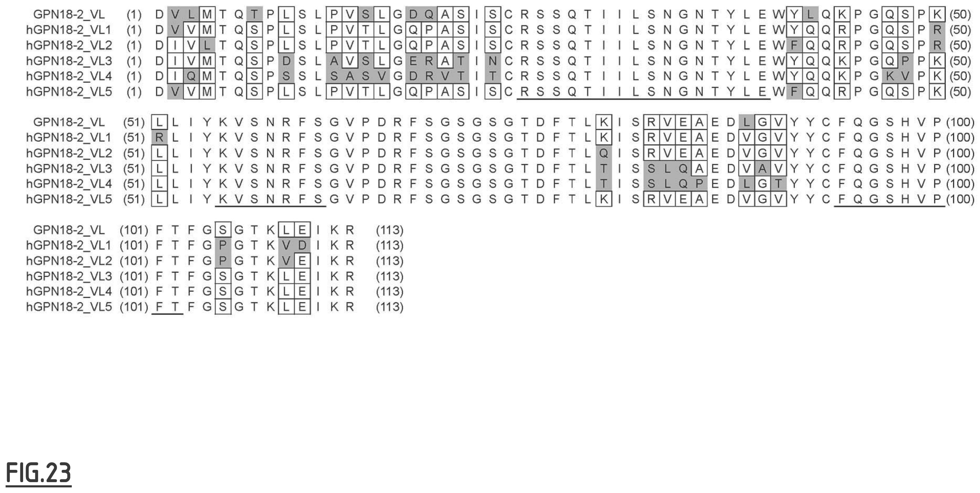

The human germline gene that most closely approximated the amino acid sequence of the mouse light chain variable region (GPN18-2_VL) (SEQ ID NO 31) was IGKV2-30*01. BLAST search was used to select 200 candidate human IgG sequences with high homology to GPN18-2_VL. Then, four framework sequences were finally selected in terms of framework homology, retention of key amino acids in the framework, and loop structure. These were subjected to CDR grafting to thereby obtain humanized mutant sequences hGPN18-2_VL1, hGPN18-2_VL2, hGPN18-2_VL3, and hGPN18-2_VL4. In addition, a sequence designed via CDR-grafting of IGKV2-30*01 as a framework was designated as humanized mutant sequence GPN18-2_VL5. The alignment of these humanized mutant sequences GPN18-2_VL1 to VL5 to mouse GPN18-2_VL is shown in Figure 23, in which the identified CDR regions are underlined. Table 16 shows the identity and homology of these humanized mutant sequences hGPN18-2_VL1 to VL5 to the mouse sequence GPN18-2_VL. The order of identity and homology of the humanized mutant sequences hGPN18-2_VL1 to VL5 to mouse GPN18-2_VL was VL5 > VL1 > VL2 > VL3 > VL4.

**[Table 16]**

| Table 16: Identity and homology of humanized mutation sequence hGPN18-2_VL1 to VL5 to GPN18-2_VL | | |
|---|---|---|
| | Amino acid sequence identity | Amino acid sequence homology |
| hGPN18-2_VL1 | 88.4% | 95.5% |
| hGPN18-2_VL2 | 86.6% | 96.4% |
| hGPN18-2_VL3 | 84.8% | 93.8% |
| hGPN18-2_VL4 | 82.1% | 90.2% |
| hGPN18-2_VL5 | 92.0% | 97.3% |

### [Example 20] Selection of GPN18-2 humanized antibodies

The five humanized heavy chain sequences and the five humanized light chain sequences derived from the anti-gpNMB antibody GPN18-2 designed in Example 19 were combined to prepare twenty-five humanized antibodies. These humanized antibodies were evaluated for binding by antigen ELISA to select humanized antibodies whose binding activity was similar to that of human chimeric antibody hGPN18-2_H0L0.

Specifically, a gene sequence encoding a signal sequence was added to the 5' end of each of the gene sequences encoding the H chains and the L chains of the humanized anti-gpNMB antibodies designed in Example 19, and the resulting sequence was inserted into the animal cell expression vector pcDNA3.4 to form a plasmid, which was transiently expressed using the ExpiCHO Expression System (A29133, Thermo Fisher Scientific) to secrete antibodies into the medium. The culture supernatant was then collected and purified using a Protein A column (Ab-Capture, Protenova) and a gel filtration column (Superdex 200 Increase, Cytiva).

Antigen ELISA was performed by adding each of serially-diluted solutions of the purified humanized antibody to a plate fixed with recombinant human soluble gpNMB (SEQ ID NO 221). The results are shown in Table 17. The results showed that hGPN18-2_H1L3, hGPN18-2_H1L4, hGPN18-2_H2L3, hGPN18-2_H2L4, hGPN18-2_H2L5, hGPN18-2_H3L3, hGPN18-2_H3L4, hGPN18-2_H5L3, and hGPN18-2_H5L4 had similar levels of binding ability to human chimeric antibody (hGPN18-2_H0L0) (i.e., EC₅₀ values were within 3-fold compared to human chimeric antibody hGPN18-2_H0L0).

**[Table 17]**

| Table 17: Evaluation results of the binding ability of each GPN18-22 humanized antibody to human gpNMB by antigen ELISA | | |
|---|---|---|
| Antibody name | EC₅₀ (M) | Relative binding activity |
| hGPN18-2_H0L0 | 1.21E-09 | 1 |
| hGPN18-2_H1L1 | 4.95E-09 | 0.59 |
| hGPN18-2_H1L2 | 1.62E-08 | 0.25 |
| hGPN18-2_H1L3 | **1.93E-09** | **0.77** |
| hGPN18-2_H1L4 | **3.07E-09** | **0.68** |
| hGPN18-2_H1L5 | 9.55E-09 | 0.36 |
| hGPN18-2_H2L1 | 9.12E-09 | 0.41 |
| hGPN18-2_H2L2 | 6.91E-09 | 0.46 |
| **hGPN18-2_H2L3** | **1.84E-09** | **0.84** |
| hGPN18-2_H2L4 | **2.00E-09** | **0.81** |
| hGPN18-2_H2L5 | **3.35E-09** | **0.61** |
| hGPN18-2_H3L1 | 5.50E-09 | 0.55 |
| hGPN18-2_H3L2 | 2.35E-08 | 0.23 |
| hGPN18-2_H3L3 | **2.11E-09** | **0.79** |
| hGPN18-2_H3L4 | **2.59E-09** | **0.73** |
| hGPN18-2_H3L5 | 1.01E-08 | 0.35 |
| hGPN18-2_H4L1 | 4.54E-08 | 0.05 |
| hGPN18-2_H4L2 | 2.99E-08 | 0.20 |
| hGPN18-2_H4L3 | 6.09E-09 | 0.46 |
| hGPN18-2_H4L4 | 5.54E-09 | 0.50 |
| hGPN18-2_H4L5 | 2.54E-08 | 0.15 |
| hGPN18-2_H5L1 | 9.25E-09 | 0.46 |
| hGPN18-2_H5L2 | 1.76E-08 | 0.23 |
| hGPN18-2_H5L3 | **2.41E-09** | **0.79** |
| hGPN18-2_H5L4 | **3.10E-09** | **0.72** |
| hGPN18-2_H5L5 | 1.10E-08 | 0.35 |

### [Example 21] Identification of amino acids in the CDR regions in GPN18-2 humanized antibodies critical for binding by alanine substitution

Twenty-five humanized GPN18-2 antibody variants obtained in Example 20 were compared in terms of the binding properties (binding and dissociation kinetics) to human gpNMB by means of surface plasmon resonance (SPR). Specifically, the same measurement and analysis conditions as in Example 7 were used, except for the conditions described below.

The temperature of the measurement system was set at 36°C. The humanized GPN18-2 antibodies produce in Example 20 were used as analytes. Each analyte was diluted with HBS-EP+ (100nmol/L) and was reacted for 300 seconds in each flow cell to obtain a binding curve, and HBS-EP+ was reacted for 300 seconds to obtain a dissociation curve. After the reaction finished, regeneration buffer 1 (0.2% SDS), regeneration buffer 2 (100 mmol/L Tris-HCl (pH 8.5), 1 mol/L NaCl, 15 mmol/L MgCl2) and regeneration buffer 3 (10 mmol/L glycine -HCl (pH 1.5)) were reacted for 1 minute each to remove and wash gpNMB-FLAG-His in the measurement system. BIACORE T200 Evaluation software (ver. 2.0) was used to analyze the data with the 1:1 Binding model, and the binding rate value (Ass: 1/Ms), binding stability value (Diss: 1/s) and equilibrium dissociation value (Diss/Ass: M) were quantified as ordinal numbers. The results are shown in Table 18.

**[Table 18]**

| Table 18: Kinetic analysis of each anti-gpNMB antibody against human gpNMB by SPR method | | | |
|---|---|---|---|
| Antibody name | Ass (1/Ms) | Diss (1/s) | Diss/Ass (M) |
| hGPN18-2_H0L0 | 1.41E+05 | 4.71E-07 | 3.46E-12 |
| hGPN18-2_H1L1 | 8.79E+04 | 6.01E-07 | 6.84E-12 |
| hGPN18-2_H1L2 | 3.19E+04 | 3.50E-08 | 1.10E-12 |
| hGPN18-2_H1L3 | 9.15E+04 | 8.15E-08 | 8.91E-13 |
| hGPN18-2_H1L4 | 7.67E+04 | 5.59E-07 | 7.30E-12 |
| hGPN18-2_H1L5 | 4.63E+04 | 4.37E-08 | 9.43E-13 |
| hGPN18-2_H2L1 | 2.84E+04 | 3.92E-06 | 1.38E-10 |
| hGPN18-2_H2L2 | 8.02E+04 | 5.20E-07 | 6.48E-12 |
| hGPN18-2_H2L3 | 9.62E+04 | 7.49E-07 | 7.78E-12 |
| hGPN18-2_H2L4 | 8.90E+04 | 1.47E-07 | 1.65E-12 |
| hGPN18-2_H2L5 | 5.65E+04 | 8.61E-08 | 1.52E-12 |
| hGPN18-2_H3L1 | 5.04E+04 | 2.53E-08 | 5.01E-13 |
| hGPN18-2_H3L2 | 6.31E+04 | 1.69E-04 | 2.68E-09 |
| hGPN18-2_H3L3 | 3.04E+04 | 1.48E-08 | 4.87E-13 |
| hGPN18-2_H3L4 | 7.79E+04 | 3.70E-10 | 4.74E-15 |
| hGPN18-2_H3L5 | 4.45E+04 | 2.49E-08 | 5.60E-13 |
| hGPN18-2_H4L1 | 1.66E+04 | 3.51E-04 | 2.11E-08 |
| hGPN18-2_H4L2 | 9.08E+04 | 9.93E-08 | 1.09E-12 |
| hGPN18-2_H4L3 | 1.03E+05 | 8.11E-07 | 7.91E-12 |
| hGPN18-2_H4L4 | 9.14E+04 | 2.06E-07 | 2.26E-12 |
| hGPN18-2_H4L5 | 9.63E+04 | 2.68E-05 | 2.78E-10 |
| hGPN18-2_H5L1 | not measured | not measured | not measured |
| hGPN18-2_H5L2 | 2.91E+04 | 2.16E-08 | 7.40E-13 |
| hGPN18-2_H5L3 | 7.45E+04 | 1.68E-07 | 2.25E-12 |
| hGPN18-2_H5L4 | 7.20E+04 | 5.19E-09 | 7.21E-14 |
| hGPN18-2_H5L5 | 2.68E+04 | 3.40E-07 | 1.27E-11 |

### [Example 22] Identification of amino acids in the CDR regions in GPN18-2 humanized antibodies critical for binding by alanine substitution

Each amino acid in the CDR regions of humanized anti-gpNMB antibody hGPN18-2_H2L3 produced in Example 20 was substituted with alanine to produce seventy-four CDR substitution mutants of humanized anti-gpNMB antibody hGPN18-2_H2L3. The binding of these alanine-substituted antibodies to recombinant human soluble gpNMB (SEQ ID NO 221) was evaluated by antigen ELISA to determine which amino acids in the CDR grafting regions are critical for binding. The alanine-substitution mutants of the humanized anti-gpNMB antibody hGPN18-2_H2L3 and their binding activity results are shown in Tables 19-1, 19-2, 20-1, and 20-2 below.

The results show that alanine-substitution of the following amino acids in each CDR region caused reduced binding activity and are therefore critical for maintaining the binding ability: in the light chain variable region of the humanized anti-gpNMB antibody hGPN18-2_H2L3 (SEQ ID NO 325), in CDR grafting region-L1 (SEQ ID NO 305), leucine at position 31, tyrosine at position 37, and glutamic acid at position 39; in CDR grafting region-L2 (SEQ ID NO 307), lysine at position 55; and in CDR grafting region-L3 (SEQ ID NO 309), phenylalanine at position 94; and in the heavy chain variable region of the humanized anti-gpNMB antibody hGPN18-2_H2L3 (SEQ ID NO 323), in CDR grafting region-H2 (SEQ ID NO 301), aspartic acid at position 52, tryptophan at position 54, aspartic acid at position 58, and proline at position 63; and in CDR grafting region-H3 (SEQ ID NO 303), arginine at position 99, threonine at position 100, tyrosine at position 102, tyrosine at position 105, tyrosine at position 107, and methionine at position 110.

**[Table 19-1]**

| Table 19-1: Evaluation results of binding activity of alanine substitutions in the light chain CDR regions | | | | |
|---|---|---|---|---|
| | Alanine substitution position | Amino acid before substitution | EC₅₀ (M) | Relative binding activity |
| hGPN18-2 H2L3 | - | - | 4.39E-09 | 1.00 |
| CDR grafting region-L1 | 24 | R | 4.73E-09 | 1.04 |
| | 25 | S | 3.76E-09 | 1.01 |
| | 26 | S | 3.14E-09 | 1.03 |
| | 27 | Q | 4.00E-09 | 1.04 |
| | 28 | T | 2.91E-09 | 1,05 |
| | 29 | I | 6.04E-09 | 0.75 |
| | 30 | I | 4.10E-09 | 1.00 |
| | **31** | **L** | **2.07E-08** | **0.41** |
| | 32 | S | 8.67E-09 | 0.92 |
| | 33 | N | 3.66E-09 | 0.82 |
| | 34 | G | 3.94E-09 | 1.00 |
| | 35 | N | 3.08E-09 | 1.11 |
| | 36 | T | 4.74E-09 | 1.06 |
| | **37** | **Y** | **2.71E-08** | **0.37** |
| | 38 | L | 4.15E-09 | 1.06 |
| | **39** | **E** | **8.48E-08** | **0.10** |
| CDR grafting region-L2 | **55** | **K** | **4.83E-08** | **0.31** |
| | 56 | V | 2.99E-09 | 1.07 |
| | 57 | S | 3.14E-09 | 1.05 |
| | 58 | N | 2.86E-09 | 1.04 |
| | 59 | R | 2.36E-09 | 1.11 |
| | 60 | F | 2.28E-09 | 1.12 |
| | 61 | S | 3.63E-09 | 0.94 |
| CDR grafting region-L3 | **94** | **F** | **1.80E-08** | **0.07** |
| | 95 | Q | 5.49E-09 | 0.79 |
| | 96 | G | 1.08E-08 | 0.91 |
| | 97 | S | 3.29E-09 | 1.04 |
| | 98 | H | 5.69E-09 | 0.80 |
| | 99 | V | 9.93E-09 | 0.81 |
| | 100 | P | 4.22E-09 | 0.84 |
| | 101 | F | 7.66E-09 | 0.69 |
| | 102 | T | 4.73E-09 | 0.81 |

**[Table 20-1]**

| Table 20-1: Evaluation results of binding activity of alanine substitutions in the heavy chain CDR regions | | | | |
|---|---|---|---|---|
| | Alanine substitution position | Amino acid before substitution | EC₅₀ (M) | Relative binding activity |
| hGPN18-2 _H2L3 | - | - | 4.39E-09 | 1.00 |
| CDR grafting region-H1 | 26 | G | 3.92E-09 | 0.84 |
| | 27 | F | 5.47E-09 | 0.69 |
| | 28 | S | 3.22E-09 | 0.88 |
| | 29 | L | 4.41E-09 | 0.82 |
| | 30 | S | 6.60E-09 | 0.97 |
| | 31 | T | 3.12E-09 | 0.97 |
| | 32 | S | 4.31E-09 | 0.78 |
| | 33 | V | 8.88E-09 | 0.55 |
| | 34 | M | 3.58E-09 | 0.91 |
| | 35 | G | 5.16E-09 | 0.68 |
| | 36 | V | 3.43E-09 | 0.92 |
| | 37 | G | 3.32E-09 | 0.87 |
| CDR grafting region-H2 | **52** | **D** | **1.00E-07** | **0.05** |
| | 53 | I | 6.94E-09 | 0.60 |
| | **54** | **W** | **3.80E-08** | **0.20** |
| | 55 | W | 8.36E-09 | 0.66 |
| | 56 | D | 8.98E-09 | 0.63 |
| | 57 | D | 5.61E-09 | 0.86 |
| | **58** | **D** | **1.02E-08** | **0.50** |
| | 59 | K | 4.42E-09 | 0.78 |
| | 60 | D | 3.13E-09 | 0.98 |

**[Table 20-2]**

| Table 20-2: Evaluation results of binding activity of alanine substitutions in the heavy chain CDR regions (continued) | | | | |
|---|---|---|---|---|
| | Alanine substitution position | Amino acid before substitution | EC₅₀ (M) | Relative binding activity |
| hGPN18-2 _H2L3 | - | - | 4.39E-09 | 1.00 |
| CDR grafting region-H2 (continued) | 61 | Y | 7.55E-09 | 0.70 |
| | 62 | N | 4.58E-09 | 0.97 |
| | **63** | **P** | **1.20E-08** | **0.43** |
| | 64 | S | 3.59E-09 | 1.09 |
| | 65 | L | 5.04E-09 | 0.94 |
| | 66 | K | 4.96E-09 | 0.96 |
| | 67 | S | 4.25E-09 | 1.01 |
| CDR grafting region-H3 | **99** | **R** | **1.06E-08** | **0.37** |
| | **100** | **T** | **8.44E-09** | **0.47** |
| | 101 | Y | 7.68E-09 | 0.61 |
| | **102** | **Y** | **4.91E-08** | **0.07** |
| | 103 | S | 4.04E-09 | 0.94 |
| | 104 | N | 5.62E-09 | 0.80 |
| | **105** | **Y** | **1.56E-08** | **0.47** |
| | 106 | E | 1.08E-08 | 0.64 |
| | **107** | **Y** | **6.09E-08** | **0.06** |
| | 108 | Y | 4.70E-09 | 0.91 |
| | 109 | G | 1.05E-08 | 0.61 |
| | **110** | **M** | **2.34E-08** | **0.41** |
| | 111 | D | 1.13E-08 | 0.62 |
| | 112 | Y | 3.73E-09 | 1.05 |

### [Reference Example 2] Investigation of microglia in a mouse model of Parkinson's disease <Pathology analysis>

Fourteen-month-old A53T mutant human α-synuclein-Tg mice were subjected to fixation of the brains with perfusion. The fixed brains were removed, and 5-µm-thick paraffin sections of the brains were prepared. After deparaffinization, the brain sections were boiled in citrate buffer (pH 6) for 30 minutes to activate the antigen, and then double stained with anti-gpNMB antibody AF2330 (R&D, 2 µg/mL) + a FITC-donkey anti-goat IgG antibody (Jackson Lab) and an anti-Iba1 antibody (WAKO, 5µg/mL) + a rhodamine-donkey anti-rabbit IgG antibody (Jackson Lab). The results are shown in Figure 24.

### [Example 23] Cognitive function improvement analysis of anti-gpNMB antibodies in Parkinson's disease model mice (A53T mutant human α-synuclein-Tg mice)

Eight- to nine-month-old A53T mutant human α-synuclein-Tg mice were used to analyze the effect of the anti-gpNMB antibodies of the present invention on cognitive function improvement after administration of the anti-gpNMB antibodies once a week for a total of five doses, using the Morris water maze test. Nine- to ten-month-old non-Tg (wild-type) mice born in the same abdomen were used as comparison subjects.

### <Group configuration>

*Evaluation antibody group 1: Anti-gpNMB mouse monoclonal antibody GPN06-1, 2.5 mg/mL in 150mM NaCl buffer (pH6.5) containing 0.02M histidine, was administered intraperitoneally at 0.4 mL once weekly for a total of 5 times (n=10).
*Evaluation antibody group 2: anti-gpNMB mouse monoclonal antibody GPN18-2, 2.5 mg/mL in 150mM NaCl buffer (pH6.5) containing 0.02M histidine, was administered intraperitoneally at 0.4 mL once weekly for a total of 5 times (n=9).
*αSyn-Tg group: 150mM NaCl buffer (pH6.5) containing 0.02M histidine was administered intraperitoneally at 0.4 mL once weekly for a total of 5 times (n=10).
*Non-Tg group: 150mM NaCl buffer containing 0.02M histidine (pH6.5) was administered intraperitoneally at 0.4 mL once weekly for a total of 5 times (n=12).

### <Water maze test>

*Preparation: A black pool with an inner diameter of 100 cm and a height of 45 cm was filled with water 16 cm deep. The water temperature was adjusted to 21 to 23°C, and the water was kept clear and colorless. After each trial day, feces were removed, and approximately 10 liters of water was replaced.
*Acquisition test: A 15 cm high transparent platform was sunk 20 cm from the wall (30 cm from the center). The pool was divided into four quadrants, including the area where the platform was sunk, and mice were randomly placed only in one of the three quadrants without the platform. Each trial was limited to 60 seconds and was conducted at a rate of 5 trials/day, with an interval of approximately 5 minutes between trials. The escape time to reach the platform (escape time) was recorded as data. Mice that did not escape within 60 seconds were guided to the platform by an operator, and the escape time was recorded as 60 seconds. Mice on the platform were removed from the pool after 10 seconds, and allowed to act freely and dry their bodies until the next trial. The average seconds of escape time for five trials was used for the daily mouse performance. Trials were conducted for four days.

The acquisition test was finished when the performance of the non-Tg group stabilized, and a probe test was conducted the next day.
*Probe test: The day after the final day of the acquisition test, the platform was removed from the pool, and a 60-second free swim of each mouse was captured by video camera. The time that the free-swimming mouse spent swimming in the target quadrant of the four quadrants was measured and expressed as a percentage of the 60-second period.

Significant differences for each test were determined using repeated measure and Fisher's least significant difference test (Fisher's PLSD), and significant differences for the probe test were determined using Fisher's least significant difference test (Fisher's PLSD).

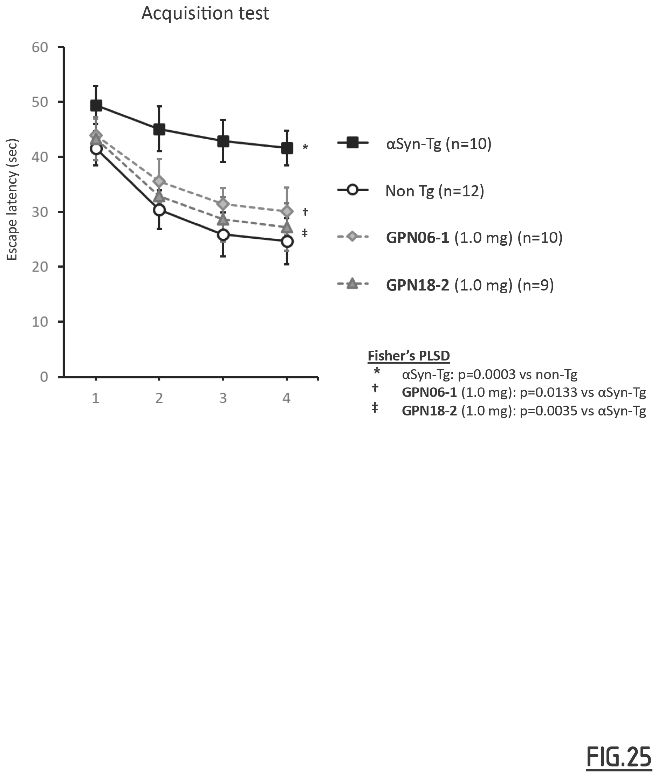

The results of the Acquisition test are shown in the graph in Figure 25. The analysis showed that the αSyn-Tg (A53T mutation human α-synuclein-Tg mice) group had a significantly longer Escape Latency in the Morris water maze test compared to Non-Tg (littermate wild-type mice). On the other hand, the A53T mutation human α-synuclein-Tg mice group that received anti-gpNMB antibody GPN06-1 or GPN18-2 showed a statistically significant reduction in the time to reach the hidden platform.

### [Example 24] Motor function disorder control analysis of anti-gpNMB antibodies in Parkinson's disease model mice (A53T mutation human α-synuclein-Tg mice)

To evaluate the therapeutic effect of anti-gpNMB antibodies on the motor function of Parkinson's disease model mice (A53T mutation human α-synuclein-Tg mice), mice that had been evaluated for cognitive function in the Morris water maze test in Example 23 were subjected to the evaluation of motor function next week. No motor deficits were observed during the Morris water maze test.

### <Rotarod test>

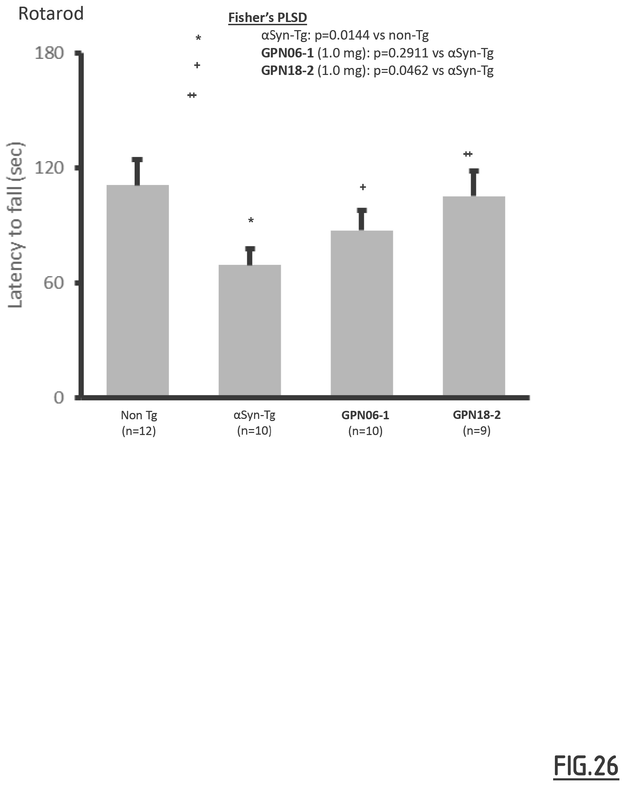

Regarding motor function, the Rotarod test was initially performed. Specifically, the mice were evaluated by the Rotarod test using the MK-610A Rotarod Treadmill for Mice (Muromachi Machinery, Tokyo, Japan). On the first day, the mice were trained to remain on the rod rotating at 5 rpm for 180 seconds. Next, the rotational speed was increased from 4 rpm to 40 rpm over 240 seconds, and this accelerated rotor-rod training was repeated twice. The next day, the accelerated Rotarod test was performed twice with a 1-hour interval, and the time the mouse fell off the rod was recorded. If the mouse clung to the rod and rotated completely downward, it was considered to have fallen off the rod; the average time for the two trials was calculated and represented in the graph (Figure 26).

### <Inverted Screen test>

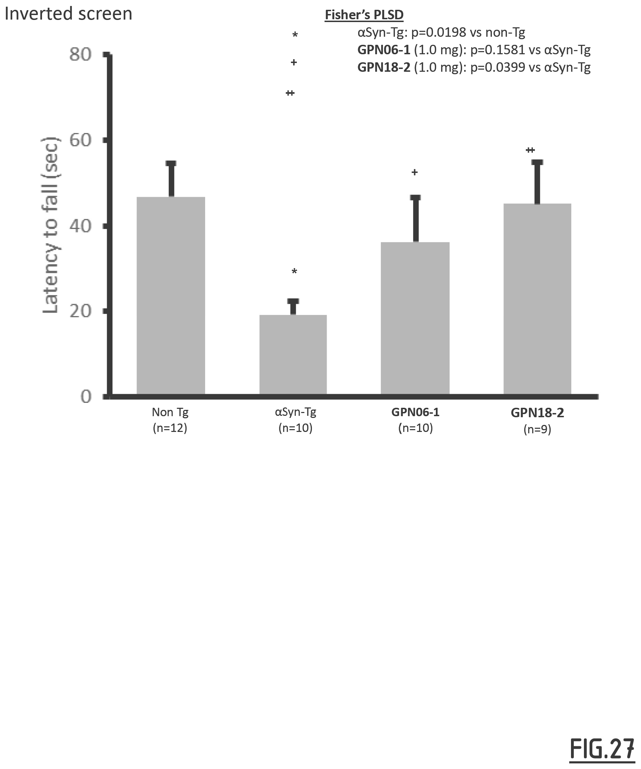

Next to the Rotarod test mentioned above, the mice were further evaluated by the Inverted Screen test with respect to motor strength and adjustment ability. The mice were individually placed on a circular wire screen (24 cm diameter, 1.2 cm x 1.2 cm aperture) mounted horizontally on a metal rod. The screen was then rotated 180°, and the behavior of the mouse during the rotation was observed for 2 minutes, and the time the mouse fell off the screen was recorded. If the mouse climbed to the top of the screen or remained clinging to the bottom of the screen, a waiting time of 120 seconds was assigned; the average time for the two trials was calculated and represented in the graph (Figure 27).

### [Example 25] Pathology analysis of administration with anti-gpNMB antibodies in Parkinson's disease model mice (A53T mutation human α-synuclein-Tg mice)

### <Pathology Analysis>

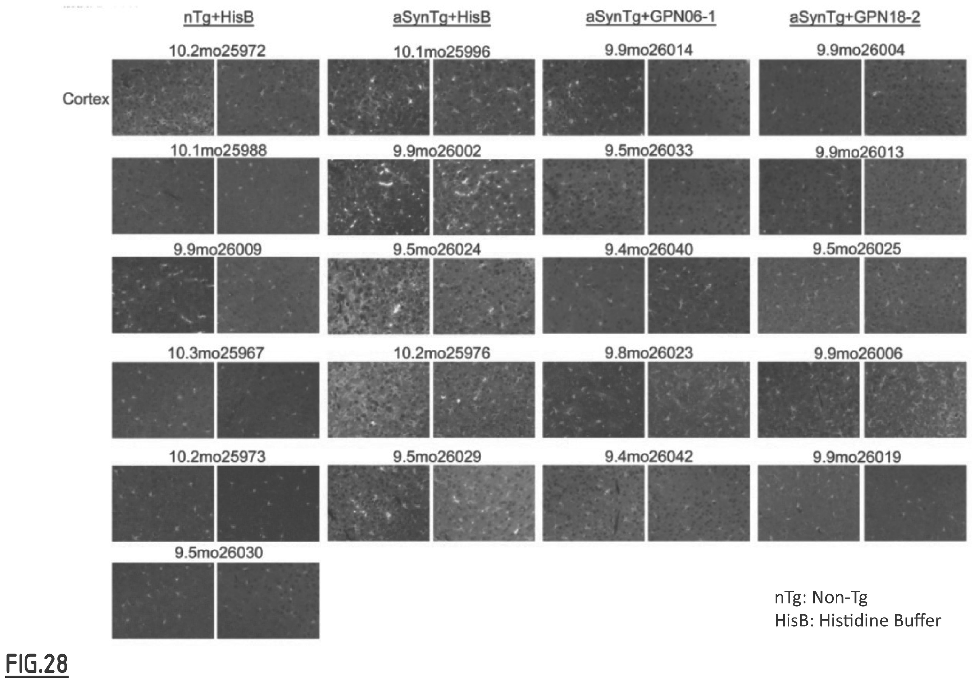

Anti-gpNMB antibodies: 9- to 10-month-old A53T mutation human α-synuclein-Tg mice treated with 1 mg/head of GPN18-2 or GPN06-1 and 9- to 10-month-old A53T mutation human α-synuclein-Tg mice treated with 1 mg/head of medium (comparative control) were subjected to fixation of the brains with perfusion. The fixed brains were removed, and eight 5-µm-thick paraffin sections of the brains were prepared. After deparaffinization, the brain sections were boiled in citrate buffer (pH 6) for 30 minutes to activate the antigen, and then double stained with anti-gpNMB antibody AF2330 (R&D, 2 µg/mL) + a FITC-donkey anti-goat IgG antibody (Jackson Lab) and an anti-Iba1 antibody (WAKO, 5µg/mL) + a rhodamine-donkey anti-rabbit IgG antibody (Jackson Lab). After treatment with lipofuscin quencher True Black plus (Biotium) for 10 minutes at room temperature, the tissue was mounted with DAPI-containing mountant (Vector). The results are shown in Figure 28.

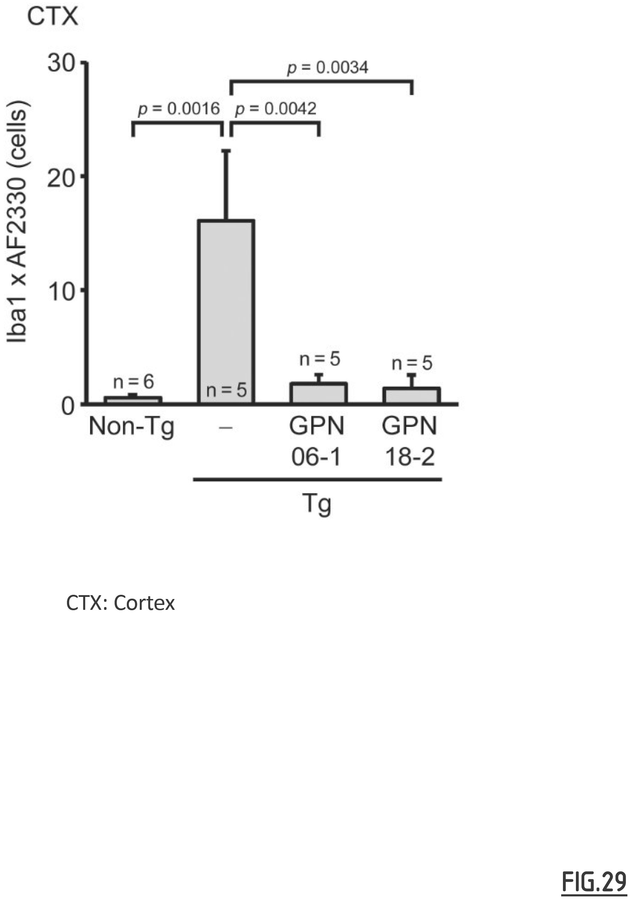

Statistical analysis was performed on the above immunostaining results. Specifically, the co-staining signals of gpNMB (AF2330) and Iba1 were extracted and the area of each stained region was quantified using ImageJ image processing software. The obtained values were used for graphing. The resulting graph is shown in Figure 29. The significance test was performed by ANOVA for the three groups using StatView, and only those values below the p-value of 0.05 by Fisher's PLSD were considered "significantly different" and their p-values are indicated in the graph.

The results of the above analysis confirmed that administration of anti-gpNMB monoclonal antibodies GPN18-2 and GPN06-1 reduced gpNMB-positive microglia in the brain with statistically significant differences. This means that mal-functional microglia, which form at least a part of gpNMB-positive microglia, were reduced. The fact that such reduction of mal-functional microglia improved the pathophysiology is also supported by the results showing that synuclein oligomers and phosphorylated synuclein were removed and the number of synapses was prevented from decreasing or restored, as described below.

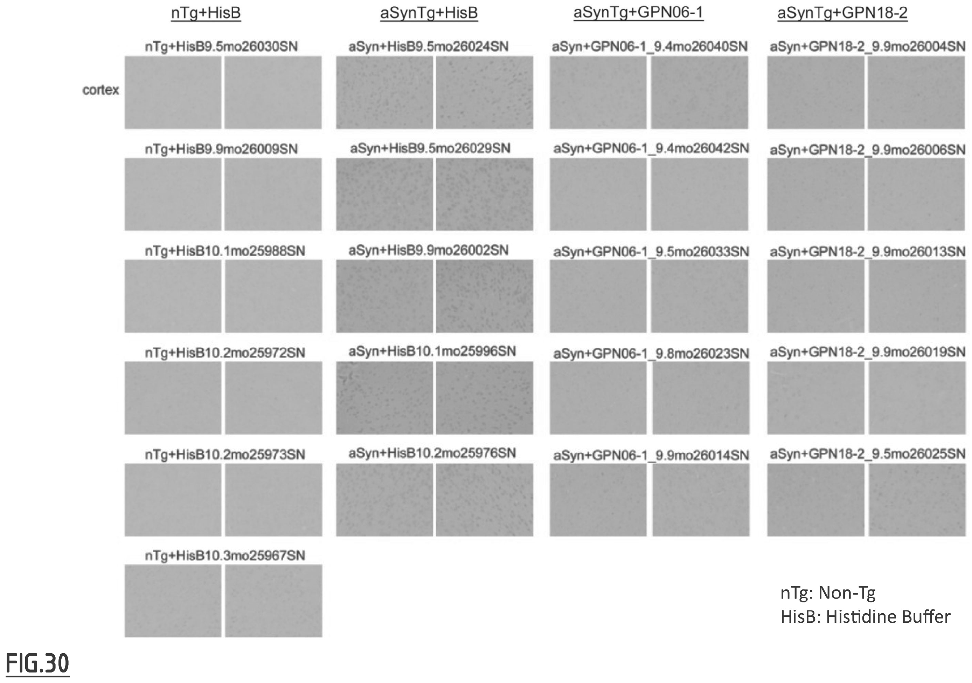

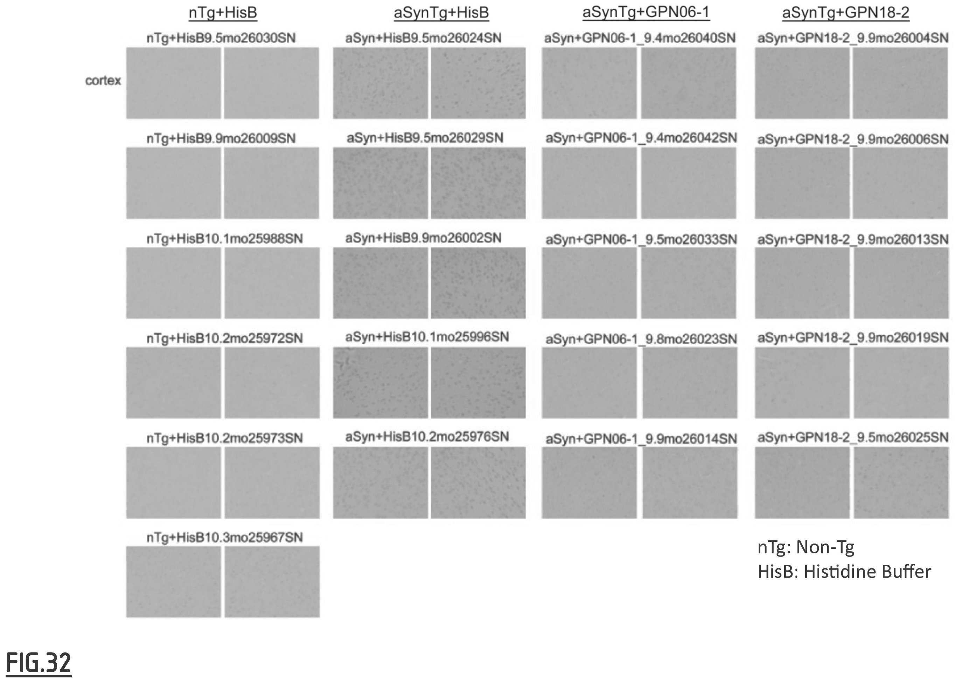

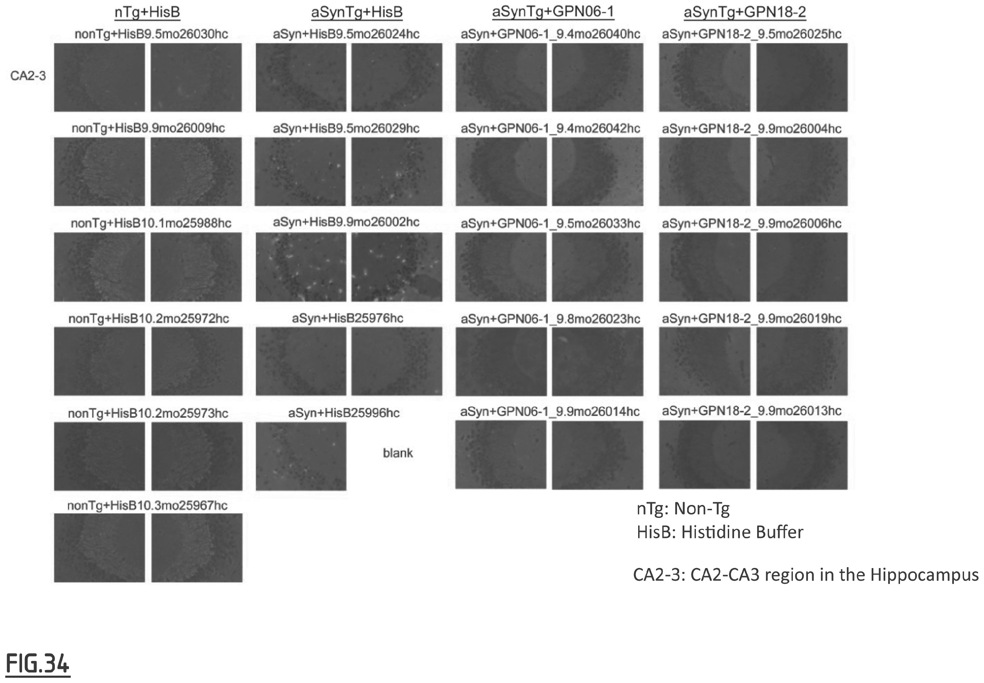

Staining of phosphorylated α-synuclein, α-synuclein oligomers, and synaptophysin was also performed in parallel. Brain sections were boiled in 10mM hydrochloric acid (pH2) for 10 minutes, and reacted with [pSer129] α-synuclein rabbit monoclonal antibody (Abcam-Epitomics, Cambridge, UK). The brain sections were also boiled in 10mM citrate buffer (pH6) for 30 minutes, and reacted with antibodies to α-synuclein oligomer (Syn33; Merck Millipore, Darmstadt, Germany) and synaptophysin (SVP-38; Sigma-Aldrich). Then, for synuclein staining, biotinylated secondary antibodies, HRP-labeled avidin-biotin complex, and DAB (diaminobentidine) were reacted to develop color. Synaptophysin, on the other hand, was stained with FITC-goat anti-mouse IgG antibody (Jackson Lab). After treatment with lipofuscin quencher True Black plus (Biotium) for 10 minutes at room temperature, the cells were mounted with DAPI-containing mountant (Vector). The results of staining for phosphorylated α-synuclein, α-synuclein oligomers, and synaptophysin are shown in Figures 30, 32, and 34, respectively.

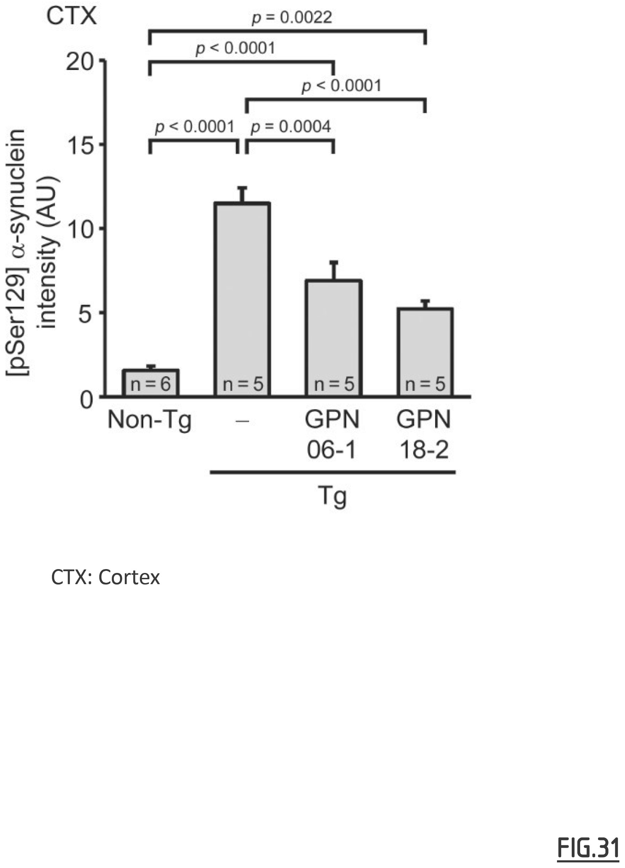

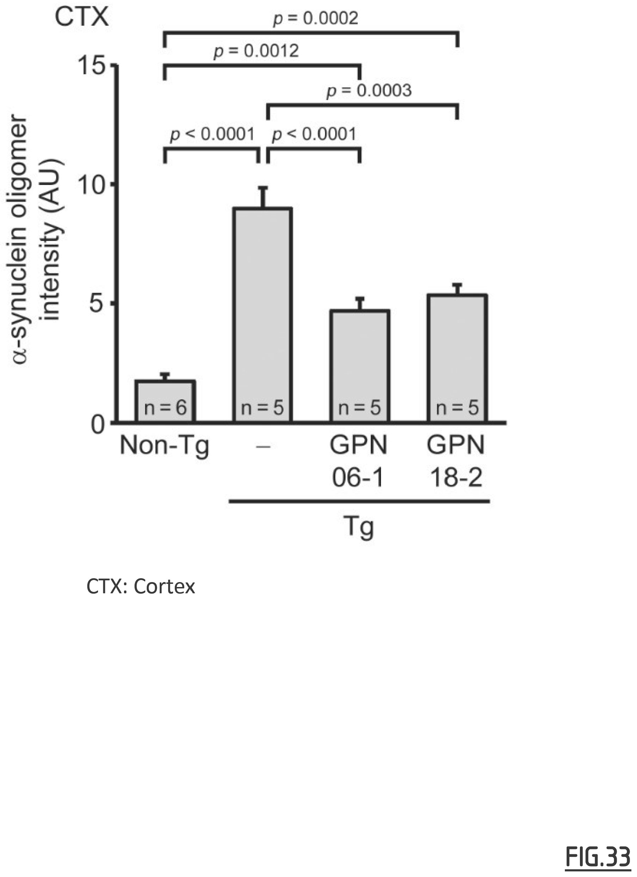

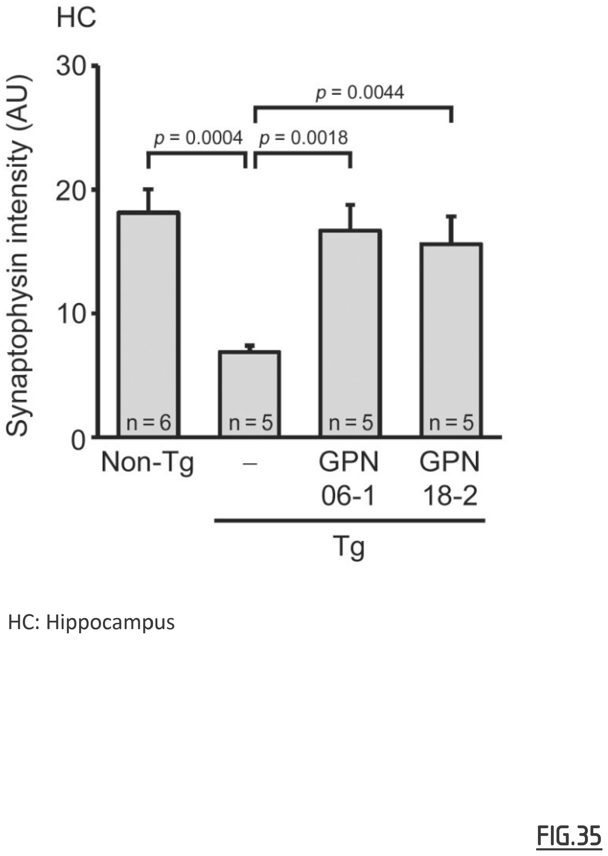

Statistical analysis was performed on the above immunostaining results. Specifically, phosphorylated α-synuclein, α-synuclein oligomers, and synaptophysin staining signals were extracted, respectively, and the area of each stained region was quantified using ImageJ image processing software. The obtained values were used for graphing. The resulting graphs are shown in Figures 31, 33, and 35 for phosphorylated α-synuclein, α-synuclein oligomers, and synaptophysin, respectively. The significance test was performed by ANOVA for the three groups using StatView, and only those values below the p-value of 0.05 by Fisher's PLSD were considered "significantly different" and their p-values are indicated in the graphs.

### INDUSTRIAL APPLICABILITY

The present invention provides an anti-gpNMB antibody that can remove mal-functional microglia and/or removing amyloid β oligomers and/or phosphorylated tau and/or synuclein oligomers, which are toxic proteins, and/or restore the number of synapses and/or inhibit or restore declines in cognitive functions or motor functions, by specifically binding to mammalian microglia expressing gpNMB. Therefore, the present invention can be used for, e.g., the treatment, prevention, or diagnosis of diseases including neurodegenerative diseases associated with microglia, such as Alzheimer's disease and Parkinson's disease.

### SEQUENCE LISTING FREE TEXT

SEQ ID NO: 1
   Sequence Name: Antibody GPN05-1 CDR-H1
   Sequence Type: AA
   Organism Name: Mus musculus
      GYAFSNHL
SEQ ID NO: 2
   Sequence Name: Antibody GPN05-1 CDR-H1
   Sequence Type: DNA
   Organism Name: synthetic construct
      GGCTACGCCTTCAGCAACCACCTG
SEQ ID NO: 3
   Sequence Name: Antibody GPN05-1 CDR-H2
   Sequence Type: AA
   Organism Name: Mus musculus
      INPGSGGT
SEQ ID NO: 4
   Sequence Name: Antibody GPN05-1 CDR-H2
   Sequence Type: DNA
   Organism Name: synthetic construct
      ATCAACCCTGGCAGCGGCGGCACC
SEQ ID NO: 5
   Sequence Name: Antibody GPN05-1 CDR-H3
   Sequence Type: AA
   Organism Name: Mus musculus
      ARGANWDVGFAY
SEQ ID NO: 6
   Sequence Name: Antibody GPN05-1 CDR-H3
   Sequence Type: DNA
   Organism Name: synthetic construct
      GCTAGAGGCGCTAATTGGGACGTGGGGTTCGCGTAT
SEQ ID NO: 7
   Sequence Name: Antibody GPN05-1 CDR-L1
   Sequence Type: AA
   Organism Name: Mus musculus
      QDIVKN
SEQ ID NO: 8
   Sequence Name: Antibody GPN05-1 CDR-L1
   Sequence Type: DNA
   Organism Name: synthetic construct
      CAAGACATCGTGAAGAAC
SEQ ID NO: 9
   Sequence Name: Antibody GPN05-1 CDR-L2
   Sequence Type: AA
   Organism Name: Mus musculus
      YAT
SEQ ID NO: 10
   Sequence Name: Antibody GPN05-1 CDR-L2
   Sequence Type: DNA
   Organism Name: synthetic construct
      TACGCCACC
SEQ ID NO: 11
   Sequence Name: Antibody GPN05-1 CDR-L3
   Sequence Type: AA
   Organism Name: Mus musculus
      LQFHEFPRT
SEQ ID NO: 12
   Sequence Name: Antibody GPN05-1 CDR-L3
   Sequence Type: DNA
   Organism Name: synthetic construct
      CTGCAGTTCCACGAGTTCCCTAGAACC
SEQ ID NO: 13
   Sequence Name: Antibody GPN05-1 VH
   Sequence Type: AA
   Organism Name: Mus musculus 
QVQLQQSGAELVRPGTSVKVSCKASGYAFSNHLIEWVKQRPGQGLEWIGVINPGS
GGTNYIEKFKGKATLTADKSSSTAYMQLSSLTSDDSAVYFCARGANWDVGFAYW
GQGTLVTVSA
SEQ ID NO: 14
   Sequence Name: Antibody GPN05-1 VH
   Sequence Type: DNA
   Organism Name: synthetic construct 
CAAGTGCAGCTGCAGCAATCTGGCGCCGAGCTGGTGAGACCTGGCACAAGCGT
GAAGGTGAGCTGCAAGGCTAGCGGCTACGCCTTCAGCAACCACCTGATCGAGT
GGGTGAAGCAGAGACCTGGCCAAGGCCTGGAGTGGATCGGCGTGATCAACCCT
GGCAGCGGCGGCACCAACTACATCGAGAAGTTCAAGGGCAAGGCCACCCTGA
CCGCCGACAAGAGCAGCAGCACCGCCTACATGCAGCTGAGCAGCCTGACAAGC
GACGACAGCGCCGTGTACTTCTGCGCTAGAGGCGCTAATTGGGACGTGGGGTT
CGCGTATTGGGGCCAAGGCACCCTGGTGACCGTGAGCGCC
SEQ ID NO: 15
   Sequence Name: Antibody GPN05-1 VL
   Sequence Type: AA
   Organism Name: Mus musculus 
EIQMTQSPSSMSASLGDRITITCQATQDIVKNLNWYQQKPGKPPSFLIYYATELAEG
VPSRFSGSGSGSDFSLTISNLESEDFADYYCLQFHEFPRTFGGGTKLEIK
SEQ ID NO: 16
   Sequence Name: Antibody GPN05-1 VL
   Sequence Type: DNA
   Organism Name: synthetic construct 
GAGATTCAGATGACACAGAGCCCTAGCAGCATGAGCGCTAGCCTGGGCGACAG
AATCACCATCACCTGCCAAGCCACCCAAGACATCGTGAAGAACCTGAACTGGT
ATCAGCAGAAGCCTGGCAAGCCTCCTAGCTTCCTGATCTACTACGCCACCGAG
CTGGCCGAGGGCGTGCCTAGCAGATTCAGCGGCAGCGGCAGCGGCAGCGACTT
CAGCCTGACCATCAGCAACCTGGAGAGCGAGGACTTCGCCGACTACTACTGCC
TGCAGTTCCACGAGTTCCCTAGAACCTTCGGCGGCGGCACCAAGCTGGAGATC
AAG
SEQ ID NO: 17
   Sequence Name: Antibody GPN06-1 CDR-H1
   Sequence Type: AA
   Organism Name: Mus musculus
      GYTFTDNW
SEQ ID NO: 18
   Sequence Name: Antibody GPN06-1 CDR-H1
   Sequence Type: DNA
   Organism Name: synthetic construct
      GGCTACACCTTCACCGACAACTGG
SEQ ID NO: 19
   Sequence Name: Antibody GPN06-1 CDR-H2
   Sequence Type: AA
   Organism Name: Mus musculus
      IDPSDSFT
SEQ ID NO: 20
   Sequence Name: Antibody GPN06-1 CDR-H2
   Sequence Type: DNA
   Organism Name: synthetic construct
      ATCGACCCTAGCGACAGCTTCACC
   SEQ ID NO: 21
   Sequence Name: Antibody GPN06-1 CDR-H3
   Sequence Type: AA
   Organism Name: Mus musculus
      TRSGYYGSPKLGGDY
   SEQ ID NO: 22
   Sequence Name: Antibody GPN06-1 CDR-H3
   Sequence Type: DNA
   Organism Name: synthetic construct
      ACAAGAAGCGGCTACTATGGCTCCCCTAAATTGGGCGGCGACTAC
   SEQ ID NO: 23
   Sequence Name: Antibody GPN06-1 CDR-L1
   Sequence Type: AA
   Organism Name: Mus musculus
      SSISY
SEQ ID NO: 24
   Sequence Name: Antibody GPN06-1 CDR-L1
   Sequence Type: DNA
   Organism Name: synthetic construct
      AGCAGCATCAGCTAC
SEQ ID NO: 25
   Sequence Name: Antibody GPN06-1 CDR-L2
   Sequence Type: AA
   Organism Name: Mus musculus
      STS
SEQ ID NO: 26
   Sequence Name: Antibody GPN06-1 CDR-L2
   Sequence Type: DNA
   Organism Name: synthetic construct
      AGCACAAGC
SEQ ID NO: 27
   Sequence Name: Antibody GPN06-1 CDR-L3
   Sequence Type: AA
   Organism Name: Mus musculus
      HQWNSYPCT
SEQ ID NO: 28
   Sequence Name: Antibody GPN06-1 CDR-L3
   Sequence Type: DNA
   Organism Name: synthetic construct
      CATCAGTGGAACAGCTACCCTTGCACC
SEQ ID NO: 29
   Sequence Name: Antibody GPN06-1 VH
   Sequence Type: AA
   Organism Name: Mus musculus 
QVQLQQPGAEIVRPGASVKLSCKASGYTFTDNWMGWVKQRPGQGLEWIGAIDPS
DSFTNYNQNFKGKATLTVDTSSSTAYMQLSSLTSEDSAVYFCTRSGYYGSPKLGG
DYWGQGTTLTVSS
SEQ ID NO: 30
   Sequence Name: Antibody GPN06-1 VH
   Sequence Type: DNA
   Organism Name: synthetic construct 
CAAGTGCAGCTGCAGCAGCCTGGCGCCGAGATCGTGAGACCTGGCGCTAGCGT
GAAGCTGAGCTGCAAGGCTAGCGGCTACACCTTCACCGACAACTGGATGGGCT
GGGTGAAGCAGAGACCTGGCCAAGGCCTGGAGTGGATCGGCGCCATCGACCCT
AGCGACAGCTTCACCAACTACAATCAGAACTTCAAGGGCAAGGCGACGTTAAC
CGTCGACACAAGCAGCAGCACCGCCTACATGCAGCTGAGCAGCCTGACAAGCG
AGGACAGCGCCGTGTACTTCTGCACAAGAAGCGGCTACTATGGCTCCCCTAAA
TTGGGCGGCGACTACTGGGGCCAAGGCACCACACTGACGGTTAGCTCC
SEQ ID NO: 31
   Sequence Name: Antibody GPN06-1 VL
   Sequence Type: AA
   Organism Name: Mus musculus 
QIVLTQSPAIMSASLGEEITLTCSASSSISYMHWYQQKSGTSPKLLIYSTSNLASGVP
SRFSGSGSGTFYSLTISSVEAEDAADYYCHQWNSYPCTFGGGTKLEIK
SEQ ID NO: 32
   Sequence Name: Antibody GPN06-1 VL
   Sequence Type: DNA
   Organism Name: synthetic construct 
CAGATCGTGCTGACACAGAGCCCTGCCATCATGAGCGCTAGCCTGGGCGAGGA
GATCACCCTGACCTGCAGCGCTAGCAGCAGCATCAGCTACATGCACTGGTATC
AGCAGAAGAGCGGTACAAGCCCTAAGCTGCTGATCTACAGCACAAGCAACCTG
GCTAGCGGCGTGCCTAGCAGATTCAGCGGCAGCGGCAGCGGCACCTTCTACAG
CCTGACCATCAGCAGCGTGGAGGCCGAGGACGCCGCCGACTACTACTGCCATC
AGTGGAACAGCTACCCTTGCACCTTCGGCGGCGGCACCAAGCTGGAGATCAAG
SEQ ID NO: 33
   Sequence Name: Antibody GPN07-1 CDR-H1
   Sequence Type: AA
   Organism Name: Mus musculus
      GYTFTDYW
SEQ ID NO: 34
   Sequence Name: Antibody GPN07-1 CDR-H1
   Sequence Type: DNA
   Organism Name: synthetic construct
      GGCTACACCTTCACCGACTACTGG
SEQ ID NO: 35
   Sequence Name: Antibody GPN07-1 CDR-H2
   Sequence Type: AA
   Organism Name: Mus musculus
      IDPSDSYT
SEQ ID NO: 36
   Sequence Name: Antibody GPN07-1 CDR-H2
   Sequence Type: DNA
   Organism Name: synthetic construct
      ATCGACCCTAGCGACAGCTACACC
SEQ ID NO: 37
   Sequence Name: Antibody GPN07-1 CDR-H3
   Sequence Type: AA
   Organism Name: Mus musculus
      ARSGYYGSPKLGGDY
SEQ ID NO: 38
   Sequence Name: Antibody GPN07-1 CDR-H3
   Sequence Type: DNA
   Organism Name: synthetic construct
      GCTAGAAGCGGCTACTATGGCTCCCCTAAATTGGGCGGCGACTAC
SEQ ID NO: 39
   Sequence Name: Antibody GPN07-1 CDR-L1
   Sequence Type: AA
   Organism Name: Mus musculus
      SSISY
SEQ ID NO: 40
   Sequence Name: Antibody GPN07-1 CDR-L1
   Sequence Type: DNA
   Organism Name: synthetic construct
      AGCAGCATCAGCTAC
SEQ ID NO: 41
   Sequence Name: Antibody GPN07-1 CDR-L2
   Sequence Type: AA
   Organism Name: Mus musculus
      STS
SEQ ID NO: 42
   Sequence Name: Antibody GPN07-1 CDR-L2
   Sequence Type: DNA
   Organism Name: synthetic construct
      AGCACAAGC
SEQ ID NO: 43
   Sequence Name: Antibody GPN07-1 CDR-L3
   Sequence Type: AA
   Organism Name: Mus musculus
      HQWNSYPCT
SEQ ID NO: 44
   Sequence Name: Antibody GPN07-1 CDR-L3
   Sequence Type: DNA
   Organism Name: synthetic construct
      CATCAGTGGAACAGCTACCCTTGCACC
SEQ ID NO: 45
   Sequence Name: Antibody GPN07-1 VH
   Sequence Type: AA
   Organism Name: Mus musculus 
QVQLQQPGAEIVRPGASVKLSCKASGYTFTDYWMNWVKQRPGQGLEWIGAIDPS
DSYTNYNQEFNGKATLTVDTSSSTAYMQLSSLTSEDSAVYFCARSGYYGSPKLGG
DYWGQGTTLTVSS
SEQ ID NO: 46
   Sequence Name: Antibody GPN07-1 VH
   Sequence Type: DNA
   Organism Name: synthetic construct 
CAAGTGCAGCTGCAGCAGCCTGGCGCCGAGATCGTGAGACCTGGCGCTAGCGT
GAAGCTGAGCTGCAAGGCTAGCGGCTACACCTTCACCGACTACTGGATGAACT
GGGTGAAGCAGAGACCTGGCCAAGGCCTGGAGTGGATCGGCGCCATCGACCCT
AGCGACAGCTACACCAACTACAACCAAGAGTTCAACGGCAAGGCGACGTTAAC
CGTCGACACAAGCAGCAGCACCGCCTACATGCAGCTGAGCAGCCTGACAAGCG
AGGACAGCGCCGTGTACTTCTGCGCTAGAAGCGGCTACTATGGCTCCCCTAAA
TTGGGCGGCGACTACTGGGGCCAAGGCACCACACTGACGGTTAGCTCC
SEQ ID NO: 47
   Sequence Name: Antibody GPN07-1 VL
   Sequence Type: AA
   Organism Name: Mus musculus 
QIVLTQSPAIMSASLGEEITLTCSASSSISYMHWYQQKSGTSPKLLIYSTSNLASGVP
SRFSGSGSGTFYSLTISSVEAEDAADYYCHQWNSYPCTFGGGTKLEIK
SEQ ID NO: 48
   Sequence Name: Antibody GPN07-1 VL
   Sequence Type: DNA
   Organism Name: synthetic construct 
CAGATCGTGCTGACACAGAGCCCTGCCATCATGAGCGCTAGCCTGGGCGAGGA
GATCACCCTGACCTGCAGCGCTAGCAGCAGCATCAGCTACATGCACTGGTATC
AGCAGAAGAGCGGTACAAGCCCTAAGCTGCTGATCTACAGCACAAGCAACCTG
GCTAGCGGCGTGCCTAGCAGATTCAGCGGCAGCGGCAGCGGCACCTTCTACAG
CCTGACCATCAGCAGCGTGGAGGCCGAGGACGCCGCCGACTACTACTGCCATC
AGTGGAACAGCTACCCTTGCACCTTCGGCGGCGGCACCAAGCTGGAGATCAAG
SEQ ID NO: 49
   Sequence Name: Antibody GPN11-10 CDR-H1
   Sequence Type: AA
   Organism Name: Mus musculus
      GYTFTDCY
SEQ ID NO: 50
   Sequence Name: Antibody GPN11-10 CDR-H1
   Sequence Type: DNA
   Organism Name: synthetic construct
      GGGTACACCTTTACCGACTGCTAC
SEQ ID NO: 51
   Sequence Name: Antibody GPN11-10 CDR-H2
   Sequence Type: AA
   Organism Name: Mus musculus
      INPNSGYT
SEQ ID NO: 52
   Sequence Name: Antibody GPN11-10 CDR-H2
   Sequence Type: DNA
   Organism Name: synthetic construct
      ATCAACCCCAACAGTGGCTACACG
SEQ ID NO: 53
   Sequence Name: Antibody GPN11-10 CDR-H3
   Sequence Type: AA
   Organism Name: Mus musculus
      TRGDYYTSNLYDWFAY
SEQ ID NO: 54
   Sequence Name: Antibody GPN11-10 CDR-H3
   Sequence Type: DNA
   Organism Name: synthetic construct
      ACAAGAGGCGATTACTACACCTCCAATCTCTATGACTGGTTTGCATAC
SEQ ID NO: 55
   Sequence Name: Antibody GPN11-10 CDR-L1
   Sequence Type: AA
   Organism Name: Mus musculus
      QTVGTV
SEQ ID NO: 56
   Sequence Name: Antibody GPN11-10 CDR-L1
   Sequence Type: DNA
   Organism Name: synthetic construct
      CAGACCGTAGGGACTGTG
SEQ ID NO: 57
   Sequence Name: Antibody GPN11-10 CDR-L2
   Sequence Type: AA
   Organism Name: Mus musculus
      WAT
SEQ ID NO: 58
   Sequence Name: Antibody GPN11-10 CDR-L2
   Sequence Type: DNA
   Organism Name: synthetic construct
      TGGGCAACG
SEQ ID NO: 59
   Sequence Name: Antibody GPN11-10 CDR-L3
   Sequence Type: AA
   Organism Name: Mus musculus
      LQYGSVPPT
SEQ ID NO: 60
   Sequence Name: Antibody GPN11-10 CDR-L3
   Sequence Type: DNA
   Organism Name: synthetic construct
      CTGCAGTATGGCAGCGTTCCACCCACC
SEQ ID NO: 61
   Sequence Name: Antibody GPN11-10 VH
   Sequence Type: AA
   Organism Name: Mus musculus 
QVNLLQSGAALVKPGASVRLSCKTSGYTFTDCYIHWVRQSHGKSLEWIGFINPNSG
YTFYNEKFKSKATLTVAKSTNTAYMELSRLTSEDSATYFCTRGDYYTSNLYDWFA
YWGQGTLVTVSS
SEQ ID NO: 62
   Sequence Name: Antibody GPN11-10 VH
   Sequence Type: DNA
   Organism Name: synthetic construct 
CAGGTCAATCTGCTGCAGTCCGGAGCTGCATTGGTCAAACCCGGAGCCAGCGT
ACGGCTTTCCTGTAAGACATCTGGGTACACCTTTACCGACTGCTACATTCACTG
GGTAAGGCAGTCACATGGGAAGAGCCTTGAGTGGATTGGCTTCATCAACCCCA
ACAGTGGCTACACGTTCTACAACGAGAAGTTCAAGTCCAAAGCTACACTGACA
GTGGCCAAAAGCACCAACACTGCCTATATGGAGCTGTCACGCTTGACATCCGA
AGATAGTGCCACATATTTCTGCACAAGAGGCGATTACTACACCTCCAATCTCTA
TGACTGGTTTGCATACTGGGGTCAAGGCACCCTGGTTACCGTGTCATCT
SEQ ID NO: 63
   Sequence Name: Antibody GPN11-10 VL
   Sequence Type: AA
   Organism Name: Mus musculus 
DTVMTQSPASMSTSVGERVTVNCKASQTVGTVVAWFQQKPGQSPKRLIYWATNR
HTGVPDRFTGSGFGRDFSLTISNVEAEDLAVYYCLQYGSVPPTFGGGTKLELK
SEQ ID NO: 64
   Sequence Name: Antibody GPN11-10 VL
   Sequence Type: DNA
   Organism Name: synthetic construct 
GACACAGTCATGACCCAATCACCAGCATCCATGAGCACTTCAGTTGGCGAGAG
AGTGACCGTGAACTGTAAGGCCTCTCAGACCGTAGGGACTGTGGTTGCCTGGT
TCCAGCAGAAACCAGGGCAAAGCCCCAAACGGCTGATATACTGGGCAACGAA
TCGACACACTGGTGTACCTGACCGCTTTACCGGCAGTGGGTTCGGAAGGGACT
TCTCCCTCACCATTTCCAACGTCGAAGCCGAAGATCTTGCCGTGTACTATTGCC
TGCAGTATGGCAGCGTTCCACCCACCTTTGGAGGAGGCACAAAGTTGGAGCTG
AAG
SEQ ID NO: 65
   Sequence Name: Antibody GPN15-2 CDR-H1
   Sequence Type: AA
   Organism Name: Mus musculus
      GYTFTNHN
SEQ ID NO: 66
   Sequence Name: Antibody GPN15-2 CDR-H1
   Sequence Type: DNA
   Organism Name: synthetic construct
      GGCTACACATTTACCAATCACAAT
SEQ ID NO: 67
   Sequence Name: Antibody GPN15-2 CDR-H2
   Sequence Type: AA
   Organism Name: Mus musculus
      FYTGNGDT
SEQ ID NO: 68
   Sequence Name: Antibody GPN15-2 CDR-H2
   Sequence Type: DNA
   Organism Name: synthetic construct
      TTTTATACAGGAAATGGTGATACT
SEQ ID NO: 69
   Sequence Name: Antibody GPN15-2 CDR-H3
   Sequence Type: AA
   Organism Name: Mus musculus
      ARERYDGYYIAMDY
SEQ ID NO: 70
   Sequence Name: Antibody GPN15-2 CDR-H3
   Sequence Type: DNA
   Organism Name: synthetic construct
      GCAAGAGAAAGGTATGATGGTTACTATATTGCTATGGACTAC
SEQ ID NO: 71
   Sequence Name: Antibody GPN15-2 CDR-L1
   Sequence Type: AA
   Organism Name: Mus musculus
      SSVYY
SEQ ID NO: 72
   Sequence Name: Antibody GPN15-2 CDR-L1
   Sequence Type: DNA
   Organism Name: synthetic construct
      TCGAGTGTGTATTAC
SEQ ID NO: 73
   Sequence Name: Antibody GPN15-2 CDR-L2
   Sequence Type: AA
   Organism Name: Mus musculus
      LTS
SEQ ID NO: 74
   Sequence Name: Antibody GPN15-2 CDR-L2
   Sequence Type: DNA
   Organism Name: synthetic construct
      CTCACATCC
SEQ ID NO: 75
   Sequence Name: Antibody GPN15-2 CDR-L3
   Sequence Type: AA
   Organism Name: Mus musculus
      QQWSSNPQWT
SEQ ID NO: 76
   Sequence Name: Antibody GPN15-2 CDR-L3
   Sequence Type: DNA
   Organism Name: synthetic construct
      CAGCAGTGGAGTAGTAACCCACAGTGGACG
SEQ ID NO: 77
   Sequence Name: Antibody GPN15-2 VH
   Sequence Type: AA
   Organism Name: Mus musculus 
QVQLQQPGAELVKPGASVKMSCKASGYTFTNHNMHWVKQTPGQGLEWIGAFYT
GNGDTSYNQKFKGKATLTADKSSSTAYIQLSSLTSEDSAVYYCARERYDGYYIAM
DYWGQGTSVTVSS
SEQ ID NO: 78
   Sequence Name: Antibody GPN15-2 VH
   Sequence Type: DNA
   Organism Name: synthetic construct 
CAGGTACAACTGCAGCAGCCTGGGGCTGAGCTGGTGAAGCCTGGGGCCTCAGT
GAAGATGTCCTGCAAGGCTTCTGGCTACACATTTACCAATCACAATATGCACTG
GGTAAAGCAGACACCTGGACAGGGCCTGGAATGGATTGGAGCTTTTTATACAG
GAAATGGTGATACTTCCTACAATCAGAAGTTCAAGGGCAAGGCCACATTGACT
GCAGACAAATCCTCCAGCACAGCCTACATACAACTCAGCAGCCTGACATCTGA
GGACTCTGCGGTCTATTACTGTGCAAGAGAAAGGTATGATGGTTACTATATTGC
TATGGACTACTGGGGTCAAGGAACCTCAGTCACCGTCTCCTCA
SEQ ID NO: 79
   Sequence Name: Antibody GPN15-2 VL
   Sequence Type: AA
   Organism Name: Mus musculus 
QIVLTQSPAFMSVSPGEKVTMTCSASSSVYYMFWYQQKPRSSPKPWIYLTSKLASG
VPARFSGSGSGTSYSVTISSMEAEDAATYYCQQWSSNPQWTFGGGTKLEIK
SEQ ID NO: 80
   Sequence Name: Antibody GPN15-2 VL
   Sequence Type: DNA
   Organism Name: synthetic construct 
CAAATTGTTCTCACCCAGTCTCCAGCATTCATGTCTGTATCTCCAGGGGAGAAG
GTCACCATGACCTGCAGTGCCAGCTCGAGTGTGTATTACATGTTCTGGTACCAG
CAGAAGCCAAGATCCTCCCCCAAACCCTGGATTTATCTCACATCCAAGCTGGCT
TCTGGAGTCCCTGCTCGCTTCAGTGGCAGTGGGTCTGGGACCTCTTACTCTGTC
ACAATCAGCAGCATGGAGGCTGAAGATGCTGCCACTTATTACTGCCAGCAGTG
GAGTAGTAACCCACAGTGGACGTTCGGTGGAGGCACCAAGCTGGAAATCAAA
SEQ ID NO: 81
   Sequence Name: Antibody GPN15-3 CDR-H1
   Sequence Type: AA
   Organism Name: Mus musculus
      GFSLTTSGMG
SEQ ID NO: 82
   Sequence Name: Antibody GPN15-3 CDR-H1
   Sequence Type: DNA
   Organism Name: synthetic construct
      GGGTTTTCACTGACCACTTCTGGTATGGGT
SEQ ID NO: 83
   Sequence Name: Antibody GPN15-3 CDR-H2
   Sequence Type: AA
   Organism Name: Mus musculus
      MWWDDDK
SEQ ID NO: 84
   Sequence Name: Antibody GPN15-3 CDR-H2
   Sequence Type: DNA
   Organism Name: synthetic construct
      ATGTGGTGGGATGACGATAAG
SEQ ID NO: 85
   Sequence Name: Antibody GPN15-3 CDR-H3
   Sequence Type: AA
   Organism Name: Mus musculus
      ARRVYSSWFAY
SEQ ID NO: 86
   Sequence Name: Antibody GPN15-3 CDR-H3
   Sequence Type: DNA
   Organism Name: synthetic construct
      GCTCGAAGAGTCTACAGTAGTTGGTTTGCTTAC
SEQ ID NO: 87
   Sequence Name: Antibody GPN15-3 CDR-L1
   Sequence Type: AA
   Organism Name: Mus musculus
      QNVGAN
SEQ ID NO: 88
   Sequence Name: Antibody GPN15-3 CDR-L1
   Sequence Type: DNA
   Organism Name: synthetic construct
      CAGAATGTGGGTGCTAAT
SEQ ID NO: 89
   Sequence Name: Antibody GPN15-3 CDR-L2
   Sequence Type: AA
   Organism Name: Mus musculus
      SAS
SEQ ID NO: 90
   Sequence Name: Antibody GPN15-3 CDR-L2
   Sequence Type: DNA
   Organism Name: synthetic construct
      TCGGCATCC
SEQ ID NO: 91
   Sequence Name: Antibody GPN15-3 CDR-L3
   Sequence Type: AA
   Organism Name: Mus musculus
      QQYNNYPLT
SEQ ID NO: 92
   Sequence Name: Antibody GPN15-3 CDR-L3
   Sequence Type: DNA
   Organism Name: synthetic construct
      CAGCAATATAACAACTATCCTCTCACG
SEQ ID NO: 93
   Sequence Name: Antibody GPN15-3 VH
   Sequence Type: AA
   Organism Name: Mus musculus 
QVTLKESGPGILQPSQTLSLTCSFSGFSLTTSGMGVGWIRQPSGKGLEWLADMWW
DDDKNYNPSLKSRLTISKDNSKKQVFLKIASVDTADTATYYCARRVYSSWFAYWG
QGTLVTVSA
SEQ ID NO: 94
   Sequence Name: Antibody GPN15-3 VH
   Sequence Type: DNA
   Organism Name: synthetic construct 
CAGGTTACTCTGAAAGAGTCTGGCCCTGGAATATTGCAGCCCTCCCAGACCCTC
AGTCTGACTTGTTCTTTCTCTGGGTTTTCACTGACCACTTCTGGTATGGGTGTAG
GCTGGATTCGTCAGCCTTCAGGAAAGGGTCTAGAGTGGCTGGCAGACATGTGG
TGGGATGACGATAAGAATTATAATCCATCCCTGAAGAGCCGGCTCACAATCTC
CAAGGATAACTCCAAAAAGCAGGTATTCCTCAAGATCGCCAGTGTGGACACTG
CAGATACTGCCACTTACTACTGTGCTCGAAGAGTCTACAGTAGTTGGTTTGCTT
ACTGGGGCCAAGGGACTCTGGTCACTGTCTCTGCA
SEQ ID NO: 95
   Sequence Name: Antibody GPN15-3 VL
   Sequence Type: AA
   Organism Name: Mus musculus 
DGDIVMTQSQKFMSTSVGDRVSVTCKASQNVGANVAWYQQKPGQSPKTLIYSAS
YRYGGVPDRFTGSGSGTDFTFTITNVQSEDLAEYFCQQYNNYPLTFGAGTKLDLK
SEQ ID NO: 96
   Sequence Name: Antibody GPN15-3 VL
   Sequence Type: DNA
   Organism Name: synthetic construct GATGGAGACATTGTGATGACCCAGTCTCAAAAATTCATGTCCACATCAGTAGG 
AGACAGGGTCAGCGTCACCTGCAAGGCCAGTCAGAATGTGGGTGCTAATGTAG
CCTGGTATCAACAGAAACCAGGGCAATCTCCTAAAACACTGATTTATTCGGCA
TCCTACCGGTACGGTGGAGTCCCTGATCGCTTCACAGGCAGTGGATCTGGGAC
AGATTTCACTTTCACCATCACTAATGTGCAGTCTGAAGACTTGGCAGAGTATTT
CTGTCAGCAATATAACAACTATCCTCTCACGTTCGGTGCTGGGACCAAGTTGGA
CCTGAAA
SEQ ID NO: 97
   Sequence Name: Antibody GPN18-4 CDR-H1
   Sequence Type: AA
   Organism Name: Mus musculus
      GYTLSSYW
SEQ ID NO: 98
   Sequence Name: Antibody GPN18-4 CDR-H1
   Sequence Type: DNA
   Organism Name: synthetic construct
      GGCTACACTTTAAGTAGTTATTGG
SEQ ID NO: 99
   Sequence Name: Antibody GPN18-4 CDR-H2
   Sequence Type: AA
   Organism Name: Mus musculus
      INPSNNYT
SEQ ID NO: 100
   Sequence Name: Antibody GPN18-4 CDR-H2
   Sequence Type: DNA
   Organism Name: synthetic construct
      ATTAATCCTAGCAATAATTATACT
SEQ ID NO: 101
   Sequence Name: Antibody GPN18-4 CDR-H3
   Sequence Type: AA
   Organism Name: Mus musculus
      ATWGY
SEQ ID NO: 102
   Sequence Name: Antibody GPN18-4 CDR-H3
   Sequence Type: DNA
   Organism Name: synthetic construct
      GCAACATGGGGCTAC
SEQ ID NO: 103
   Sequence Name: Antibody GPN18-4 CDR-L1
   Sequence Type: AA
   Organism Name: Mus musculus
      QDVSTA
SEQ ID NO: 104
   Sequence Name: Antibody GPN18-4 CDR-L1
   Sequence Type: DNA
   Organism Name: synthetic construct
      CAGGATGTGAGTACTGCT
SEQ ID NO: 105
   Sequence Name: Antibody GPN18-4 CDR-L2
   Sequence Type: AA
   Organism Name: Mus musculus
      SAS
SEQ ID NO: 106
   Sequence Name: Antibody GPN18-4 CDR-L2
   Sequence Type: DNA
   Organism Name: synthetic construct
      TCGGCATCT
SEQ ID NO: 107
   Sequence Name: Antibody GPN18-4 CDR-L3
   Sequence Type: AA
   Organism Name: Mus musculus
      QHHYTTTWT
SEQ ID NO: 108
   Sequence Name: Antibody GPN18-4 CDR-L3 Sequence Type: DNA
   Organism Name: synthetic construct
      CAGCACCATTATACTACTACGTGGACG
SEQ ID NO: 109
   Sequence Name: Antibody GPN18-4 VH
   Sequence Type: AA
   Organism Name: Mus musculus 
QVQLQQSGAELTKPGTSVKMSCKASGYTLSSYWMNWIKQRPGQGLEWIGTINPSN
NYTAYNLKFKDKAMLTADKSSSTAYMQLSSLTSEDSAVYYCATWGYWGQGTTL
TVSS
SEQ ID NO: 110
   Sequence Name: Antibody GPN18-4 VH
   Sequence Type: DNA
   Organism Name: synthetic construct 
CAGGTCCAGCTGCAGCAGTCTGGCGCTGAACTGACAAAACCTGGGACCTCAGT
GAAGATGTCCTGCAAGGCCTCTGGCTACACTTTAAGTAGTTATTGGATGAACTG
GATAAAACAGAGGCCTGGACAAGGTCTGGAATGGATTGGGACTATTAATCCTA
GCAATAATTATACTGCATACAATCTGAAGTTTAAGGACAAGGCCATGTTGACT
GCAGACAAATCCTCCAGTACAGCCTACATGCAACTCAGCAGCCTGACATCTGA
GGACTCTGCAGTCTATTACTGTGCAACATGGGGCTACTGGGGCCAAGGCACCA
CTCTCACAGTCTCCTCA
SEQ ID NO: 111
   Sequence Name: Antibody GPN18-4 VL
   Sequence Type: AA
   Organism Name: Mus musculus 
DIVMTQSHKFMSTSVGDRVSITCKASQDVSTAVAWHQQKPGQPPKLLIYSASYRY
TGVPDRFTGSGSGTDFTFTISSVQTEDLAIYYCQHHYTTTWTFGGGTKLEIR
SEQ ID NO: 112
   Sequence Name: Antibody GPN18-4 VL
   Sequence Type: DNA
   Organism Name: synthetic construct 
GACATTGTGATGACCCAGTCTCACAAATTCATGTCCACATCAGTAGGAGACAG
GGTCAGCATCACCTGCAAGGCCAGTCAGGATGTGAGTACTGCTGTAGCCTGGC
ATCAACAGAAACCAGGACAGCCTCCTAAGCTACTGATTTACTCGGCATCTTACC
GGTACACTGGAGTCCCTGATCGCTTCACTGGCAGTGGATCTGGGACGGATTTCA
CTTTCACCATCAGCAGTGTGCAGACTGAAGACCTGGCAATTTATTACTGTCAGC
ACCATTATACTACTACGTGGACGTTCGGTGGAGGCACCAAGCTGGAAATCAGA
SEQ ID NO: 113
   Sequence Name: Antibody GPN18-1 CDR-H1
   Sequence Type: AA
   Organism Name: Mus musculus
      GFSLSTSVMG
SEQ ID NO: 114
   Sequence Name: Antibody GPN18-1 CDR-H1 Sequence Type: DNA
   Organism Name: synthetic construct
      GGCTTCTCTCTGTCCACATCTGTGATGGGC
SEQ ID NO: 115
   Sequence Name: Antibody GPN18-1 CDR-H2
   Sequence Type: AA
   Organism Name: Mus musculus
      IWWDDDK
SEQ ID NO: 116
   Sequence Name: Antibody GPN18-1 CDR-H2
   Sequence Type: DNA
   Organism Name: synthetic construct
      ATCTGGTGGGACGACGATAAG
SEQ ID NO: 117
   Sequence Name: Antibody GPN18-1 CDR-H3
   Sequence Type: AA
   Organism Name: Mus musculus
      ARAYYSNYEYYGMDY
SEQ ID NO: 118
   Sequence Name: Antibody GPN18-1 CDR-H3
   Sequence Type: DNA
   Organism Name: synthetic construct
      GCCCGCGCTTATTACTCTAACTACGAGTACTATGGCATGGATTAC
SEQ ID NO: 119
   Sequence Name: Antibody GPN18-1 CDR-L1
   Sequence Type: AA
   Organism Name: Mus musculus
      QSIVHSNGNTY
SEQ ID NO: 120
   Sequence Name: Antibody GPN18-1 CDR-L1
   Sequence Type: DNA
   Organism Name: synthetic construct
      CAGAGCATTGTTCATAGTAATGGAAACACCTAT
SEQ ID NO: 121
   Sequence Name: Antibody GPN18-1 CDR-L2
   Sequence Type: AA
   Organism Name: Mus musculus
      KVS
SEQ ID NO: 122
   Sequence Name: Antibody GPN18-1 CDR-L2
   Sequence Type: DNA
   Organism Name: synthetic construct
      AAGGTTTCC
SEQ ID NO: 123
   Sequence Name: Antibody GPN18-1 CDR-L3
   Sequence Type: AA
   Organism Name: Mus musculus
      FQGSHVPFT
SEQ ID NO: 124
   Sequence Name: Antibody GPN18-1 CDR-L3
   Sequence Type: DNA
   Organism Name: synthetic construct
      TTTCAAGGTTCACATGTTCCATTCACG
SEQ ID NO: 125
   Sequence Name: Antibody GPN18-1 VH
   Sequence Type: AA
   Organism Name: Mus musculus 
QVTLKESGPGILQPSQTLSLTCSFSGFSLSTSVMGVGWIRQPSGKGLEWLADIWWD
DDKDYNPSLKSRLTISKDTSKNQVFLKIASVDTADTATYYCARAYYSNYEYYGMD
YWGQGTSVTVSS
SEQ ID NO: 126
   Sequence Name: Antibody GPN18-1 VH
   Sequence Type: DNA
   Organism Name: synthetic construct 
CAGGTCACACTGAAAGAGTCTGGTCCCGGCATCCTGCAGCCTTCTCAGACCCTG
TCTCTGACCTGTAGCTTCTCCGGCTTCTCTCTGTCCACATCTGTGATGGGCGTGG
GCTGGATCAGACAGCCTTCTGGCAAGGGCCTGGAGTGGCTGGCTGACATCTGG
TGGGACGACGATAAGGATTACAACCCTTCCCTGAAGTCTCGGCTGACCATCAG
CAAGGATACATCCAAGAATCAGGTGTTCCTGAAGATCGCTTCTGTGGACACCG
CCGACACCGCCACATATTACTGTGCCCGCGCTTATTACTCTAACTACGAGTACT
ATGGCATGGATTACTGGGGCCAGGGCACATCTGTGACAGTGTCTTCC
SEQ ID NO: 127
   Sequence Name: Antibody GPN18-1 VL
   Sequence Type: AA
   Organism Name: Mus musculus 
DVLMTQTPLSLPVSLGDQASISCRSSQSIVHSNGNTYLEWYLQKPGQSPKLLIYKVS
NRFSGVPDRFSGSGSGTDFTLKISRVEAEDLGVYYCFQGSHVPFTFGSGTKLEIK
SEQ ID NO: 128
   Sequence Name: Antibody GPN18-1 VL
   Sequence Type: DNA
   Organism Name: synthetic construct 
GATGTTTTGATGACCCAAACTCCACTCTCCCTGCCTGTCAGTCTTGGAGATCAA
GCCTCCATCTCTTGCAGATCTAGTCAGAGCATTGTTCATAGTAATGGAAACACC
TATTTAGAATGGTACCTGCAGAAACCAGGCCAGTCTCCAAAGCTCCTGATCTAC
AAGGTTTCCAACCGATTTTCTGGGGTCCCAGACAGGTTCAGTGGCAGTGGATC
AGGGACAGATTTCACACTCAAGATCAGCAGAGTGGAGGCTGAGGATCTGGGA
 
GTTTATTACTGCTTTCAAGGTTCACATGTTCCATTCACGTTCGGCTCGGGGACA
AAGTTGGAAATAAAA
SEQ ID NO: 129
   Sequence Name: Antibody GPN18-2 CDR-H1
   Sequence Type: AA
   Organism Name: Mus musculus
      GFSLSTSVMG
SEQ ID NO: 130
   Sequence Name: Antibody GPN18-2 CDR-H1
   Sequence Type: DNA
   Organism Name: synthetic construct
      GGGTTTTCACTGAGTACTTCTGTT
SEQ ID NO: 131
   Sequence Name: Antibody GPN18-2 CDR-H2
   Sequence Type: AA
   Organism Name: Mus musculus
      IWWDDDK
SEQ ID NO: 132
   Sequence Name: Antibody GPN18-2 CDR-H2
   Sequence Type: DNA
   Organism Name: synthetic construct
      ATTTGGTGGGATGACGATAAG
SEQ ID NO: 133
   Sequence Name: Antibody GPN18-2 CDR-H3
   Sequence Type: AA
   Organism Name: Mus musculus
      ARTYYSNYEYYGMDY
SEQ ID NO: 134
   Sequence Name: Antibody GPN18-2 CDR-H3
   Sequence Type: DNA
   Organism Name: synthetic construct
      GCTCGAACCTACTATAGTAACTACGAATACTATGGTATGGACTAC
SEQ ID NO: 135
   Sequence Name: Antibody GPN18-2 CDR-L1
   Sequence Type: AA
   Organism Name: Mus musculus
      QTIILSNGNTY
SEQ ID NO: 136
   Sequence Name: Antibody GPN18-2 CDR-L1
   Sequence Type: DNA
   Organism Name: synthetic construct
      CAGACCATTATACTTAGTAATGGAAACACCTAT
SEQ ID NO: 137
   Sequence Name: Antibody GPN18-2 CDR-L2
   Sequence Type: AA
   Organism Name: Mus musculus
      KVS
SEQ ID NO: 138
   Sequence Name: Antibody GPN18-2 CDR-L2
   Sequence Type: DNA
   Organism Name: synthetic construct
      AAAGTTTCC
SEQ ID NO: 139
   Sequence Name: Antibody GPN18-2 CDR-L3
   Sequence Type: AA
   Organism Name: Mus musculus
      FQGSHVPFT
SEQ ID NO: 140
   Sequence Name: Antibody GPN18-2 CDR-L3
   Sequence Type: DNA
   Organism Name: synthetic construct
      TTTCAAGGTTCACATGTTCCATTCACG
SEQ ID NO: 141
   Sequence Name: Antibody GPN18-2 VH
   Sequence Type: AA
   Organism Name: Mus musculus 
QVTLKESGPGILQPSQTLSLTCSFSGFSLSTSVMGVGWIRQPSGKGLEWLADIWWD
DDKDYNPSLKSRLTISKDTSKNQVFLKIASVDTADTATYYCARTYYSNYEYYGMD
YWGQGTSVTVSS
SEQ ID NO: 142
   Sequence Name: Antibody GPN18-2 VH
   Sequence Type: DNA
   Organism Name: synthetic construct 
CAGGTTACTCTGAAAGAGTCTGGCCCTGGAATATTGCAGCCCTCCCAGACCCTC
AGTCTGACTTGTTCTTTCTCTGGGTTTTCACTGAGTACTTCTGTTATGGGTGTAG
GCTGGATTCGTCAGCCTTCAGGAAAGGGTCTAGAGTGGCTGGCAGACATTTGG
 
TGGGATGACGATAAGGACTATAACCCATCCCTGAAGAGCCGGCTCACAATCTC
CAAGGATACCTCCAAAAACCAGGTATTCCTCAAGATCGCCAGTGTGGACACTG
CAGATACTGCCACTTACTACTGTGCTCGAACCTACTATAGTAACTACGAATACT
ATGGTATGGACTACTGGGGTCAAGGAACCTCAGTCACCGTCTCCTCA
SEQ ID NO: 143
   Sequence Name: Antibody GPN18-2 VL
   Sequence Type: AA
   Organism Name: Mus musculus 
DVLMTQTPLSLPVSLGDQASISCRSSQTIILSNGNTYLEWYLQKPGQSPKLLIYKVS
NRFSGVPDRFSGSGSGTDFTLKISRVEAEDLGVYYCFQGSHVPFTFGSGTKLEIK
SEQ ID NO: 144
   Sequence Name: Antibody GPN18-2 VL
   Sequence Type: DNA
   Organism Name: synthetic construct 
GACGTTTTGATGACCCAAACTCCACTCTCCCTGCCTGTCAGTCTTGGAGATCAA
GCCTCCATCTCTTGCAGATCTAGTCAGACCATTATACTTAGTAATGGAAACACC
TATTTAGAATGGTACCTGCAGAAACCAGGCCAGTCTCCAAAGCTCCTGATCTAC
AAAGTTTCCAACCGATTTTCTGGGGTCCCAGACAGGTTCAGTGGCAGTGGATC
GGGGACAGATTTCACACTCAAGATCAGCAGAGTGGAGGCTGAGGATCTGGGA
GTTTATTACTGTTTTCAAGGTTCACATGTTCCATTCACGTTCGGCTCGGGGACA
AAGTTAGAAATAAAA
SEQ ID NO: 145
   Sequence Name: Antibody GPN18-6 CDR-H1
   Sequence Type: AA
   Organism Name: Mus musculus
      GYTFTDYY
SEQ ID NO: 146
   Sequence Name: Antibody GPN18-6 CDR-H1
   Sequence Type: DNA
   Organism Name: synthetic construct
      GGATACACATTCACTGACTACTAC
SEQ ID NO: 147
   Sequence Name: Antibody GPN18-6 CDR-H2
   Sequence Type: AA
   Organism Name: Mus musculus
      INPINGDI
SEQ ID NO: 148
   Sequence Name: Antibody GPN18-6 CDR-H2
   Sequence Type: DNA
   Organism Name: synthetic construct
      ATTAATCCGATCAATGGTGATATT
SEQ ID NO: 149
   Sequence Name: Antibody GPN18-6 CDR-H3
   Sequence Type: AA
   Organism Name: Mus musculus
      AREAFFGYKGNYFDY
SEQ ID NO: 150
   Sequence Name: Antibody GPN18-6 CDR-H3
   Sequence Type: DNA
   Organism Name: synthetic construct
      GCAAGAGAGGCTTTCTTTGGTTACAAGGGGAACTACTTTGACTAC
SEQ ID NO: 151
   Sequence Name: Antibody GPN18-6 CDR-L1
   Sequence Type: AA
   Organism Name: Mus musculus
      ESVDNYGISF
SEQ ID NO: 152
   Sequence Name: Antibody GPN18-6 CDR-L1
   Sequence Type: DNA
   Organism Name: synthetic construct
      GAAAGTGTTGATAATTATGGCATTAGTTTT
SEQ ID NO: 153
   Sequence Name: Antibody GPN18-6 CDR-L2 Sequence Type: AA
   Organism Name: Mus musculus
      AAS
SEQ ID NO: 154
   Sequence Name: Antibody GPN18-6 CDR-L2
   Sequence Type: DNA
   Organism Name: synthetic construct
      GCTGCATCC
SEQ ID NO: 155
   Sequence Name: Antibody GPN18-6 CDR-L3
   Sequence Type: AA
   Organism Name: Mus musculus
      QQTKEVPYT
SEQ ID NO: 156
   Sequence Name: Antibody GPN18-6 CDR-L3
   Sequence Type: DNA
   Organism Name: synthetic construct
      CAGCAAACTAAGGAGGTTCCGTACACG
SEQ ID NO: 157
   Sequence Name: Antibody GPN18-6 VH
   Sequence Type: AA
   Organism Name: Mus musculus 
EVQLQQSGPELVMPGSSVKISCKASGYTFTDYYMNWVRQSHGKSLEWIGDINPIN
GDITYNQKFKGKATLTVDKSSSTDYMELRGLTSEDSAVYYCAREAFFGYKGNYFD
YWGQGTTLTVSS
SEQ ID NO: 158
   Sequence Name: Antibody GPN18-6 VH
   Sequence Type: DNA
   Organism Name: synthetic construct 
GAGGTCCAGCTGCAACAGTCTGGGCCTGAGCTGGTGATGCCTGGGTCTTCAGT
GAAGATATCATGTAAGGCTTCTGGATACACATTCACTGACTACTACATGAACTG
GGTGAGGCAGAGCCATGGAAAGAGCCTTGAGTGGATTGGAGATATTAATCCGA
TCAATGGTGATATTACCTACAACCAGAAGTTCAAGGGCAAGGCCACATTGACT
GTAGACAAGTCCTCCAGCACAGACTACATGGAGCTCCGCGGCCTGACATCTGA
GGACTCTGCAGTCTATTACTGTGCAAGAGAGGCTTTCTTTGGTTACAAGGGGAA
CTACTTTGACTACTGGGGCCAAGGCACCACTCTCACAGTCTCCTCA
SEQ ID NO: 159
   Sequence Name: Antibody GPN18-6 VL
   Sequence Type: AA
   Organism Name: Mus musculus 
DIVLTQSPASLAVSLGQRATISCRASESVDNYGISFMSWFQQKPGQPPKLLIYAASN
QGSGVPAKFSGSGSGTDFSLNIHPMEEDDPAMYFCQQTKEVPYTFGGGTKLEIK
SEQ ID NO: 160
   Sequence Name: Antibody GPN18-6 VL
   Sequence Type: DNA
   Organism Name: synthetic construct 
GACATTGTGCTGACCCAATCTCCAGCTTCTTTGGCTGTGTCTCTAGGGCAGAGG
GCCACCATCTCCTGCAGAGCCAGCGAAAGTGTTGATAATTATGGCATTAGTTTT
ATGAGCTGGTTCCAACAGAAACCAGGACAGCCACCCAAACTCCTCATCTATGC
TGCATCCAACCAAGGATCCGGGGTCCCTGCCAAGTTTAGTGGCAGTGGGTCTG
GGACAGACTTCAGCCTCAACATCCATCCTATGGAGGAGGATGATCCTGCAATG
TATTTCTGTCAGCAAACTAAGGAGGTTCCGTACACGTTCGGAGGGGGGACCAA
GCTGGAAATAAAA
SEQ ID NO: 161
   Sequence Name: Antibody GPN18-7 CDR-H1
   Sequence Type: AA
   Organism Name: Mus musculus
      GFSLSTSVMG
SEQ ID NO: 162
   Sequence Name: Antibody GPN18-7 CDR-H1
   Sequence Type: DNA
   Organism Name: synthetic construct
      GGGTTTTCACTGAGTACTTCTGTTATGGGT
SEQ ID NO: 163
   Sequence Name: Antibody GPN18-7 CDR-H2
   Sequence Type: AA
   Organism Name: Mus musculus
      IWWDDDK
SEQ ID NO: 164
   Sequence Name: Antibody GPN18-7 CDR-H2
   Sequence Type: DNA
   Organism Name: synthetic construct
      ATTTGGTGGGATGACGATAAG
SEQ ID NO: 165
   Sequence Name: Antibody GPN18-7 CDR-H3
   Sequence Type: AA
   Organism Name: Mus musculus
      ARAYYSNFEYYGMDY
SEQ ID NO: 166
   Sequence Name: Antibody GPN18-7 CDR-H3
   Sequence Type: DNA
   Organism Name: synthetic construct
      GCTCGAGCCTACTATAGTAACTTCGAATACTATGGTATGGACTAC
SEQ ID NO: 167
   Sequence Name: Antibody GPN18-7 CDR-L1
   Sequence Type: AA
   Organism Name: Mus musculus
      QTIVHSNGNTY
SEQ ID NO: 168
   Sequence Name: Antibody GPN18-7 CDR-L1
   Sequence Type: DNA
   Organism Name: synthetic construct
      CAGACCATTGTACATAGTAATGGAAACACCTAT
SEQ ID NO: 169
   Sequence Name: Antibody GPN18-7 CDR-L2
   Sequence Type: AA
   Organism Name: Mus musculus
      KVS
SEQ ID NO: 170
   Sequence Name: Antibody GPN18-7 CDR-L2
   Sequence Type: DNA
   Organism Name: synthetic construct
      AAAGTTTCC
SEQ ID NO: 171
   Sequence Name: Antibody GPN18-7 CDR-L3
   Sequence Type: AA
   Organism Name: Mus musculus
      FQGSHVPFT
SEQ ID NO: 172
   Sequence Name: Antibody GPN18-7 CDR-L3
   Sequence Type: DNA
   Organism Name: synthetic construct
      TTTCAAGGTTCACATGTTCCATTCACG
SEQ ID NO: 173
   Sequence Name: Antibody GPN18-7 VH
   Sequence Type: AA
   Organism Name: Mus musculus 
QVTLKESGPGILQPSQTLSLTCSFSGFSLSTSVMGVGWIRQPSGKGLEWLADIWWD
DDKDYNPSLKSRLTISKDTSKNQVFLKIASVDTADTATYYCARAYYSNFEYYGMD
YWGQGTSVTVSS
SEQ ID NO: 174
   Sequence Name: Antibody GPN18-7 VH
   Sequence Type: DNA
   Organism Name: synthetic construct 
CAGGTTACTCTGAAAGAGTCTGGCCCTGGAATATTGCAGCCCTCCCAGACCCTC
AGTCTGACTTGTTCTTTCTCTGGGTTTTCACTGAGTACTTCTGTTATGGGTGTAG
GCTGGATTCGTCAGCCTTCAGGAAAGGGTCTTGAGTGGCTGGCAGACATTTGG
TGGGATGACGATAAGGACTATAACCCATCTCTGAAGAGCCGGCTCACAATCTC
CAAGGATACCTCCAAAAACCAGGTATTCCTCAAGATCGCCAGTGTGGACACTG
CAGATACTGCCACTTACTACTGTGCTCGAGCCTACTATAGTAACTTCGAATACT
 ATGGTATGGACTACTGGGGTCAAGGAACCTCAGTCACCGTCTCCTCA
SEQ ID NO: 175
   Sequence Name: Antibody GPN18-7 VL
   Sequence Type: AA
   Organism Name: Mus musculus 
DVLMTQIPLSLPVSLGDQASISCRSSQTIVHSNGNTYLEWYLQKPGQSPKLLIYKVS
NRFSGVPDRFSGSGSGTDFTLKISRVEAEDLGVYYCFQGSHVPFTFGSGTKLEIK
SEQ ID NO: 176
   Sequence Name: Antibody GPN18-7 VL
   Sequence Type: DNA
   Organism Name: synthetic construct 
GATGTTTTGATGACCCAAATTCCACTCTCCCTGCCTGTCAGTCTTGGAGATCAA
GCCTCCATCTCTTGCAGATCTAGTCAGACCATTGTACATAGTAATGGAAACACC
TATTTAGAATGGTACCTGCAGAAACCAGGCCAGTCTCCAAAGCTCCTGATCTAC
AAAGTTTCCAACCGATTTTCTGGGGTCCCAGACAGGTTCAGTGGCAGTGGATC
GGGGACAGATTTCACACTCAAGATCAGCAGAGTGGAGGCTGAGGATCTGGGA
GTTTATTACTGCTTTCAAGGTTCACATGTTCCATTCACGTTCGGCTCGGGGACA
AAGTTGGAAATAAAA
SEQ ID NO: 177
   Sequence Name: Antibody GPN18-5 CDR-H1
   Sequence Type: AA
   Organism Name: Mus musculus
      GYTFTTYWMN
SEQ ID NO: 178
   Sequence Name: Antibody GPN18-5 CDR-H1 Sequence Type: DNA
   Organism Name: synthetic construct
      GGCTACACCTTTACTACCTACTGGATGAAC
SEQ ID NO: 179
   Sequence Name: Antibody GPN18-5 CDR-H2
   Sequence Type: AA
   Organism Name: Mus musculus
      INPSNDYT
SEQ ID NO: 180
   Sequence Name: Antibody GPN18-5 CDR-H2
   Sequence Type: DNA
   Organism Name: synthetic construct
      ATTAATCCTAGCAATGATTATACT
SEQ ID NO: 181
   Sequence Name: Antibody GPN18-5 CDR-H3
   Sequence Type: AA
   Organism Name: Mus musculus
      GRSYSPDY
SEQ ID NO: 182
   Sequence Name: Antibody GPN18-5 CDR-H3
   Sequence Type: DNA
   Organism Name: synthetic construct
      GGAAGGAGTTACTCCCCTGACTAC
SEQ ID NO: 183
   Sequence Name: Antibody GPN18-5 CDR-L1
   Sequence Type: AA
   Organism Name: Mus musculus
      QNVDNH
SEQ ID NO: 184
   Sequence Name: Antibody GPN18-5 CDR-L1
   Sequence Type: DNA
   Organism Name: synthetic construct
      CAGAATGTGGATAATCAT
SEQ ID NO: 185
   Sequence Name: Antibody GPN18-5 CDR-L2
   Sequence Type: AA
   Organism Name: Mus musculus
      SAS
SEQ ID NO: 186
   Sequence Name: Antibody GPN18-5 CDR-L2
   Sequence Type: DNA
   Organism Name: synthetic construct
      TCGGCATCC
SEQ ID NO: 187
   Sequence Name: Antibody GPN18-5 CDR-L3
   Sequence Type: AA
   Organism Name: Mus musculus
      QQYKSYPFT
SEQ ID NO: 188
   Sequence Name: Antibody GPN18-5 CDR-L3
   Sequence Type: DNA
   Organism Name: synthetic construct
      CAGCAATATAAAAGCTATCCATTCACG
SEQ ID NO: 189
   Sequence Name: Antibody GPN18-5 VH
   Sequence Type: AA
   Organism Name: Mus musculus 
QVQLQQSGAELAKPGTSVKMSCKASGYTFTTYWMNWVKQRPGQDLEWIGAINPS
NDYTEYNQKFKDKAILTADKSSNTAYMQLSSLTSEDSAVYYCGRSYSPDYWGQG
TTLTVSS
SEQ ID NO: 190
   Sequence Name: Antibody GPN18-5 VH
   Sequence Type: DNA
   Organism Name: synthetic construct 
CAGGTCCAGCTGCAGCAGTCTGGCGCTGAACTGGCAAAACCTGGGACCTCAGT
GAAGATGTCCTGCAAGGCTTCTGGCTACACCTTTACTACCTACTGGATGAACTG
GGTAAAACAGAGGCCTGGACAGGATCTGGAATGGATTGGGGCTATTAATCCTA
GCAATGATTATACTGAGTACAATCAGAAGTTCAAGGACAAGGCCATATTGACT
GCAGACAAATCCTCCAACACAGCCTACATGCAACTGAGTAGCCTGACATCTGA
GGACTCTGCAGTCTATTACTGTGGAAGGAGTTACTCCCCTGACTACTGGGGCCA
AGGCACCACTCTCACAGTCTCATCA
SEQ ID NO: 191
   Sequence Name: Antibody GPN18-5 VL
   Sequence Type: AA
   Organism Name: Mus musculus 
DIVMTQSQKFMSTSVGDRVSVTCKASQNVDNHVAWHQQKPGQSPKALIYSASYR
YSGVPDRFTGSGSGTDFTLTIRDVQSEDLADYFCQQYKSYPFTFGSGTELEIK
SEQ ID NO: 192
   Sequence Name: Antibody GPN18-5 VL
   Sequence Type: DNA
   Organism Name: synthetic construct 
GACATTGTGATGACCCAGTCTCAAAAATTCATGTCCACATCAGTAGGAGACAG
GGTCAGCGTCACCTGCAAGGCCAGTCAGAATGTGGATAATCATGTAGCCTGGC
ATCAACAGAAACCAGGGCAGTCTCCTAAGGCACTGATTTACTCGGCATCCTAC
CGGTACAGTGGAGTCCCTGATCGCTTCACAGGCAGTGGATCTGGGACAGATTT
CACTCTCACCATCCGCGATGTGCAGTCTGAAGACTTGGCAGACTATTTCTGTCA
GCAATATAAAAGCTATCCATTCACGTTCGGCTCGGGGACAGAGTTGGAAATAA
AA
SEQ ID NO: 193
   Sequence Name: Antibody 1-5E CDR-H1
   Sequence Type: AA
   Organism Name: Mus musculus
      GFNIKDYY
SEQ ID NO: 194
   Sequence Name: Antibody 1-5E CDR-H1
   Sequence Type: DNA
   Organism Name: synthetic construct
      GGCTTCAACATTAAAGACTACTAT
SEQ ID NO: 195
   Sequence Name: Antibody 1-5E CDR-H2
   Sequence Type: AA
   Organism Name: Mus musculus
      IDPENGDT
SEQ ID NO: 196
   Sequence Name: Antibody 1-5E CDR-H2
   Sequence Type: DNA
   Organism Name: synthetic construct
      ATTGATCCTGAGAATGGTGATACT
SEQ ID NO: 197
   Sequence Name: Antibody 1-5E CDR-H3
   Sequence Type: AA
   Organism Name: Mus musculus
      NGVYYGSRGFFDY
SEQ ID NO: 198
   Sequence Name: Antibody 1-5E CDR-H3
   Sequence Type: DNA
   Organism Name: synthetic construct
      AATGGAGTTTACTACGGTAGTAGAGGGTTCTTTGACTAC
SEQ ID NO: 199
   Sequence Name: Antibody 1-5E CDR-L1
   Sequence Type: AA
   Organism Name: Mus musculus
      QSLLDSDGKTY
SEQ ID NO: 200
   Sequence Name: Antibody 1-5E CDR-L1
   Sequence Type: DNA
   Organism Name: synthetic construct
      CAGAGCCTCTTAGATAGTGATGGAAAGACATAT
SEQ ID NO: 201
   Sequence Name: Antibody 1-5E CDR-L2
   Sequence Type: AA
   Organism Name: Mus musculus
      LVS
SEQ ID NO: 202
   Sequence Name: Antibody 1-5E CDR-L2 Sequence Type: DNA
   Organism Name: synthetic construct
      CTGGTGTCT
SEQ ID NO: 203
   Sequence Name: Antibody 1-5E CDR-L3
   Sequence Type: AA
   Organism Name: Mus musculus
      CQITHFPFT
SEQ ID NO: 204
   Sequence Name: Antibody 1-5E CDR-L3
   Sequence Type: DNA
   Organism Name: synthetic construct
      TGCCAAATTACACATTTTCCATTCACG
SEQ ID NO: 205
   Sequence Name: Antibody 1-5E VH
   Sequence Type: AA
   Organism Name: Mus musculus 
EVQLQQSGAELVRSGASVKLSCTASGFNIKDYYMHWVKQRPDQGLEWIGWIDPE
NGDTEYAPKFQGKATMTADTSPNTAYLQLSSLTSEDTAVYYCNGVYYGSRGFFD
YWGQGTTLTVSS
SEQ ID NO: 206
   Sequence Name: Antibody 1-5E VH
   Sequence Type: DNA
   Organism Name: synthetic construct 
GAGGTTCAGCTGCAGCAGTCTGGGGCAGAGCTTGTGAGGTCAGGGGCCTCAGT
CAAGTTGTCCTGCACAGCTTCTGGCTTCAACATTAAAGACTACTATATGCACTG
GGTGAAGCAGAGGCCTGATCAGGGCCTGGAGTGGATTGGATGGATTGATCCTG
AGAATGGTGATACTGAATACGCCCCGAAGTTCCAGGGCAAGGCCACTATGACT
GCAGACACATCCCCCAACACAGCCTACCTGCAGCTCAGCAGCCTGACATCTGA
GGACACTGCCGTCTATTACTGTAATGGAGTTTACTACGGTAGTAGAGGGTTCTT
TGACTACTGGGGCCAAGGCACCACTCTCACAGTCTCCTCA
SEQ ID NO: 207
   Sequence Name: Antibody 1-5E VL
   Sequence Type: AA
   Organism Name: Mus musculus 
DVVMTQTPLTLSVTIGQPASISCKSSQSLLDSDGKTYLNWLLQRPGQSPKRLIYLVS
KLDSGVPDRFTGSGSGTDFTLKISRVEAEDLGVYYCCQITHFPFTFGSGTKLEIK
SEQ ID NO: 208
   Sequence Name: Antibody 1-5E VL
   Sequence Type: DNA
   Organism Name: synthetic construct 
GATGTTGTGATGACCCAGACTCCACTCACTTTGTCGGTTACCATTGGACAACCA
GCCTCCATCTCTTGCAAGTCAAGTCAGAGCCTCTTAGATAGTGATGGAAAGAC
ATATTTGAATTGGTTGTTACAGAGGCCAGGCCAGTCTCCAAAGCGCCTAATCTA
TCTGGTGTCTAAACTGGACTCTGGAGTCCCTGACAGGTTCACTGGCAGTGGATC
AGGGACAGATTTCACACTGAAAATCAGCAGAGTGGAGGCTGAGGATTTGGGA
GTTTATTATTGCTGCCAAATTACACATTTTCCATTCACGTTCGGCTCGGGGACA
AAGTTGGAAATAAAA
SEQ ID NO: 209
   Sequence Name: h_gpNMB FL (NP_001005340)
   Sequence Type: AA
   Organism Name: Homo sapiens 
MECLYYFLGFLLLAARLPLDAAKRFHDVLGNERPSAYMREHNQLNGWSSDENDW
NEKLYPVWKRGDMRWKNSWKGGRVQAVLTSDSPALVGSNITFAVNLIFPRCQKE
DANGNIVYEKNCRNEAGLSADPYVYNWTAWSEDSDGENGTGQSHHNVFPDGKPF
PHHPGWRRWNFIYVFHTLGQYFQKLGRCSVRVSVNTANVTLGPQLMEVTVYRRH
GRAYVPIAQVKDVYVVTDQIPVFVTMFQKNDRNSSDETFLKDLPIMFDVLIHDPSH
FLNYSTINYKWSFGDNTGLFVSTNHTVNHTYVLNGTFSLNLTVKAAAPGPCPPPPP
PPRPSKPTPSLATTLKSYDSNTPGPAGDNPLELSRIPDENCQINRYGHFQATITIVEGI
LEVNIIQMTDVLMPVPWPESSLIDFVVTCQGSIPTEVCTIISDPTCEITQNTVCSPVDV
DEMCLLTVRRTFNGSGTYCVNLTLGDDTSLALTSTLISVPDRDPASPLRMANSALIS
VGCLAIFVTVISLLVYKKHKEYNPIENSPGNVVRSKGLSVFLNRAKAVFFPGNQEK
DPLLKNQEFKGVS
SEQ ID NO: 210
   Sequence Name: h_gpNMB FL (NM_001005340)
   Sequence Type: DNA
   Organism Name: Homo sapiens 
ATGGAATGTCTCTACTATTTCCTGGGATTTCTGCTCCTGGCTGCAAGATTGCCA
CTTGATGCCGCCAAACGATTTCATGATGTGCTGGGCAATGAAAGACCTTCTGCT
TACATGAGGGAGCACAATCAATTAAATGGCTGGTCTTCTGATGAAAATGACTG
GAATGAAAAACTCTACCCAGTGTGGAAGCGGGGAGACATGAGGTGGAAAAAC
TCCTGGAAGGGAGGCCGTGTGCAGGCGGTCCTGACCAGTGACTCACCAGCCCT
CGTGGGCTCAAATATAACATTTGCGGTGAACCTGATATTCCCTAGATGCCAAA
AGGAAGATGCCAATGGCAACATAGTCTATGAGAAGAACTGCAGAAATGAGGC
 
TGGTTTATCTGCTGATCCGTATGTTTACAACTGGACAGCATGGTCAGAGGACAG
TGACGGGGAAAATGGCACCGGCCAAAGCCATCATAACGTCTTCCCTGATGGGA
AACCTTTTCCTCACCACCCCGGATGGAGAAGATGGAATTTCATCTACGTCTTCC
ACACACTTGGTCAGTATTTCCAGAAATTGGGACGATGTTCAGTGAGAGTTTCTG
TGAACACAGCCAATGTGACACTTGGGCCTCAACTCATGGAAGTGACTGTCTAC
AGAAGACATGGACGGGCATATGTTCCCATCGCACAAGTGAAAGATGTGTACGT
GGTAACAGATCAGATTCCTGTGTTTGTGACTATGTTCCAGAAGAACGATCGAA
ATTCATCCGACGAAACCTTCCTCAAAGATCTCCCCATTATGTTTGATGTCCTGA
TTCATGATCCTAGCCACTTCCTCAATTATTCTACCATTAACTACAAGTGGAGCT
TCGGGGATAATACTGGCCTGTTTGTTTCCACCAATCATACTGTGAATCACACGT
ATGTGCTCAATGGAACCTTCAGCCTTAACCTCACTGTGAAAGCTGCAGCACCA
GGACCTTGTCCGCCACCGCCACCACCACCCAGACCTTCAAAACCCACCCCTTCT
TTAGCAACTACTCTAAAATCTTATGATTCAAACACCCCAGGACCTGCTGGTGAC
AACCCCCTGGAGCTGAGTAGGATTCCTGATGAAAACTGCCAGATTAACAGATA
TGGCCACTTTCAAGCCACCATCACAATTGTAGAGGGAATCTTAGAGGTTAACA
TCATCCAGATGACAGACGTCCTGATGCCGGTGCCATGGCCTGAAAGCTCCCTA
ATAGACTTTGTCGTGACCTGCCAAGGGAGCATTCCCACGGAGGTCTGTACCATC
ATTTCTGACCCCACCTGCGAGATCACCCAGAACACAGTCTGCAGCCCTGTGGAT
GTGGATGAGATGTGTCTGCTGACTGTGAGACGAACCTTCAATGGGTCTGGGAC
GTACTGTGTGAACCTCACCCTGGGGGATGACACAAGCCTGGCTCTCACGAGCA
CCCTGATTTCTGTTCCTGACAGAGACCCAGCCTCGCCTTTAAGGATGGCAAACA
GCGCCCTGATCTCCGTTGGCTGCTTGGCCATATTTGTCACTGTGATCTCCCTCTT
GGTGTACAAAAAACACAAGGAATACAACCCAATAGAAAATAGTCCTGGGAAT
GTGGTCAGAAGCAAAGGCCTGAGTGTCTTTCTCAACCGTGCAAAAGCCGTGTT
CTTCCCGGGAAACCAGGAAAAGGATCCGCTACTCAAAAACCAAGAATTTAAAG
GAGTTTCT
SEQ ID NO: 211
   Sequence Name: m_gpNMB FL (NP_444340)
   Sequence Type: AA
   Organism Name: Mus musculus 
MESLCGVLGFLLLAAGLPLQAAKRFRDVLGHEQYPNHMREHNQLRGWSSDENE
WDEHLYPVWRRGDGRWKDSWEGGRVQAVLTSDSPALVGSNITFVVNLVFPRCQ
KEDANGNIVYEKNCRNDLGLTSDLHVYNWTAGADDGDWEDGTSRSQHLRFPDR
RPFPRPHGWKKWSFVYVFHTLGQYFQKLGRCSARVSINTVNLTAGPQVMEVTVF
RRYGRAYIPISKVKDVYVITDQIPVFVTMSQKNDRNLSDEIFLRDLPIVFDVLIHDPS
HFLNDSAISYKWNFGDNTGLFVSNNHTLNHTYVLNGTFNLNLTVQTAVPGPCPPP
SPSTPPPPSTPPSPPPSPLPTLSTPSPSLMPTGYKSMELSDISNENCRINRYGYFRATITI
VEGILEVSIMQIADVPMPTPQPANSLMDFTVTCKGATPMEACTIISDPTCQIAQNRV
CSPVAVDGLCLLSVRRAFNGSGTYCVNFTLGDDASLALTSTLISIPGKDPDSPLRAV
NGVLISIGCLAVLVTMVTILLYKKHKAYKPIGNCPRNTVKGKGLSVLLSHAKAPFF
RGDQEKDPLLQDKPRTL
SEQ ID NO: 212
   Sequence Name: m_gpNMB FL (NM_053110)
   Sequence Type: DNA
   Organism Name: Mus musculus 
ATGGAAAGTCTCTGCGGGGTCCTGGGATTTCTGCTGCTGGCTGCAGGACTGCCT
CTCCAGGCTGCCAAGCGATTTCGTGATGTGCTGGGCCATGAACAGTATCCCAAT
CACATGAGAGAGCACAACCAATTACGTGGCTGGTCTTCGGATGAAAATGAATG
GGATGAACACCTGTATCCAGTGTGGAGGAGGGGAGACGGCAGGTGGAAGGAC
TCCTGGGAAGGAGGCCGTGTGCAGGCAGTCCTGACCAGTGACTCACCGGCTCT
GGTGGGTTCCAATATCACCTTTGTGGTGAACCTGGTGTTCCCCAGATGCCAGAA
GGAAGATGCTAATGGCAATATCGTCTATGAGAAGAACTGCAGGAATGATTTGG
GACTGACCTCTGACCTGCATGTCTACAACTGGACTGCAGGGGCAGATGATGGT
GACTGGGAAGATGGCACCAGCCGAAGCCAGCATCTCAGGTTCCCGGACAGGA
GGCCCTTCCCTCGCCCCCATGGATGGAAGAAATGGAGCTTTGTCTACGTCTTTC
ACACACTTGGCCAGTATTTCCAAAAACTGGGTCGGTGTTCAGCACGGGTTTCTA
TAAACACAGTCAACTTGACAGCTGGCCCTCAGGTCATGGAAGTGACTGTCTTTC
GAAGATACGGCCGGGCATACATTCCCATCTCGAAGGTGAAAGATGTGTATGTG
ATAACAGATCAGATCCCTGTATTCGTGACCATGTCCCAGAAGAATGACAGGAA
CTTGTCTGATGAGATCTTCCTCAGAGACCTCCCCATCGTCTTCGATGTCCTCATT
CATGATCCCAGCCACTTCCTCAACGACTCTGCCATTTCCTACAAGTGGAACTTT
GGGGACAACACTGGCCTGTTTGTCTCCAACAATCACACTTTGAATCACACTTAT
GTGCTCAATGGAACCTTCAACCTTAACCTCACCGTGCAAACTGCAGTGCCCGG
GCCATGCCCTCCCCCTTCGCCTTCGACTCCGCCTCCACCTTCAACTCCGCCCTCA
CCTCCGCCCTCACCTCTGCCCACATTATCAACACCTAGCCCCTCTTTAATGCCT
ACTGGTTACAAATCCATGGAGCTGAGTGACATTTCCAATGAAAACTGCCGAAT
AAACAGATATGGCTACTTCAGAGCCACCATCACAATTGTAGAGGGGATCCTGG
AAGTCAGCATCATGCAGATAGCAGATGTCCCCATGCCCACACCGCAGCCTGCC
AACTCCCTGATGGACTTCACTGTGACCTGCAAAGGGGCCACCCCCATGGAAGC
CTGTACGATCATCTCCGACCCCACCTGCCAGATCGCCCAGAACCGGGTCTGCA
GCCCTGTGGCTGTGGATGGGCTGTGCCTGCTGTCTGTGAGAAGAGCCTTCAATG
GGTCTGGCACCTACTGTGTGAATTTCACTCTGGGAGATGATGCAAGCCTGGCCC
TCACCAGCACCCTGATCTCTATCCCTGGCAAAGACCCAGACTCCCCTCTGAGAG
CAGTGAATGGTGTCCTGATCTCCATTGGCTGCCTGGCTGTGCTTGTCACCATGG
TTACCATCTTGCTGTACAAAAAACACAAGGCGTACAAGCCAATAGGAAACTGC
CCCAGGAACACGGTCAAGGGCAAAGGCCTGAGTGTTCTCCTCAGCCACGCGAA
AGCCCCGTTCTTCCGAGGAGACCAGGAGAAGGATCCATTGCTCCAGGACAAGC
CAAGGACACTC
SEQ ID NO: 213
   Sequence Name: r_gpNMB FL (NP_579832)
   Sequence Type: AA
   Organism Name: Rattus norvegicus 
MESLCGVLVFLLLAAGLPLQAAKRFRDVLGHEQYPDHMRENNQLRGWSSDENE
WDEQLYPVWRRGEGRWKDSWEGGRVQAALTSDSPALVGSNITFVVNLVFPRCQK
EDANGNIVYERNCRSDLELASDPYVYNWTTGADDEDWEDSTSQGQHLRFPDGKP
FPRPHGRKKWNFVYVFHTLGQYFQKLGRCSARVSINTVNLTVGPQVMEVIVFRRH
GRAYIPISKVKDVYVITDQIPIFVTMYQKNDRNSSDETFLRDLPIFFDVLIHDPSHFL
 
NYSAISYKWNFGDNTGLFVSNNHTLNHTYVLNGTFNFNLTVQTAVPGPCPSPTPSP
SSSTSPSPASSPSPTLSTPSPSLMPTGHKSMELSDISNENCRINRYGYFRATITIVDGIL
EVNIIQVADVPIPTPQPDNSLMDFIVTCKGATPTEACTIISDPTCQIAQNRVCSPVAV
DELCLLSVRRAFNGSGTYCVNFTLGDDASLALTSALISIPGKDLGSPLRTVNGVLISI
GCLAMFVTMVTILLYKKHKTYKPIGNCTRNVVKGKGLSVFLSHAKAPFSRGDREK
DPLLQDKPWML
SEQ ID NO: 214
   Sequence Name: r_gpNMB FL (NM_133298) Sequence Type: DNA
   Organism Name: Rattus norvegicus 
ATGGAAAGTCTCTGCGGGGTCCTGGTATTTCTGCTGCTGGCTGCAGGACTGCCG
CTCCAGGCGGCCAAGCGGTTCCGTGATGTGCTGGGCCATGAGCAGTATCCGGA
TCACATGAGGGAGAACAACCAATTACGTGGCTGGTCTTCAGATGAAAATGAAT
GGGATGAACAGCTGTATCCAGTGTGGAGGAGGGGAGAGGGCAGATGGAAGGA
CTCCTGGGAAGGAGGCCGTGTGCAGGCAGCCCTAACCAGTGATTCACCGGCCT
TGGTGGGTTCCAATATCACCTTCGTAGTGAACCTGGTGTTCCCCAGATGCCAGA
AGGAAGATGCCAACGGCAATATCGTCTATGAGAGGAACTGCAGAAGTGATTTG
GAGCTGGCTTCTGACCCGTATGTCTACAACTGGACCACAGGGGCAGACGATGA
GGACTGGGAAGACAGCACCAGCCAAGGCCAGCACCTCAGGTTCCCCGACGGG
AAGCCCTTCCCTCGCCCCCACGGACGGAAGAAATGGAACTTCGTCTACGTCTTC
CACACACTTGGTCAGTATTTTCAAAAGCTGGGTCGGTGTTCAGCACGAGTTTCT
ATAAACACAGTCAACTTGACAGTTGGCCCTCAGGTCATGGAAGTGATTGTCTTT
CGAAGACACGGCCGGGCATACATTCCCATCTCCAAAGTGAAAGACGTGTATGT
GATAACAGATCAGATCCCTATATTCGTGACCATGTACCAGAAGAATGACCGGA
ACTCGTCTGATGAAACCTTCCTCAGAGACCTCCCCATTTTCTTCGATGTCCTCAT
TCACGATCCCAGTCATTTCCTCAACTACTCTGCCATTTCCTACAAGTGGAACTTT
GGGGACAACACTGGCCTGTTTGTCTCCAACAATCACACTTTGAATCACACGTAT
GTGCTCAATGGAACCTTCAACTTTAACCTCACCGTGCAAACTGCAGTGCCGGG
ACCATGCCCCTCACCCACACCTTCGCCTTCTTCTTCGACTTCTCCTTCGCCTGCA
TCTTCGCCTTCACCCACATTATCAACACCTAGTCCCTCTTTAATGCCTACTGGCC
ACAAATCCATGGAGCTGAGTGACATTTCCAATGAAAACTGCCGAATAAACAGA
TATGGTTACTTCAGAGCCACCATCACAATTGTAGATGGAATCCTAGAAGTCAA
CATCATCCAGGTAGCAGATGTCCCAATCCCCACACCGCAGCCTGACAACTCAC
TGATGGACTTCATTGTGACCTGCAAAGGGGCCACTCCCACGGAAGCCTGTACG
ATCATCTCTGACCCCACCTGCCAGATCGCCCAGAACAGGGTGTGCAGCCCGGT
GGCTGTGGATGAGCTGTGCCTCCTGTCCGTGAGGAGAGCCTTCAATGGGTCCG
GCACGTACTGTGTGAATTTCACTCTGGGAGACGATGCAAGCCTGGCCCTCACC
AGCGCCCTGATCTCTATCCCTGGCAAAGACCTAGGCTCCCCTCTGAGAACAGTG
AATGGTGTCCTGATCTCCATTGGCTGCCTGGCCATGTTTGTCACCATGGTTACC
ATCTTGCTGTACAAAAAACACAAGACGTACAAGCCAATAGGAAACTGCACCAG
GAACGTGGTCAAGGGCAAAGGCCTGAGTGTTTTTCTCAGCCATGCAAAAGCCC
CGTTCTCCCGAGGAGACCGGGAGAAGGATCCACTGCTCCAGGACAAGCCATGG
ATGCTC
SEQ ID NO: 215
   Sequence Name: h_gpNMB ECD
   Sequence Type: AA
   Organism Name: Homo sapiens 
MECLYYFLGFLLLAARLPLDAAKRFHDVLGNERPSAYMREHNQLNGWSSDENDW
NEKLYPVWKRGDMRWKNSWKGGRVQAVLTSDSPALVGSNITFAVNLIFPRCQKE
DANGNIVYEKNCRNEAGLSADPYVYNWTAWSEDSDGENGTGQSHHNVFPDGKPF
PHHPGWRRWNFIYVFHTLGQYFQKLGRCSVRVSVNTANVTLGPQLMEVTVYRRH
GRAYVPIAQVKDVYVVTDQIPVFVTMFQKNDRNSSDETFLKDLPIMFDVLIHDPSH
FLNYSTINYKWSFGDNTGLFVSTNHTVNHTYVLNGTFSLNLTVKAAAPGPCPPPPP
PPRPSKPTPSLATTLKSYDSNTPGPAGDNPLELSRIPDENCQINRYGHFQATITIVEGI
LEVNIIQMTDVLMPVPWPESSLIDFVVTCQGSIPTEVCTIISDPTCEITQNTVCSPVDV
DEMCLLTVRRTFNGSGTYCVNLTLGDDTSLALTSTLISVPDRDPASPLRMAN
SEQ ID NO: 216
   Sequence Name: h_gpNMB ECD
   Sequence Type: DNA
   Organism Name: Homo sapiens 
ATGGAATGTCTCTACTATTTCCTGGGATTTCTGCTCCTGGCTGCAAGATTGCCA
CTTGATGCCGCCAAACGATTTCATGATGTGCTGGGCAATGAAAGACCTTCTGCT
TACATGAGGGAGCACAATCAATTAAATGGCTGGTCTTCTGATGAAAATGACTG
GAATGAAAAACTCTACCCAGTGTGGAAGCGGGGAGACATGAGGTGGAAAAAC
TCCTGGAAGGGAGGCCGTGTGCAGGCGGTCCTGACCAGTGACTCACCAGCCCT
CGTGGGCTCAAATATAACATTTGCGGTGAACCTGATATTCCCTAGATGCCAAA
AGGAAGATGCCAATGGCAACATAGTCTATGAGAAGAACTGCAGAAATGAGGC
TGGTTTATCTGCTGATCCGTATGTTTACAACTGGACAGCATGGTCAGAGGACAG
TGACGGGGAAAATGGCACCGGCCAAAGCCATCATAACGTCTTCCCTGATGGGA
AACCTTTTCCTCACCACCCCGGATGGAGAAGATGGAATTTCATCTACGTCTTCC
ACACACTTGGTCAGTATTTCCAGAAATTGGGACGATGTTCAGTGAGAGTTTCTG
TGAACACAGCCAATGTGACACTTGGGCCTCAACTCATGGAAGTGACTGTCTAC
AGAAGACATGGACGGGCATATGTTCCCATCGCACAAGTGAAAGATGTGTACGT
GGTAACAGATCAGATTCCTGTGTTTGTGACTATGTTCCAGAAGAACGATCGAA
ATTCATCCGACGAAACCTTCCTCAAAGATCTCCCCATTATGTTTGATGTCCTGA
TTCATGATCCTAGCCACTTCCTCAATTATTCTACCATTAACTACAAGTGGAGCT
TCGGGGATAATACTGGCCTGTTTGTTTCCACCAATCATACTGTGAATCACACGT
ATGTGCTCAATGGAACCTTCAGCCTTAACCTCACTGTGAAAGCTGCAGCACCA
GGACCTTGTCCGCCACCGCCACCACCACCCAGACCTTCAAAACCCACCCCTTCT
TTAGCAACTACTCTAAAATCTTATGATTCAAACACCCCAGGACCTGCTGGTGAC
AACCCCCTGGAGCTGAGTAGGATTCCTGATGAAAACTGCCAGATTAACAGATA
TGGCCACTTTCAAGCCACCATCACAATTGTAGAGGGAATCTTAGAGGTTAACA
TCATCCAGATGACAGACGTCCTGATGCCGGTGCCATGGCCTGAAAGCTCCCTA
ATAGACTTTGTCGTGACCTGCCAAGGGAGCATTCCCACGGAGGTCTGTACCATC
ATTTCTGACCCCACCTGCGAGATCACCCAGAACACAGTCTGCAGCCCTGTGGAT
GTGGATGAGATGTGTCTGCTGACTGTGAGACGAACCTTCAATGGGTCTGGGAC
GTACTGTGTGAACCTCACCCTGGGGGATGACACAAGCCTGGCTCTCACGAGCA
 CCCTGATTTCTGTTCCTGACAGAGACCCAGCCTCGCCTTTAAGGATGGCAAAC
SEQ ID NO: 217
   Sequence Name: m_gpNMB ECD
   Sequence Type: AA
   Organism Name: Mus musculus 
MESLCGVLGFLLLAAGLPLQAAKRFRDVLGHEQYPDHMREHNQLRGWSSDENE
WDEHLYPVWRRGDGRWKDSWEGGRVQAVLTSDSPALVGSNITFVVNLVFPRCQ
KEDANGNIVYEKNCRNDLGLTSDLHVYNWTAGADDGDWEDGTSRSQHLRFPDR
RPFPRPHGWKKWSFVYVFHTLGQYFQKLGRCSARVSINTVNLTAGPQVMEVTVF
RRYGRAYIPISKVKDVYVITDQIPVFVTMSQKNDRNLSDEIFLRDLPIVFDVLIHDPS
HFLNDSAISYKWNFGDNTGLFVSNNHTLNHTYVLNGTFNLNLTVQTAVPGPCPPP
SPSTPPPPSTPPSPPPSPLPTLSTPSPSLMPTGYKSMELSDISNENCRINRYGYFRATITI
VEGILEVSIMQIADVPMPTPQPANSLMDFTVTCKGATPMEACTIISDPTCQIAQNRV
CSPVAVDGLCLLSVRRAFNGSGTYCVNFTLGDDASLALTSTLISIPGKDPDSPLRAV
N
SEQ ID NO: 218
   Sequence Name: m_gpNMB ECD
   Sequence Type: DNA
   Organism Name: Mus musculus 
ATGGAAAGTCTCTGCGGGGTCCTGGGATTTCTGCTGCTGGCTGCAGGACTGCCT
CTCCAGGCTGCCAAGCGATTTCGTGATGTGCTGGGCCATGAACAGTATCCCGAT
CACATGAGAGAGCACAACCAATTACGTGGCTGGTCTTCGGATGAAAATGAATG
GGATGAACACCTGTATCCAGTGTGGAGGAGGGGAGACGGCAGGTGGAAGGAC
TCCTGGGAAGGAGGCCGTGTGCAGGCAGTCCTGACCAGTGACTCACCGGCTCT
GGTGGGTTCCAATATCACTTTTGTGGTGAACCTGGTGTTCCCCAGATGCCAGAA
GGAAGATGCTAATGGCAATATCGTCTATGAGAAGAACTGCAGGAATGATTTGG
GACTGACATCTGACCTGCATGTCTACAACTGGACTGCAGGGGCAGATGATGGT
GACTGGGAAGATGGCACCAGCCGAAGCCAGCATCTCAGGTTCCCGGACAGGA
GGCCCTTCCCTCGCCCCCATGGATGGAAGAAATGGAGCTTTGTCTACGTCTTTC
ACACACTTGGCCAGTATTTCCAAAAACTGGGTCGGTGTTCAGCACGGGTTTCTA
TAAACACAGTCAACTTGACAGCTGGCCCTCAGGTCATGGAAGTGACTGTCTTTC
GAAGATACGGCCGGGCATACATTCCCATCTCGAAGGTGAAAGATGTGTATGTG
ATAACAGATCAGATCCCTGTATTCGTGACCATGTCCCAGAAGAATGACAGGAA
CTTGTCTGATGAGATCTTCCTCAGAGACCTCCCCATCGTCTTCGATGTCCTCATT
CATGATCCCAGCCACTTCCTCAACGACTCTGCCATTTCCTACAAGTGGAACTTT
GGGGACAACACTGGCCTGTTTGTCTCCAACAATCACACTTTGAATCACACTTAT
GTGCTCAATGGAACCTTCAACCTTAACCTCACCGTGCAAACTGCAGTGCCCGG
GCCATGCCCTCCCCCTTCGCCTTCGACTCCGCCTCCACCTTCAACTCCGCCCTCA
CCTCCGCCCTCACCTCTGCCCACATTATCAACACCTAGCCCCTCTTTAATGCCT
ACTGGTTACAAATCCATGGAGCTGAGTGACATTTCCAATGAAAACTGCCGAAT
AAACAGATATGGCTACTTCAGAGCCACCATCACAATTGTAGAGGGGATCCTGG
AAGTCAGCATCATGCAGATAGCAGATGTCCCCATGCCCACACCGCAGCCTGCC
AACTCCCTGATGGACTTCACTGTGACCTGCAAAGGGGCCACCCCCATGGAAGC
 
CTGTACGATCATCTCCGACCCCACCTGCCAGATCGCCCAGAACCGGGTCTGCA
GCCCTGTGGCTGTGGATGGGCTGTGCCTGCTGTCTGTGAGAAGAGCCTTCAATG
GGTCTGGCACCTACTGTGTGAATTTCACTCTGGGAGATGATGCAAGCCTGGCCC
TCACCAGCACCCTGATCTCTATCCCTGGCAAAGACCCAGACTCCCCTCTGAGAG
CAGTGAAT
SEQ ID NO: 219
   Sequence Name: r_gpNMB ECD
   Sequence Type: AA
   Organism Name: Rattus norvegicus 
MESLCGVLVFLLLAAGLPLQAAKRFRDVLGHEQYPDHMRENNQLRGWSSDENE
WDEQLYPVWRRGEGRWKDSWEGGRVQAALTSDSPALVGSNITFVVNLVFPRCQK
EDANGNIVYERNCRSDLELASDPYVYNWTTGADDEDWEDNTSQGQHLRFPDGKP
FPRPHGRKKWNFVYVFHTLGQYFQKLGQCSARVSINTVNLTVGPQVMEVIVFRRH
GRAYIPISKVKDVYVITDQIPIFVTMYQKNDRNSSDETFLRDLPIFFDVLIHDPSHFL
NYSAISYKWNFGDNTGLFVSNNHTLNHTYVLNGTFNFNLTVQTAVPGPCPSPTPSP
SSSTSPSPASSPSPTLSTPSPSLMPTGYKSMELSDISNENCRINRYGYFRATITIVDGIL
EVNIIQVADVPIPTLQPDNSLMDFIVTCKGATPTEACTIISDPTCQIAQNRVCSPVAV
DELCLLSVRRAFNGSGTYCVNFTLGDDASLALTSALISIPGKDLGSPLRTVN
SEQ ID NO: 220
   Sequence Name: r_gpNMB ECD
   Sequence Type: DNA
   Organism Name: Rattus norvegicus 
ATGGAAAGTCTCTGCGGGGTCCTGGTATTTCTGCTGCTGGCTGCAGGACTGCCG
CTCCAGGCGGCCAAGCGGTTCCGTGATGTGCTGGGCCATGAGCAGTATCCGGA
TCACATGAGGGAGAACAACCAATTACGTGGCTGGTCTTCAGATGAAAATGAAT
GGGATGAACAGCTGTATCCAGTGTGGAGGAGGGGAGAGGGCAGATGGAAGGA
CTCCTGGGAAGGAGGCCGTGTGCAGGCAGCCCTAACCAGTGATTCACCGGCCT
TGGTGGGTTCCAATATCACCTTCGTAGTGAACCTGGTGTTCCCCAGATGCCAGA
AGGAAGATGCCAACGGCAATATCGTCTATGAGAGGAACTGCAGAAGTGATTTG
GAGCTGGCTTCTGACCCGTATGTCTACAACTGGACCACAGGGGCAGACGATGA
GGACTGGGAAGACAACACCAGCCAAGGCCAGCACCTCAGGTTCCCCGACGGG
AAGCCCTTCCCTCGCCCCCACGGACGGAAGAAATGGAACTTCGTCTACGTCTTC
CACACACTTGGTCAGTATTTTCAAAAGCTGGGTCAGTGTTCAGCACGAGTTTCT
ATAAACACAGTCAACTTGACAGTTGGCCCTCAGGTCATGGAAGTGATTGTCTTT
CGAAGACACGGCCGGGCATACATTCCCATCTCCAAAGTGAAAGACGTGTATGT
GATAACAGATCAGATCCCTATATTCGTGACCATGTACCAGAAGAATGACCGGA
ACTCGTCTGATGAAACCTTCCTCAGAGACCTCCCCATTTTCTTCGATGTCCTCAT
TCACGATCCCAGTCATTTCCTCAACTACTCTGCCATTTCCTACAAGTGGAACTTT
GGGGACAACACTGGCCTGTTTGTCTCCAACAATCACACTTTGAATCACACGTAT
GTGCTCAATGGAACCTTCAACTTTAACCTCACCGTGCAAACTGCAGTGCCGGG
ACCATGCCCCTCACCCACACCTTCGCCTTCTTCTTCGACTTCTCCTTCGCCTGCA
TCTTCGCCTTCACCCACATTATCAACACCTAGTCCCTCTTTAATGCCTACTGGCT
ACAAATCCATGGAGCTGAGTGACATTTCCAATGAAAACTGCCGAATAAACAGA
 
TATGGTTACTTCAGAGCCACCATCACAATTGTAGATGGAATCCTAGAAGTCAA
CATCATCCAGGTAGCAGATGTCCCAATCCCCACACTGCAGCCTGACAACTCACT
GATGGACTTCATTGTGACCTGCAAAGGGGCCACTCCCACGGAAGCCTGTACGA
TCATCTCTGACCCCACCTGCCAGATCGCCCAGAACAGGGTGTGCAGCCCGGTG
GCTGTGGATGAGCTGTGCCTCCTGTCCGTGAGGAGAGCCTTCAATGGGTCCGG
CACGTACTGTGTGAATTTCACTCTGGGAGACGATGCAAGCCTGGCCCTCACCA
GCGCCCTGATCTCTATCCCTGGCAAAGACCTAGGCTCCCCTCTGAGAACAGTGA
AT
SEQ ID NO: 221
   Sequence Name: h_gpNMB ECD-FH
   Sequence Type: AA
   Organism Name: synthetic construct 
MECLYYFLGFLLLAARLPLDAAKRFHDVLGNERPSAYMREHNQLNGWSSDENDW
NEKLYPVWKRGDMRWKNSWKGGRVQAVLTSDSPALVGSNITFAVNLIFPRCQKE
DANGNIVYEKNCRNEAGLSADPYVYNWTAWSEDSDGENGTGQSHHNVFPDGKPF
PHHPGWRRWNFIYVFHTLGQYFQKLGRCSVRVSVNTANVTLGPQLMEVTVYRRH
GRAYVPIAQVKDVYVVTDQIPVFVTMFQKNDRNSSDETFLKDLPIMFDVLIHDPSH
FLNYSTINYKWSFGDNTGLFVSTNHTVNHTYVLNGTFSLNLTVKAAAPGPCPPPPP
PPRPSKPTPSLATTLKSYDSNTPGPAGDNPLELSRIPDENCQINRYGHFQATITIVEGI
LEVNIIQMTDVLMPVPWPESSLIDFVVTCQGSIPTEVCTIISDPTCEITQNTVCSPVDV
DEMCLLTVRRTFNGSGTYCVNLTLGDDTSLALTSTLISVPDRDPASPLRMANEFDY
KDDDDKLEHHHHHH
SEQ ID NO: 222
   Sequence Name: h_gpNMB ECD-FH
   Sequence Type: DNA
   Organism Name: synthetic construct 
ATGGAATGTCTCTACTATTTCCTGGGATTTCTGCTCCTGGCTGCAAGATTGCCA
CTTGATGCCGCCAAACGATTTCATGATGTGCTGGGCAATGAAAGACCTTCTGCT
TACATGAGGGAGCACAATCAATTAAATGGCTGGTCTTCTGATGAAAATGACTG
GAATGAAAAACTCTACCCAGTGTGGAAGCGGGGAGACATGAGGTGGAAAAAC
TCCTGGAAGGGAGGCCGTGTGCAGGCGGTCCTGACCAGTGACTCACCAGCCCT
CGTGGGCTCAAATATAACATTTGCGGTGAACCTGATATTCCCTAGATGCCAAA
AGGAAGATGCCAATGGCAACATAGTCTATGAGAAGAACTGCAGAAATGAGGC
TGGTTTATCTGCTGATCCGTATGTTTACAACTGGACAGCATGGTCAGAGGACAG
TGACGGGGAAAATGGCACCGGCCAAAGCCATCATAACGTCTTCCCTGATGGGA
AACCTTTTCCTCACCACCCCGGATGGAGAAGATGGAATTTCATCTACGTCTTCC
ACACACTTGGTCAGTATTTCCAGAAATTGGGACGATGTTCAGTGAGAGTTTCTG
TGAACACAGCCAATGTGACACTTGGGCCTCAACTCATGGAAGTGACTGTCTAC
AGAAGACATGGACGGGCATATGTTCCCATCGCACAAGTGAAAGATGTGTACGT
GGTAACAGATCAGATTCCTGTGTTTGTGACTATGTTCCAGAAGAACGATCGAA
ATTCATCCGACGAAACCTTCCTCAAAGATCTCCCCATTATGTTTGATGTCCTGA
TTCATGATCCTAGCCACTTCCTCAATTATTCTACCATTAACTACAAGTGGAGCT
TCGGGGATAATACTGGCCTGTTTGTTTCCACCAATCATACTGTGAATCACACGT
 
ATGTGCTCAATGGAACCTTCAGCCTTAACCTCACTGTGAAAGCTGCAGCACCA
GGACCTTGTCCGCCACCGCCACCACCACCCAGACCTTCAAAACCCACCCCTTCT
TTAGCAACTACTCTAAAATCTTATGATTCAAACACCCCAGGACCTGCTGGTGAC
AACCCCCTGGAGCTGAGTAGGATTCCTGATGAAAACTGCCAGATTAACAGATA
TGGCCACTTTCAAGCCACCATCACAATTGTAGAGGGAATCTTAGAGGTTAACA
TCATCCAGATGACAGACGTCCTGATGCCGGTGCCATGGCCTGAAAGCTCCCTA
ATAGACTTTGTCGTGACCTGCCAAGGGAGCATTCCCACGGAGGTCTGTACCATC
ATTTCTGACCCCACCTGCGAGATCACCCAGAACACAGTCTGCAGCCCTGTGGAT
GTGGATGAGATGTGTCTGCTGACTGTGAGACGAACCTTCAATGGGTCTGGGAC
GTACTGTGTGAACCTCACCCTGGGGGATGACACAAGCCTGGCTCTCACGAGCA
CCCTGATTTCTGTTCCTGACAGAGACCCAGCCTCGCCTTTAAGGATGGCAAACG
AATTCGATTATAAGGATGACGATGACAAGCTCGAGCATCACCACCATCACCAC
SEQ ID NO: 223
   Sequence Name: m_gpNMB ECD-FH
   Sequence Type: AA
   Organism Name: synthetic construct 
MESLCGVLGFLLLAAGLPLQAAKRFRDVLGHEQYPDHMREHNQLRGWSSDENE
WDEHLYPVWRRGDGRWKDSWEGGRVQAVLTSDSPALVGSNITFVVNLVFPRCQ
KEDANGNIVYEKNCRNDLGLTSDLHVYNWTAGADDGDWEDGTSRSQHLRFPDR
RPFPRPHGWKKWSFVYVFHTLGQYFQKLGRCSARVSINTVNLTAGPQVMEVTVF
RRYGRAYIPISKVKDVYVITDQIPVFVTMSQKNDRNLSDEIFLRDLPIVFDVLIHDPS
HFLNDSAISYKWNFGDNTGLFVSNNHTLNHTYVLNGTFNLNLTVQTAVPGPCPPP
SPSTPPPPSTPPSPPPSPLPTLSTPSPSLMPTGYKSMELSDISNENCRINRYGYFRATITI
VEGILEVSIMQIADVPMPTPQPANSLMDFTVTCKGATPMEACTIISDPTCQIAQNRV
CSPVAVDGLCLLSVRRAFNGSGTYCVNFTLGDDASLALTSTLISIPGKDPDSPLRAV
NEFDYKDDDDKLEHHHHHH
SEQ ID NO: 224
   Sequence Name: m_gpNMB ECD-FH
   Sequence Type: DNA
   Organism Name: synthetic construct 
ATGGAAAGTCTCTGCGGGGTCCTGGGATTTCTGCTGCTGGCTGCAGGACTGCCT
CTCCAGGCTGCCAAGCGATTTCGTGATGTGCTGGGCCATGAACAGTATCCCGAT
CACATGAGAGAGCACAACCAATTACGTGGCTGGTCTTCGGATGAAAATGAATG
GGATGAACACCTGTATCCAGTGTGGAGGAGGGGAGACGGCAGGTGGAAGGAC
TCCTGGGAAGGAGGCCGTGTGCAGGCAGTCCTGACCAGTGACTCACCGGCTCT
GGTGGGTTCCAATATCACTTTTGTGGTGAACCTGGTGTTCCCCAGATGCCAGAA
GGAAGATGCTAATGGCAATATCGTCTATGAGAAGAACTGCAGGAATGATTTGG
GACTGACATCTGACCTGCATGTCTACAACTGGACTGCAGGGGCAGATGATGGT
GACTGGGAAGATGGCACCAGCCGAAGCCAGCATCTCAGGTTCCCGGACAGGA
GGCCCTTCCCTCGCCCCCATGGATGGAAGAAATGGAGCTTTGTCTACGTCTTTC
ACACACTTGGCCAGTATTTCCAAAAACTGGGTCGGTGTTCAGCACGGGTTTCTA
TAAACACAGTCAACTTGACAGCTGGCCCTCAGGTCATGGAAGTGACTGTCTTTC
GAAGATACGGCCGGGCATACATTCCCATCTCGAAGGTGAAAGATGTGTATGTG
 
ATAACAGATCAGATCCCTGTATTCGTGACCATGTCCCAGAAGAATGACAGGAA
CTTGTCTGATGAGATCTTCCTCAGAGACCTCCCCATCGTCTTCGATGTCCTCATT
CATGATCCCAGCCACTTCCTCAACGACTCTGCCATTTCCTACAAGTGGAACTTT
GGGGACAACACTGGCCTGTTTGTCTCCAACAATCACACTTTGAATCACACTTAT
GTGCTCAATGGAACCTTCAACCTTAACCTCACCGTGCAAACTGCAGTGCCCGG
GCCATGCCCTCCCCCTTCGCCTTCGACTCCGCCTCCACCTTCAACTCCGCCCTCA
CCTCCGCCCTCACCTCTGCCCACATTATCAACACCTAGCCCCTCTTTAATGCCT
ACTGGTTACAAATCCATGGAGCTGAGTGACATTTCCAATGAAAACTGCCGAAT
AAACAGATATGGCTACTTCAGAGCCACCATCACAATTGTAGAGGGGATCCTGG
AAGTCAGCATCATGCAGATAGCAGATGTCCCCATGCCCACACCGCAGCCTGCC
AACTCCCTGATGGACTTCACTGTGACCTGCAAAGGGGCCACCCCCATGGAAGC
CTGTACGATCATCTCCGACCCCACCTGCCAGATCGCCCAGAACCGGGTCTGCA
GCCCTGTGGCTGTGGATGGGCTGTGCCTGCTGTCTGTGAGAAGAGCCTTCAATG
GGTCTGGCACCTACTGTGTGAATTTCACTCTGGGAGATGATGCAAGCCTGGCCC
TCACCAGCACCCTGATCTCTATCCCTGGCAAAGACCCAGACTCCCCTCTGAGAG
CAGTGAATGAATTCGATTATAAGGATGACGATGACAAGCTCGAGCATCACCAC
CATCACCAC
SEQ ID NO: 225
   Sequence Name: r_gpNMB ECD-FH
   Sequence Type: AA
   Organism Name: synthetic construct 
MESLCGVLVFLLLAAGLPLQAAKRFRDVLGHEQYPDHMRENNQLRGWSSDENE
WDEQLYPVWRRGEGRWKDSWEGGRVQAALTSDSPALVGSNITFVVNLVFPRCQK
EDANGNIVYERNCRSDLELASDPYVYNWTTGADDEDWEDNTSQGQHLRFPDGKP
FPRPHGRKKWNFVYVFHTLGQYFQKLGQCSARVSINTVNLTVGPQVMEVIVFRRH
GRAYIPISKVKDVYVITDQIPIFVTMYQKNDRNSSDETFLRDLPIFFDVLIHDPSHFL
NYSAISYKWNFGDNTGLFVSNNHTLNHTYVLNGTFNFNLTVQTAVPGPCPSPTPSP
SSSTSPSPASSPSPTLSTPSPSLMPTGYKSMELSDISNENCRINRYGYFRATITIVDGIL
EVNIIQVADVPIPTLQPDNSLMDFIVTCKGATPTEACTIISDPTCQIAQNRVCSPVAV
DELCLLSVRRAFNGSGTYCVNFTLGDDASLALTSALISIPGKDLGSPLRTVNEFDYK
DDDDKLEHHHHHH
SEQ ID NO: 226
   Sequence Name: r_gpNMB ECD-FH
   Sequence Type: DNA
   Organism Name: synthetic construct 
ATGGAAAGTCTCTGCGGGGTCCTGGTATTTCTGCTGCTGGCTGCAGGACTGCCG
CTCCAGGCGGCCAAGCGGTTCCGTGATGTGCTGGGCCATGAGCAGTATCCGGA
TCACATGAGGGAGAACAACCAATTACGTGGCTGGTCTTCAGATGAAAATGAAT
GGGATGAACAGCTGTATCCAGTGTGGAGGAGGGGAGAGGGCAGATGGAAGGA
CTCCTGGGAAGGAGGCCGTGTGCAGGCAGCCCTAACCAGTGATTCACCGGCCT
TGGTGGGTTCCAATATCACCTTCGTAGTGAACCTGGTGTTCCCCAGATGCCAGA
AGGAAGATGCCAACGGCAATATCGTCTATGAGAGGAACTGCAGAAGTGATTTG
GAGCTGGCTTCTGACCCGTATGTCTACAACTGGACCACAGGGGCAGACGATGA
 
GGACTGGGAAGACAACACCAGCCAAGGCCAGCACCTCAGGTTCCCCGACGGG
AAGCCCTTCCCTCGCCCCCACGGACGGAAGAAATGGAACTTCGTCTACGTCTTC
CACACACTTGGTCAGTATTTTCAAAAGCTGGGTCAGTGTTCAGCACGAGTTTCT
ATAAACACAGTCAACTTGACAGTTGGCCCTCAGGTCATGGAAGTGATTGTCTTT
CGAAGACACGGCCGGGCATACATTCCCATCTCCAAAGTGAAAGACGTGTATGT
GATAACAGATCAGATCCCTATATTCGTGACCATGTACCAGAAGAATGACCGGA
ACTCGTCTGATGAAACCTTCCTCAGAGACCTCCCCATTTTCTTCGATGTCCTCAT
TCACGATCCCAGTCATTTCCTCAACTACTCTGCCATTTCCTACAAGTGGAACTTT
GGGGACAACACTGGCCTGTTTGTCTCCAACAATCACACTTTGAATCACACGTAT
GTGCTCAATGGAACCTTCAACTTTAACCTCACCGTGCAAACTGCAGTGCCGGG
ACCATGCCCCTCACCCACACCTTCGCCTTCTTCTTCGACTTCTCCTTCGCCTGCA
TCTTCGCCTTCACCCACATTATCAACACCTAGTCCCTCTTTAATGCCTACTGGCT
ACAAATCCATGGAGCTGAGTGACATTTCCAATGAAAACTGCCGAATAAACAGA
TATGGTTACTTCAGAGCCACCATCACAATTGTAGATGGAATCCTAGAAGTCAA
CATCATCCAGGTAGCAGATGTCCCAATCCCCACACTGCAGCCTGACAACTCACT
GATGGACTTCATTGTGACCTGCAAAGGGGCCACTCCCACGGAAGCCTGTACGA
TCATCTCTGACCCCACCTGCCAGATCGCCCAGAACAGGGTGTGCAGCCCGGTG
GCTGTGGATGAGCTGTGCCTCCTGTCCGTGAGGAGAGCCTTCAATGGGTCCGG
CACGTACTGTGTGAATTTCACTCTGGGAGACGATGCAAGCCTGGCCCTCACCA
GCGCCCTGATCTCTATCCCTGGCAAAGACCTAGGCTCCCCTCTGAGAACAGTGA
ATGAATTCGATTATAAGGATGACGATGACAAGCTCGAGCATCACCACCATCAC
CAC
SEQ ID NO: 227
   Sequence Name: hgpNMBd03_1-251
   Sequence Type: AA
   Organism Name: synthetic construct 
MECLYYFLGFLLLAARLPLDAAKRFHDVLGNERPSAYMREHNQLNGWSSDENDW
NEKLYPVWKRGDMRWKNSWKGGRVQAVLTSDSPALVGSNITFAVNLIFPRCQKE
DANGNIVYEKNCRNEAGLSADPYVYNWTAWSEDSDGENGTGQSHHNVFPDGKPF
PHHPGWRRWNFIYVFHTLGQYFQKLGRCSVRVSVNTANVTLGPQLMEVTVYRRH
GRAYVPIAQVKDVYVVTDQIPVFVTMFQKNDRNSSEFDYKDDDDKLEHHHHHH
SEQ ID NO: 228
   Sequence Name: hgpNMBd03_1-251
   Sequence Type: DNA
   Organism Name: synthetic construct 
ATGGAATGTCTCTACTATTTCCTGGGATTTCTGCTCCTGGCTGCAAGATTGCCA
CTTGATGCCGCCAAACGATTTCATGATGTGCTGGGCAATGAAAGACCTTCTGCT
TACATGAGGGAGCACAATCAATTAAATGGCTGGTCTTCTGATGAAAATGACTG
GAATGAAAAACTCTACCCAGTGTGGAAGCGGGGAGACATGAGGTGGAAAAAC
TCCTGGAAGGGAGGCCGTGTGCAGGCGGTCCTGACCAGTGACTCACCAGCCCT
CGTGGGCTCAAATATAACATTTGCGGTGAACCTGATATTCCCTAGATGCCAAA
AGGAAGATGCCAATGGCAACATAGTCTATGAGAAGAACTGCAGAAATGAGGC
TGGTTTATCTGCTGATCCGTATGTTTACAACTGGACAGCATGGTCAGAGGACAG
 
TGACGGGGAAAATGGCACCGGCCAAAGCCATCATAACGTCTTCCCTGATGGGA
AACCTTTTCCTCACCACCCCGGATGGAGAAGATGGAATTTCATCTACGTCTTCC
ACACACTTGGTCAGTATTTCCAGAAATTGGGACGATGTTCAGTGAGAGTTTCTG
TGAACACAGCCAATGTGACACTTGGGCCTCAACTCATGGAAGTGACTGTCTAC
AGAAGACATGGACGGGCATATGTTCCCATCGCACAAGTGAAAGATGTGTACGT
GGTAACAGATCAGATTCCTGTGTTTGTGACTATGTTCCAGAAGAACGATCGAA
ATTCATCCGAATTCGATTATAAGGATGACGATGACAAGCTCGAGCATCACCAC
CATCACCAC
SEQ ID NO: 229
   Sequence Name: hgpNMBd04_1-321
   Sequence Type: AA
   Organism Name: synthetic construct 
MECLYYFLGFLLLAARLPLDAAKRFHDVLGNERPSAYMREHNQLNGWSSDENDW
NEKLYPVWKRGDMRWKNSWKGGRVQAVLTSDSPALVGSNITFAVNLIFPRCQKE
DANGNIVYEKNCRNEAGLSADPYVVYNWTAWSEDSDGENGTGQSHHNVFPDGKPF
PHHPGWRRWNFIYVFHTLGQYFQKLGRCSVRVSVNTANVTLGPQLMEVTVYRRH
GRAYVPIAQVKDVYVVTDQIPVFVTMFQKNDRNSSDETFLKDLPIMFDVLIHDPSH
FLNYSTINYKWSFGDNTGLFVSTNHTVNHTYVLNGTFSLNLTVKAAAPGEFDYKD
DDDKLEHHHHHH
SEQ ID NO: 230
   Sequence Name: hgpNMBd04_1-321
   Sequence Type: DNA
   Organism Name: synthetic construct 
ATGGAATGTCTCTACTATTTCCTGGGATTTCTGCTCCTGGCTGCAAGATTGCCA
CTTGATGCCGCCAAACGATTTCATGATGTGCTGGGCAATGAAAGACCTTCTGCT
TACATGAGGGAGCACAATCAATTAAATGGCTGGTCTTCTGATGAAAATGACTG
GAATGAAAAACTCTACCCAGTGTGGAAGCGGGGAGACATGAGGTGGAAAAAC
TCCTGGAAGGGAGGCCGTGTGCAGGCGGTCCTGACCAGTGACTCACCAGCCCT
CGTGGGCTCAAATATAACATTTGCGGTGAACCTGATATTCCCTAGATGCCAAA
AGGAAGATGCCAATGGCAACATAGTCTATGAGAAGAACTGCAGAAATGAGGC
TGGTTTATCTGCTGATCCGTATGTTTACAACTGGACAGCATGGTCAGAGGACAG
TGACGGGGAAAATGGCACCGGCCAAAGCCATCATAACGTCTTCCCTGATGGGA
AACCTTTTCCTCACCACCCCGGATGGAGAAGATGGAATTTCATCTACGTCTTCC
ACACACTTGGTCAGTATTTCCAGAAATTGGGACGATGTTCAGTGAGAGTTTCTG
TGAACACAGCCAATGTGACACTTGGGCCTCAACTCATGGAAGTGACTGTCTAC
AGAAGACATGGACGGGCATATGTTCCCATCGCACAAGTGAAAGATGTGTACGT
GGTAACAGATCAGATTCCTGTGTTTGTGACTATGTTCCAGAAGAACGATCGAA
ATTCATCCGACGAAACCTTCCTCAAAGATCTCCCCATTATGTTTGATGTCCTGA
TTCATGATCCTAGCCACTTCCTCAATTATTCTACCATTAACTACAAGTGGAGCT
TCGGGGATAATACTGGCCTGTTTGTTTCCACCAATCATACTGTGAATCACACGT
ATGTGCTCAATGGAACCTTCAGCCTTAACCTCACTGTGAAAGCTGCAGCACCA
GGAGAATTCGATTATAAGGATGACGATGACAAGCTCGAGCATCACCACCATCA
CCAC
SEQ ID NO: 231
   Sequence Name: hgpNMBd05_1-375
   Sequence Type: AA
   Organism Name: synthetic construct 
MECLYYFLGFLLLAARLPLDAAKRFHDVLGNERPSAYMREHNQLNGWSSDENDW
NEKLYPVWKRGDMRWKNSWKGGRVQAVLTSDSPALVGSNITFAVNLIFPRCQKE
DANGNIVYEKNCRNEAGLSADPYVYNWTAWSEDSDGENGTGQSHHNVFPDGKPF
PHHPGWRRWNFIYVFHTLGQYFQKLGRCSVRVSVNTANVTLGPQLMEVTVYRRH
GRAYVPIAQVKDVYVVTDQIPVFVTMFQKNDRNSSDETFLKDLPIMFDVLIHDPSH
FLNYSTINYKWSFGDNTGLFVSTNHTVNHTYVLNGTFSLNLTVKAAAPGPCPPPPP
PPRPSKPTPSLATTLKSYDSNTPGPAGDNPLELSRIPDENCQINRYGEFDYKDDDDK
LEHHHHHH
SEQ ID NO: 232
   Sequence Name: hgpNMBd05_1-375 Sequence Type: DNA
   Organism Name: synthetic construct 
ATGGAATGTCTCTACTATTTCCTGGGATTTCTGCTCCTGGCTGCAAGATTGCCA
CTTGATGCCGCCAAACGATTTCATGATGTGCTGGGCAATGAAAGACCTTCTGCT
TACATGAGGGAGCACAATCAATTAAATGGCTGGTCTTCTGATGAAAATGACTG
GAATGAAAAACTCTACCCAGTGTGGAAGCGGGGAGACATGAGGTGGAAAAAC
TCCTGGAAGGGAGGCCGTGTGCAGGCGGTCCTGACCAGTGACTCACCAGCCCT
CGTGGGCTCAAATATAACATTTGCGGTGAACCTGATATTCCCTAGATGCCAAA
AGGAAGATGCCAATGGCAACATAGTCTATGAGAAGAACTGCAGAAATGAGGC
TGGTTTATCTGCTGATCCGTATGTTTACAACTGGACAGCATGGTCAGAGGACAG
TGACGGGGAAAATGGCACCGGCCAAAGCCATCATAACGTCTTCCCTGATGGGA
AACCTTTTCCTCACCACCCCGGATGGAGAAGATGGAATTTCATCTACGTCTTCC
ACACACTTGGTCAGTATTTCCAGAAATTGGGACGATGTTCAGTGAGAGTTTCTG
TGAACACAGCCAATGTGACACTTGGGCCTCAACTCATGGAAGTGACTGTCTAC
AGAAGACATGGACGGGCATATGTTCCCATCGCACAAGTGAAAGATGTGTACGT
GGTAACAGATCAGATTCCTGTGTTTGTGACTATGTTCCAGAAGAACGATCGAA
ATTCATCCGACGAAACCTTCCTCAAAGATCTCCCCATTATGTTTGATGTCCTGA
TTCATGATCCTAGCCACTTCCTCAATTATTCTACCATTAACTACAAGTGGAGCT
TCGGGGATAATACTGGCCTGTTTGTTTCCACCAATCATACTGTGAATCACACGT
ATGTGCTCAATGGAACCTTCAGCCTTAACCTCACTGTGAAAGCTGCAGCACCA
GGACCTTGTCCGCCACCGCCACCACCACCCAGACCTTCAAAACCCACCCCTTCT
TTAGCAACTACTCTAAAATCTTATGATTCAAACACCCCAGGACCTGCTGGTGAC
AACCCCCTGGAGCTGAGTAGGATTCCTGATGAAAACTGCCAGATTAACAGATA
TGGCGAATTCGATTATAAGGATGACGATGACAAGCTCGAGCATCACCACCATC
ACCAC
SEQ ID NO: 233
   Sequence Name: hgpNMBd06_1-418
   Sequence Type: AA
   Organism Name: synthetic construct 
MECLYYFLGFLLLAARLPLDAAKRFHDVLGNERPSAYMREHNQLNGWSSDENDW
NEKLYPVWKRGDMRWKNSWKGGRVQAVLTSDSPALVGSNITFAVNLIFPRCQKE
DANGNIVYEKNCRNEAGLSADPYVYNWTAWSEDSDGENGTGQSHHNVFPDGKPF
PHHPGWRRWNFIYVFHTLGQYFQKLGRCSVRVSVNTANVTLGPQLMEVTVYRRH
GRAYVPIAQVKDVYVVTDQIPVFVTMFQKNDRNSSDETFLKDLPIMFDVLIHDPSH
FLNYSTINYKWSFGDNTGLFVSTNHTVNHTYVLNGTFSLNLTVKAAAPGPCPPPPP
PPRPSKPTPSLATTLKSYDSNTPGPAGDNPLELSRIPDENCQINRYGHFQATITIVEGI
LEVNIIQMTDVLMPVPWPESSLIDFVVTCQGEFDYKDDDDKLEHHHHHH
SEQ ID NO: 234
   Sequence Name: hgpNMBd06_1-418
   Sequence Type: DNA
   Organism Name: synthetic construct 
ATGGAATGTCTCTACTATTTCCTGGGATTTCTGCTCCTGGCTGCAAGATTGCCA
CTTGATGCCGCCAAACGATTTCATGATGTGCTGGGCAATGAAAGACCTTCTGCT
TACATGAGGGAGCACAATCAATTAAATGGCTGGTCTTCTGATGAAAATGACTG
GAATGAAAAACTCTACCCAGTGTGGAAGCGGGGAGACATGAGGTGGAAAAAC
TCCTGGAAGGGAGGCCGTGTGCAGGCGGTCCTGACCAGTGACTCACCAGCCCT
CGTGGGCTCAAATATAACATTTGCGGTGAACCTGATATTCCCTAGATGCCAAA
AGGAAGATGCCAATGGCAACATAGTCTATGAGAAGAACTGCAGAAATGAGGC
TGGTTTATCTGCTGATCCGTATGTTTACAACTGGACAGCATGGTCAGAGGACAG
TGACGGGGAAAATGGCACCGGCCAAAGCCATCATAACGTCTTCCCTGATGGGA
AACCTTTTCCTCACCACCCCGGATGGAGAAGATGGAATTTCATCTACGTCTTCC
ACACACTTGGTCAGTATTTCCAGAAATTGGGACGATGTTCAGTGAGAGTTTCTG
TGAACACAGCCAATGTGACACTTGGGCCTCAACTCATGGAAGTGACTGTCTAC
AGAAGACATGGACGGGCATATGTTCCCATCGCACAAGTGAAAGATGTGTACGT
GGTAACAGATCAGATTCCTGTGTTTGTGACTATGTTCCAGAAGAACGATCGAA
ATTCATCCGACGAAACCTTCCTCAAAGATCTCCCCATTATGTTTGATGTCCTGA
TTCATGATCCTAGCCACTTCCTCAATTATTCTACCATTAACTACAAGTGGAGCT
TCGGGGATAATACTGGCCTGTTTGTTTCCACCAATCATACTGTGAATCACACGT
ATGTGCTCAATGGAACCTTCAGCCTTAACCTCACTGTGAAAGCTGCAGCACCA
GGACCTTGTCCGCCACCGCCACCACCACCCAGACCTTCAAAACCCACCCCTTCT
TTAGCAACTACTCTAAAATCTTATGATTCAAACACCCCAGGACCTGCTGGTGAC
AACCCCCTGGAGCTGAGTAGGATTCCTGATGAAAACTGCCAGATTAACAGATA
TGGCCACTTTCAAGCCACCATCACAATTGTAGAGGGAATCTTAGAGGTTAACA
TCATCCAGATGACAGACGTCCTGATGCCGGTGCCATGGCCTGAAAGCTCCCTA
ATAGACTTTGTCGTGACCTGCCAAGGGGAATTCGATTATAAGGATGACGATGA
CAAGCTCGAGCATCACCACCATCACCAC
SEQ ID NO: 235
   Sequence Name: hgpNMBd07_76-498
   Sequence Type: AA
   Organism Name: synthetic construct MECLYYFLGFLLLAARLPLDAAGSGRVQAVLTSDSPALVGSNITFAVNLIFPRCQK 
EDANGNIVYEKNCRNEAGLSADPYVYNWTAWSEDSDGENGTGQSHHNVFPDGKP
FPHHPGWRRWNFIYVFHTLGQYFQKLGRCSVRVSVNTANVTLGPQLMEVTVYRR
HGRAYVPIAQVKDVYVVTDQIPVFVTMFQKNDRNSSDETFLKDLPIMFDVLIHDPS
HFLNYSTINYKWSFGDNTGLFVSTNHTVNHTYVLNGTFSLNLTVKAAAPGPCPPPP
PPPRPSKPTPSLATTLKSYDSNTPGPAGDNPLELSRIPDENCQINRYGHFQATITIVE
GILEVNIIQMTDVLMPVPWPESSLIDFVVTCQGSIPTEVCTIISDPTCEITQNTVCSPV
DVDEMCLLTVRRTFNGSGTYCVNLTLGDDTSLALTSTLISVPDRDPASPLRMANEF
DYKDDDDKLEHHHHHH
SEQ ID NO: 236
   Sequence Name: hgpNMBd07_76-498
   Sequence Type: DNA
   Organism Name: synthetic construct 
ATGGAATGTCTCTACTATTTCCTGGGATTTCTGCTCCTGGCTGCAAGATTGCCA
CTTGATGCCGCCGGATCCGGCCGTGTGCAGGCGGTCCTGACCAGTGACTCACC
AGCCCTCGTGGGCTCAAATATAACATTTGCGGTGAACCTGATATTCCCTAGATG
CCAAAAGGAAGATGCCAATGGCAACATAGTCTATGAGAAGAACTGCAGAAAT
GAGGCTGGTTTATCTGCTGATCCGTATGTTTACAACTGGACAGCATGGTCAGAG
GACAGTGACGGGGAAAATGGCACCGGCCAAAGCCATCATAACGTCTTCCCTGA
TGGGAAACCTTTTCCTCACCACCCCGGATGGAGAAGATGGAATTTCATCTACGT
CTTCCACACACTTGGTCAGTATTTCCAGAAATTGGGACGATGTTCAGTGAGAGT
TTCTGTGAACACAGCCAATGTGACACTTGGGCCTCAACTCATGGAAGTGACTGT
CTACAGAAGACATGGACGGGCATATGTTCCCATCGCACAAGTGAAAGATGTGT
ACGTGGTAACAGATCAGATTCCTGTGTTTGTGACTATGTTCCAGAAGAACGATC
GAAATTCATCCGACGAAACCTTCCTCAAAGATCTCCCCATTATGTTTGATGTCC
TGATTCATGATCCTAGCCACTTCCTCAATTATTCTACCATTAACTACAAGTGGA
GCTTCGGGGATAATACTGGCCTGTTTGTTTCCACCAATCATACTGTGAATCACA
CGTATGTGCTCAATGGAACCTTCAGCCTTAACCTCACTGTGAAAGCTGCAGCAC
CAGGACCTTGTCCGCCACCGCCACCACCACCCAGACCTTCAAAACCCACCCCTT
CTTTAGCAACTACTCTAAAATCTTATGATTCAAACACCCCAGGACCTGCTGGTG
ACAACCCCCTGGAGCTGAGTAGGATTCCTGATGAAAACTGCCAGATTAACAGA
TATGGCCACTTTCAAGCCACCATCACAATTGTAGAGGGAATCTTAGAGGTTAA
CATCATCCAGATGACAGACGTCCTGATGCCGGTGCCATGGCCTGAAAGCTCCC
TAATAGACTTTGTCGTGACCTGCCAAGGGAGCATTCCCACGGAGGTCTGTACC
ATCATTTCTGACCCCACCTGCGAGATCACCCAGAACACAGTCTGCAGCCCTGTG
GATGTGGATGAGATGTGTCTGCTGACTGTGAGACGAACCTTCAATGGGTCTGG
GACATACTGTGTGAACCTCACCCTGGGGGATGACACAAGCCTGGCTCTCACGA
GCACCCTGATTTCTGTTCCTGACAGAGACCCAGCCTCGCCTTTAAGGATGGCAA
ACGAATTCGATTATAAGGATGACGATGACAAGCTCGAGCATCACCACCATCAC
CAC
SEQ ID NO: 237
   Sequence Name: hgpNMBd13_1-412
   Sequence Type: AA
   Organism Name: synthetic construct 
MECLYYFLGFLLLAARLPLDAAKRFHDVLGNERPSAYMREHNQLNGWSSDENDW
NEKLYPVWKRGDMRWKNSWKGGRVQAVLTSDSPALVGSNITFAVNLIFPRCQKE
DANGNIVYEKNCRNEAGLSADPYVYNWTAWSEDSDGENGTGQSHHNVFPDGKPF
PHHPGWRRWNFIYVFHTLGQYFQKLGRCSVRVSVNTANVTLGPQLMEVTVYRRH
GRAYVPIAQVKDVYVVTDQIPVFVTMFQKNDRNSSDETFLKDLPIMFDVLIHDPSH
FLNYSTINYKWSFGDNTGLFVSTNHTVNHTYVLNGTFSLNLTVKAAAPGPCPPPPP
PPRPSKPTPSLATTLKSYDSNTPGPAGDNPLELSRIPDENCQINRYGHFQATITIVEGI
LEVNIIQMTDVLMPVPWPESSLIDFEFDYKDDDDKLEHHHHHH
SEQ ID NO: 238
   Sequence Name: hgpNMBd13_1-412
   Sequence Type: DNA
   Organism Name: synthetic construct 
ATGGAATGTCTCTACTATTTCCTGGGATTTCTGCTCCTGGCTGCAAGATTGCCA
CTTGATGCCGCCAAACGATTTCATGATGTGCTGGGCAATGAAAGACCTTCTGCT
TACATGAGGGAGCACAATCAATTAAATGGCTGGTCTTCTGATGAAAATGACTG
GAATGAAAAACTCTACCCAGTGTGGAAGCGGGGAGACATGAGGTGGAAAAAC
TCCTGGAAGGGAGGCCGTGTGCAGGCGGTCCTGACCAGTGACTCACCAGCCCT
CGTGGGCTCAAATATAACATTTGCGGTGAACCTGATATTCCCTAGATGCCAAA
AGGAAGATGCCAATGGCAACATAGTCTATGAGAAGAACTGCAGAAATGAGGC
TGGTTTATCTGCTGATCCGTATGTTTACAACTGGACAGCATGGTCAGAGGACAG
TGACGGGGAAAATGGCACCGGCCAAAGCCATCATAACGTCTTCCCTGATGGGA
AACCTTTTCCTCACCACCCCGGATGGAGAAGATGGAATTTCATCTACGTCTTCC
ACACACTTGGTCAGTATTTCCAGAAATTGGGACGATGTTCAGTGAGAGTTTCTG
TGAACACAGCCAATGTGACACTTGGGCCTCAACTCATGGAAGTGACTGTCTAC
AGAAGACATGGACGGGCATATGTTCCCATCGCACAAGTGAAAGATGTGTACGT
GGTAACAGATCAGATTCCTGTGTTTGTGACTATGTTCCAGAAGAACGATCGAA
ATTCATCCGACGAAACCTTCCTCAAAGATCTCCCCATTATGTTTGATGTCCTGA
TTCATGATCCTAGCCACTTCCTCAATTATTCTACCATTAACTACAAGTGGAGCT
TCGGGGATAATACTGGCCTGTTTGTTTCCACCAATCATACTGTGAATCACACGT
ATGTGCTCAATGGAACCTTCAGCCTTAACCTCACTGTGAAAGCTGCAGCACCA
GGACCTTGTCCGCCACCGCCACCACCACCCAGACCTTCAAAACCCACCCCTTCT
TTAGCAACTACTCTAAAATCTTATGATTCAAACACCCCAGGACCTGCTGGTGAC
AACCCCCTGGAGCTGAGTAGGATTCCTGATGAAAACTGCCAGATTAACAGATA
TGGCCACTTTCAAGCCACCATCACAATTGTAGAGGGAATCTTAGAGGTTAACA
TCATCCAGATGACAGACGTCCTGATGCCGGTGCCATGGCCTGAAAGCTCCCTA
ATAGACTTTGAATTCGATTATAAGGATGACGATGACAAGCTCGAGCATCACCA
CCATCACCAC
SEQ ID NO: 239
   Sequence Name: hgpNMBd14_1-406
   Sequence Type: AA
   Organism Name: synthetic construct 
MECLYYFLGFLLLAARLPLDAAKRFHDVLGNERPSAYMREHNQLNGWSSDENDW
NEKLYPVWKRGDMRWKNSWKGGRVQAVLTSDSPALVGSNITFAVNLIFPRCQKE
 
DANGNIVYEKNCRNEAGLSADPYVYNWTAWSEDSDGENGTGQSHHNVFPDGKPF
PHHPGWRRWNFIYVFHTLGQYFQKLGRCSVRVSVNTANVTLGPQLMEVTVYRRH
GRAYVPIAQVKDVYVVTDQIPVFVTMFQKNDRNSSDETFLKDLPIMFDVLIHDPSH
FLNYSTINYKWSFGDNTGLFVSTNHTVNHTYVLNGTFSLNLTVKAAAPGPCPPPPP
PPRPSKPTPSLATTLKSYDSNTPGPAGDNPLELSRIPDENCQINRYGHFQATITIVEGI
LEVNIIQMTDVLMPVPWPEEFDYKDDDDKLEHHHHHH
SEQ ID NO: 240
   Sequence Name: hgpNMBd14_1-406
   Sequence Type: DNA
   Organism Name: synthetic construct 
ATGGAATGTCTCTACTATTTCCTGGGATTTCTGCTCCTGGCTGCAAGATTGCCA
CTTGATGCCGCCAAACGATTTCATGATGTGCTGGGCAATGAAAGACCTTCTGCT
TACATGAGGGAGCACAATCAATTAAATGGCTGGTCTTCTGATGAAAATGACTG
GAATGAAAAACTCTACCCAGTGTGGAAGCGGGGAGACATGAGGTGGAAAAAC
TCCTGGAAGGGAGGCCGTGTGCAGGCGGTCCTGACCAGTGACTCACCAGCCCT
CGTGGGCTCAAATATAACATTTGCGGTGAACCTGATATTCCCTAGATGCCAAA
AGGAAGATGCCAATGGCAACATAGTCTATGAGAAGAACTGCAGAAATGAGGC
TGGTTTATCTGCTGATCCGTATGTTTACAACTGGACAGCATGGTCAGAGGACAG
TGACGGGGAAAATGGCACCGGCCAAAGCCATCATAACGTCTTCCCTGATGGGA
AACCTTTTCCTCACCACCCCGGATGGAGAAGATGGAATTTCATCTACGTCTTCC
ACACACTTGGTCAGTATTTCCAGAAATTGGGACGATGTTCAGTGAGAGTTTCTG
TGAACACAGCCAATGTGACACTTGGGCCTCAACTCATGGAAGTGACTGTCTAC
AGAAGACATGGACGGGCATATGTTCCCATCGCACAAGTGAAAGATGTGTACGT
GGTAACAGATCAGATTCCTGTGTTTGTGACTATGTTCCAGAAGAACGATCGAA
ATTCATCCGACGAAACCTTCCTCAAAGATCTCCCCATTATGTTTGATGTCCTGA
TTCATGATCCTAGCCACTTCCTCAATTATTCTACCATTAACTACAAGTGGAGCT
TCGGGGATAATACTGGCCTGTTTGTTTCCACCAATCATACTGTGAATCACACGT
ATGTGCTCAATGGAACCTTCAGCCTTAACCTCACTGTGAAAGCTGCAGCACCA
GGACCTTGTCCGCCACCGCCACCACCACCCAGACCTTCAAAACCCACCCCTTCT
TTAGCAACTACTCTAAAATCTTATGATTCAAACACCCCAGGACCTGCTGGTGAC
AACCCCCTGGAGCTGAGTAGGATTCCTGATGAAAACTGCCAGATTAACAGATA
TGGCCACTTTCAAGCCACCATCACAATTGTAGAGGGAATCTTAGAGGTTAACA
TCATCCAGATGACAGACGTCCTGATGCCGGTGCCATGGCCTGAAGAATTCGAT
TATAAGGATGACGATGACAAGCTCGAGCATCACCACCATCACCAC
SEQ ID NO: 241
   Sequence Name: hgpNMBd15_1-400
   Sequence Type: AA
   Organism Name: synthetic construct 
MECLYYFLGFLLLAARLPLDAAKRFHDVLGNERPSAYMREHNQLNGWSSDENDW
NEKLYPVWKRGDMRWKNSWKGGRVQAVLTSDSPALVGSNITFAVNLIFPRCQKE
DANGNIVYEKNCRNEAGLSADPYVVYNWTAWSEDSDGENGTGQSHHNVFPDGKPF
PHHPGWRRWNFIYVFHTLGQYFQKLGRCSVRVSVNTANVTLGPQLMEVTVYRRH
GRAYVPIAQVKDVYVVTDQIPVFVTMFQKNDRNSSDETFLKDLPIMFDVLIHDPSH
 
FLNYSTINYKWSFGDNTGLFVSTNHTVNHTYVLNGTFSLNLTVKAAAPGPCPPPPP
PPRPSKPTPSLATTLKSYDSNTPGPAGDNPLELSRIPDENCQINRYGHFQATITIVEGI
LEVNIIQMTDVLMEFDYKDDDDKLEHHHHHH
SEQ ID NO: 242
   Sequence Name: hgpNMBd15_1-400
   Sequence Type: DNA
   Organism Name: synthetic construct 
ATGGAATGTCTCTACTATTTCCTGGGATTTCTGCTCCTGGCTGCAAGATTGCCA
CTTGATGCCGCCAAACGATTTCATGATGTGCTGGGCAATGAAAGACCTTCTGCT
TACATGAGGGAGCACAATCAATTAAATGGCTGGTCTTCTGATGAAAATGACTG
GAATGAAAAACTCTACCCAGTGTGGAAGCGGGGAGACATGAGGTGGAAAAAC
TCCTGGAAGGGAGGCCGTGTGCAGGCGGTCCTGACCAGTGACTCACCAGCCCT
CGTGGGCTCAAATATAACATTTGCGGTGAACCTGATATTCCCTAGATGCCAAA
AGGAAGATGCCAATGGCAACATAGTCTATGAGAAGAACTGCAGAAATGAGGC
TGGTTTATCTGCTGATCCGTATGTTTACAACTGGACAGCATGGTCAGAGGACAG
TGACGGGGAAAATGGCACCGGCCAAAGCCATCATAACGTCTTCCCTGATGGGA
AACCTTTTCCTCACCACCCCGGATGGAGAAGATGGAATTTCATCTACGTCTTCC
ACACACTTGGTCAGTATTTCCAGAAATTGGGACGATGTTCAGTGAGAGTTTCTG
TGAACACAGCCAATGTGACACTTGGGCCTCAACTCATGGAAGTGACTGTCTAC
AGAAGACATGGACGGGCATATGTTCCCATCGCACAAGTGAAAGATGTGTACGT
GGTAACAGATCAGATTCCTGTGTTTGTGACTATGTTCCAGAAGAACGATCGAA
ATTCATCCGACGAAACCTTCCTCAAAGATCTCCCCATTATGTTTGATGTCCTGA
TTCATGATCCTAGCCACTTCCTCAATTATTCTACCATTAACTACAAGTGGAGCT
TCGGGGATAATACTGGCCTGTTTGTTTCCACCAATCATACTGTGAATCACACGT
ATGTGCTCAATGGAACCTTCAGCCTTAACCTCACTGTGAAAGCTGCAGCACCA
GGACCTTGTCCGCCACCGCCACCACCACCCAGACCTTCAAAACCCACCCCTTCT
TTAGCAACTACTCTAAAATCTTATGATTCAAACACCCCAGGACCTGCTGGTGAC
AACCCCCTGGAGCTGAGTAGGATTCCTGATGAAAACTGCCAGATTAACAGATA
TGGCCACTTTCAAGCCACCATCACAATTGTAGAGGGAATCTTAGAGGTTAACA
TCATCCAGATGACAGACGTCCTGATGGAATTCGATTATAAGGATGACGATGAC
AAGCTCGAGCATCACCACCATCACCAC
SEQ ID NO: 243
   Sequence Name: hgpNMBd16_1-394
   Sequence Type: AA
   Organism Name: synthetic construct 
MECLYYFLGFLLLAARLPLDAAKRFHDVLGNERPSAYMREHNQLNGWSSDENDW
NEKLYPVWKRGDMRWKNSWKGGRVQAVLTSDSPALVGSNITFAVNLIFPRCQKE
DANGNIVYEKNCRNEAGLSADPYVYNWTAWSEDSDGENGTGQSHHNVFPDGKPF
PHHPGWRRWNFIYVFHTLGQYFQKLGRCSVRVSVNTANVTLGPQLMEVTVYRRH
GRAYVPIAQVKDVYVVTDQIPVFVTMFQKNDRNSSDETFLKDLPIMFDVLIHDPSH
FLNYSTINYKWSFGDNTGLFVSTNHTVNHTYVLNGTFSLNLTVKAAAPGPCPPPPP
PPRPSKPTPSLATTLKSYDSNTPGPAGDNPLELSRIPDENCQINRYGHFQATITIVEGI
LEVNIIQEFDYKDDDDKLEHHHHHH
SEQ ID NO: 244
   Sequence Name: hgpNMBd16_1-394
   Sequence Type: DNA
   Organism Name: synthetic construct 
ATGGAATGTCTCTACTATTTCCTGGGATTTCTGCTCCTGGCTGCAAGATTGCCA
CTTGATGCCGCCAAACGATTTCATGATGTGCTGGGCAATGAAAGACCTTCTGCT
TACATGAGGGAGCACAATCAATTAAATGGCTGGTCTTCTGATGAAAATGACTG
GAATGAAAAACTCTACCCAGTGTGGAAGCGGGGAGACATGAGGTGGAAAAAC
TCCTGGAAGGGAGGCCGTGTGCAGGCGGTCCTGACCAGTGACTCACCAGCCCT
CGTGGGCTCAAATATAACATTTGCGGTGAACCTGATATTCCCTAGATGCCAAA
AGGAAGATGCCAATGGCAACATAGTCTATGAGAAGAACTGCAGAAATGAGGC
TGGTTTATCTGCTGATCCGTATGTTTACAACTGGACAGCATGGTCAGAGGACAG
TGACGGGGAAAATGGCACCGGCCAAAGCCATCATAACGTCTTCCCTGATGGGA
AACCTTTTCCTCACCACCCCGGATGGAGAAGATGGAATTTCATCTACGTCTTCC
ACACACTTGGTCAGTATTTCCAGAAATTGGGACGATGTTCAGTGAGAGTTTCTG
TGAACACAGCCAATGTGACACTTGGGCCTCAACTCATGGAAGTGACTGTCTAC
AGAAGACATGGACGGGCATATGTTCCCATCGCACAAGTGAAAGATGTGTACGT
GGTAACAGATCAGATTCCTGTGTTTGTGACTATGTTCCAGAAGAACGATCGAA
ATTCATCCGACGAAACCTTCCTCAAAGATCTCCCCATTATGTTTGATGTCCTGA
TTCATGATCCTAGCCACTTCCTCAATTATTCTACCATTAACTACAAGTGGAGCT
TCGGGGATAATACTGGCCTGTTTGTTTCCACCAATCATACTGTGAATCACACGT
ATGTGCTCAATGGAACCTTCAGCCTTAACCTCACTGTGAAAGCTGCAGCACCA
GGACCTTGTCCGCCACCGCCACCACCACCCAGACCTTCAAAACCCACCCCTTCT
TTAGCAACTACTCTAAAATCTTATGATTCAAACACCCCAGGACCTGCTGGTGAC
AACCCCCTGGAGCTGAGTAGGATTCCTGATGAAAACTGCCAGATTAACAGATA
TGGCCACTTTCAAGCCACCATCACAATTGTAGAGGGAATCTTAGAGGTTAACA
TCATCCAGGAATTCGATTATAAGGATGACGATGACAAGCTCGAGCATCACCAC
CATCACCAC
SEQ ID NO: 245
   Sequence Name: hgpNMBd17_1-388
   Sequence Type: AA
   Organism Name: synthetic construct 
MECLYYFLGFLLLAARLPLDAAKRFHDVLGNERPSAYMREHNQLNGWSSDENDW
NEKLYPVWKRGDMRWKNSWKGGRVQAVLTSDSPALVGSNITFAVNLIFPRCQKE
DANGNIVYEKNCRNEAGLSADPYVYNWTAWSEDSDGENGTGQSHHNVFPDGKPF
PHHPGWRRWNFIYVFHTLGQYFQKLGRCSVRVSVNTANVTLGPQLMEVTVYRRH
GRAYVPIAQVKDVYVVTDQIPVFVTMFQKNDRNSSDETFLKDLPIMFDVLIHDPSH
FLNYSTINYKWSFGDNTGLFVSTNHTVNHTYVLNGTFSLNLTVKAAAPGPCPPPPP
PPRPSKPTPSLATTLKSYDSNTPGPAGDNPLELSRIPDENCQINRYGHFQATITIVEGI
LEFDYKDDDDKLEHHHHHH
SEQ ID NO: 246
   Sequence Name: hgpNMBd17_1-388
   Sequence Type: DNA
   Organism Name: synthetic construct 
ATGGAATGTCTCTACTATTTCCTGGGATTTCTGCTCCTGGCTGCAAGATTGCCA
CTTGATGCCGCCAAACGATTTCATGATGTGCTGGGCAATGAAAGACCTTCTGCT
TACATGAGGGAGCACAATCAATTAAATGGCTGGTCTTCTGATGAAAATGACTG
GAATGAAAAACTCTACCCAGTGTGGAAGCGGGGAGACATGAGGTGGAAAAAC
TCCTGGAAGGGAGGCCGTGTGCAGGCGGTCCTGACCAGTGACTCACCAGCCCT
CGTGGGCTCAAATATAACATTTGCGGTGAACCTGATATTCCCTAGATGCCAAA
AGGAAGATGCCAATGGCAACATAGTCTATGAGAAGAACTGCAGAAATGAGGC
TGGTTTATCTGCTGATCCGTATGTTTACAACTGGACAGCATGGTCAGAGGACAG
TGACGGGGAAAATGGCACCGGCCAAAGCCATCATAACGTCTTCCCTGATGGGA
AACCTTTTCCTCACCACCCCGGATGGAGAAGATGGAATTTCATCTACGTCTTCC
ACACACTTGGTCAGTATTTCCAGAAATTGGGACGATGTTCAGTGAGAGTTTCTG
TGAACACAGCCAATGTGACACTTGGGCCTCAACTCATGGAAGTGACTGTCTAC
AGAAGACATGGACGGGCATATGTTCCCATCGCACAAGTGAAAGATGTGTACGT
GGTAACAGATCAGATTCCTGTGTTTGTGACTATGTTCCAGAAGAACGATCGAA
ATTCATCCGACGAAACCTTCCTCAAAGATCTCCCCATTATGTTTGATGTCCTGA
TTCATGATCCTAGCCACTTCCTCAATTATTCTACCATTAACTACAAGTGGAGCT
TCGGGGATAATACTGGCCTGTTTGTTTCCACCAATCATACTGTGAATCACACGT
ATGTGCTCAATGGAACCTTCAGCCTTAACCTCACTGTGAAAGCTGCAGCACCA
GGACCTTGTCCGCCACCGCCACCACCACCCAGACCTTCAAAACCCACCCCTTCT
TTAGCAACTACTCTAAAATCTTATGATTCAAACACCCCAGGACCTGCTGGTGAC
AACCCCCTGGAGCTGAGTAGGATTCCTGATGAAAACTGCCAGATTAACAGATA
TGGCCACTTTCAAGCCACCATCACAATTGTAGAGGGAATCTTAGAATTCGATTA
TAAGGATGACGATGACAAGCTCGAGCATCACCACCATCACCAC
SEQ ID NO: 247
   Sequence Name: hgpNMBd18_1-382
   Sequence Type: AA
   Organism Name: synthetic construct 
MECLYYFLGFLLLAARLPLDAAKRFHDVLGNERPSAYMREHNQLNGWSSDENDW
NEKLYPVWKRGDMRWKNSWKGGRVQAVLTSDSPALVGSNITFAVNLIFPRCQKE
DANGNIVYEKNCRNEAGLSADPYVYNWTAWSEDSDGENGTGQSHHNVFPDGKPF
PHHPGWRRWNFIYVFHTLGQYFQKLGRCSVRVSVNTANVTLGPQLMEVTVYRRH
GRAYVPIAQVKDVYVVTDQIPVFVTMFQKNDRNSSDETFLKDLPIMFDVLIHDPSH
FLNYSTINYKWSFGDNTGLFVSTNHTVNHTYVLNGTFSLNLTVKAAAPGPCPPPPP
PPRPSKPTPSLATTLKSYDSNTPGPAGDNPLELSRIPDENCQINRYGHFQATITEFDY
KDDDDKLEHHHHHH
SEQ ID NO: 248
   Sequence Name: hgpNMBd18_1-382
   Sequence Type: DNA
   Organism Name: synthetic construct 
ATGGAATGTCTCTACTATTTCCTGGGATTTCTGCTCCTGGCTGCAAGATTGCCA
CTTGATGCCGCCAAACGATTTCATGATGTGCTGGGCAATGAAAGACCTTCTGCT
 
TACATGAGGGAGCACAATCAATTAAATGGCTGGTCTTCTGATGAAAATGACTG
GAATGAAAAACTCTACCCAGTGTGGAAGCGGGGAGACATGAGGTGGAAAAAC
TCCTGGAAGGGAGGCCGTGTGCAGGCGGTCCTGACCAGTGACTCACCAGCCCT
CGTGGGCTCAAATATAACATTTGCGGTGAACCTGATATTCCCTAGATGCCAAA
AGGAAGATGCCAATGGCAACATAGTCTATGAGAAGAACTGCAGAAATGAGGC
TGGTTTATCTGCTGATCCGTATGTTTACAACTGGACAGCATGGTCAGAGGACAG
TGACGGGGAAAATGGCACCGGCCAAAGCCATCATAACGTCTTCCCTGATGGGA
AACCTTTTCCTCACCACCCCGGATGGAGAAGATGGAATTTCATCTACGTCTTCC
ACACACTTGGTCAGTATTTCCAGAAATTGGGACGATGTTCAGTGAGAGTTTCTG
TGAACACAGCCAATGTGACACTTGGGCCTCAACTCATGGAAGTGACTGTCTAC
AGAAGACATGGACGGGCATATGTTCCCATCGCACAAGTGAAAGATGTGTACGT
GGTAACAGATCAGATTCCTGTGTTTGTGACTATGTTCCAGAAGAACGATCGAA
ATTCATCCGACGAAACCTTCCTCAAAGATCTCCCCATTATGTTTGATGTCCTGA
TTCATGATCCTAGCCACTTCCTCAATTATTCTACCATTAACTACAAGTGGAGCT
TCGGGGATAATACTGGCCTGTTTGTTTCCACCAATCATACTGTGAATCACACGT
ATGTGCTCAATGGAACCTTCAGCCTTAACCTCACTGTGAAAGCTGCAGCACCA
GGACCTTGTCCGCCACCGCCACCACCACCCAGACCTTCAAAACCCACCCCTTCT
TTAGCAACTACTCTAAAATCTTATGATTCAAACACCCCAGGACCTGCTGGTGAC
AACCCCCTGGAGCTGAGTAGGATTCCTGATGAAAACTGCCAGATTAACAGATA
TGGCCACTTTCAAGCCACCATCACAGAATTCGATTATAAGGATGACGATGACA
AGCTCGAGCATCACCACCATCACCAC
SEQ ID NO: 249
   Sequence Name: hgpNMBd19_1-234 Sequence Type: AA
   Organism Name: synthetic construct 
MECLYYFLGFLLLAARLPLDAAKRFHDVLGNERPSAYMREHNQLNGWSSDENDW
NEKLYPVWKRGDMRWKNSWKGGRVQAVLTSDSPALVGSNITFAVNLIFPRCQKE
DANGNIVYEKNCRNEAGLSADPYVYNWTAWSEDSDGENGTGQSHHNVFPDGKPF
PHHPGWRRWNFIYVFHTLGQYFQKLGRCSVRVSVNTANVTLGPQLMEVTVYRRH
GRAYVPIAQVKDVYVVTDEFDYKDDDDKLEHHHHHH
SEQ ID NO: 250
   Sequence Name: hgpNMBd19_1-234
   Sequence Type: DNA
   Organism Name: synthetic construct 
ATGGAATGTCTCTACTATTTCCTGGGATTTCTGCTCCTGGCTGCAAGATTGCCA
CTTGATGCCGCCAAACGATTTCATGATGTGCTGGGCAATGAAAGACCTTCTGCT
TACATGAGGGAGCACAATCAATTAAATGGCTGGTCTTCTGATGAAAATGACTG
GAATGAAAAACTCTACCCAGTGTGGAAGCGGGGAGACATGAGGTGGAAAAAC
TCCTGGAAGGGAGGCCGTGTGCAGGCGGTCCTGACCAGTGACTCACCAGCCCT
CGTGGGCTCAAATATAACATTTGCGGTGAACCTGATATTCCCTAGATGCCAAA
AGGAAGATGCCAATGGCAACATAGTCTATGAGAAGAACTGCAGAAATGAGGC
TGGTTTATCTGCTGATCCGTATGTTTACAACTGGACAGCATGGTCAGAGGACAG
TGACGGGGAAAATGGCACCGGCCAAAGCCATCATAACGTCTTCCCTGATGGGA
 
AACCTTTTCCTCACCACCCCGGATGGAGAAGATGGAATTTCATCTACGTCTTCC
ACACACTTGGTCAGTATTTCCAGAAATTGGGACGATGTTCAGTGAGAGTTTCTG
TGAACACAGCCAATGTGACACTTGGGCCTCAACTCATGGAAGTGACTGTCTAC
AGAAGACATGGACGGGCATATGTTCCCATCGCACAAGTGAAAGATGTGTACGT
GGTAACAGATGAATTCGATTATAAGGATGACGATGACAAGCTCGAGCATCACC
ACCATCACCAC
SEQ ID NO: 251
   Sequence Name: Antibody hGPN06-1_H0L0 VH
   Sequence Type: AA
   Organism Name: synthetic construct 
QVQLQQPGAEIVRPGASVKLSCKASGYTFTDNWMGWVKQRPGQGLEWIGAIDPS
DSFTNYNQNFKGKATLTVDTSSSTAYMQLSSLTSEDSAVYFCTRSGYYGSPKLGG
DYWGQGTTLTVSS
SEQ ID NO: 252
   Sequence Name: Antibody hGPN06-1_H0L0 VH
   Sequence Type: DNA
   Organism Name: synthetic construct 
CAGGTCCAGCTGCAGCAGCCTGGGGCCGAAATCGTGAGACCTGGCGCTAGCGT
GAAGCTGTCTTGCAAGGCCAGCGGCTACACCTTCACAGACAACTGGATGGGCT
GGGTGAAGCAGAGACCTGGCCAGGGCCTGGAGTGGATCGGCGCCATCGATCCC
TCCGACAGCTTCACCAATTACAATCAGAACTTTAAGGGCAAGGCCACCCTGAC
AGTGGATACCTCTTCCAGCACAGCTTACATGCAGCTGTCTAGCCTGACAAGCG
AGGATTCTGCCGTGTATTTTTGTACAAGAAGCGGCTATTACGGCAGCCCTAAGC
TGGGCGGCGACTACTGGGGCCAGGGCACCACACTGACAGTGTCTTCC
SEQ ID NO: 253
   Sequence Name: Antibody hGPN06-1_H0L0 VL
   Sequence Type: AA
   Organism Name: synthetic construct 
QIVLTQSPAIMSASLGEEITLTCSASSSISYMHWYQQKSGTSPKLLIYSTSNLASGVP
SRFSGSGSGTFYSLTISSVEAEDAADYYCHQWNSYPCTFGGGTKLEIK
SEQ ID NO: 254
   Sequence Name: Antibody hGPN06-1_H0L0 VL
   Sequence Type: DNA
   Organism Name: synthetic construct 
CAGATTGTGCTGACTCAGTCACCCGCTATCATGTCCGCTTCTCTGGGCGAGGAG
ATCACACTGACCTGTTCTGCCTCCAGCTCTATCTCCTACATGCACTGGTATCAG
CAGAAGTCTGGCACCTCTCCAAAGCTGCTGATCTACTCCACCTCTAACCTGGCT
TCTGGCGTGCCTTCCAGATTCTCTGGCAGCGGCTCTGGCACCTTCTATTCCCTG
ACAATCTCCAGCGTGGAGGCTGAGGATGCCGCTGACTACTATTGCCACCAGTG
GAACTCTTACCCATGCACATTTGGCGGCGGCACAAAGCTGGAGATCAAG
SEQ ID NO: 255
   Sequence Name: Antibody GPN09-1 CDR-H1
   Sequence Type: AA
   Organism Name: Mus musculus
      GLSLTSIN
SEQ ID NO: 256
   Sequence Name: Antibody GPN09-1 CDR-H1
   Sequence Type: DNA
   Organism Name: synthetic construct
      GGCCTTTCCCTGACGAGCATCAAC
SEQ ID NO: 257
   Sequence Name: Antibody GPN09-1 CDR-H2
   Sequence Type: AA
   Organism Name: Mus musculus
      IWSDGDT
SEQ ID NO: 258
   Sequence Name: Antibody GPN09-1 CDR-H2
   Sequence Type: DNA
   Organism Name: synthetic construct
      ATTTGGAGTGATGGGGATACT
SEQ ID NO: 259
   Sequence Name: Antibody GPN09-1 CDR-H3
   Sequence Type: AA
   Organism Name: Mus musculus
      ARGIRD
SEQ ID NO: 260
   Sequence Name: Antibody GPN09-1 CDR-H3
   Sequence Type: DNA
   Organism Name: synthetic construct
      GCTAGAGGCATAAGGGAC
SEQ ID NO: 261
   Sequence Name: Antibody GPN09-1 CDR-L1
   Sequence Type: AA
   Organism Name: Mus musculus
      QSLKYSDGKTY
SEQ ID NO: 262
   Sequence Name: Antibody GPN09-1 CDR-L1
   Sequence Type: DNA
   Organism Name: synthetic construct
      CAGTCCCTGAAGTATAGCGATGGGAAAACCTAC
SEQ ID NO: 263
   Sequence Name: Antibody GPN09-1 CDR-L2
   Sequence Type: AA
   Organism Name: Mus musculus
      QVS
SEQ ID NO: 264
   Sequence Name: Antibody GPN09-1 CDR-L2 Sequence Type: DNA
   Organism Name: synthetic construct
      CAGGTCAGC
SEQ ID NO: 265
   Sequence Name: Antibody GPN09-1 CDR-L3 Sequence Type: AA
   Organism Name: Mus musculus
      CQGSYSPHT
SEQ ID NO: 266
   Sequence Name: Antibody GPN09-1 CDR-L3
   Sequence Type: DNA
   Organism Name: synthetic construct
      TGTCAAGGGTCCTATTCACCCCATACC
SEQ ID NO: 267
   Sequence Name: Antibody GPN09-1 VH Sequence Type: AA
   Organism Name: Mus musculus 
QVQLKASGPGLVQPSQTLSLTCTVSGLSLTSININWIRQPPGKGLEWMGVIWSDGD
TDFNSAVKSRLSISRDTSKSQVFLNMNSLQTEDTAMYFCARGIRDWGQGVMVTVS
S
SEQ ID NO: 268
   Sequence Name: Antibody GPN09-1 VH Sequence Type: DNA
   Organism Name: synthetic construct 
CAGGTCCAGCTGAAAGCCAGTGGTCCTGGACTGGTTCAGCCAAGCCAGACCCT
CTCACTCACATGCACCGTTTCTGGCCTTTCCCTGACGAGCATCAACATCAATTG
GATTCGGCAACCACCTGGCAAAGGATTGGAGTGGATGGGCGTGATTTGGAGTG
ATGGGGATACTGACTTCAACTCCGCTGTGAAGTCCCGACTGTCCATTTCACGCG
ATACCAGCAAGTCTCAGGTGTTCCTGAACATGAATAGCCTGCAGACTGAGGAC
ACTGCCATGTACTTCTGTGCTAGAGGCATAAGGGACTGGGGTCAAGGAGTCAT
 GGTTACCGTGTCATCT
SEQ ID NO: 269
   Sequence Name: Antibody GPN09-1 VL
   Sequence Type: AA
   Organism Name: Mus musculus 
DIVMTQTPLSLSVAIGQSASISCKSSQSLKYSDGKTYLNWVFQSPGQSPKRLIYQVS
KLDSGVPDRFSGTGSETDFTLKISRVEAEDLGVYYCCQGSYSPHTFGSGTKLEIK
SEQ ID NO: 270
   Sequence Name: Antibody GPN09-1 VL
   Sequence Type: DNA
   Organism Name: synthetic construct 
GACATAGTGATGACCCAGACACCCTTGAGTCTGAGTGTTGCCATTGGCCAAAG
TGCCTCTATCTCCTGCAAGTCAAGTCAGTCCCTGAAGTATAGCGATGGGAAAA
CCTACCTGAACTGGGTCTTTCAGAGCCCAGGTCAGTCACCCAAACGGCTGATTT
ACCAGGTCAGCAAACTCGACTCTGGAGTACCAGACCGCTTTTCCGGAACAGGT
AGCGAGACTGACTTCACCCTCAAGATCTCTAGGGTGGAAGCCGAGGATCTTGG
CGTGTATTACTGCTGTCAAGGGTCCTATTCACCCCATACCTTTGGTTCCGGCAC
AAAGCTGGAGATCAAG
SEQ ID NO: 271
   Sequence Name: Antibody hGPN06-1_H1L1 graft-H1
   Sequence Type: AA
   Organism Name: synthetic construct
      GYTFTDNWMG
SEQ ID NO: 272
   Sequence Name: Antibody hGPN06-1_H1L1 graft-H1 Sequence Type: DNA
   Organism Name: synthetic construct
      GGCTACACCTTCACCGATAATTGGATGGGC
SEQ ID NO: 273
   Sequence Name: Antibody hGPN06-1_H1L1 graft-H2
   Sequence Type: AA
   Organism Name: synthetic construct
      AIDPSDSFTNYNQNFKG
SEQ ID NO: 274
   Sequence Name: Antibody hGPN06-1_H1L1 graft-H2
   Sequence Type: DNA
   Organism Name: synthetic construct
      GCCATCGACCCTTCTGACTCCTTTACCAACTACAACCAGAACTTCAAGGGC
SEQ ID NO: 275
   Sequence Name: Antibody hGPN06-1_H1L1 graft-H3
   Sequence Type: AA
   Organism Name: synthetic construct
      TRSGYYGSPKLGGDY
SEQ ID NO: 276
   Sequence Name: Antibody hGPN06-1_H1L1 graft-H3
   Sequence Type: DNA
   Organism Name: synthetic construct
      ACCAGATCTGGATACTACGGCTCTCCCAAGCTGGGAGGCGACTAC
SEQ ID NO: 277
   Sequence Name: Antibody hGPN06-1_H1L1 graft-L1
   Sequence Type: AA
   Organism Name: synthetic construct
      SASSSISYMH
SEQ ID NO: 278
   Sequence Name: Antibody hGPN06-1_H1L1 graft-L1
   Sequence Type: DNA
   Organism Name: synthetic construct
      TCCGCCTCCTCCTCAATCTCCTACATGCAC
SEQ ID NO: 279
   Sequence Name: Antibody hGPN06-1_H1L1 graft-L2
   Sequence Type: AA
   Organism Name: synthetic construct
      STSNLAS
SEQ ID NO: 280
   Sequence Name: Antibody hGPN06-1_H1L1 graft-L2
   Sequence Type: DNA
   Organism Name: synthetic construct
      TCCACCTCCAACCTGGCCTCT
SEQ ID NO: 281
   Sequence Name: Antibody hGPN06-1_H1L1 graft-L3
   Sequence Type: AA
   Organism Name: synthetic construct
      HQWNSYPCT
SEQ ID NO: 282
   Sequence Name: Antibody hGPN06-1_H1L1 graft-L3
   Sequence Type: DNA
   Organism Name: synthetic construct
      CACCAGTGGAATTCTTACCCCTGCACC
SEQ ID NO: 283
   Sequence Name: Antibody hGPN06-1_H1L1 CDR-H1
   Sequence Type: AA
   Organism Name: synthetic construct
      GYTFTDNW
SEQ ID NO: 284
   Sequence Name: Antibody hGPN06-1_H1L1 CDR-H1
   Sequence Type: DNA
   Organism Name: synthetic construct
      GGCTACACCTTCACCGATAATTGG
SEQ ID NO: 285
   Sequence Name: Antibody hGPN06-1_H1L1 CDR-H2
   Sequence Type: AA
   Organism Name: synthetic construct
      IDPSDSFT
SEQ ID NO: 286
   Sequence Name: Antibody hGPN06-1_H1L1 CDR-H2 Sequence Type: DNA
   Organism Name: synthetic construct
      ATCGACCCTTCTGACTCCTTTACC
SEQ ID NO: 287
   Sequence Name: Antibody hGPN06-1_H1L1 CDR-H3 Sequence Type: AA
   Organism Name: synthetic construct
      TRSGYYGSPKLGGDY
SEQ ID NO: 288
   Sequence Name: Antibody hGPN06-1_H1L1 CDR-H3
   Sequence Type: DNA
   Organism Name: synthetic construct
      ACCAGATCTGGATACTACGGCTCTCCCAAGCTGGGAGGCGACTAC
SEQ ID NO: 289
   Sequence Name: Antibody hGPN06-1_H1L1 CDR-L1
   Sequence Type: AA
   Organism Name: synthetic construct
      SSISY
SEQ ID NO: 290
   Sequence Name: Antibody hGPN06-1_H1L1 CDR-L1
   Sequence Type: DNA
   Organism Name: synthetic construct
      TCCTCAATCTCCTAC
SEQ ID NO: 291
   Sequence Name: Antibody hGPN06-1_H1L1 CDR-L2
   Sequence Type: AA
   Organism Name: synthetic construct
      STS
SEQ ID NO: 292
   Sequence Name: Antibody hGPN06-1_H1L1 CDR-L2
   Sequence Type: DNA
   Organism Name: synthetic construct
      TCCACCTCC
SEQ ID NO: 293
   Sequence Name: Antibody hGPN06-1_H1L1 CDR-L3
   Sequence Type: AA
   Organism Name: synthetic construct
      HQWNSYPCT
SEQ ID NO: 294
   Sequence Name: Antibody hGPN06-1_H1L1 CDR-L3
   Sequence Type: DNA
   Organism Name: synthetic construct
      CACCAGTGGAATTCTTACCCCTGCACC
SEQ ID NO: 295
   Sequence Name: Antibody hGPN06-1_H1L1 VH
   Sequence Type: AA
   Organism Name: synthetic construct 
QVQLVQSGAEVKKPGASVKVSCKASGYTFTDNWMGWVKQRPGQGLEWIGAIDP
SDSFTNYNQNFKGRVTMTVDTSISTAYMELSRLRSDDSAVYFCTRSGYYGSPKLG
GDYWGQGTLVTVSS
SEQ ID NO: 296
   Sequence Name: Antibody hGPN06-1_H1L1 VH
   Sequence Type: DNA
   Organism Name: synthetic construct 
CAGGTGCAGCTGGTCCAGTCCGGCGCCGAGGTGAAGAAGCCCGGCGCTAGCGT
GAAAGTGTCCTGCAAGGCCTCTGGCTACACCTTCACCGATAATTGGATGGGCT
GGGTCAAGCAGAGACCTGGCCAAGGCCTGGAATGGATCGGCGCCATCGACCCT
 
TCTGACTCCTTTACCAACTACAACCAGAACTTCAAGGGCCGGGTGACCATGAC
AGTGGATACCTCCATCAGCACCGCCTACATGGAACTGTCCAGACTGAGATCCG
ACGACAGCGCTGTGTACTTCTGCACCAGATCTGGATACTACGGCTCTCCCAAGC
TGGGAGGCGACTACTGGGGCCAGGGCACCCTGGTGACCGTGTCCTCC
SEQ ID NO: 297
   Sequence Name: Antibody hGPN06-1_H1L1 VL
   Sequence Type: AA
   Organism Name: synthetic construct 
EIVLTQSPSSLSASVGDRVTITCSASSSISYMHWYQQKSGTAPKLLIYSTSNLASGVP
SRFSGSGSGTDFTFTISSLQPEDAATYYCHQWNSYPCTFGGGTKLEIK
SEQ ID NO: 298
   Sequence Name: Antibody hGPN06-1_H1L1 VL
   Sequence Type: DNA
   Organism Name: synthetic construct 
GAGATCGTGCTGACCCAATCTCCTTCTTCTCTGTCTGCTTCCGTGGGCGACAGA
GTGACCATCACCTGCTCCGCCTCCTCCTCAATCTCCTACATGCACTGGTACCAG
CAGAAGTCTGGAACAGCTCCTAAACTTCTGATCTACTCCACCTCCAACCTGGCC
TCTGGCGTGCCATCTCGCTTCTCCGGCTCTGGCAGCGGAACCGACTTTACATTT
ACCATCTCTAGCCTGCAGCCTGAGGACGCCGCTACATACTACTGCCACCAGTG
GAATTCTTACCCCTGCACCTTCGGCGGCGGCACCAAGCTGGAAATCAAG
SEQ ID NO: 299
   Sequence Name: Antibody hGPN18-2_H2L3 graft-H1
   Sequence Type: AA
   Organism Name: synthetic construct
      GFSLSTSVMGVG
SEQ ID NO: 300
   Sequence Name: Antibody hGPN18-2_H2L3 graft-H1
   Sequence Type: DNA
   Organism Name: synthetic construct
      GGCTTCAGCCTGTCTACCTCCGTGATGGGCGTGGGC
SEQ ID NO: 301
   Sequence Name: Antibody hGPN18-2_H2L3 graft-H2
   Sequence Type: AA
   Organism Name: synthetic construct
      DIWWDDDKDYNPSLKS
SEQ ID NO: 302
   Sequence Name: Antibody hGPN18-2_H2L3 graft-H2 Sequence Type: DNA
   Organism Name: synthetic construct
      GACATCTGGTGGGACGACGACAAGGACTACAATCCCTCTCTGAAGTCC
SEQ ID NO: 303
   Sequence Name: Antibody hGPN18-2_H2L3 graft-H3
   Sequence Type: AA
   Organism Name: synthetic construct
      ARTYYSNYEYYGMDY
SEQ ID NO: 304
   Sequence Name: Antibody hGPN18-2_H2L3 graft-H3
   Sequence Type: DNA
   Organism Name: synthetic construct
      GCCAGAACCTACTACTCCAACTACGAGTACTACGGCATGGATTAC
SEQ ID NO: 305
   Sequence Name: Antibody hGPN18-2_H2L3 graft-L1
   Sequence Type: AA
   Organism Name: synthetic construct
      RSSQTIILSNGNTYLE
SEQ ID NO: 306
   Sequence Name: Antibody hGPN18-2_H2L3 graft-L1
   Sequence Type: DNA
   Organism Name: synthetic construct
      AGATCCTCTCAGACCATCATCCTGTCCAACGGCAACACCTACCTGGAA
SEQ ID NO: 307
   Sequence Name: Antibody hGPN18-2_H2L3 graft-L2
   Sequence Type: AA
   Organism Name: synthetic construct
      KVSNRFS
SEQ ID NO: 308
   Sequence Name: Antibody hGPN18-2_H2L3 graft-L2
   Sequence Type: DNA
   Organism Name: synthetic construct
      AAGGTGTCCAATCGGTTCTCT
SEQ ID NO: 309
   Sequence Name: Antibody hGPN18-2_H2L3 graft-L3
   Sequence Type: AA
   Organism Name: synthetic construct
      FQGSHVPFT
SEQ ID NO: 310
   Sequence Name: Antibody hGPN18-2_H2L3 graft-L3
   Sequence Type: DNA
   Organism Name: synthetic construct
      TTCCAAGGCTCTCACGTGCCCTTTACC
SEQ ID NO: 311
   Sequence Name: Antibody hGPN18-2_H2L3 CDR-H1
   Sequence Type: AA
   Organism Name: synthetic construct
      GFSLSTSVMG
SEQ ID NO: 312
   Sequence Name: Antibody hGPN18-2_H2L3 CDR-H1
   Sequence Type: DNA
   Organism Name: synthetic construct
      GGCTTCAGCCTGTCTACCTCCGTGATGGGC
SEQ ID NO: 313
   Sequence Name: Antibody hGPN18-2_H2L3 CDR-H2
   Sequence Type: AA
   Organism Name: synthetic construct
      IWWDDDK
SEQ ID NO: 314
   Sequence Name: Antibody hGPN18-2_H2L3 CDR-H2
   Sequence Type: DNA
   Organism Name: synthetic construct
      ATCTGGTGGGACGACGACAAG
SEQ ID NO: 315
   Sequence Name: Antibody hGPN18-2_H2L3 CDR-H3
   Sequence Type: AA
   Organism Name: synthetic construct
      ARTYYSNYEYYGMDY
SEQ ID NO: 316
   Sequence Name: Antibody hGPN18-2_H2L3 CDR-H3
   Sequence Type: DNA
   Organism Name: synthetic construct
      GCCAGAACCTACTACTCCAACTACGAGTACTACGGCATGGATTAC
SEQ ID NO: 317
   Sequence Name: Antibody hGPN18-2_H2L3 CDR-L1
   Sequence Type: AA
   Organism Name: synthetic construct
      QTIILSNGNTY
SEQ ID NO: 318
   Sequence Name: Antibody hGPN18-2_H2L3 CDR-L1
   Sequence Type: DNA
   Organism Name: synthetic construct
      CAGACCATCATCCTGAGCAACGGCAACACCTAC
SEQ ID NO: 319
   Sequence Name: Antibody hGPN18-2_H2L3 CDR-L2
   Sequence Type: AA
   Organism Name: synthetic construct
      KVS
SEQ ID NO: 320
   Sequence Name: Antibody hGPN18-2_H2L3 CDR-L2
   Sequence Type: DNA
   Organism Name: synthetic construct
      AAGGTGTCC
SEQ ID NO: 321
   Sequence Name: Antibody hGPN18-2_H2L3 CDR-L3
   Sequence Type: AA
   Organism Name: synthetic construct
      FQGSHVPFT
SEQ ID NO: 322
   Sequence Name: Antibody hGPN18-2_H2L3 CDR-L3
   Sequence Type: DNA
   Organism Name: synthetic construct
      TTCCAGGGCTCTCACGTGCCCTTCACC
SEQ ID NO: 323
   Sequence Name: Antibody hGPN18-2_H2L3 VH
   Sequence Type: AA
   Organism Name: synthetic construct 
QVQLVQSGPGLVQPTQTLTLTCSFSGFSLSTSVMGVGWIRQPSGKGLEWLADIWW
DDDKDYNPSLKSRLTISKDTSKNQVVLTMTNMDTVDTATYYCARTYYSNYEYYG
MDYWGQGTLVTVSS
SEQ ID NO: 324
   Sequence Name: Antibody hGPN18-2_H2L3 VH
   Sequence Type: DNA
   Organism Name: synthetic construct CAAGTGCAACTAGTGCAAAGTGGTCCAGGTCTGGTGCAGCCTACCCAGACCCT 
GACACTGACCTGTAGCTTCTCCGGCTTCAGCCTGTCTACCTCCGTGATGGGCGT
GGGCTGGATCCGGCAACCTTCTGGCAAGGGCCTGGAATGGCTGGCCGACATCT
GGTGGGACGACGACAAGGACTACAATCCCTCTCTGAAGTCCAGACTGACCATC
TCCAAAGATACCTCTAAGAACCAGGTCGTGCTGACCATGACCAACATGGATAC
CGTGGACACCGCTACATATTACTGCGCCAGAACCTACTACTCCAACTACGAGT
ACTACGGCATGGATTACTGGGGCCAGGGAACACTCGTGACCGTGTCCTCT
SEQ ID NO: 325
   Sequence Name: Antibody hGPN18-2_H2L3 VL
   Sequence Type: AA
   Organism Name: synthetic construct 
DIVMTQSPDSLAVSLGERATINCRSSQTIILSNGNTYLEWYQQKPGQPPKLLIYKVS
NRFSGVPDRFSGSGSGTDFTLTISSLQAEDVAVYYCFQGSHVPFTFGSGTKLEIKR
SEQ ID NO: 326
   Sequence Name: Antibody hGPN18-2_H2L3 VL
   Sequence Type: DNA
   Organism Name: synthetic construct

GATATTGTGATGACTCAAAGTCCAGATAGTCTGGCTGTCTCTCTGGGCGAGAG
AGCTACAATCAACTGTAGATCCTCTCAGACCATCATCCTGTCCAACGGCAACAC
CTACCTGGAATGGTATCAGCAGAAACCAGGCCAGCCTCCTAAGCTGCTGATCT
ACAAGGTGTCCAATCGGTTCTCTGGCGTGCCTGATAGATTCTCCGGCTCTGGAA
GCGGAACCGACTTCACCCTGACCATCTCCAGCCTGCAGGCCGAGGACGTGGCC
GTGTACTACTGCTTCCAAGGCTCTCACGTGCCCTTTACCTTCGGCTCCGGCACC
AAGCTGGAGATCAAGCGG

## Claims

1. An anti-gpNMB humanized antibody or its fragment or a derivative thereof which binds specifically to at least one site in a region from a PMEL-CAF-like (PMEL core amyloid fragment-like) domain to a PKD domain of human gpNMB (glycoprotein nonmetastatic melanoma protein B).

2. The anti-gpNMB humanized antibody or its fragment or a derivative thereof according to claim 1, which binds specifically to a region containing amino acid residue(s) D287 and/or H301 of human gpNMB having the amino acid sequence defined in SEQ ID NO 209.

3. The anti-gpNMB humanized antibody or its fragment or a derivative thereof according to claim 1 or 2, which binds specifically to a region containing amino acid residue(s) R214 and/or R215 of human gpNMB having the amino acid sequence defined in SEQ ID NO 209.

4. The anti-gpNMB humanized antibody or its fragment or a derivative thereof according to claim 2 or 3, which also binds specifically to one or more regions selected from of a region containing amino acid residue(s) K257 and/or D258, a region containing amino acid residue(s) H268 and/or D269, a region containing an amino acid residue K282, a region containing an amino acid residue K316, a region containing amino acid residue K186, and a region containing amino acid residue(s) H216 and/or R218, of human gpNMB having the amino acid sequence defined in SEQ ID NO 209.

5. The anti-gpNMB humanized antibody or its fragment or a derivative thereof according to any one of claims 1 to 4, which also binds specifically to at least one site in a region from a PMEL-CAF-like domain to a PKD domain of mouse gpNMB.

6. The anti-gpNMB humanized antibody or its fragment or a derivative thereof according to any one of claims 1 to 5, having one or more activities selected from: an activity to reduce the number of mal-functional microglia; an activity to remove amyloid beta oligomers; an activity to increase the number of synapses; and an activity to restore cognitive function.

7. The anti-gpNMB humanized antibody or its fragment or a derivative thereof according to any one of claims 1 to 6, which is a monoclonal antibody or its fragment or a derivative thereof.

8. The anti-gpNMB humanized antibody or its fragment or a derivative thereof according to any one of claims 1 to 7, which comprises at least a heavy chain variable region comprising:
(13) as a CDR-H1 sequence, the amino acid sequence defined in SEQ ID NO 283, or an amino acid sequence derived from SEQ ID NO 283 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 283,
as a CDR-H2 sequence, the amino acid sequence defined in SEQ ID NO 285, or an amino acid sequence derived from SEQ ID NO 285 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 285,
as a CDR-H3 sequence, the amino acid sequence defined in SEQ ID NO 287, or an amino acid sequence derived from SEQ ID NO 287 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 287, or
(14) as a CDR-H1 sequence, the amino acid sequence defined in SEQ ID NO 311, or an amino acid sequence derived from SEQ ID NO 311 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 311,
as a CDR-H2 sequence, the amino acid sequence defined in SEQ ID NO 313, or an amino acid sequence derived from SEQ ID NO 313 via substitution, deletion, or insertion of any one or two amino acid residues, or an amino acid sequence having a homology (preferably an identity) of 71.4 % or more or 85.7 % or more to the amino acid sequence defined in SEQ ID NO 313,
as a CDR-H3 sequence, the amino acid sequence defined in SEQ ID NO 315, or an amino acid sequence derived from SEQ ID NO 315 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 315, or
(15) as a CDR grafting region H1 sequence, the amino acid sequence defined in SEQ ID NO 271, or an amino acid sequence defined in SEQ ID NO 271 via substitution, deletion, or insertion of any one, two, three, or four amino acid residues other than tyrosine at position 27, asparagine at position 32, and tryptophan at position 33,
as a CDR grafting region H2 sequence, the amino acid sequence defined in SEQ ID NO 273, or an amino acid sequence defined in SEQ ID NO 273 via substitution, deletion, or insertion of any one, two, three, four, five, or six amino acid residues other than aspartic acid at position 55, phenylalanine at position 57, threonine at position 58, asparagine at position 59, tyrosine at position 60, and asparagine at position 61,
as a CDR grafting region H3 sequence, the amino acid sequence defined in SEQ ID NO 275, or an amino acid sequence defined in SEQ ID NO 275 via substitution, deletion, or insertion of any one, two, three, four, five, or six amino acid residues other than arginine at position 98, glycine at position 100, and glycine at position 109, or
(16) as a CDR grafting region H1 sequence, the amino acid sequence defined in SEQ ID NO 299, or an amino acid sequence derived from SEQ ID NO 299 via substitution, deletion, or insertion of any one, two, three, or four amino acid residues,
as a CDR grafting region H2 sequence, the amino acid sequence defined in SEQ ID NO 301, or an amino acid sequence defined in SEQ ID NO 301 via substitution, deletion, or insertion of any one, two, three, four, five, or six amino acid residues other than aspartic acid at position 52, tryptophan at position 54, aspartic acid at position 58, and proline at position 63,
as a CDR grafting region H3 sequence, the amino acid sequence defined in SEQ ID NO 303, or an amino acid sequence defined in SEQ ID NO 303 via substitution, deletion, or insertion of any one, two, three, four, five, or six amino acid residues other than arginine at position 99, threonine at position 100, tyrosine at position 102, tyrosine at position 105, tyrosine at position 107, and methionine at position 110.

9. The anti-gpNMB humanized antibody or its fragment or a derivative thereof according to any one of claims 1 to 7, which comprises at least a heavy chain variable region comprising:
(1) the amino acid sequence defined in SEQ ID NO 295, or an amino acid sequence having a homology of 60 % or more to the amino acid sequence defined in SEQ ID NO 295, or
(2) the amino acid sequence defined in SEQ ID NO 323, or an amino acid sequence having a homology of 60 % or more to the amino acid sequence defined in SEQ ID NO 32.

10. An anti-gpNMB humanized antibody or its fragment or a derivative thereof according to claim 8 or 9, wherein the heavy chain variable region comprises, as a framework sequence, a framework sequence of a class of human immunoglobulin.

11. The anti-gpNMB humanized antibody or its fragment or a derivative thereof according to any one of claims 8 to 10, which further comprises a heavy chain constant region having an amino acid sequence of a heavy chain constant region of a class of human immunoglobulin.

12. The anti-gpNMB humanized antibody or its fragment or a derivative thereof according to any one of claims 1 to 11, which comprises at least a light chain variable region comprising:
(13) as a CDR-L1 sequence, the amino acid sequence defined in SEQ ID NO 289, or an amino acid sequence derived from SEQ ID NO 289 via substitution, deletion, or insertion of any one or two amino acid residues, or an amino acid sequence having a homology (preferably an identity) of 60.0 % or more or 80.0 % or more to the amino acid sequence defined in SEQ ID NO 289,
as a CDR-L2 sequence, the amino acid sequence defined in SEQ ID NO 291, or an amino acid sequence derived from SEQ ID NO 291 via substitution, deletion, or insertion of any one amino acid residue, or an amino acid sequence having a homology (preferably an identity) of 66.6 % or more to the amino acid sequence defined in SEQ ID NO 291, and
as a CDR-L3 sequence, the amino acid sequence defined in SEQ ID NO 293, or an amino acid sequence derived from SEQ ID NO 293 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 293, or
(14) as a CDR-L1 sequence, the amino acid sequence defined in SEQ ID NO 317, or an amino acid sequence derived from SEQ ID NO 317 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 317,
as a CDR-L2 sequence, the amino acid sequence defined in SEQ ID NO 319, or an amino acid sequence derived from SEQ ID NO 319 via substitution, deletion, or insertion of any one amino acid residue, or an amino acid sequence having a homology (preferably an identity) of 66.6 % or more to the amino acid sequence defined in SEQ ID NO 319, and
as a CDR-L3 sequence, the amino acid sequence defined in SEQ ID NO 321, or an amino acid sequence derived from SEQ ID NO 321 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 321, or
(15) as a CDR grafting region L1 sequence, the amino acid sequence defined in SEQ ID NO 277, or an amino acid sequence defined in SEQ ID NO 277 via substitution, deletion, or insertion of any one, two, three, or four amino acid residues other than isoleucine at position 29, tyrosine at position 31, and histidine at position 33,
as a CDR grafting region L2 sequence, the amino acid sequence defined in SEQ ID NO 279, or an amino acid sequence defined in SEQ ID NO 279 via substitution, deletion, or insertion of any one or two amino acid residue other than threonine at position 50,
as a CDR grafting region L3 sequence, the amino acid sequence defined in SEQ ID NO 281, or an amino acid sequence defined in SEQ ID NO 281 via substitution, deletion, or insertion of any one, two, or three amino acid residues other than histidine at position 88, glutamine at position 89, tryptophan at position 90, serine at position 92, tyrosine at position 93, proline at position 94, and cysteine at position 95, or
(16) as a CDR grafting region L1 sequence, the amino acid sequence defined in SEQ ID NO 305, or an amino acid sequence defined in SEQ ID NO 305 via substitution, deletion, or insertion of any one, two, three, four, five, or six amino acid residues other than leucine at position 31, tyrosine at position 37 and glutamic acid at position 39,
as a CDR grafting region L2 sequence, the amino acid sequence defined in SEQ ID NO 307, or an amino acid sequence defined in SEQ ID NO 307 via substitution, deletion, or insertion of any one or two amino acid residue other than lysine at position 55,
as a CDR grafting region L3 sequence, the amino acid sequence defined in SEQ ID NO 309, or an amino acid sequence defined in SEQ ID NO 309 via substitution, deletion, or insertion of any one, two, or three amino acid residues other than phenylalanine at position 94.

13. The anti-gpNMB humanized antibody or its fragment or a derivative thereof according to any one of claims 1 to 11, which comprises at least a light chain variable region comprising:
(1) the amino acid sequence defined in SEQ ID NO 297, or an amino acid sequence having a homology of 60 % or more to the amino acid sequence defined in SEQ ID NO 297, or
(2) the amino acid sequence defined in SEQ ID NO 325, or an amino acid sequence having a homology of 60 % or more to the amino acid sequence defined in SEQ ID NO 325.

14. The anti-gpNMB humanized antibody or its fragment or a derivative thereof according to claim 12 or 13, wherein the light chain variable region comprises, as a framework sequence, a framework sequence of a class of human immunoglobulin.

15. The anti-gpNMB humanized antibody or its fragment or a derivative thereof according to any one of claims 12 to 14, which further comprises a light chain constant region having an amino acid sequence of a light chain constant region of a class of human immunoglobulin.

16. The anti-gpNMB humanized antibody or its fragment or a derivative thereof according to any one of claims 1 to 15, which is a Fab, scFv, Diabody, Nanobody, VHH, bispecific antibody, or multispecific antibody, or a derivative thereof.

17. An anti-gpNMB humanized antibody or its fragment or a derivative thereof which binds to at least one site in a region from a PMEL-CAF-like domain to a PKD domain competitively with an anti-gpNMB antibody or its fragment or a derivative thereof according to any one of claims 1 to 16.

18. A nucleic acid molecule comprising a polynucleotide sequence encoding an anti-gpNMB humanized antibody or its fragment or a derivative thereof according to any one of claims 1 to 17.

19. A cloning vector or expression vector carrying at least one nucleic acid molecule according to claim 18.

20. A recombinant cell transformed with a vector according to claim 19.

21. A method for producing an anti-gpNMB humanized antibody or its fragment or a derivative thereof according to any one of claims 1 to 17, comprising culturing a recombinant cell according to claim 20.

22. A method for producing an anti-gpNMB humanized antibody or its fragment or a derivative thereof according to any one of claims 1 to 17, comprising:
administering to an animal a polypeptide having the same amino acid sequence as at least one region selected from:
a region containing amino acid residue(s) D287 and/or H301,
a region containing amino acid residue(s) R214 and/or R215,
a region containing amino acid residue(s) K257 and/or D258,
a region containing amino acid residue(s) H268 and/or D269,
a region containing amino acid residue K282,
a region containing amino acid residue K316,
a region containing amino acid residue K186, and
a region containing amino acid residue(s) H216 and/or R218 of human gpNMB having the amino acid sequence defined in SEQ ID NO 209; and
collecting an antibody or its fragment or a derivative thereof produced in the body of the animal.

23. A vaccine having an activity to stimulate the production of an anti-gpNMB humanized antibody or its fragment or a derivative thereof according to any one of claims 1 to 17, comprising a polypeptide having the same amino acid sequence as a region containing amino acid residue(s) D287 and/or H301 and /or a region containing amino acid residue(s) R214 and /or R215 of human gpNMB having the amino acid sequence defined in SEQ ID NO 209.

24. A pharmaceutical composition comprising, as an active ingredient, one or more selected from the group consisting of an anti-gpNMB humanized antibody or its fragment or a derivative thereof according to any one of claims 1 to 17, a nucleic acid molecule according to claim 18, a vector according to claim 19, and a recombinant cell according to claim 20.

25. The pharmaceutical composition according to claim 24, for at least one use selected from;
decreasing the number of mal-functional microglia in a subject;
removing amyloid β oligomers in a subject;
increasing the number of synapses in a subject;
removing phosphorylated tau and/or inhibiting accumulation of tau in a subject;
removing synuclein and/or synuclein aggregates in a subject; and
treating or preventing neurodegenerative disease in a subject.

26. A pharmaceutical composition for at least one use selected from;
removing amyloid β oligomers in a subject;
increasing the number of synapses in a subject;
removing phosphorylated tau and/or inhibiting accumulation of tau in a subject;
removing synuclein and/or synuclein aggregates in a subject; and
treating or preventing neurodegenerative disease in a subject,
the pharmaceutical composition comprising, as an active ingredient, an agent for decreasing the number of mal-functional microglia.

27. The pharmaceutical composition according to claim 24, for use in treating or preventing neurodegenerative disease in a subject.

28. The pharmaceutical composition according to any one of claims 25 to 27, wherein the neurodegenerative disease is a disease associated with accumulation of at least one protein selected from amyloid β, tau, synuclein, polyglutamine, RAN translation-generated protein, and prion.

29. The pharmaceutical composition according to any one of claims 25 to 27, wherein the neurodegenerative disease is at least one disease selected from Alzheimer's disease, frontotemporal lobar degeneration (FTLD-Tau), frontotemporal dementia (FTD), primary age-related tauopathy (PART), chronic traumatic encephalopathy (CTE), Pick's disease, cerebral cortex basal ganglia degeneration (CBD or CBS), progressive supranuclear paralysis (PSP), globular glial tauopathy (GGT), argyrophilic grain dementia (AGD) (argyrophilic grain disease), aging-related tau astroglial tauopathy (ARTAG), familial British dementia (FBD), familial Danish dementia (FDD), FTDP17, multisystem tauopathy with dementia (MSTD), dementia of the neurofibrillary tangle type, diffuse neurofibrillary tangles with calcification (DNTC), white matter tauopathy with globular glial inclusions (WMT-GGI), Parkinson's disease, Huntington's disease, spinal cord cerebellum degeneration, hereditary spinocerebellar ataxia, spinal and bulbar muscular atrophy, Lewy body dementia, Creutzfeldt-Jakob disease, amyotrophic lateral sclerosis (ALS), frontotemporal lobar degeneration (FTLD-TDP, FTLD-FUS), and repeat diseases.

30. The pharmaceutical composition according to claim 29, wherein the neurodegenerative disease is Alzheimer's disease.

31. The pharmaceutical composition according to claim 29, wherein the neurodegenerative disease is Parkinson's disease, Lewy body dementia, multisystem atrophy (MSA), or amyotrophic lateral sclerosis (ALS).

32. The pharmaceutical composition according to claim 30 or 31, further comprising a second active ingredient.

33. The pharmaceutical composition according to claim 32, wherein the second active ingredient one or more selected from an anti-Tau antibody, anti-amyloid β antibody, anti-CD33 antibody, anti-semaphorin 4D antibody, anti-TNFα antibody, anti-sortilin antibody, anti-galactose-specific lectin (galectin) 3 antibody, anti-TREM2 (Triggering receptor expressed on myeloid cells 2) antibody, anti-IL-1β antibody, anti-CD38 antibody, anti-MS4A antibody, and anti-synuclein antibody.

34. The pharmaceutical composition according to claim 32, wherein the second active ingredient is a vaccine comprising a full-length or partial-length polypeptide of one or more proteins selected from Tau, amyloid β, CD33, semaphorin 4D, TNFα, sortilin, galactose-specific lectin (galectin) 3, and TREM2 (triggering receptor expressed on myeloid cells 2), or a nucleic acid encoding the polypeptide.
